# EUROPEAN PATENT SPECIFICATION

(11) **EP 3 066 118 B1**
(45) Date of publication and mention of the grant of the patent: **08.01.2020**
(21) Application number: 14805427.3
(22) Date of filing: 06.11.2014
(51) Int. Cl.: C07K 14/725, C07K 16/30, C07K 16/46

(54) **ALK ANTIBODIES, CONJUGATES, AND CHIMERIC ANTIGEN RECEPTORS, AND THEIR USE**
ALK-ANTIKÖRPER, KONJUGATE UND CHIMÄRE ANTIGENREZEPTOREN UND DEREN VERWENDUNG
ANTICORPS ANTI-ALK, CONJUGUES, ET RECEPTEURS ANTIGENIQUES CHIMERIQUES, ET LEUR UTILISATION

(30) Priority: 06.11.2013 US 201361900806 P
(43) Date of publication of application: 14.09.2016
(73) Proprietor: The U.S.A. as represented by the Secretary, Department of Health and Human Services, Bethesda, MD 20892-7660 (US)
(72) Inventor: ORENTAS, Rimas, Gaithersburg, MD 20878 (US); MACKALL, Crystal, Bethesda, MD 20892 (US)
(74) Representative: Gowshall, Jonathan Vallance
(86) International application number: PCT/US2014/064383
(87) International publication number: WO 2015/069922

(56) References cited:
- G. BARONE ET AL: "New Strategies in Neuroblastoma: Therapeutic Targeting of MYCN and ALK", CLINICAL CANCER RESEARCH, vol. 19, no. 21, 21 August 2013 (2013-08-21), pages 5814-5821, XP055163577, ISSN: 1078-0432, DOI: 10.1158/1078-0432.CCR-13-0680
- WEBB THOMAS R ET AL: "Anaplastic lymphoma kinase: role in cancer pathogenesis and small-molecule inhibitor development for therapy", EXPERT REVIEW OF ANTICANCER THE, FUTURE DRUGS LTD, UK, vol. 9, no. 3, 1 March 2009 (2009-03-01), pages 331-356, XP009182621, ISSN: 1744-8328, DOI: 10.1586/14737140.9.3.331
- P MAZOT ET AL: "The constitutive activity of the ALK mutated at positions F1174 or R1275 impairs receptor trafficking", ONCOGENE, vol. 30, no. 17, 17 January 2011 (2011-01-17), pages 2017-2025, XP055466126, London ISSN: 0950-9232, DOI: 10.1038/onc.2010.595
- Orentas, RJ et al.: "Designing a New Chimeric Antigen Receptor for the TumorAntigen ALK", , vol. 33, no. 8 1 October 2010 (2010-10-01), pages 905-905, XP055169988, Retrieved from the Internet: URL:http://pdfs.journals.lww.com/immunothe rapy-journal/2010/10000/Abstracts_for_the_ 25th_Annual_Scientific_Meeting.13.pdf?toke n=method|ExpireAbsolute;source|Journals;tt l|1424098503278;payload|mY8D3u1TCCsNvP5E42 1JYPPlNl9ZUXrQDsjmMHeXqBgfxP56d5BAis WhfSrPR1S6lcHrAT5WTvTkrI7Jc1zUq2UlEn8N1x7q r2heZXbSZE2/LnQ [retrieved on 2015-02-16]
- RIMAS J ORENTAS ET AL: "ALK (anaplastic lymphoma kinase, CD246)-specific CARs: new immunotherapeutic agents for the treatment of pediatric solid tumors", JOURNAL FOR IMMUNOTHERAPY OF CANCER, BIOMED CENTRAL LTD, LONDON, UK, vol. 1, no. Suppl 1, 7 November 2013 (2013-11-07), page P27, XP021167231, ISSN: 2051-1426, DOI: 10.1186/2051-1426-1-S1-P27
- RIMAS J. ORENTAS ET AL: "Immunotherapy Targets in Pediatric Cancer", FRONTIERS IN ONCOLOGY, vol. 2, 1 January 2012 (2012-01-01), XP055170174, DOI: 10.3389/fonc.2012.00003
- RIMAS J. ORENTAS ET AL: "Identification of Cell Surface Proteins as Potential Immunotherapy Targets in 12 Pediatric Cancers", FRONTIERS IN ONCOLOGY, vol. 2, 1 January 2012 (2012-01-01), XP055127272, DOI: 10.3389/fonc.2012.00194
- YAËL P. MOSSÉ ET AL: "Identification of ALK as a major familial neuroblastoma predisposition gene", NATURE, vol. 455, no. 7215, 16 October 2008 (2008-10-16), pages 930-935, XP055004093, ISSN: 0028-0836, DOI: 10.1038/nature07261 cited in the application
- MAZOT PIERRE ET AL: "Internalization and Down-Regulation of the ALK Receptor in Neuroblastoma Cell Lines upon Monoclonal Antibodies Treatment", PLOS ONE, vol. 7, no. 3, March 2012 (2012-03), XP002736064, cited in the application
- LEE D W ET AL: "The Future Is Now: Chimeric Antigen Receptors as New Targeted Therapies for Childhood Cancer", CLINICAL CANCER RESEARCH, THE AMERICAN ASSOCIATION FOR CANCER RESEARCH, US, vol. 18, no. 10, 15 May 2012 (2012-05-15), pages 2780-2790, XP002725492, ISSN: 1078-0432, DOI: 10.1158/1078-0432.CCR-11-1920 cited in the application
- MOOG-LUTZ CHRISTEL ET AL: "Activation and inhibition of anaplastic lymphoma kinase receptor tyrosine kinase by monoclonal antibodies and absence of agonist activity of pleiotrophin", JOURNAL OF BIOLOGICAL CHEMISTRY, AMERICAN SOCIETY FOR BIOCHEMISTRY AND MOLECULAR BIOLOGY, US, vol. 280, no. 28, 1 July 2005 (2005-07-01) , pages 26039-26048, XP002424604, ISSN: 0021-9258, DOI: 10.1074/JBC.M501972200 cited in the application
- ALEC WALKER ET AL: "Targeting high-risk pediatric solid tumors with CAR T cells directed against ALK (anaplastic lymphoma kinase, CD246)", JOURNAL FOR IMMUNOTHERAPY OF CANCER, BIOMED CENTRAL LTD, LONDON, UK, vol. 2, no. Suppl 3, 6 November 2014 (2014-11-06), page P40, XP021202601, ISSN: 2051-1426, DOI: 10.1186/2051-1426-2-S3-P40
- G. BARONE ET AL: "New Strategies in Neuroblastoma: Therapeutic Targeting of MYCN and ALK", CLINICAL CANCER RESEARCH, vol. 19, no. 21, 21 August 2013 (2013-08-21), pages 5814-5821, XP055163577, ISSN: 1078-0432, DOI: 10.1158/1078-0432.CCR-13-0680
- WEBB THOMAS R ET AL: "Anaplastic lymphoma kinase: role in cancer pathogenesis and small-molecule inhibitor development for therapy", EXPERT REVIEW OF ANTICANCER THE, FUTURE DRUGS LTD, UK, vol. 9, no. 3, 1 March 2009 (2009-03-01), pages 331-356, XP009182621, ISSN: 1744-8328, DOI: 10.1586/14737140.9.3.331
- P MAZOT ET AL: "The constitutive activity of the ALK mutated at positions F1174 or R1275 impairs receptor trafficking", ONCOGENE, vol. 30, no. 17, 17 January 2011 (2011-01-17), pages 2017-2025, XP055466126, London ISSN: 0950-9232, DOI: 10.1038/onc.2010.595

## Description

### FIELD OF THE DISCLOSURE

This application relates to the field of cancer, particularly to chimeric antigen receptors (CARs) that specifically bind anaplastic lymphoma kinase (ALK) and their use.

### BACKGROUND

Cancer is a public health concern. It is one of the primary causes of death in the industrialized world. Despite advances in treatments such as chemotherapy, the prognosis for many cancers, including neuroblastoma, can be poor. One treatment approach includes genetic modification of T cells to express CARs that target antigens expressed on tumor cells. CARs are antigen receptors that are designed to recognize cell surface antigens in a human leukocyte antigen-independent manner. Additional treatment approaches include use of therapeutic antibodies, or conjugates thereof. However, a need exists for additional and improved treatments for cancer, particularly neuroblastoma. Orentas etal, Immunotherapy Journal, V33, no8, p905, 2010 discloses a nucleic acid encoding a CAR.

### SUMMARY

The invention is defined in the claims. Novel CARs that specifically bind to the extracellular domain of ALK protein are provided herein, as well as host cells (e.g., T cells) expressing the receptors, nucleic acid molecules encoding the receptors. Methods of using the disclosed CARs, host cells, and nucleic acid molecules are also provided, for example, to treat a tumor in a subject.

In some embodiments, a nucleic acid molecule encoding a CAR is provided. The CAR comprises, from N-terminus to C-terminus, an antigen binding domain, a transmembrane domain, and at least one intracellular T-cell signaling domain. The antigen binding domain comprises a heavy chain variable region and a light chain variable region comprising one of: (a) a heavy chain complementarity determining region (H-CDR)1, a H-CDR2, and a H-CDR3 of the heavy chain variable region set forth as SEQ ID NO: 1, and a light chain complementarity determining region (L-CDR) 1, a L-CDR2, and a L-CDR3 of the light chain variable region set forth as SEQ ID NO: 2; (b) a H-CDR1, a H-CDR2, and a H-CDR3 of the heavy chain variable region sequence set forth as SEQ ID NO: 3, and a L-CDR1, a L-CDR2, and a L-CDR3 of the light chain variable region sequence set forth as SEQ ID NO: 4; (c) a H-CDR1, a H-CDR2, and a H-CDR3 of the heavy chain variable region sequence set forth as SEQ ID NO: 5, and a L-CDR1, a L-CDR2, and a L-CDR3 of the light chain variable region sequence set forth as SEQ ID NO: 6; or (d) a H-CDR1, a H-CDR2, and a H-CDR3 of the heavy chain variable region sequence set forth as SEQ ID NO: 7, and a L-CDR1, a L-CDR2, and a L-CDR3 of the light chain variable sequence region set forth as SEQ ID NO: 8. The CAR specifically binds to an extracellular domain of anaplastic lymphoma kinase.

In some embodiments, the H-CDR1, H-CDR2, and H-CDR3 comprise amino acids 26-33, 51-57, and 95-109 of SEQ ID NO: 1, respectively, and the L-CDR1, L-CDR2, and L-CDR3 comprise amino acids 27-37, 55-57, and 93-103 of SEQ ID NO: 2, respectively. In other embodiments, the H-CDR1, H-CDR2, and H-CDR3 comprise amino acids 26-33, 51-58, and 96-110 of SEQ ID NO: 3, respectively, and the L-CDR1, L-CDR2, and L-CDR3 comprise amino acids 27-36, 54-56, and 92-102 of SEQ ID NO: 4, respectively. In additional embodiments, the H-CDR1, H-CDR2, and H-CDR3 comprise amino acids 26-33, 51-58, and 96-108 of SEQ ID NO: 5, respectively, and the L-CDR1, L-CDR2, and L-CDR3 comprise amino acids 27-37, 55-57, and 93-103 of SEQ ID NO: 6, respectively. In still other embodiments, the H-CDR1, H-CDR2, and H-CDR3 comprise amino acids 26-33, 51-58, and 96-110 of SEQ ID NO: 7, respectively, and the L-CDR1, L-CDR2, and L-CDR3 comprise amino acids 27-32, 50-52, and 88-98 of SEQ ID NO: 8, respectively.

In more embodiments the heavy and light chain variable regions of the antigen binding domain includes amino acid sequences set forth as SEQ ID NO: 9 and SEQ ID NO: 10, respectively; SEQ ID NO: 11 and SEQ ID NO: 12, respectively; SEQ ID NO: 13 and SEQ ID NO: 14, respectively; or SEQ ID NO: 15 and SEQ ID NO: 16, respectively.

In some embodiments, the at least one T-cell signaling domain comprises, from N-terminus to C-terminus, (a) a CD3 zeta signaling domain; (b) a CD28 signaling domain and a CD3 zeta signaling domain; (c) a CD137 (4-1BB) signaling domain and a CD3 zeta signaling domain; (d) an OX40 signaling domain and a CD3 zeta signaling domain; (e) a CD28 signaling domain, a CD137 (4-1BB) signaling domain, and a CD3 zeta signaling domain; or (f) a CD28 signaling domain, an OX40 (CD134) signaling domain, and a CD3 zeta signaling domain.

In some embodiments, the CAR includes the amino acid sequence set forth as one of SEQ ID NOs: 43-90.

The nucleic acid molecule encoding the CAR can be included on a vector, such as a viral vector. In some embodiments, the viral vector is a lentiviral vector.

Host cells including the nucleic acid molecule encoding the CAR are also provided. In some embodiments, the host cell is a T cell, such as a primary T cell obtained from a subject.

Methods of making CAR T cells are provided. The methods include transducing a T cell with the vector or nucleic acid molecule encoding a disclosed CAR that specifically binds ALK, thereby making the CAR T cell.

Also provided are methods of treating a subject with a tumor using the disclosed CARs that specifically bind ALK. The methods include administering to the subject a therapeutically effective amount of host cells expressing a disclosed CAR that specifically binds ALK, under conditions sufficient to form an immune complex of the antigen binding domain on the CAR and the extracellular domain of ALK in the subject. In some embodiments, the host cells are T cells from the subject that have been transformed or transduced with a nucleic acid molecule or vector encoding the disclosed CAR that specifically binds ALK. In additional embodiments, the methods further include the steps of obtaining T cells from the subject, and transforming or transducing the T cells with the nucleic acid molecule or vector encoding the disclosed CAR that specifically binds ALK.

In several embodiments, the tumor comprises cell surface expression of anaplastic lymphoma kinase. In additional embodiments, the tumor is a neuroblastoma, a rhabdomyosarcoma, or a glioblastoma.

Isolated human monoclonal neutralizing antibodies and antigen binding fragments thereof that specifically bind to ALK on the cell surface, and conjugates thereof are also provided. Nucleic acid molecules encoding the disclosed antibodies, antigen binding fragments, or conjugates, expression vectors including such nucleic acid molecules, and host cells including the nucleic acid molecules and/or expression vectors are also provided. In several embodiments, the antibodies, antigen binding fragments, conjugates, nucleic acid molecules, expression vectors and/or host cells can be used in methods of treating a subject with a tumor, for example treating a subject with a neuroblastoma.

It will be understood that the antibodies, antigen binding fragments, CARs, host cells, nucleic acids, and methods are useful beyond the specific circumstances that are described in detail herein. The foregoing and features and advantages of the disclosure will become more apparent from the following detailed description, which proceeds with reference to the accompanying figures.

### BRIEF DESCRIPTION OF THE FIGURES

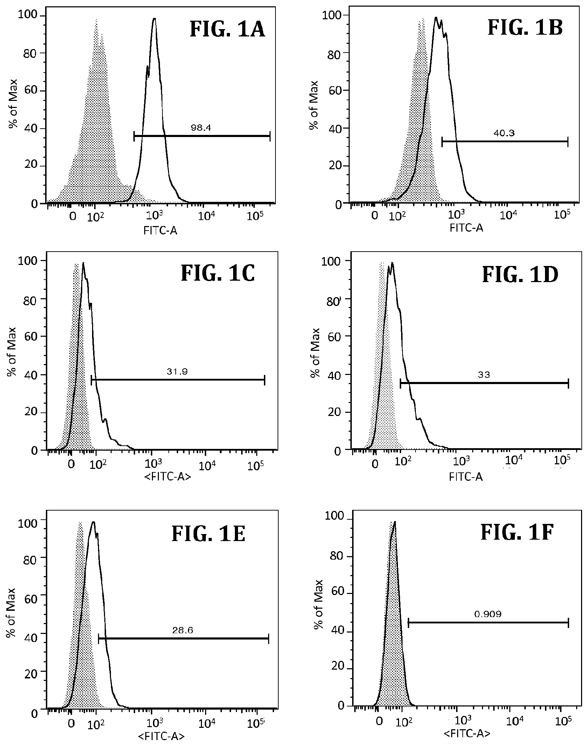

**FIGs. 1A-1F** show a series of graphs illustrating ALK expression on tumor cell lines. ALK expression on tumor cell lines was evaluated by flow cytomeric analysis using the ALK specific monoclonal antibody (mAb) ALK 48. Cell lines analyzed were of neuroblastoma origin: (A) SY5Y, (C) LAN5, (D) KCNR, (E), IMR32; rhabdomyosarcoma origin (B) Rh18; or a control leukemia cell line (F), K562. Control is shown in grey, and ALK positive cells are shown with a solid line, gated as indicated.

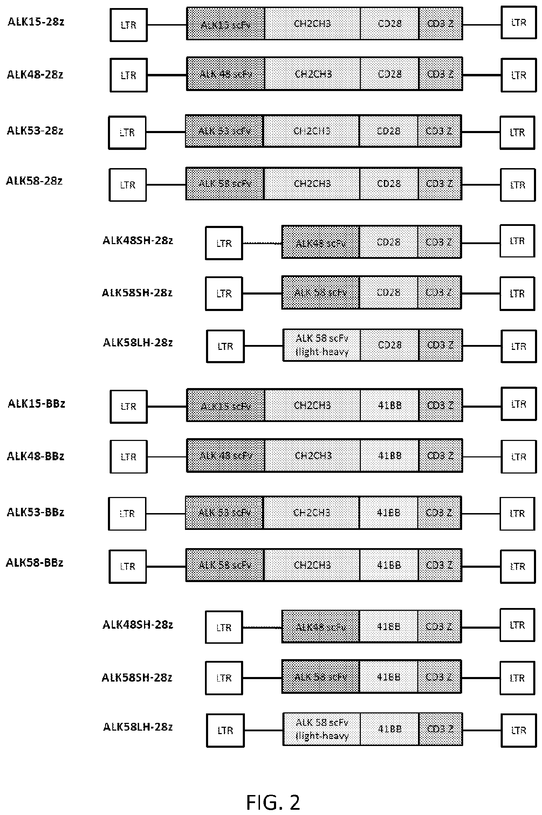

FIG. 2 shows a series of schematic diagrams illustrating ALK-specific CAR constructs. CAR constructs were created by linking the variable heavy and light chain regions of four different anti-ALK scFv antibody fragments (clones 15, 43, 53, 58) with CD28 transmembrane and signaling domains (CD28), 4-1BB (also known as CD137) transmembrane and signaling domain (41BB), and/or CD3 zeta-chain intracellular signaling domains (CD3 Z). For some constructs a CH2CH3 spacer domain (CH2CH3) was also included. The names of some of the constructs used herein are listed to the left of the schematic diagrams. All constructs include heavy chain first, followed by light chain in the linear sequence except for ALK58-LH which includes the light chain first.

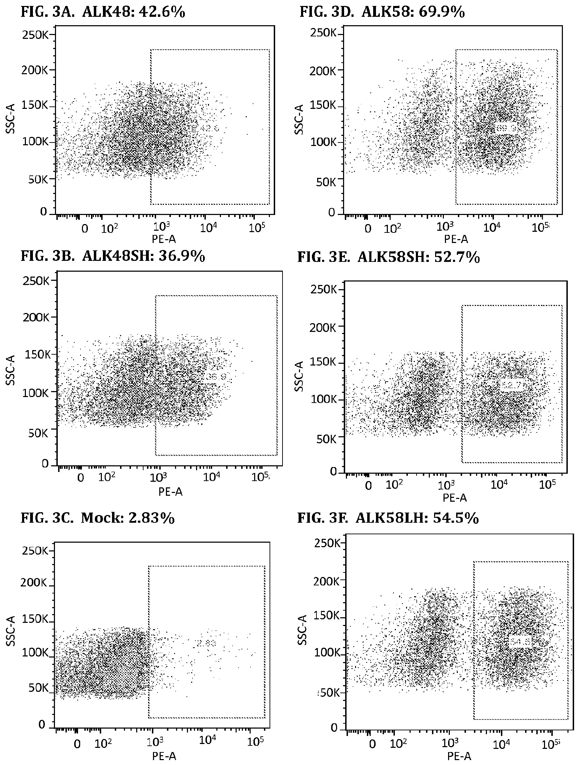

**FIGs. 3A-3F** show a series of graphs illustrating expression of ALK specific CARs on the T cell surface. Peripheral blood mononuclear cells (PBMCs) were transduced with a) ALK48, b) ALK48SH, c) non-transduced control (Mock), d) ALK58, e) ALK58SH, or f) ALK58LH, and then stained for CAR expression. The expression of cell-surface CAR was evaluated using flow cytometry. Percent transduction according to the indicated gates is listed at the top of each panel.

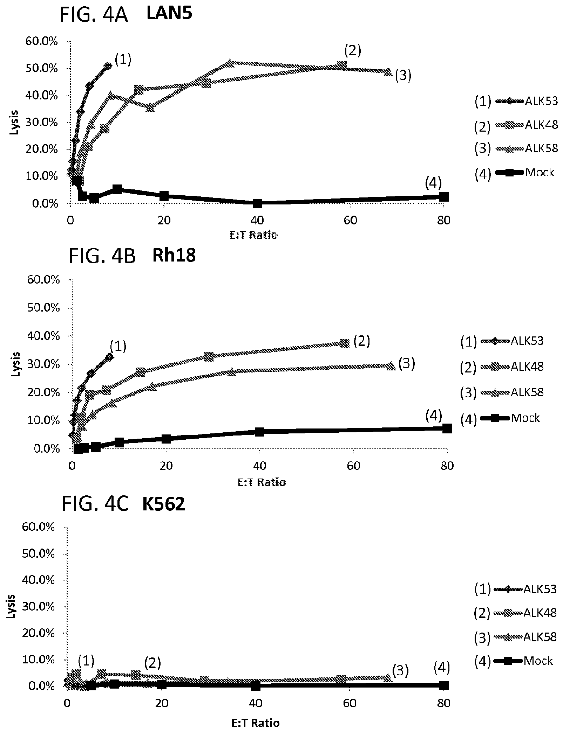

**FIGs. 4A-4C** show a series of graphs illustrating cytotoxic T Cell activity mediated by the disclosed CARs that specifically bind ALK. PBMCs were transduced with the indicated vectors: ALK48, ALK53, ALK58, and incubated in 96-well plates with ⁵¹Cr-labeled LAN5 (A), Rh18 (B), or K562 (C) target cell lines at the E:T ratios indicated. Effector number was corrected for percent transduction.

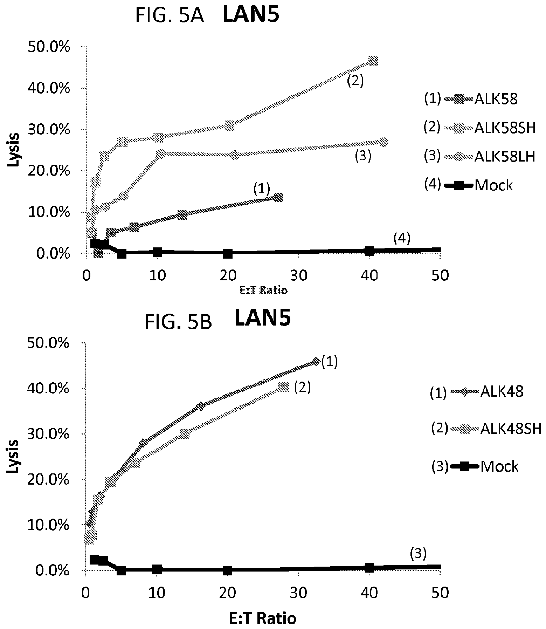

**FIGs. 5A and 5B** show a set of graphs illustrating cytotoxic T cell activity of SH-ALK CAR. PBMC were transduced with A) ALK58, ALK58SH, or ALK58LH, or B) ALK48 or ALK48SH and incubated in 96-well plates with ⁵¹Cr-labeled LAN5 to compare relative CAR format efficiency. Effector number was corrected for percent transduction.

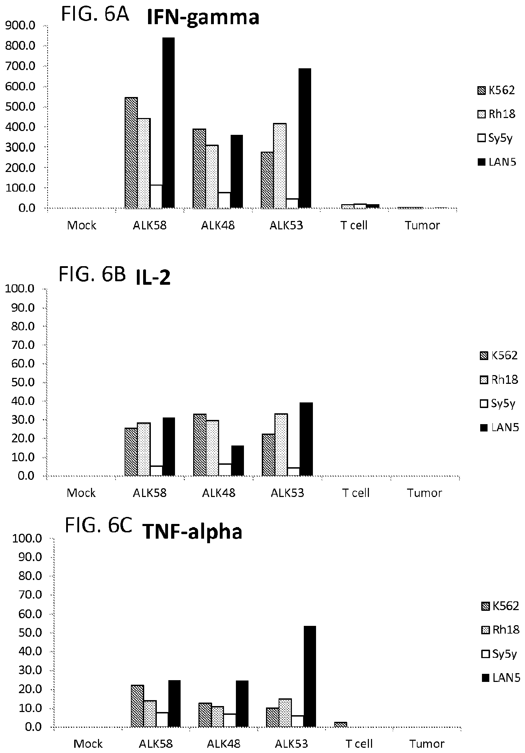

**FIGs. 6A-6C** show a series of graphs illustrating cytokine activity of the disclosed CAR T Cells. PBMC were transduced with the indicated CAR constructs, and incubated with tumor targets (LAN5 SY5Y, Rh18, K562 as indicated in legend), and supernatants collected after 24 hours and tested for the presence of cytokine by a MesoScale mutli-analyte detection system. Results for A) IFN-gamma, B) Interleukin-2, and C) TNF-alpha are shown. Controls include mock transduced cells, tumor only, and T cells without tumor target, as indicated.

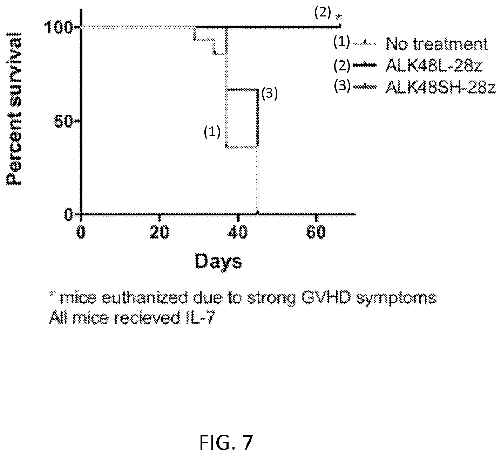

**FIG. 7** is a graph illustrating effective CAR T cell therapy with CAR T-cells expressing a CAR based on the ALK48 mAb. NOD *scid* gamma (NSG) mice were inoculated with ALK positive neuroblastoma cells (SY5Y cells) to produce xenograft. Following inoculation, the mice were treated with primary human T cells transduced to express either ALK48L-28z (second generation CAR, long-format that contains a CH2CH3 spaced domain from IgG); ALK48SH-28z (second generation CAR in a short format, no spacer), or mock transduced T cells.

### SEQUENCES

The nucleic and amino acid sequences listed below are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand. The Sequence Listing is submitted as an ASCII text file in the form of the file named "Sequence.txt" (∼400 kb), which was created on October 30, 2014. In the accompanying sequence listing:
**SEQ ID NO: 1** is the amino acid sequence of the V_{H} of the ALK15 mAb.
**SEQ ID NO: 2** is the amino acid sequence of the V_{L} of the ALK15 mAb.
**SEQ ID NO: 3** is the amino acid sequence of the V_{H} of the ALK48 mAb.
**SEQ ID NO: 4** is the amino acid sequence of the V_{L} of the ALK48 mAb.
**SEQ ID NO: 5** is the amino acid sequence of the V_{H} of the ALK53 mAb.
**SEQ ID NO: 6** is the amino acid sequence of the V_{L} of the ALK53 mAb.
**SEQ ID NO: 7** is the amino acid sequence of the V_{H} of the ALK58 mAb.
**SEQ ID NO: 8** is the amino acid sequence of the V_{L} of the ALK58 mAb.
**SEQ ID NO: 9** is the amino acid sequence of a humanized V_{H} of the ALK15 mAb.
**SEQ ID NO: 10** is the amino acid sequence of a humanized V_{L} of the ALK15 mAb.
**SEQ ID NO: 11** is the amino acid sequence of a humanized V_{H} of the ALK48 mAb.
**SEQ ID NO: 12** is the amino acid sequence of a humanized V_{L} of the ALK48 mAb
**SEQ ID NO: 13** is the amino acid sequence of a humanized V_{H} of the ALK53 mAb.
**SEQ ID NO: 14** is the amino acid sequence of a humanized V_{L} of the ALK53 mAb.
**SEQ ID NO: 15** is the amino acid sequence of a humanized V_{H} of the ALK58 mAb.
**SEQ ID NO: 16** is the amino acid sequence of a humanized V_{L} of the ALK58 mAb.
**SEQ ID NO: 17** is the amino acid sequence of a scFv including the V_{H} and V_{L} of the ALK15 mAb.
**SEQ ID NO: 18** is the amino acid sequence of a scFv including the V_{H} and V_{L} of the ALK48 mAb.
**SEQ ID NO: 19** is the amino acid sequence of a scFv including the V_{H} and V_{L} of the ALK53 mAb.
**SEQ ID NO: 20** is the amino acid sequence of a scFv including the V_{H} and V_{L} of the ALK58 mAb.
**SEQ ID NO: 21** is the amino acid sequence of a scFv including humanized V_{H} and V_{L} of the ALK15 mAb.
**SEQ ID NO: 22** is the amino acid sequence of a scFv including humanized V_{H} and V_{L} of the ALK48 mAb.
**SEQ ID NO: 23** is the amino acid sequence of a scFv including humanized V_{H} and V_{L} of the ALK53 mAb.
**SEQ ID NO: 24** is the amino acid sequence of a scFv including humanized V_{H} and V_{L} of the ALK58 mAb.
**SEQ ID NO: 25** is the amino acid sequence of a peptide linker.
   GGGGSGGGGSGGGGS
**SEQ ID NO: 26** is the amino acid sequence of an exemplary signal peptide. LLVTSLLLCELPHPAFLLIPDT
**SEQ ID NO: 27** is the amino acid sequence of an exemplary CD28 transmembrane domain. IEVMYPPPYLDNEKSNGTIIHVKGKHLCPSPLFPGPSKPFWVLVVVGGVLACYSLLVTVAFIIFWVR
**SEQ ID NO: 28** is the amino acid sequence of an exemplary CD28 signaling domain. SKRSRLLHSDYMNMTPRRPGPTRKHYQPYAPPRDFAAYRS
**SEQ ID NO: 29** is the amino acid sequence of exemplary CD28 transmembrane and signaling domains.
**SEQ ID NO: 30** is the amino acid sequence of an exemplary CD8 transmembrane domain. TTTPAPRPPTPAPTIASQPLSLRPEACRPAAGGAVHTRGLDFACDIYIWAPLAGTCGVLLLSLVITLYC
**SEQ ID NO: 31** is the amino acid sequence of an exemplary CD8 signaling domain.
**SEQ ID NO: 32** is the amino acid sequence of an exemplary CD137 signaling domain. KRGRKKLLYIFKQPFMRPVQTTQEEDGCSCRFPEEEEGGCEL
**SEQ ID NO: 33** is the amino acid sequence of an exemplary CD137 signaling domain. RFSVVKRGRKKLLYIFKQPFMRPVQTTQEEDGCSCRFPEEEEGGCEL
**SEQ ID NO: 34** is the amino acid sequence of an exemplary CD3 zeta signaling domain.
**SEQ ID NO: 35** is the amino acid sequence of an exemplary CH2CH3 spacer domain.
**SEQ ID NO: 36** is an exemplary nucleic acid sequence encoding a CH2CH3 spacer domain.
**SEQ ID NO: 37** is the amino acid sequence of the transmembrane and intracellular domains of an exemplary 2n^{d} generation CAR including a CD28 transmembrane domain and a CD3 zeta signaling domain ("28z").
**SEQ ID NO: 38** is an exemplary nucleic acid sequence encoding the transmembrane and intracellular domains of an exemplary 2^{nd} generation CAR including a CD28 transmembrane domain and a CD3 zeta signaling domain ("28z").
**SEQ ID NO: 39** is the amino acid sequence of the transmembrane and intracellular domains of an exemplary 2^{nd} generation CAR including a CD8 transmembrane domain, CD137 (4-1BB) signaling domain, and a CD3 zeta signaling domain ("BBz").
**SEQ ID NO: 40** is an exemplary nucleic acid sequence encoding the transmembrane and intracellular domains of an exemplary 2^{nd} generation CAR including a CD8 transmembrane domain, CD137 (4-1BB) signaling domain, and a CD3 zeta signaling domain ("BBz").
**SEQ ID NO: 41** is the amino acid sequence of the transmembrane and intracellular domains of an exemplary 3^{rd} generation CAR including a CD8 transmembrane domain, a CD28 signaling domain, a CD137 (4-1BB) signaling domain, and a CD3 zeta signaling domain ("28BBz").
**SEQ ID NO: 42** is an exemplary nucleic acid sequence encoding the transmembrane and intracellular domains of an exemplary 3^{rd} generation CAR including a CD8 transmembrane domain, a CD28 signaling domain, a CD137 (4-1BB) signaling domain, and a CD3 zeta signaling domain ("28BBz").
**SEQ ID NOs: 43-90** are amino acid sequences of exemplary chimeric antigen receptors including an antigen binding domain that specifically binds to the extracellular domain of ALK protein.
**SEQ ID NOs: 91-114** are exemplary nucleic acid sequences encoding chimeric antigen receptors including an antigen binding domain that specifically binds to the extracellular domain of ALK protein.
**SEQ ID NO: 115** is an exemplary amino acid sequence of human ALK protein (UniProt Acc. No Q9UM73, as present in the database on October 31, 2013). The extracellular domain is composed of amino acids 19-1038.
**SEQ ID NO: 116** is an exemplary nucleic acid sequence encoding human ALK protein (GENBANK Acc. No. NP_004295.2, as present in the database on October 31, 2013).

### DETAILED DESCRIPTION

The developmentally-regulated cell surface receptor tyrosine kinase ALK is known to be expressed as a tumor-associated antigen as a fusion protein resulting from a chromosomal translocation. Cancer-associated ALK was first described as a 2;5 chromosomal translocation associated with nucleophosmin (NPM) in anaplastic large cell leukemia (ALCL; Morris et al., (1994) Science, 263, 1281-1284). The fusion protein was composed of the intracellular domain of NPM and the intracellular kinase domain of ALK. However, ALK fusion proteins are not known to be expressed on the cell surface.

Chimeric antigen receptors are an example of synthetic biology used for adoptive immunotherapy for cancer, wherein a protein not encoded by the genome, is designed in the laboratory and is expressed in normal human tissues for a therapeutic effect. There are a number of CAR constructs in clinical trials, but most of the activity has been in hematologic malignancies, most notably in B cell leukemias (Lee et al., (2012) Clin Cancer Res, 18, 2780-2790; Sadelain et al., (2013) Cancer Discov, 3, 388-398). This is due in part to the acceptable safety profile of B cell antigen-specific CAR-modified T cells.

Chimeric antigen receptors are composed of an extracellular antigen binding domain, transmembrane domain, and one or more intracellular T cell signaling domains (Long et al., (2013) Oncoimmunology, 2, e23621). In addition to variations in these structural design elements, how these elements are linked to one another by joining domains provides another level of variability. First-generation CARs include only the CD3 zeta chain-derived cytoplasmic signaling domain. Second generation CARs additionally include CD28 or CD137-derived signaling domains. Third generation CARs encode three signaling domains and may also include sequences derived from CD137, OX40, or GITR.

An overarching rule for the assembly of CAR domains into a functional chimeric receptor that is effective for cancer therapy has yet to be developed, particularly in the context of CARs for use to treat non-hematological cancers. Accordingly, recombinant scFv domains specific for the extracellular domain of ALK were designed and synthesized, and linked to CAR structural domains to create a series of CARs specific for ALK. The ALK-specific CARs have utility, for example, as a new generation of adoptive immunotherapeutic agents for treatment of tumors, such as neuroblastoma.

### I. Summary of Terms

Unless otherwise noted, technical terms are used according to conventional usage. Definitions of common terms in molecular biology can be found in Benjamin Lewin, Genes VII, published by Oxford University Press, 1999; Kendrew et al. (eds.), The Encyclopedia of Molecular Biology, published by Blackwell Science Ltd., 1994; and Robert A. Meyers (ed.), Molecular Biology and Biotechnology: a Comprehensive Desk Reference, published by VCH Publishers, Inc., 1995; and other similar references.

As used herein, the singular forms "a," "an," and "the," refer to both the singular as well as plural, unless the context clearly indicates otherwise. For example, the term "an antigen" includes single or plural antigens and can be considered equivalent to the phrase "at least one antigen." As used herein, the term "comprises" means "includes." Thus, "comprising an antigen" means "including an antigen" without excluding other elements. The phrase "and/or" means "and" or "or." It is further to be understood that any and all base sizes or amino acid sizes, and all molecular weight or molecular mass values, given for nucleic acids or polypeptides are approximate, and are provided for descriptive purposes, unless otherwise indicated. Although many methods and materials similar or equivalent to those described herein can be used, particular suitable methods and materials are described below. In case of conflict, the present specification, including explanations of terms, will control. In addition, the materials, methods, and examples are illustrative only and not intended to be limiting. To facilitate review of the various embodiments, the following explanations of terms are provided:
**Administration:** To provide or give to a subject an agent, for example, a composition that includes a monoclonal antibody or antigen binding fragment that specifically binds ALK, or a CAR including the antigen binding fragment, by any effective route. Exemplary routes of administration include, but are not limited to, oral, injection (such as subcutaneous, intramuscular, intradermal, intraperitoneal, and intravenous), sublingual, rectal, transdermal (for example, topical), intranasal, vaginal, and inhalation routes.
**Agent:** Any substance or any combination of substances that is useful for achieving an end or result; for example, a substance or combination of substances useful for decreasing or reducing tumor growth in a subject. Agents include effector molecules and detectable markers. In some embodiments, the agent is a chemotherapeutic agent. The skilled artisan will understand that particular agents may be useful to achieve more than one result; for example, an agent may be useful as both a detectable marker and a chemotherapeutic agent.
**ALK inhibitor:** An agent that that inhibits or decreases ALK activity, such as ALK tyrosine kinase activity. In some examples, an ALK inhibitor can be a small molecule, a protein (such as an antibody), or a nucleic acid (such as an antisense molecule). An ALK inhibitor may inhibit or decrease binding of a ligand (such as pleiotrophin) to ALK and thus decrease ALK tyrosine kinase activity. An ALK inhibitor may also directly inhibit or decrease ALK tyrosine kinase activity, for example, an ATP-competitive inhibitor (such as crizotinib). Molecules that decrease or inhibit expression of ALK, such as antisense molecules, are also ALK inhibitors. The ALK inhibitor may specifically inhibit ALK tyrosine kinase activity or may inhibit other receptor tyrosine kinase activity (such as c-Met/HGFR activity), in addition to inhibiting ALK tyrosine kinase activity. In one example, and ALK inhibitor is the protein kinase inhibitor (PKI) crizotinib. PKIs or other agents that affect ALK may render ALK positive cancers more susceptible to immune targeting with anti-ALK antibody or with CAR-expressing T cells specific for ALK.
**Amino acid substitution:** The replacement of one amino acid in peptide with a different amino acid.
**Anaplastic lymphoma kinase (ALK):** A receptor tyrosine kinase belonging to the insulin receptor superfamily. The protein includes an extracellular domain, a hydrophobic stretch corresponding to a single pass transmembrane region, and an intracellular kinase domain. Human ALK sequences are publically available, for example from the GENBANK® sequence database (e.g., accession numbers NP_004295 (protein), and NM_004304 (nucleic acid), respectively, as available on October 31, 2013). Human ALK sequences can also be found at UniProt Acc. No Q9UM73, as present in the database on October 31, 2013). The extracellular domain of human ALK is composed of amino acids 19-1038 of UniProt Acc. No Q9UM73. One of ordinary skill in the art can identify additional ALK nucleic acid and protein sequences, including ALK variants.
**Antigen Binding Domain** and **Antibody:** A polypeptide or polypeptides that specifically bind and recognizes an analyte (antigen) such as ALK protein or an antigenic fragment thereof. As used herein, and "antigen binding domain" can include an antibody and antigen binding fragments thereof. The term "antibody" is used herein in the broadest sense and encompasses various antibody structures, including but not limited to monoclonal antibodies, polyclonal antibodies, multispecific antibodies (e.g., bispecific antibodies), and antigen binding fragments thereof, so long as they exhibit the desired antigen-binding activity. Non-limiting examples of antibodies include, for example, intact immunoglobulins and variants and fragments thereof known in the art that retain binding affinity for the antigen.

A "monoclonal antibody" is an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a single antigenic epitope. The modifier "monoclonal" indicates the character of the antibody as being obtained from a substantially homogeneous population of antibodies, and is not to be construed as requiring production of the antibody by any particular method. In some examples, a monoclonal antibody is an antibody produced by a single clone of B-lymphocytes or by a cell into which nucleic acid encoding the light and heavy variable regions of the antibody of a single antibody (or an antigen binding fragment thereof) have been transfected, or a progeny thereof. In some examples monoclonal antibodies are isolated from a subject. Monoclonal antibodies can have conservative amino acid substitutions which have substantially no effect on antigen binding or other immunoglobulin functions. Exemplary methods of production of monoclonal antibodies are known, for example, see Harlow & Lane, Antibodies, A Laboratory Manual, 2nd ed. Cold Spring Harbor Publications, New York (2013).

Typically, an immunoglobulin has heavy (H) chains and light (L) chains interconnected by disulfide bonds. Immunoglobulin genes include the kappa, lambda, alpha, gamma, delta, epsilon and mu constant region genes, as well as the myriad immunoglobulin variable domain genes. There are two types of light chain, lambda (λ) and kappa (κ). There are five main heavy chain classes (or isotypes) which determine the functional activity of an antibody molecule: IgM, IgD, IgG, IgA and IgE.

Each heavy and light chain contains a constant region (or constant domain) and a variable region (or variable domain; see, e.g., Kindt et al. Kuby Immunology, 6.sup.th ed., W.H. Freeman and Co., page 91 (2007).) In several embodiments, the heavy and the light chain variable regions combine to specifically bind the antigen. In additional embodiments, only the heavy chain variable region is required. For example, naturally occurring camelid antibodies consisting of a heavy chain only are functional and stable in the absence of light chain (see, *e.g*., Hamers-Casterman et al., Nature, 363:446-448, 1993; Sheriff et al., Nat. Struct. Biol., 3:733-736, 1996). References to "V_{H}" or "VH" refer to the variable region of an antibody heavy chain, including that of an antigen binding fragment, such as Fv, scFv, dsFv or Fab. References to "V_{L}" or "VL" refer to the variable domain of an antibody light chain, including that of an Fv, scFv, dsFv or Fab.

Light and heavy chain variable regions contain a "framework" region interrupted by three hypervariable regions, also called "complementarity-determining regions" or "CDRs" (see, e.g., Kabat et al., Sequences of Proteins of Immunological Interest, U.S. Department of Health and Human Services, 1991). The sequences of the framework regions of different light or heavy chains are relatively conserved within a species. The framework region of an antibody, that is the combined framework regions of the constituent light and heavy chains, serves to position and align the CDRs in three-dimensional space.

The CDRs are primarily responsible for binding to an epitope of an antigen. The amino acid sequence boundaries of a given CDR can be readily determined using any of a number of well-known schemes, including those described by Kabat et al. ("Sequences of Proteins of Immunological Interest," 5th Ed. Public Health Service, National Institutes of Health, Bethesda, MD, 1991; "Kabat" numbering scheme), Al-Lazikani et al., (JMB 273,927-948, 1997; "Chothia" numbering scheme), and Lefranc et al. ("IMGT unique numbering for immunoglobulin and T cell receptor variable domains and Ig superfamily V-like domains," Dev. Comp. Immunol., 27:55-77, 2003; "IMGT" numbering scheme). The CDRs of each chain are typically referred to as CDR1, CDR2, and CDR3 (from the N-terminus to C-terminus), and are also typically identified by the chain in which the particular CDR is located. Thus, a V_{H} CDR3 is the CDR3 from the variable domain of the heavy chain of the antibody in which it is found, whereas a V_{L} CDR1 is the CDR1 from the variable domain of the light chain of the antibody in which it is found. Light chain CDRs are sometimes referred to as LCDR1, LCDR2, and LCDR3. Heavy chain CDRs are sometimes referred to as LCDR1, LCDR2, and LCDR3.

An "antigen binding fragment" is a portion of a full length antibody that retains the ability to specifically recognize the cognate antigen, as well as various combinations of such portions. Non-limiting examples of antigen binding fragments include Fv, Fab, Fab', Fab'-SH, F(ab')₂; diabodies; linear antibodies; single-chain antibody molecules (e.g. scFv); and multispecific antibodies formed from antibody fragments. Antibody fragments include antigen binding fragments either produced by the modification of whole antibodies or those synthesized *de novo* using recombinant DNA methodologies (see, e.g., Kontermann and Dubel (Ed), Antibody Engineering, Vols. 1-2, 2nd Ed., Springer Press, 2010).

A single-chain antibody (scFv) is a genetically engineered molecule containing the V_{H} and V_{L} domains of one or more antibody(ies) linked by a suitable polypeptide linker as a genetically fused single chain molecule (see, for example, Bird et al., Science, 242:423-426, 1988; Huston et al., Proc. Natl. Acad. Sci., 85:5879-5883, 1988; Ahmad et al., Clin. Dev. Immunol., 2012, doi:10.1155/2012/980250; Marbry, IDrugs, 13:543-549, 2010). The intramolecular orientation of the V_{H}-domain and the V_{L}-domain in a scFv, is typically not decisive for scFvs. Thus, scFvs with both possible arrangements (V_{H}-domain-linker domain-V_{L}-domain; V_{L}-domain-linker domain-V_{H}-domain) may be used.

In a dsFv the heavy and light chain variable chains have been mutated to introduce a disulfide bond to stabilize the association of the chains. Diabodies also are included, which are bivalent, bispecific antibodies in which V_{H} and V_{L} domains are expressed on a single polypeptide chain, but using a linker that is too short to allow for pairing between the two domains on the same chain, thereby forcing the domains to pair with complementary domains of another chain and creating two antigen binding sites (see, for example, Holliger et al., Proc. Natl. Acad. Sci., 90:6444-6448, 1993; Poljak et al., Structure, 2:1121-1123, 1994).

Antibodies also include genetically engineered forms such as chimeric antibodies (such as humanized murine antibodies) and heteroconjugate antibodies (such as bispecific antibodies). See also, Pierce Catalog and Handbook, 1994-1995 (Pierce Chemical Co., Rockford, IL); Kuby, J., Immunology, 3rd Ed., W.H. Freeman & Co., New York, 1997.

Non-naturally occurring antibodies can be constructed using solid phase peptide synthesis, can be produced recombinantly, or can be obtained, for example, by screening combinatorial libraries consisting of variable heavy chains and variable light chains as described by Huse et al., Science 246:1275-1281 (1989). These and other methods of making, for example, chimeric, humanized, CDR-grafted, single chain, and bifunctional antibodies, are well known to those skilled in the art (Winter and Harris, Immunol. Today 14:243-246 (1993); Ward et al., Nature 341:544-546 (1989); Harlow and Lane, *supra*, 1988; Hilyard et al., Protein Engineering: A practical approach (IRL Press 1992); Borrabeck, Antibody Engineering, 2d ed. (Oxford University Press 1995).

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. Antibody competition assays are known, and an exemplary competition assay is provided herein.

A "humanized" antibody or antigen binding fragment includes a human framework region and one or more CDRs from a non-human (such as a mouse, rat, or synthetic) antibody or antigen binding fragment. The non-human antibody or antigen binding fragment providing the CDRs is termed a "donor," and the human antibody or antigen binding fragment providing the framework is termed an "acceptor." In one embodiment, all the CDRs are from the donor immunoglobulin in a humanized immunoglobulin. Constant regions need not be present, but if they are, they can be substantially identical to human immunoglobulin constant regions, such as at least about 85-90%, such as about 95% or more identical. Hence, all parts of a humanized antibody or antigen binding fragment, except possibly the CDRs, are substantially identical to corresponding parts of natural human antibody sequences.

A "chimeric antibody" is an antibody which includes sequences derived from two different antibodies, which typically are of different species. In some examples, a chimeric antibody includes one or more CDRs and/or framework regions from one human antibody and CDRs and/or framework regions from another human antibody.

A "fully human antibody" or "human antibody" is an antibody which includes sequences from (or derived from) the human genome, and does not include sequence from another species. In some embodiments, a human antibody includes CDRs, framework regions, and (if present) an Fc region from (or derived from) the human genome. Human antibodies can be identified and isolated using technologies for creating antibodies based on sequences derived from the human genome, for example by phage display or using transgenic animals (see, e.g., Barbas et al. Phage display: A Laboratory Manuel. 1st Ed. New York: Cold Spring Harbor Laboratory Press, 2004. Print.; Lonberg, Nat. Biotech., 23: 1117-1125, 2005; Lonenberg, Curr. Opin. Immunol., 20:450-459, 2008).

An antibody may have one or more binding sites. If there is more than one binding site, the binding sites may be identical to one another or may be different. For instance, a naturally-occurring immunoglobulin has two identical binding sites, a single-chain antibody or Fab fragment has one binding site, while a bispecific or bifunctional antibody has two different binding sites.

**Biological sample:** A sample obtained from a subject. Biological samples include all clinical samples useful for detection of disease or infection (for example, cancer) in subjects, including, but not limited to, cells, tissues, and bodily fluids, such as blood, derivatives and fractions of blood (such as serum), cerebrospinal fluid; as well as biopsied or surgically removed tissue, for example tissues that are unfixed, frozen, or fixed in formalin or paraffin. In a particular example, a biological sample is obtained from a subject having or suspected of having a tumor; for example, a subject having or suspected of having a neuroblastoma. In some examples, the subject has or is suspected of having a carcinoma.

**Chemotherapeutic agent:** Any chemical agent with therapeutic usefulness in the treatment of diseases characterized by abnormal cell growth. For example, chemotherapeutic agents are useful for the treatment of neuroblastoma. Particular examples of additional therapeutic agents that can be used include microtubule binding agents, DNA intercalators or cross-linkers, DNA synthesis inhibitors, DNA and RNA transcription inhibitors, antibodies, enzymes, enzyme inhibitors, gene regulators, and angiogenesis inhibitors. In one embodiment, a chemotherapeutic agent is a radioactive compound. One of skill in the art can readily identify a chemotherapeutic agent of use (see for example, Slapak and Kufe, Principles of Cancer Therapy, Chapter 86 in Harrison's Principles of Internal Medicine, 14th edition; Perry et al., Chemotherapy, Ch. 17 in Abeloff, Clinical Oncology 2nd ed., © 2000 Churchill Livingstone, Inc; Baltzer, L., Berkery, R. (eds): Oncology Pocket Guide to Chemotherapy, 2nd ed. St. Louis, Mosby-Year Book, 1995; Fischer, D.S., Knobf, M.F., Durivage, H.J. (eds): The Cancer Chemotherapy Handbook, 4th ed. St. Louis, Mosby-Year Book, 1993; Chabner and Longo, Cancer Chemotherapy and Biotherapy: Principles and Practice (4th ed.). Philadelphia: Lippincott Willians & Wilkins, 2005; Skeel,. Handbook of Cancer Chemotherapy (6th ed.). Lippincott Williams & Wilkins, 2003). Combination chemotherapy is the administration of more than one agent to treat cancer.

**Chimeric Antigen Receptor (CAR):** An engineered T cell receptor having an extracellular antibody-derived targeting domain (such as an scFv) joined to one or more intracellular signaling domains of a T cell receptor. A **"chimeric antigen receptor T cell"** or **"CAR T cell**"is a T cell expressing a CAR, and has antigen specificity determined by the antibody-derived targeting domain of the CAR. Methods of making CARs (e.g., for treatment of cancer) are available (see, e.g., Park et al., Trends Biotechnol., 29:550-557, 2011; Grupp et al., N Engl J Med., 368:1509-1518, 2013; Han et al., J. Hematol Oncol., 6:47, 2013; Haso et al., (2013) Blood, 121, 1165-1174; PCT Pubs. WO2012/079000, WO2013/059593; and U.S. Pub. 2012/0213783.

**Conditions sufficient to form an immune complex:** Conditions which allow an antibody or antigen binding fragment thereof to bind to its cognate epitope to a detectably greater degree than, and/or to the substantial exclusion of, binding to substantially all other epitopes. Conditions sufficient to form an immune complex are dependent upon the format of the binding reaction and typically are those utilized in immunoassay protocols or those conditions encountered *in vivo.* See Harlow & Lane, *supra,* for a description of immunoassay formats and conditions. The conditions employed in the methods are "physiological conditions" which include reference to conditions (e.g., temperature, osmolarity, pH) that are typical inside a living mammal or a mammalian cell. While it is recognized that some organs are subject to extreme conditions, the intra-organismal and intracellular environment normally lies around pH 7 (e.g., from pH 6.0 to pH 8.0, more typically pH 6.5 to 7.5), contains water as the predominant solvent, and exists at a temperature above 0°C and below 50°C. Osmolarity is within the range that is supportive of cell viability and proliferation.

**Conjugate:** A complex of two molecules linked together, for example, linked together by a covalent bond. In one embodiment, an antibody is linked to an effector molecule; for example, an antibody that specifically binds to ALK covalently linked to an effector molecule. The linkage can be by chemical or recombinant means. In one embodiment, the linkage is chemical, wherein a reaction between the antibody moiety and the effector molecule has produced a covalent bond formed between the two molecules to form one molecule. A peptide linker (short peptide sequence) can optionally be included between the antibody and the effector molecule. Because conjugates can be prepared from two molecules with separate functionalities, such as an antibody and an effector molecule, they are also sometimes referred to as "chimeric molecules."

**Conservative variants:** "Conservative" amino acid substitutions are those substitutions that do not substantially decrease the binding affinity of an antibody for an antigen (for example, the binding affinity of an antibody for ALK). For example, a human antibody that specifically binds ALK can include at most about 1, at most about 2, at most about 5, at most about 10, or at most about 15 conservative substitutions and specifically bind the ALK polypeptide. The term conservative variation also includes the use of a substituted amino acid in place of an unsubstituted parent amino acid, provided that antibody retains binding affinity for ALK. Non-conservative substitutions are those that reduce an activity or binding to ALK.

Conservative amino acid substitution tables providing functionally similar amino acids are well known to one of ordinary skill in the art. The following six groups are examples of amino acids that are considered to be conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

**Contacting:** Placement in direct physical association; includes both in solid and liquid form, which can take place either *in vivo* or *in vitro.* Contacting includes contact between one molecule and another molecule, for example the amino acid on the surface of one polypeptide, such as an antigen, that contacts another polypeptide, such as an antibody. Contacting can also include contacting a cell for example by placing an antibody in direct physical association with a cell.

**Control:** A reference standard. In some embodiments, the control is a negative control, such as tissue sample obtained from a patient that does not have cancer, or a tissue sample from a tissue that is non-cancerous. In other embodiments, the control is a positive control, such as a tissue sample obtained from a patient diagnosed with cancer, or a tissue sample from a cancerous tissue. In still other embodiments, the control is a historical control or standard reference value or range of values (such as a previously tested control sample, such as a group of cancer patients with known prognosis or outcome, or group of samples that represent baseline or normal values).

A difference between a test sample and a control can be an increase or conversely a decrease. The difference can be a qualitative difference or a quantitative difference, for example a statistically significant difference. In some examples, a difference is an increase or decrease, relative to a control, of at least about 5%, such as at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, or at least about 500%.

**Crizotinib:** A receptor tyrosine kinase inhibitor that inhibits ALK. Crizotinib (also known as PF-02341066 or XALKORI, Pfizer) is an orally available selective ATP-competitive small molecule inhibitor of ALK and c-Met/HGFR tyrosine kinases and their oncogenic variants. See, *e.g.,* U.S. Pat. Nos. 7,230,098; 7,825,137; 7,858,643; and 8,217,057. Crizotinib can be used to treat patients with ALK-positive tumors.

**Decrease** or **Reduce:** To reduce the quality, amount, or strength of something; for example a reduction in tumor burden. In one example, a therapy reduces a tumor (such as the size of a tumor, the number of tumors, the metastasis of a tumor, or combinations thereof), or one or more symptoms associated with a tumor, for example as compared to the response in the absence of the therapy. In a particular example, a therapy decreases the size of a tumor, the number of tumors, the metastasis of a tumor, or combinations thereof, subsequent to the therapy, such as a decrease of at least 10%, at least 20%, at least 30%, at least 40%, at least 50%, at least 60%, at least 70%, at least 80%, or at least 90%. Such decreases can be measured using the methods disclosed herein.

**Degenerate variant:** In the context of the present disclosure, a "degenerate variant" refers to a polynucleotide encoding a protein (for example, an antibody that specifically binds ALK) that includes a sequence that is degenerate as a result of the genetic code. There are twenty natural amino acids, most of which are specified by more than one codon. Therefore, all degenerate nucleotide sequences are included as long as the amino acid sequence of the antibody that binds ALK encoded by the nucleotide sequence is unchanged.

**Detectable marker:** A detectable molecule (also known as a label) that is conjugated directly or indirectly to a second molecule, such as an antibody, to facilitate detection of the second molecule. For example, the detectable marker can be capable of detection by ELISA, spectrophotometry, flow cytometry, microscopy or diagnostic imaging techniques (such as CT scans, MRIs, ultrasound, fiberoptic examination, and laparoscopic examination). Specific, non-limiting examples of detectable markers include fluorophores, chemiluminescent agents, enzymatic linkages, radioactive isotopes and heavy metals or compounds (for example super paramagnetic iron oxide nanocrystals for detection by MRI). In one example, a "labeled antibody" refers to incorporation of another molecule in the antibody. For example, the label is a detectable marker, such as the incorporation of a radiolabeled amino acid or attachment to a polypeptide of biotinyl moieties that can be detected by marked avidin (for example, streptavidin containing a fluorescent marker or enzymatic activity that can be detected by optical or colorimetric methods). Various methods of labeling polypeptides and glycoproteins are known in the art and may be used. Examples of labels for polypeptides include, but are not limited to, the following: radioisotopes or radionuclides (such as ³⁵S or ¹³¹I), fluorescent labels (such as fluorescein isothiocyanate (FITC), rhodamine, lanthanide phosphors), enzymatic labels (such as horseradish peroxidase, beta-galactosidase, luciferase, alkaline phosphatase), chemiluminescent markers, biotinyl groups, predetermined polypeptide epitopes recognized by a secondary reporter (such as a leucine zipper pair sequences, binding sites for secondary antibodies, metal binding domains, epitope tags), or magnetic agents, such as gadolinium chelates. In some embodiments, labels are attached by spacer arms of various lengths to reduce potential steric hindrance. Methods for using detectable markers and guidance in the choice of detectable markers appropriate for various purposes are discussed for example in Sambrook et al. (Molecular Cloning: A Laboratory Manual, 4th ed, Cold Spring Harbor, New York, 2012) and Ausubel et al. (In Current Protocols in Molecular Biology, John Wiley & Sons, New York, through supplement 104, 2013).

**Detecting:** To identify the existence, presence, or fact of something. General methods of detecting are known to the skilled artisan and may be supplemented with the protocols and reagents disclosed herein. For example, included herein are methods of detecting an ALK-positive tumor in a subject.

**Effector molecule:** A molecule intended to have or produce a desired effect; for example, a desired effect on a cell to which the effector molecule is targeted. Effector molecules include such molecules as polypeptides, radioisotopes and small molecules. Non-limiting examples of effector molecules include toxins, chemotherapeutic agents and anti-angiogenic agents. The skilled artisan will understand that some effector molecules may have or produce more than one desired effect. In one example, an effector molecule is the portion of a chimeric molecule, for example a chimeric molecule that includes a disclosed antibody or fragment thereof, that is intended to have a desired effect on a cell to which the chimeric molecule is targeted.

**Epitope:** An antigenic determinant. These are particular chemical groups or peptide sequences on a molecule that are antigenic, *i.e*. that elicit a specific immune response. An antibody specifically binds a particular antigenic epitope on a polypeptide. In some examples a disclosed antibody specifically binds to an epitope on ALK.

**Expressed:** Translation of a nucleic acid into a protein. Proteins may be expressed and remain intracellular, become a component of the cell surface membrane, or be secreted into the extracellular matrix or medium.

**Expression Control Sequences:** Nucleic acid sequences that regulate the expression of a heterologous nucleic acid sequence to which it is operatively linked. Expression control sequences are operatively linked to a nucleic acid sequence when the expression control sequences control and regulate the transcription and, as appropriate, translation of the nucleic acid sequence. Thus expression control sequences can include appropriate promoters, enhancers, transcription terminators, a start codon (i.e., ATG) in front of a protein-encoding gene, splicing signal for introns, maintenance of the correct reading frame of that gene to permit proper translation of mRNA, and stop codons. The term "control sequences" is intended to include, at a minimum, components whose presence can influence expression, and can also include additional components whose presence is advantageous, for example, leader sequences and fusion partner sequences. Expression control sequences can include a promoter.

A promoter is a minimal sequence sufficient to direct transcription. Also included are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters are included (see for example, Bitter et al., Methods in Enzymology 153:516-544, 1987). For example, when cloning in bacterial systems, inducible promoters such as pL of bacteriophage lambda, plac, ptrp, ptac (ptrp-lac hybrid promoter) and the like may be used. In one embodiment, when cloning in mammalian cell systems, promoters derived from the genome of mammalian cells (such as metallothionein promoter) or from mammalian viruses (such as the retrovirus long terminal repeat; the adenovirus late promoter; the vaccinia virus 7.5K promoter) can be used. Promoters produced by recombinant DNA or synthetic techniques may also be used to provide for transcription of the nucleic acid sequences. A polynucleotide can be inserted into an expression vector that contains a promoter sequence which facilitates the efficient transcription of the inserted genetic sequence of the host. The expression vector typically contains an origin of replication, a promoter, as well as specific nucleic acid sequences that allow phenotypic selection of the transformed cells.

**Expression vector:** A vector comprising a recombinant polynucleotide comprising expression control sequences operatively linked to a nucleotide sequence to be expressed. An expression vector comprises sufficient cis- acting elements for expression; other elements for expression can be supplied by the host cell or in an in vitro expression system. Expression vectors include all those known in the art, such as cosmids, plasmids (e.g., naked or contained in liposomes) and viruses (e.g., lentiviruses, retroviruses, adenoviruses, and adeno-associated viruses) that incorporate the recombinant polynucleotide.

**Immune complex:** The binding of antibody or antigen binding fragment (such as an antigen binding domain on a CAR) to a soluble antigen forms an immune complex. The formation of an immune complex can be detected through conventional methods known to the skilled artisan, for instance immunohistochemistry, immunoprecipitation, flow cytometry, immunofluorescence microscopy, ELISA, immunoblotting (for example, Western blot), magnetic resonance imaging, CT scans, X-ray and affinity chromatography. Immunological binding properties of selected antibodies may be quantified using methods well known in the art.

**Inhibiting or Treating a Disease:** A therapeutic intervention (for example, administration of a therapeutically effective amount of an antibody that specifically binds ALK or a conjugate thereof) that reduces a sign or symptom of a disease or pathological condition related to a disease (such as a tumor). Treatment can also induce remission or cure of a condition, such as a tumor. In particular examples, treatment includes preventing a tumor, for example by inhibiting the full development of a tumor, such as preventing development of a metastasis or the development of a primary tumor. Prevention does not require a total absence of a tumor.

Reducing a sign or symptom of a disease or pathological condition related to a disease, refers to any observable beneficial effect of the treatment. Reducing a sign or symptom associated with a tumor can be evidenced, for example, by a delayed onset of clinical symptoms of the disease in a susceptible subject (such as a subject having a tumor which has not yet metastasized), a reduction in severity of some or all clinical symptoms of the disease, a slower progression of the disease (for example by prolonging the life of a subject having tumor), a reduction in the number of relapses of the disease, an improvement in the overall health or well-being of the subject, or by other parameters well known in the art that are specific to the particular tumor. A "prophylactic" treatment is a treatment administered to a subject who does not exhibit signs of a disease or exhibits only early signs for the purpose of decreasing the risk of developing pathology.

**Isolated:** A biological component (such as a nucleic acid, peptide, protein or protein complex, for example an antibody) that has been substantially separated, produced apart from, or purified away from other biological components in the cell of the organism in which the component naturally occurs, that is, other chromosomal and extra-chromosomal DNA and RNA, and proteins. Thus, isolated nucleic acids, peptides and proteins include nucleic acids and proteins purified by standard purification methods. The term also embraces nucleic acids, peptides and proteins prepared by recombinant expression in a host cell, as well as, chemically synthesized nucleic acids. A isolated nucleic acid, peptide or protein, for example an antibody, can be at least 50%, at least 60%, at least 70%, at least 80%, at least 90%, at least 95%, at least 96%, at least 97%, at least 98%, or at least 99% pure.

**K_{D}**: The dissociation constant for a given interaction, such as a polypeptide ligand interaction or an antibody antigen interaction. For example, for the bimolecular interaction of an antibody or antigen binding fragment (such as an ALK specific antibody or an antigen binding fragment thereof) and an antigen (such as ALK protein) it is the concentration of the individual components of the bimolecular interaction divided by the concentration of the complex.

**Linker:** A bi-functional molecule that can be used to link two molecules into one contiguous molecule, for example, to link an effector molecule to an antibody. In some embodiments, the provided conjugates include a linker between the effector molecule or detectable marker and an antibody. In some embodiments, the linker is cleavable under intracellular conditions, such that cleavage of the linker releases the effector molecule or detectable marker from the antibody in the intracellular environment. In yet other embodiments, the linker is not cleavable and the effector molecule or detectable marker can be released, for example, by antibody degradation. In some cases, a linker is a peptide within an antigen binding fragment (such as an Fv fragment) which serves to indirectly bond the variable heavy chain to the variable light chain.

The terms "conjugating," "joining," "bonding," or "linking" refer to making two molecules into one contiguous molecule; for example, linking two polypeptides into one contiguous polypeptide, or covalently attaching an effector molecule or detectable marker radionuclide or other molecule to a polypeptide, such as an scFv. In the specific context, the terms include reference to joining a ligand, such as an antibody moiety, to an effector molecule. The linkage can be either by chemical or recombinant means. "Chemical means" refers to a reaction between the antibody moiety and the effector molecule such that there is a covalent bond formed between the two molecules to form one molecule.

**Neutralizing antibody:** An antibody that is able to specifically bind to a target protein in such a way as to inhibit a biological function associated with that target protein. In general, any protein that can perform this type of specific blocking activity is considered a neutralizing protein; neutralizing antibodies are therefore a specific class of neutralizing protein.

**Neoplasia, cancer, or tumor:** A neoplasm is an abnormal growth of tissue or cells that results from excessive cell division. Neoplastic growth can produce a tumor. The amount of a tumor in an individual is the "tumor burden" which can be measured as the number, volume, or weight of the tumor. A tumor that does not metastasize is referred to as "benign." A tumor that invades the surrounding tissue or can metastasize (or both) is referred to as "malignant."

Tumors of the same tissue type are primary tumors originating in a particular organ and may be divided into tumors of different sub-types. For examples, lung carcinomas can be divided into an adenocarcinoma, small cell, squamous cell, or non-small cell tumors.

Examples of solid tumors, such as sarcomas (connective tissue cancer) and carcinomas (epithelial cell cancer), include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colorectal carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, glioblastoma, medulloblastoma, craniopharyogioma, ependymoma, pinealoma, hemangioblastoma, acoustic neuroma, oligodendroglioma, menangioma, melanoma, neuroblastoma and retinoblastoma).

**Neuroblastoma:** A solid cancerous tumor that usually originates in the abdomen in adrenal gland tissue, but can also originate from nerve tissue in the neck, chest, abdomen, and pelvis. By the time it is diagnosed, the cancer has usually metastasized to the lymph nodes, liver, lungs, bones and bone marrow. Neuroblastoma is the most common heterogenous and malignant tumor of early childhood, and two thirds of individuals with neuroblastoma are diagnosed when they are younger than 5 years.

Neuroblastoma is derived from the neural crest and is characterized by a marked clinical heterogeneity (aggressive, unremitting growth to spontaneous remission). As classified by International Neuroblastoma Staging System (INSS) there are six stages of neuroblastoma: Stage 1 (localized resectable), Stage 2A and 2B (localized unresectable or ipsilateral lymph node involvement), Stage 3 (regional, unresectable and crossing the midline), Stage 4 (disseminated) and Stage 4S (localized with limited spread; less than one year of age) referred to as "special" neuroblastoma. (See, e.g., Hayat (Ed.), Neuroblastoma, Pediatric Cancer, Volume 1, New York: Springer, 2011.)

**Nucleic acid:** A polymer composed of nucleotide units (ribonucleotides, deoxyribonucleotides, related naturally occurring structural variants, and synthetic non-naturally occurring analogs thereof) linked via phosphodiester bonds, related naturally occurring structural variants, and synthetic non-naturally occurring analogs thereof. Thus, the term includes nucleotide polymers in which the nucleotides and the linkages between them include non-naturally occurring synthetic analogs, such as, for example and without limitation, phosphorothioates, phosphoramidates, methyl phosphonates, chiral-methyl phosphonates, 2-O-methyl ribonucleotides, peptide-nucleic acids (PNAs), and the like. Such polynucleotides can be synthesized, for example, using an automated DNA synthesizer. The term "oligonucleotide" typically refers to short polynucleotides, generally no greater than about 50 nucleotides. It will be understood that when a nucleotide sequence is represented by a DNA sequence (i.e., A, T, G, C), this also includes an RNA sequence (i.e., A, U, G, C) in which "U" replaces "T. "

"Nucleotide" includes, but is not limited to, a monomer that includes a base linked to a sugar, such as a pyrimidine, purine or synthetic analogs thereof, or a base linked to an amino acid, as in a peptide nucleic acid (PNA). A nucleotide is one monomer in a polynucleotide. A nucleotide sequence refers to the sequence of bases in a polynucleotide.

Conventional notation is used herein to describe nucleotide sequences: the left-hand end of a single-stranded nucleotide sequence is the 5'-end; the left-hand direction of a double-stranded nucleotide sequence is referred to as the 5'-direction. The direction of 5' to 3' addition of nucleotides to nascent RNA transcripts is referred to as the transcription direction. The DNA strand having the same sequence as an mRNA is referred to as the "coding strand;" sequences on the DNA strand having the same sequence as an mRNA transcribed from that DNA and which are located 5' to the 5'-end of the RNA transcript are referred to as "upstream sequences;" sequences on the DNA strand having the same sequence as the RNA and which are 3' to the 3' end of the coding RNA transcript are referred to as "downstream sequences."

"cDNA" refers to a DNA that is complementary or identical to an mRNA, in either single stranded or double stranded form.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA produced by that gene produces the protein in a cell or other biological system. Both the coding strand, the nucleotide sequence of which is identical to the mRNA sequence and is usually provided in sequence listings, and non-coding strand, used as the template for transcription, of a gene or cDNA can be referred to as encoding the protein or other product of that gene or cDNA. Unless otherwise specified, a "nucleotide sequence encoding an amino acid sequence" includes all nucleotide sequences that are degenerate versions of each other and that encode the same amino acid sequence. Nucleotide sequences that encode proteins and RNA may include introns.

A first sequence is an "antisense" with respect to a second sequence if a polynucleotide whose sequence is the first sequence specifically hybridizes with a polynucleotide whose sequence is the second sequence.

**Operably linked:** A first nucleic acid sequence is operably linked with a second nucleic acid sequence when the first nucleic acid sequence is placed in a functional relationship with the second nucleic acid sequence. For instance, a promoter, such as the CMV promoter, is operably linked to a coding sequence if the promoter affects the transcription or expression of the coding sequence. Generally, operably linked DNA sequences are contiguous and, where necessary to join two protein-coding regions, in the same reading frame.

**Pharmaceutically acceptable carriers:** The pharmaceutically acceptable carriers of use are conventional. Remington's Pharmaceutical Sciences, by E. W. Martin, Mack Publishing Co., Easton, PA, 19th Edition, 1995, describes compositions and formulations suitable for pharmaceutical delivery of the disclosed immunogens.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e.g., powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate. In particular embodiments, suitable for administration to a subject the carrier may be sterile, and/or suspended or otherwise contained in a unit dosage form containing one or more measured doses of the composition suitable to induce the desired anti- MERS-CoV immune response. It may also be accompanied by medications for its use for treatment purposes. The unit dosage form may be, for example, in a sealed vial that contains sterile contents or a syringe for injection into a subject, or lyophilized for subsequent solubilization and administration or in a solid or controlled release dosage.

**Polypeptide:** Any chain of amino acids, regardless of length or post-translational modification (e.g., glycosylation or phosphorylation). "Polypeptide" applies to amino acid polymers including naturally occurring amino acid polymers and non-naturally occurring amino acid polymer as well as in which one or more amino acid residue is a non-natural amino acid, for example an artificial chemical mimetic of a corresponding naturally occurring amino acid. A "residue" refers to an amino acid or amino acid mimetic incorporated in a polypeptide by an amide bond or amide bond mimetic. A polypeptide has an amino terminal (N-terminal) end and a carboxy terminal (C-terminal) end. "Polypeptide" is used interchangeably with peptide or protein, and is used herein to refer to a polymer of amino acid residues. A protein can include multiple polypeptide chains; for example, mature MERS-CoV S protein includes S1 and S2 polypeptide chains.

Amino acids in a peptide, polypeptide or protein generally are chemically bound together via amide linkages (CONH). Additionally, amino acids may be bound together by other chemical bonds. For example, linkages for amino acids or amino acid analogs can include CH₂NH-, -CH₂S-, -CH₂-CH₂ -, -CH=CH-- (cis and trans), -COCH₂ --, -CH(OH)CH₂-, and -CHH₂SO- (These and others can be found in Spatola, in Chemistry and Biochemistry of Amino Acids, Peptides, and Proteins, B. Weinstein, eds., Marcel Dekker, New York, p. 267 (1983); Spatola, A. F., Vega Data (March 1983), Vol. 1, Issue 3, Peptide Backbone Modifications (general review); Morley, Trends Pharm Sci pp. 463-468, 1980; Hudson, et al., Int J Pept Prot Res 14:177-185, 1979; Spatola et al. Life Sci 38:1243-1249, 1986; Harm J. Chem. Soc Perkin Trans. 1307-314, 1982; Almquist et al. J. Med. Chem. 23:1392-1398, 1980; Jennings-White et al. Tetrahedron Lett 23:2533, 1982; Holladay et al. Tetrahedron. Lett 24:4401-4404, 1983; and Hruby Life Sci 31:189-199, 1982.

**Polypeptide modifications:** Polypeptides and peptides, such as the MERS-CoV S proteins disclosed herein can be modified by a variety of chemical techniques to produce derivatives having essentially the same activity as the unmodified peptides, and optionally having other desirable properties. For example, carboxylic acid groups of the protein, whether carboxyl-terminal or side chain, may be provided in the form of a salt of a pharmaceutically-acceptable cation or esterified to form a C₁-C₁₆ ester, or converted to an amide of formula NR₁R₂ wherein R₁ and R₂ are each independently H or C₁-C₁₆ alkyl, or combined to form a heterocyclic ring, such as a 5- or 6- membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to C₁-C₁₆ alkyl or dialkyl amino or further converted to an amide.

Hydroxyl groups of the peptide side chains can be converted to C₁-C₁₆ alkoxy or to a C₁-C₁₆ ester using well-recognized techniques. Phenyl and phenolic rings of the peptide side chains can be substituted with one or more halogen atoms, such as F, Cl, Br or I, or with C₁-C₁₆ alkyl, C₁-C₁₆ alkoxy, carboxylic acids and esters thereof, or amides of such carboxylic acids. Methylene groups of the peptide side chains can be extended to homologous C₂-C₄ alkylenes. Thiols can be protected with any one of a number of well-recognized protecting groups, such as acetamide groups. Those skilled in the art will also recognize methods for introducing cyclic structures into the peptides of this disclosure to select and provide conformational constraints to the structure that result in enhanced stability. For example, a C- or N-terminal cysteine can be added to the peptide, so that when oxidized the peptide will contain a disulfide bond, generating a cyclic peptide. Other peptide cyclizing methods include the formation of thioethers and carboxyl- and amino-terminal amides and esters.

**Purified:** The term purified does not require absolute purity; rather, it is intended as a relative term. Thus, for example, a purified peptide preparation is one in which the peptide or protein (such as an antibody) is more enriched than the peptide or protein is in its natural environment within a cell. In one embodiment, a preparation is purified such that the protein or peptide represents at least 50% of the total peptide or protein content of the preparation.

**Recombinant:** A recombinant nucleic acid is one that has a sequence that is not naturally occurring or has a sequence that is made by an artificial combination of two otherwise separated segments of sequence. This artificial combination can be accomplished by chemical synthesis or, more commonly, by the artificial manipulation of isolated segments of nucleic acids, for example, by genetic engineering techniques. A recombinant protein is a protein encoded by a heterologous (for example, recombinant) nucleic acid, that has been introduced into a host cell, such as a bacterial or eukaryotic cell. The nucleic acid can be introduced, for example, on an expression vector having signals capable of expressing the protein encoded by the introduced nucleic acid or the nucleic acid can be integrated into the host cell chromosome.

**Sequence identity:** The similarity between amino acid sequences is expressed in terms of the similarity between the sequences, otherwise referred to as sequence identity. Sequence identity is frequently measured in terms of percentage identity (or similarity or homology); the higher the percentage, the more similar the two sequences are. Homologs or variants of a polypeptide will possess a relatively high degree of sequence identity when aligned using standard methods.

Methods of alignment of sequences for comparison are well known in the art. Various programs and alignment algorithms are described in: Smith and Waterman, Adv. Appl. Math. 2:482, 1981; Needleman and Wunsch, J. Mol. Biol. 48:443, 1970; Pearson and Lipman, Proc. Natl. Acad. Sci. U.S.A. 85:2444, 1988; Higgins and Sharp, Gene 73:237, 1988; Higgins and Sharp, CABIOS 5:151, 1989; Corpet et al., Nucleic Acids Research 16:10881, 1988; and Pearson and Lipman, Proc. Natl. Acad. Sci. U.S.A. 85:2444, 1988. Altschul et al., Nature Genet. 6:119, 1994, presents a detailed consideration of sequence alignment methods and homology calculations.

The NCBI Basic Local Alignment Search Tool (BLAST) (Altschul et al., J. Mol. Biol. 215:403, 1990) is available from several sources, including the National Center for Biotechnology Information (NCBI, Bethesda, MD) and on the internet, for use in connection with the sequence analysis programs blastp, blastn, blastx, tblastn and tblastx. A description of how to determine sequence identity using this program is available on the NCBI website on the internet.

Homologs and variants of a V_{L} or a V_{H} of an antibody that specifically binds a polypeptide are typically characterized by possession of at least about 75%, for example at least about 80%, 85%, 90%, 91%, 92%, 93%, 94%, 95%, 96%, 97%, 98% or 99% sequence identity counted over the full length alignment with the amino acid sequence of interest. Proteins with even greater similarity to the reference sequences will show increasing percentage identities when assessed by this method, such as at least 80%, at least 85%, at least 90%, at least 95%, at least 98%, or at least 99% sequence identity. When less than the entire sequence is being compared for sequence identity, homologs and variants will typically possess at least 80% sequence identity over short windows of 10-20 amino acids, and may possess sequence identities of at least 85% or at least 90% or 95% depending on their similarity to the reference sequence. Methods for determining sequence identity over such short windows are available at the NCBI website on the internet. One of skill in the art will appreciate that these sequence identity ranges are provided for guidance only; it is entirely possible that strongly significant homologs could be obtained that fall outside of the ranges provided.

Terms used to describe sequence relationships between two or more nucleotide sequences or amino acid sequences include "reference sequence," "selected from," "comparison window," "identical," "percentage of sequence identity," "substantially identical," "complementary," and "substantially complementary."

For sequence comparison of nucleic acid sequences, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters are used. Methods of alignment of sequences for comparison are well known in the art. Optimal alignment of sequences for comparison can be conducted, e.g., by the local homology algorithm of Smith & Waterman, Adv. Appl. Math. 2:482, 1981, by the homology alignment algorithm of Needleman & Wunsch, J. Mol. Biol. 48:443, 1970, by the search for similarity method of Pearson & Lipman, Proc. Nat'l. Acad. Sci. USA 85:2444, 1988, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (see, e.g., Current Protocols in Molecular Biology (Ausubel et al., eds 1995 supplement)).

One example of a useful algorithm is PILEUP. PILEUP uses a simplification of the progressive alignment method of Feng & Doolittle, J. Mol. Evol. 35:351-360, 1987. The method used is similar to the method described by Higgins & Sharp, CABIOS 5:151-153, 1989. Using PILEUP, a reference sequence is compared to other test sequences to determine the percent sequence identity relationship using the following parameters: default gap weight (3.00), default gap length weight (0.10), and weighted end gaps. PILEUP can be obtained from the GCG sequence analysis software package, e.g., version 7.0 (Devereaux et al., Nuc. Acids Res. 12:387-395, 1984.

Another example of algorithms that are suitable for determining percent sequence identity and sequence similarity are the BLAST and the BLAST 2.0 algorithm, which are described in Altschul et al., J. Mol. Biol. 215:403-410, 1990 and Altschul et al., Nucleic Acids Res. 25:3389-3402, 1977. Software for performing BLAST analyses is publicly available through the National Center for Biotechnology Information (ncbi.nlm.nih.gov). The BLASTN program (for nucleotide sequences) uses as defaults a word length (W) of 11, alignments (B) of 50, expectation (E) of 10, M=5, N=-4, and a comparison of both strands. The BLASTP program (for amino acid sequences) uses as defaults a word length (W) of 3, and expectation (E) of 10, and the BLOSUM62 scoring matrix (see Henikoff & Henikoff, Proc. Natl. Acad. Sci. USA 89:10915, 1989). An oligonucleotide is a linear polynucleotide sequence of up to about 100 nucleotide bases in length.

**Signal Peptide:** A short amino acid sequence (e.g., approximately 18-25 amino acids in length) that directs newly synthesized secretory or membrane proteins to and through membranes (for example, the endoplasmic reticulum membrane). Signal peptides are typically located at the N-terminus of a polypeptide and can be removed by signal peptidases after the polypeptide has crossed the membrane. Signal peptide sequences typically contain three common structural features: an N-terminal polar basic region (n-region), a hydrophobic core, and a hydrophilic c-region). An exemplary signal peptide sequence is provided as SEQ ID NO: 26.

**Specifically bind:** When referring to an antibody, refers to a binding reaction which determines the presence of a target protein, peptide, or polysaccharide in the presence of a heterogeneous population of proteins and other biologies. Thus, under designated conditions, an antibody binds preferentially to a particular target protein, peptide or polysaccharide (such as an epitope of ALK) and does not bind in a significant amount to other proteins or polysaccharides present in the sample or subject. Specific binding can be determined by methods known in the art. With reference to an antibody antigen complex, specific binding of the antigen and antibody has a K_{d} of less than about 10⁻⁷ Molar (M), such as less than about 10⁻⁸ M, 10⁻⁹ M, 10⁻¹⁰ M, or even less than about 10⁻¹¹ M.

The antibodies disclosed herein specifically bind only to a defined target (or multiple targets, in the case of a bispecific antibody). Thus, an antibody that specifically binds to ALK is an antibody that binds substantially to ALK, including cells or tissue expressing ALK, substrate to which the ALK is attached, or ALK in a biological specimen. It is, of course, recognized that a certain degree of non-specific interaction may occur between an antibody or conjugate including an antibody (such as an antibody that specifically binds ALK or conjugate including such antibody) and a non-target (such as a cell that does not express ALK). Typically, specific binding results in a much stronger association between the antibody and protein or cells bearing the antigen than between the antibody and protein or cells lacking the antigen. Specific binding typically results in greater than 2-fold, such as greater than 5-fold, greater than 10-fold, or greater than 100-fold increase in amount of bound antibody (per unit time) to a protein including the epitope or cell or tissue expressing the target epitope as compared to a protein or cell or tissue lacking this epitope. Specific binding to a protein under such conditions requires an antibody that is selected for its specificity for a particular protein. A variety of immunoassay formats are appropriate for selecting antibodies or other ligands specifically immunoreactive with a particular protein. For example, solid-phase ELISA immunoassays are routinely used to select monoclonal antibodies specifically immunoreactive with a protein. See Harlow & Lane, Antibodies, A Laboratory Manual, 2nd ed., Cold Spring Harbor Publications, New York (2013), for a description of immunoassay formats and conditions that can be used to determine specific immunoreactivity.

**Subject:** Any mammal, such as humans, non-human primates, pigs, sheep, cows, rodents, and the like. In two non-limiting examples, a subject is a human subject or a murine subject. Thus, the term "subject" includes both human and veterinary subjects.

**T Cell:** A white blood cell critical to the immune response. T cells include, but are not limited to, CD4⁺ T cells and CD8⁺ T cells. A CD4⁺ T lymphocyte is an immune cell that carries a marker on its surface known as "cluster of differentiation 4" (CD4). These cells, also known as helper T cells, help orchestrate the immune response, including antibody responses as well as killer T cell responses. CD8⁺ T cells carry the "cluster of differentiation 8" (CD8) marker. In one embodiment, a CD8 T cell is a cytotoxic T lymphocyte. In another embodiment, a CD8 cell is a suppressor T cell. An **effector function of a T cell** is a specialized function of the T cell, such as cytolytic activity or helper activity including the secretion of cytokines.

T **Cell Signaling Domain:** An intracellular portion of a protein expressed in a T cell that transduces a T cell effector function signal (e.g., an activation signal) and directs the T cell to perform a specialized function. T cell activation can be induced by a number of factors, including binding of cognate antigen to the T cell receptor on the surface of T cells and binding of cognate ligand to co-stimulatory molecules on the surface of the T cell. A T cell co-stimulatory molecule is a cognate binding partner on a T cell that specifically binds with a co-stimulatory ligand, thereby mediating a co-stimulatory response by the T cell, such as, but not limited to, proliferation. Co-stimulatory molecules include, but are not limited to an MHC class I molecule, BTLA and a Toll ligand receptor. Activation of a T cell leads to immune response, such as T cell proliferation and differentiation (see, e.g., Smith-Garvin et al., Annu. Rev. Immunol., 27:591-619, 2009). Exemplary T cell signaling domains are known and described herein. Non-limiting examples include the CD3 zeta, CD8, CD28, CD27, CD154, GITR (TNFRSF18), CD134 (OX40), and CD137 (4-1BB) signaling domains.

**Therapeutic agent:** Used in a generic sense, it includes treating agents, prophylactic agents, and replacement agents. A therapeutic agent is used to ameliorate a specific set of conditions in a subject with a disease or a disorder.

**Therapeutically effective amount:** The amount of an agent (such as a ALK specific antibody, antigen binding fragment, CAR or CAR T cell, or nucleic acid molecule encoding thereof) that alone, or together with one or more additional agents, induces the desired response, such as, for example treatment of a tumor in a subject. Ideally, a therapeutically effective amount provides a therapeutic effect without causing a substantial cytotoxic effect in the subject.

In one example, a desired response is to decrease the size, volume, or number (such as metastases) of a tumor in a subject. For example, the agent or agents can decrease the size, volume, or number of tumors by a desired amount, for example by at least 5%, at least 10%, at least 15%, at least 20%, at least 25%, at least 30%, at least 50%, at least 75%, at least 90%, or at least 95% as compared to a response in the absence of the agent.

Several preparations disclosed herein are administered in therapeutically effective amounts. A therapeutically effective amount of an antibody that specifically binds ALK or antigen binding fragment thereof, or conjugate thereof (or a composition including one or more of these molecules) that is administered to a human or veterinary subject will vary depending upon a number of factors associated with that subject, for example the overall health of the subject. A therapeutically effective amount can be determined by varying the dosage and measuring the resulting therapeutic response, such as the regression of a tumor. Therapeutically effective amounts also can be determined through various *in vitro, in vivo* or *in situ* immunoassays. The disclosed agents can be administered in a single dose, or in several doses, as needed to obtain the desired response. However, the therapeutically effective amount of can be dependent on the source applied, the subject being treated, the severity and type of the condition being treated, and the manner of administration.

**Toxin:** An effector molecule that induces cytotoxicity when it contacts a cell. Specific, non-limiting examples of toxins include, but are not limited to, abrin, ricin, auristatins (such as monomethyl auristatin E (MMAE; see for example, Francisco et al., Blood, 102: 1458-1465, 2003)) and monomethyl auristatin F (MMAF; see, for example, Doronina et al., BioConjugate Chem., 17: 114-124, 2006), maytansinoids (such as DM1; see, for example, Phillips et al., Cancer Res., 68:9280-9290, 2008), *Pseudomonas* exotoxin (PE, such as PE35, PE37, PE38, and PE40), diphtheria toxin (DT), botulinum toxin, saporin, restrictocin or gelonin, or modified toxins thereof, or other toxic agents that directly or indirectly inhibit cell growth or kill cells. For example, PE and DT are highly toxic compounds that typically bring about death through liver toxicity. PE and DT, however, can be modified into a form for use as an immunotoxin by removing the native targeting component of the toxin (such as the domain Ia of PE and the B chain of DT) and replacing it with a different targeting moiety, such as an antibody.

**Transmembrane domain:** An amino acid sequence that inserts into a lipid bilayer, such as the lipid bilayer of a cell or virus or virus-like particle. A transmembrane domain can be used to anchor a protein of interest (such as a CAR) to a membrane. Exemplary transmembrane domains are familiar to the person of ordinary skill in the art, and provided herein.

**Transformed:** A transformed cell is a cell into which a nucleic acid molecule has been introduced by molecular biology techniques. As used herein, the term transformation encompasses all techniques by which a nucleic acid molecule might be introduced into such a cell, including transfection with viral vectors, transformation with plasmid vectors, and introduction of DNA by electroporation, lipofection, and particle gun acceleration.

**Tumor burden:** The total volume, number, metastasis, or combinations thereof of tumor or tumors in a subject.

**Under conditions sufficient for:** A phrase that is used to describe any environment that permits a desired activity. In one example the desired activity is formation of an immune complex. In particular examples the desired activity is treatment of a tumor.

### II. Description of Several Embodiments

Isolated monoclonal antibodies that specifically bind to ALK on the cell surface, antigen binding fragments of such antibodies, conjugates thereof, nucleic acid molecules encoding the antibodies and/or antigen binding fragments, and methods of using these molecules, are provided. Several embodiments include a chimeric antigen receptor including a disclosed antigen binding fragment that specifically binds to ALK, or a nucleic acid molecule encoding the CAR. The nucleic acid molecule can be included in an expression vector (such as a viral vector) for expression in a host cell (such as an autologous T cell). Isolated host cells (such as a T-cell) that express the nucleic acid molecules are also provided.

Compositions including the antibodies, antigen binding fragments, conjugates, CARs, nucleic acid molecules, and/or host cells, and a pharmaceutically acceptable carrier as also provided. The compositions can be used for research, diagnostic and therapeutic purposes, for example for treatment of a tumor (such as is a neuroblastoma, a rhabdomyosarcoma, or a glioblastoma) in a subject.

### A. Chimeric Antigen Receptors (CARs)

Disclosed herein are CARs that are artificially constructed chimeric proteins including an extracellular antigen binding domain (e.g., single chain variable fragment (scFv)) that specifically binds to ALK), linked to a transmembrane domain, linked to one or more intracellular T-cell signaling domains. Characteristics of the disclosed CARs include their ability to redirect T-cell specificity and reactivity towards ALK expressing cells in a non-MHC-restricted manner. The non-MHC-restricted ALK recognition gives T cells expressing a disclosed CAR the ability to recognize antigen independent of antigen processing, thus bypassing a major mechanism of tumor escape.

The intracellular T cell signaling domains can include, for example, a T cell receptor signaling domain, a T cell costimulatory signaling domain, or both. The T cell receptor signaling domain refers to a portion of the CAR comprising the intracellular domain of a T cell receptor, such as the intracellular portion of the CD3 zeta protein. The costimulatory signaling domain refers to a portion of the CAR comprising the intracellular domain of a costimulatory molecule, which is a cell surface molecule other than an antigen receptor or their ligands that are required for an efficient response of lymphocytes to antigen.

In some embodiments, the CAR includes or consists of the amino acid sequence set forth as one of SEQ ID NOs: 43-90.

### 1. Extracellular Region

Several embodiments provide a CAR including an antigen binding domain that specifically binds to ALK as disclosed herein (see, e.g., section II.B below). For example, the antigen binding domain can be a scFv including the heavy chain variable region and the light chain variable region of any of the antibodies or antigen binding fragments thereof disclosed in section II.B below.

In some embodiments, the antigen binding domain can include a heavy chain variable region and a light chain variable region including the HCDR1, HCDR2, and HCDR3, and LCDR1, LCDR2, and LCDR3 of the of the heavy and light chain variable regions, respectively, of one of the ALK15, ALK48, ALK53, or ALK58 antibodies (e.g., as set forth in Table 1 or Table 2 below). In some embodiments, the antigen binding domain includes a heavy chain variable region and a light chain variable region including the amino acid sequences set forth as SEQ ID NOs: 1 and 2, respectively; SEQ ID NOs: 3 and 4, respectively; SEQ ID NOs: 5 and 6, respectively; SEQ ID NOs: 7 and 8, respectively, SEQ ID NOs: 9 and 10, respectively; SEQ ID NOs: 11 and 12, respectively; SEQ ID NOs: 13 and 14, respectively; or SEQ ID NOs: 15 and 16, respectively.

In several embodiments, the antigen binding domain can be a scFv. In some embodiments, the scFv includes a heavy chain variable region and a light chain variable region joined by a peptide linker, such as a linker including the amino acid sequence set forth as SEQ ID NO: 25. In some such embodiments, the antigen binding domain comprises an amino acid sequence set forth as one of SEQ ID NOs: 17-24.

The CAR can include a signal peptide sequence, e.g., N-terminal to the antigen binding domain. The signal peptide sequence may comprise any suitable signal peptide sequence. In an embodiment, the signal peptide sequence is a human granulocyte-macrophage colony-stimulating factor (GM-CSF) receptor sequence, such as an amino acid sequence including or consisting of SEQ ID NO: 26. While the signal peptide sequence may facilitate expression of the CAR on the surface of the cell, the presence of the signal peptide sequence in an expressed CAR is not necessary in order for the CAR to function. Upon expression of the CAR on the cell surface, the signal peptide sequence may be cleaved off of the CAR. Accordingly, in some embodiments, the CAR lacks a signal peptide sequence.

Between the antigen binding domain and the transmembrane domain of the CAR, there may be a spacer domain, which includes a polypeptide sequence. The spacer domain may comprise up to 300 amino acids, preferably 10 to 100 amino acids and most preferably 25 to 50 amino acids. In some embodiments, the spacer domain can include an immunoglobulin domain, such as a human immunoglobulin sequence. In an embodiment, the immunoglobulin domain comprises an immunoglobulin CH2 and CH3 immunoglobulin G (IgGl) domain sequence (CH2CH3). In this regard, the spacer domain can include an immunoglobulin domain comprising or consisting of the amino acid sequence set forth as SEQ ID NO: 35. Without being bound to a particular theory, it is believed that the CH2CH3 domain extends the antigen binding domain of the CAR away from the membrane of CAR-expressing cells and may more accurately mimic the size and domain structure of a native TCR.

### 2. Transmembrane Domain

With respect to the transmembrane domain, the CAR can be designed to comprise a transmembrane domain that is fused to the extracellular domain of the CAR. In one embodiment, the transmembrane domain that naturally is associated with one of the domains in the CAR is used.

The transmembrane domain may be derived either from a natural or from a synthetic source. Where the source is natural, the domain may be derived from any membrane-bound or transmembrane protein. Exemplary transmembrane domains for use in the disclosed CARs can include at least the transmembrane region(s) of) the alpha, beta or zeta chain of the T-cell receptor, CD28, CD3 epsilon, CD45, CD4, CD5, CDS, CD9, CD 16, CD22, CD33, CD37, CD64, CD80, CD86, CD 134, CD137, CD 154. Alternatively the transmembrane domain may be synthetic, in which case it will comprise predominantly hydrophobic residues such as leucine and valine. In several embodiments, a triplet of phenylalanine, tryptophan and valine will be found at each end of a synthetic transmembrane domain.

Optionally, a short oligo- or polypeptide linker, preferably between 2 and 10 amino acids in length may form the linkage between the transmembrane domain and the intracellular T cell signaling domain and/or T cell costimulatory domain of the CAR. An exemplary linker sequence includes one or more glycine-serine doublets.

In some embodiments, the transmembrane domain comprises the transmembrane domain of a T cell receptor, such as a CD8 transmembrane domain. Thus, the CAR can include a CD8 transmembrane domain including or consisting of SEQ ID NO: 30. In another embodiment, the transmembrane domain comprises the transmembrane domain of a T cell costimulatory molecule, such as CD137 or CD28. Thus, the CAR can include a CD28 transmembrane domain including or consisting of SEQ ID NO: 27.

### 3. Intracellular Region

The intracellular region of the CAR includes one or more intracellular T cell signaling domains responsible for activation of at least one of the normal effector functions of a T cell in which the CAR is expressed or placed in. Exemplary T cell signaling domains are provided herein, and are known to the person of ordinary skill in the art.

While an entire intracellular T cell signaling domain can be employed in a CAR, in many cases it is not necessary to use the entire chain. To the extent that a truncated portion of the intracellular T cell signaling domain is used, such truncated portion may be used in place of the intact chain as long as it transduces the relevant T cell effector function signal.

Examples of intracellular T cell signaling domains for use in the CAR include the cytoplasmic sequences of the T cell receptor (TCR) and co-stimulatory molecules that act in concert to initiate signal transduction following antigen receptor engagement, as well as any derivative or variant of these sequences and any synthetic sequence that has the same functional capability.

T cell receptor signaling domains regulate primary activation of the T cell receptor complex either in a stimulatory way, or in an inhibitory way. The disclosed CARs can include primary cytoplasmic signaling sequences that act in a stimulatory manner, which may contain signaling motifs that are known as immunoreceptor tyrosine-based activation motifs or ITAMs. Examples of IT AM containing primary cytoplasmic signaling sequences that can be included in a disclosed CAR include those from CD3 zeta, FcR gamma, FcR beta, CD3 gamma, CD3 delta, CD3 epsilon, CDS, CD22, CD79a, CD79b, and CD66d proteins. It is particularly preferred that cytoplasmic signaling molecule in the CAR include an intracellular T cell signaling domain from CD3 zeta.

The intracellular region of the CAR can include the IT AM containing primary cytoplasmic signaling domain (such as CD3-zeta) by itself or combined with any other desired cytoplasmic domain(s) useful in the context of a CAR. For example, the cytoplasmic domain of the CAR can include a CD3 zeta chain portion and an intracellular costimulatory signaling domain. The costimulatory signaling domain refers to a portion of the CAR comprising the intracellular domain of a costimulatory molecule. A costimulatory molecule is a cell surface molecule other than an antigen receptor or their ligands that is required for an efficient response of lymphocytes to an antigen. Examples of such molecules include CD27, CD28, 4-1BB (CD137), OX40 (CD134), CD30, CD40, PD-1, ICOS, lymphocyte function-associated antigen 1 (LFA-1), CD2, CD7, LIGHT, NKG2C, and B7-H3. An additional example of a signaling domain that can be included in a disclosed CARs is a Tumor necrosis factor receptor superfamily member 18 (TNFRSF18; also known as glucocorticoid-induced TNFR-related protein, GITR) signaling domain.

In some embodiments, the CAR can include a CD3 zeta signaling domain, a CD8 signaling domain, a CD28 signaling domain, a CD137 signaling domain or a combination of two or more thereof. In one embodiment, the cytoplasmic domain includes the signaling domain of CD3-zeta and the signaling domain of CD28. In another embodiment, the cytoplasmic domain includes the signaling domain of CD3 zeta and the signaling domain of CD137. In yet another embodiment, the cytoplasmic domain includes the signaling domain of CD3-zeta and the signaling domain of CD28 and CD137. The order of the one or more T cell signaling domains on the CAR can be varied as needed by the person of ordinary skill in the art.

Exemplary amino acid sequences for such T cell signaling domains are provided. For example, the CD3 zeta signaling domain can include or consist of the amino acid sequence set forth as SEQ ID NO: 34, the CD8 signaling domain can include or consist of the amino acid sequence set forth as SEQ ID NO: 31, the CD28 signaling domain can include or consist of the amino acid sequence set forth as SEQ ID NO: 28, the CD137 signaling domain can include or consist of the amino acid sequences set forth as SEQ ID NO: 32 or SEQ ID NO: 33.

The cytoplasmic signaling sequences within the cytoplasmic signaling portion of the CAR of the invention may be linked to each other in a random or specified order. Optionally, a short polypeptide linker, preferably between 2 and 10 amino acids in length may form the linkage. A glycine-serine doublet provides a particularly suitable linker. Further, between the signaling domain and the transmembrane domain of the CAR, there may be a spacer domain, which includes a polypeptide sequence. The spacer domain may comprise up to 300 amino acids, preferably 10 to 100 amino acids and most preferably 25 to 50 amino acids.

### 4. Additional Description of CARs

Also provided are functional portions of the CARs described herein. The term "functional portion" when used in reference to a CAR refers to any part or fragment of the CAR, which part or fragment retains the biological activity of the CAR of which it is a part (the parent CAR). Functional portions encompass, for example, those parts of a CAR that retain the ability to recognize target cells, or detect, treat, or prevent a disease, to a similar extent, the same extent, or to a higher extent, as the parent CAR. In reference to the parent CAR, the functional portion can comprise, for instance, about 10%, 25%, 30%, 50%, 68%, 80%, 90%, 95%, or more, of the parent CAR.

The CAR or functional portion thereof, can include additional amino acids at the amino or carboxy terminus, or at both termini, which additional amino acids are not found in the amino acid sequence of the parent CAR. Desirably, the additional amino acids do not interfere with the biological function of the CAR or functional portion, e.g., recognize target cells, detect cancer, treat or prevent cancer, etc. More desirably, the additional amino acids enhance the biological activity, as compared to the biological activity of the parent CAR.

Also provided are functional variants of the CARs described herein, which have substantial or significant sequence identity or similarity to a parent CAR, which functional variant retains the biological activity of the CAR of which it is a variant. Functional variants encompass, for example, those variants of the CAR described herein (the parent CAR) that retain the ability to recognize target cells to a similar extent, the same extent, or to a higher extent, as the parent CAR. In reference to the parent CAR, the functional variant can, for instance, be at least about 30%, about 50%, about 75%, about 80%, about 85%, about 90%, about 91 %, about 92%, about 93%, about 94%, about 95%, about 96%), about 97%, about 98%, about 99% or more identical in amino acid sequence to the parent CAR.

A functional variant can, for example, comprise the amino acid sequence of the parent CAR with at least one conservative amino acid substitution. Alternatively or additionally, the functional variants can comprise the amino acid sequence of the parent CAR with at least one non-conservative amino acid substitution. In this case, it is preferable for the non-conservative amino acid substitution to not interfere with or inhibit the biological activity of the functional variant. The non-conservative amino acid substitution may enhance the biological activity of the functional variant, such that the biological activity of the functional variant is increased as compared to the parent CAR.

The CARs (including functional portions and functional variants) can be of any length, i.e., can comprise any number of amino acids, provided that the CARs (or functional portions or functional variants thereof) retain their biological activity, e.g., the ability to specifically bind to antigen, detect diseased cells in a mammal, or treat or prevent disease in a mammal, etc. For example, the CAR can be about 50 to about 5000 amino acids long, such as 50, 70, 75, 100, 125, 150, 175, 200, 300, 400, 500, 600, 700, 800, 900, 1000 or more amino acids in length.

The CARs (including functional portions and functional variants of the invention) can comprise synthetic amino acids in place of one or more naturally-occurring amino acids. Such synthetic amino acids are known in the art, and include, for example, aminocyclohexane carboxylic acid, norleucine, a-amino n-decanoic acid, homoserine, S-acetylaminomethyl-cysteine, trans-3- and trans-4-hydroxyproline, 4- aminophenylalanine, 4- nitrophenylalanine, 4-chlorophenylalanine, 4-carboxyphenylalanine, β-phenylserine β-hydroxyphenylalanine, phenylglycine, α-naphthylalanine, cyclohexylalanine, cyclohexylglycine, indoline-2-carboxylic acid, 1 ,2,3,4- tetrahydroisoquinoline-3-carboxylic acid, aminomalonic acid, aminomalonic acid monoamide, N'-benzyl-N'-methyl-lysine, N',N'-dibenzyl-lysine, 6-hydroxylysine, ornithine, α-aminocyclopentane carboxylic acid, α-aminocyclohexane carboxylic acid, oc- aminocycloheptane carboxylic acid, -(2-amino-2-norbornane)-carboxylic acid, γ-diaminobutyric acid, α,β-diaminopropionic acid, homophenylalanine, and α-tert-butylglycine.

The CARs (including functional portions and functional variants) can be glycosylated, amidated, carboxylated, phosphorylated, esterified, N-acylated, cyclized via, e.g., a disulfide bridge, or converted into an acid addition salt and/or optionally dimerized or polymerized, or conjugated.

Methods of generating chimeric antigen receptors, T cells including such receptors, and their use (e.g., for treatment of cancer) are known in the art and further described herein (see, e.g., Brentjens et al., 2010, Molecular Therapy, 18:4, 666-668; Morgan et al., 2010, Molecular Therapy, published online February 23, 2010, pages 1 -9; Till et al., 2008, Blood, 1 12:2261 -2271; Park et al., Trends Biotechnol., 29:550-557, 2011; Grupp et al., N Engl J Med., 368:1509-1518, 2013; Han et al., J. Hematol Oncol., 6:47, 2013; Tumaini et al., Cytotherapy, 15, 1406-1417, 2013; Haso et al., (2013) Blood, 121, 1165-1174; PCT Pubs. WO2012/079000, WO2013/126726; and U.S. Pub. 2012/0213783. For example, a nucleic acid molecule encoding a disclosed chimeric antigen binding receptor can be included in an expression vector (such as a lentiviral vector) used to transduce a host cell, such as a T cell, to make the disclosed CAR. In some embodiments, methods of using the chimeric antigen receptor include isolating T cells from a subject, transducing the T cells with an expression vector (such as a lentiviral vector) encoding the chimeric antigen receptor, and administering the CAR-expressing T cells to the subject for treatment, for example for treatment of a tumor in the subject.

### B. Antibodies and Antigen Binding Fragments

Isolated monoclonal antibodies that specifically bind to ALK on the cell surface, and antigen binding fragments thereof are provided. The antibodies can be fully human and/or neutralizing.

In several embodiments, the monoclonal antibodies include a heavy chain comprising a heavy chain complementarity determining region (HCDR)1, a HCDR2 and an HCDR3, and a light chain comprising a light chain complementarity determining region (LCDR) 1, LCDR2 and LCDR3. The disclosed antibodies specifically bind to an epitope of ALK and are neutralizing. In some embodiments, the ALK specific antibodies include a variable heavy (V_{H}) and a variable light (V_{L}) chain and specifically bind ALK. In several embodiments, the antibody or antigen binding fragment thereof includes heavy and light chain variable regions including the HCDR1, HCDR2, and HCDR3, and LCDR1, LCDR2, and LCDR3, respectively, of one of the ALK15, ALK48, ALK53, or ALK58 antibodies.

The discussion of monoclonal antibodies below refers to isolated monoclonal antibodies that include heavy and light chain variable domains including at least one complementarity determining region (CDR), such as a CDR1, CDR2 and CDR3. The person of ordinary skill in the art will understand that various CDR numbering schemes (such as the Kabat, Chothia or IMGT numbering schemes) can be used to determine CDR positions. The amino acid sequence and the CDR positions of the heavy and light chain of the ALK15, ALK48, ALK53, or ALK58 monoclonal antibodies according to the IMGT and Kabat numbering schemes are shown in Table 1 (IMGT) and Table 2 (Kabat). The person of skill in the art will readily understand use of various CDR numbering schemes when referencing particular amino acids of the antibodies disclosed herein.

**Table 1. IMGT CDR sequences of ALK specific antibodies**

| **ALK15** | | | | | |
|---|---|---|---|---|---|
| | SEQ ID NO: 1 | A.A. Sequence | | SEQ ID NO: 2 | A.A. Sequence |
| HCDR1 | 26-33 | GFSLTSYA | LCDR1 | 27-37 | QSIVHSYGNTY |
| HCDR2 | 51-57 | IWSGGAT | LCDR2 | 55-57 | RVS |
| HCDR3 | 95-109 | | LCDR3 | 93-103 | CFQGTHVPYTF |

| **ALK48** | | | | | |
|---|---|---|---|---|---|
| | SEQ ID NO: 3 | A.A. Sequence | | SEQ ID NO: 4 | A.A. Sequence |
| HCDR1 | 26-33 | GYAFSSYW | LCDR1 | 27-36 | ESVDNYGISF |
| HCDR2 | 51-58 | IYPGDGDT | LCDR2 | 54-56 | RAS |
| HCDR3 | 96-110 | | LCDR3 | 92-102 | CQQNNKDPPTF |

| **ALK53** | | | | | |
|---|---|---|---|---|---|
| | SEQ ID NO: 5 | A.A. Sequence | | SEQ ID NO: 6 | A.A. Sequence |
| HCDR1 | 26-33 | GYTFTDHF | LCDR1 | 27-37 | KSLLHSNGNTY |
| HCDR2 | 51-58 | LNPYSGGT | LCDR2 | 55-57 | YMS |
| HCDR3 | 96-108 | CARHNWGAYFDYW | LCDR3 | 93-103 | CMQGLEDPYTF |

| **ALK58** | | | | | |
|---|---|---|---|---|---|
| | SEQ ID NO: 7 | A.A. Sequence | | SEQ ID NO: 8 | A.A. Sequence |
| HCDR1 | 26-33 | GYTFTDYE | LCDR1 | 27-32 | QDIGNY |
| HCDR2 | 51-58 | IDPETGGT | LCDR2 | 50-52 | YTS |
| HCDR3 | 96-110 | | LCDR3 | 88-98 | CQQGSALPPTF |

In some embodiments, the antibody includes IMGT CDRs, such as those listed in Table 1. For example, in some embodiments, the antibody includes a heavy chain variable region including a HCDR1, HCDR2, and/or HCDR3 including amino acids amino acids 26-33, 51-57, and 95-109 of SEQ ID NO: 1, respectively. In further embodiments, the antibody includes a heavy chain variable region including a HCDR1, HCDR2, and/or HCDR3 including amino acids amino acids 26-33, 51-58, and 96-110 of SEQ ID NO: 3, respectively. In additional embodiments, the antibody includes a heavy chain variable region including a HCDR1, HCDR2, and/or HCDR3 including amino acids 26-33, 51-58, and 96-108 of SEQ ID NO: 5, respectively. In more embodiments, the antibody includes a heavy chain variable region including a HCDR1, HCDR2, and/or HCDR3 including amino acids 26-33, 51-58, and 96-110 of SEQ ID NO: 7, respectively.

In some embodiments, the antibody includes a light chain variable region including a LCDR1, LCDR2, and/or LCDR3 including amino acids 27-37, 55-57, and 93-103 of SEQ ID NO: 2, respectively. In further embodiments, the antibody includes a light chain variable region including a LCDR1, LCDR2, and/or LCDR3 including amino acids 27-36, 54-56, and 92-102 of SEQ ID NO: 4, respectively. In additional embodiments, the antibody includes a light chain variable region including a LCDR1, LCDR2, and/or LCDR3 including amino acids 27-37, 55-57, and 93-103 of SEQ ID NO: 6, respectively. In more embodiments, the antibody includes a light chain variable region including a LCDR1, LCDR2, and/or LCDR3 including amino acids 27-32, 50-52, and 88-98 of SEQ ID NO: 8, respectively.

In some embodiments, the antibody includes a heavy chain variable region including a HCDR1, HCDR2, and HCDR3 including amino acids 26-33, 51-57, and 95-109 of SEQ ID NO: 1, respectively, and a light chain variable region including a LCDR1, LCDR2, and LCDR3 including amino acids 27-37, 55-57, and 93-103 of SEQ ID NO: 2, respectively. In further embodiments, the antibody includes a heavy chain variable region including a HCDR1, HCDR2, and HCDR3 including amino acids 26-33, 51-58, and 96-110 of SEQ ID NO: 3, respectively, and a light chain variable region including a LCDR1, LCDR2, and LCDR3 including amino acids 27-36, 54-56, and 92-102 of SEQ ID NO: 4, respectively. In additional embodiments, the antibody includes a heavy chain variable region including a HCDR1, HCDR2, and HCDR3 including amino acids 26-33, 51-58, and 96-108 of SEQ ID NO: 5, respectively, and a light chain variable region including a LCDR1, LCDR2, and LCDR3 including amino acids 27-37, 55-57, and 93-103 of SEQ ID NO: 6, respectively. In more embodiments, the antibody includes a heavy chain variable region including a HCDR1, HCDR2, and HCDR3 including amino acids 26-33, 51-58, and 96-110 of SEQ ID NO: 7, respectively, and a light chain variable region including a LCDR1, LCDR2, and LCDR3 including amino acids 27-32, 50-52, and 88-98 of SEQ ID NO: 8, respectively.

**Table 2. Kabat CDR sequences of ALK specific antibodies**

| **ALK15** | | | | | |
|---|---|---|---|---|---|
| | SEQ ID NO: 1 | A.A. Sequence | | SEQ ID NO: 2 | A.A. Sequence |
| HCDR1 | 31-35 | SYAVS | LCDR1 | 24-39 | |
| HCDR2 | 50-65 | IIWSGGATNYNSALKS | LCDR2 | 55-61 | RVSNRFS |
| HCDR3 | 98-108 | EHYYGSSAMDY | LCDR3 | 94-102 | FQGTHVPYT |

| **ALK48** | | | | | |
|---|---|---|---|---|---|
| | SEQ ID NO: 3 | A.A. Sequence | | SEQ ID NO: 4 | A.A. Sequence |
| HCDR1 | 31-35 | SYWMN | LCDR1 | 24-38 | |
| HCDR2 | 50-66 | | LCDR2 | 54-60 | RASNLES |
| HCDR3 | 99-109 | YYYGSSGYFDY | LCDR3 | 93-101 | QQNNKDPPT |

| **ALK53** | | | | | |
|---|---|---|---|---|---|
| | SEQ ID NO: 5 | A.A. Sequence | | SEQ ID NO: 6 | A.A. Sequence |
| HCDR1 | 31-35 | DHFMD | LCDR1 | 24-39 | |
| HCDR2 | 50-66 | | LCDR2 | 55-61 | YMSNLAS |
| HCDR3 | 99-107 | HNWGAYFDY | LCDR3 | 94-102 | MQGLEDPYT |

| **ALK58** | | | | | |
|---|---|---|---|---|---|
| | SEQ ID NO: 7 | A.A. Sequence | | SEQ ID NO: 8 | A.A. Sequence |
| HCDR1 | 31-35 | DYEMH | LCDR1 | 24-34 | RASQDIGNYLN |
| HCDR2 | 50-66 | | LCDR2 | 50-56 | YTSRLHS |
| HCDR3 | 99-109 | RRYYGSSSFDY | LCDR3 | 89-97 | QQGSALPPT |

In some embodiments, the antibody includes Kabat CDRs, such as those listed in Table 2. In some embodiments, the antibody includes a heavy chain variable region including a HCDR1, HCDR2, and/or HCDR3 including amino acids 31-35, 50-65, and 98-108 of SEQ ID NO: 1, respectively. In further embodiments, the antibody includes a heavy chain variable region including a HCDR1, HCDR2, and/or HCDR3 including amino acids 31-35, 50-66, and 99-109 of SEQ ID NO: 3, respectively. In additional embodiments, the antibody includes a heavy chain variable region including a HCDR1, HCDR2, and/or HCDR3 including amino acids 31-35, 50-66, and 99-107 of SEQ ID NO: 5, respectively. In more embodiments, the antibody includes a heavy chain variable region including a HCDR1, HCDR2, and/or HCDR3 including amino acids 31-35, 50-66, and 99-109 of SEQ ID NO: 7, respectively.

In some embodiments, the antibody includes a light chain variable region including a LCDR1, LCDR2, and/or LCDR3 including amino acids 24-39, 55-61, and 94-102 of SEQ ID NO: 2, respectively. In further embodiments, the antibody includes a light chain variable region including a LCDR1, LCDR2, and/or LCDR3 including amino acids 24-38, 55-60, and 93-101 of SEQ ID NO: 4, respectively. In additional embodiments, the antibody includes a light chain variable region including a LCDR1, LCDR2, and/or LCDR3 including amino acids 24-39, 55-61, and 94-102 of SEQ ID NO: 6, respectively. In more embodiments, the antibody includes a light chain variable region including a LCDR1, LCDR2, and/or LCDR3 including amino acids 24-34, 50-56, and 89-97 of SEQ ID NO: 8, respectively.

In some embodiments, the antibody includes a heavy chain variable region including a HCDR1, HCDR2, and HCDR3 including amino acids 31-35, 50-65, and 98-108 of SEQ ID NO: 1, respectively, and a light chain variable region including a LCDR1, LCDR2, and LCDR3 including amino acids 24-39, 55-61, and 94-102 of SEQ ID NO: 2, respectively. In further embodiments, the antibody includes a heavy chain variable region including a HCDR1, HCDR2, and HCDR3 including amino acids 31-35, 50-66, and 99-109 of SEQ ID NO: 3, respectively, and a light chain variable region including a LCDR1, LCDR2, and LCDR3 including amino acids 24-38, 55-60, and 93-101 of SEQ ID NO: 4, respectively. In additional embodiments, the antibody includes a heavy chain variable region including a HCDR1, HCDR2, and HCDR3 including amino acids 31-35, 50-66, and 99-107 of SEQ ID NO: 5, respectively, and a light chain variable region including a LCDR1, LCDR2, and LCDR3 including amino acids 24-39, 55-61, and 94-102 of SEQ ID NO: 6, respectively. In more embodiments, the antibody includes a heavy chain variable region including a HCDR1, HCDR2, and HCDR3 including amino acids 31-35, 50-66, and 99-109 of SEQ ID NO: 7, respectively, and a light chain variable region including a LCDR1, LCDR2, and LCDR3 including amino acids 24-34, 50-56, and 89-97 of SEQ ID NO: 8, respectively.

In some embodiments, the antibody includes a heavy chain variable region including an amino acid sequence at least 95%, 96%, 97%, 98%, or 99% identical to the amino acid sequence set forth as one of SEQ ID NO: 1, 3, 5, 7, 9, 11, 13, or 15. In more embodiments, the antibody includes a light chain variable region including an amino acid sequence at least 95%, 96%, 97%, 98%, or 99% identical to the amino acid sequence set forth as one of SEQ ID NO: 2, 4, 6, 8, 10, 12, 14, or 16. In additional embodiments, the antibody includes a heavy chain variable region including the amino acid sequence set forth as one of SEQ ID NO: 1, 3, 5, 7, 9, 11, 13, or 15. In more embodiments, the antibody includes a light chain variable region including the amino acid sequence set forth as one of SEQ ID NO: 2, 4, 6, 8, 10, 12, 14, or 16.

In additional embodiments, the antibody includes a heavy chain variable region including an amino acid sequence at least 95%, 96%, 97%, 98%, or 99% identical to the amino acid sequence set forth as SEQ ID NO: 1, and a light chain variable region including an amino acid sequence at least 95%, 96%, 97%, 98%, or 99% identical to the amino acid sequence set forth as SEQ ID NO: 2. In additional embodiments, the antibody includes a heavy chain variable region including an amino acid sequence at least 95%, 96%, 97%, 98%, or 99% identical to the amino acid sequence set forth as SEQ ID NO: 3, and a light chain variable region including an amino acid sequence at least 95%, 96%, 97%, 98%, or 99% identical to the amino acid sequence set forth as SEQ ID NO: 4. In additional embodiments, the antibody includes a heavy chain variable region including an amino acid sequence at least 95%, 96%, 97%, 98%, or 99% identical to the amino acid sequence set forth as SEQ ID NO: 5, and a light chain variable region including an amino acid sequence at least 95%, 96%, 97%, 98%, or 99% identical to the amino acid sequence set forth as SEQ ID NO: 6. In additional embodiments, the antibody includes a heavy chain variable region including an amino acid sequence at least 95%, 96%, 97%, 98%, or 99% identical to the amino acid sequence set forth as SEQ ID NO: 7, and a light chain variable region including an amino acid sequence at least 95%, 96%, 97%, 98%, or 99% identical to the amino acid sequence set forth as SEQ ID NO: 8.

In additional embodiments, the antibody includes a heavy chain variable region including an amino acid sequence at least 95%, 96%, 97%, 98%, or 99% identical to the amino acid sequence set forth as SEQ ID NO: 9, and a light chain variable region including an amino acid sequence at least 95%, 96%, 97%, 98%, or 99% identical to the amino acid sequence set forth as SEQ ID NO: 10. In additional embodiments, the antibody includes a heavy chain variable region including an amino acid sequence at least 95%, 96%, 97%, 98%, or 99% identical to the amino acid sequence set forth as SEQ ID NO: 11, and a light chain variable region including an amino acid sequence at least 95%, 96%, 97%, 98%, or 99% identical to the amino acid sequence set forth as SEQ ID NO: 12. In additional embodiments, the antibody includes a heavy chain variable region including an amino acid sequence at least 95%, 96%, 97%, 98%, or 99% identical to the amino acid sequence set forth as SEQ ID NO: 13, and a light chain variable region including an amino acid sequence at least 95%, 96%, 97%, 98%, or 99% identical to the amino acid sequence set forth as SEQ ID NO: 14. In additional embodiments, the antibody includes a heavy chain variable region including an amino acid sequence at least 95%, 96%, 97%, 98%, or 99% identical to the amino acid sequence set forth as SEQ ID NO: 15, and a light chain variable region including an amino acid sequence at least 95%, 96%, 97%, 98%, or 99% identical to the amino acid sequence set forth as SEQ ID NO: 16.

In some embodiments, the antibody includes a heavy chain variable region and a light chain variable region including the amino acid sequences set forth as SEQ ID NOs: 1 and 2, respectfully. In some embodiments, the antibody includes a heavy chain variable region and a light chain variable region including the amino acid sequences set forth as SEQ ID NOs: 3 and 4, respectfully. In some embodiments, the antibody includes a heavy chain variable region and a light chain variable region including the amino acid sequences set forth as SEQ ID NOs: 5 and 6, respectfully. In some embodiments, the antibody includes a heavy chain variable region and a light chain variable region including the amino acid sequences set forth as SEQ ID NOs: 7 and 8, respectfully. In some embodiments, the antibody includes a heavy chain variable region and a light chain variable region including the amino acid sequences set forth as SEQ ID NOs: 9 and 10, respectfully. In some embodiments, the antibody includes a heavy chain variable region and a light chain variable region including the amino acid sequences set forth as SEQ ID NOs: 11 and 12, respectfully. In some embodiments, the antibody includes a heavy chain variable region and a light chain variable region including the amino acid sequences set forth as SEQ ID NOs: 13 and 14, respectfully. In some embodiments, the antibody includes a heavy chain variable region and a light chain variable region including the amino acid sequences set forth as SEQ ID NOs: 15 and 16, respectfully.

In several embodiments, the antibody can specifically bind ALK with an affinity of at least about 1.0 x 10⁻⁸ M, at least about 5.0 x 10⁻⁸ M, at least about 1.0 x 10⁻⁹ M, at least about 5.0 x 10⁻⁹ M, at least about 1.0 x 10⁻¹⁰ M, at least about 5.0 x 10⁻¹⁰ M, or at least about 1.0 x 10⁻¹¹ M.

The monoclonal antibodies can be human monoclonal antibodies. Chimeric antibodies are also provided. The antibodies can include any suitable framework region, such as (but not limited to) a human framework region. Human framework regions, and mutations that can be made in a human antibody framework regions, are known in the art (see, for example, in U.S. Patent No. 5,585,089. Alternatively, a heterologous framework region, such as, but not limited to a mouse framework region, can be included in the heavy or light chain of the antibodies. (See, for example, Jones et al., Nature 321:522, 1986; Riechmann et al., Nature 332:323, 1988; Verhoeyen et al., Science 239:1534, 1988; Carter et al., Proc. Natl. Acad. Sci. U.S.A. 89:4285, 1992; Sandhu, Crit. Rev. Biotech. 12:437, 1992; and Singer et al., J. Immunol. 150:2844, 1993.)

In some embodiments, an antibody that specifically binds ALK as disclosed herein includes up to 10 amino acid substitutions (such as up to 1, 2, 3, 4, 5, 6, 7, 8, or up to 9 amino acid substitutions) in the framework regions of the heavy chain of the antibody, or the light chain of the antibody, or the heavy and light chains of the antibody.

The antibodies or antigen binding fragments disclosed herein can be derivatized or linked to another molecule (such as another peptide or protein). In general, the antibodies or portion thereof is derivatized such that the binding to ALK is not affected adversely by the derivatization or labeling. For example, the antibody can be functionally linked (by chemical coupling, genetic fusion, noncovalent association or otherwise) to one or more other molecular entities, such as another antibody (for example, a bi-specific antibody or a diabody), a detectable marker, an effector molecule, or a protein or peptide that can mediate association of the antibody or antibody portion with another molecule (such as a streptavidin core region or a polyhistidine tag).

One type of derivatized antibody is produced by crosslinking two or more antibodies (of the same type or of different types, such as to create bispecific antibodies). Suitable crosslinkers include those that are heterobifunctional, having two distinctly reactive groups separated by an appropriate spacer (such as m-maleimidobenzoyl-N-hydroxysuccinimide ester) or homobifunctional (such as disuccinimidyl suberate). Such linkers are available from Pierce Chemical Company, Rockford, I11.

The monoclonal antibodies disclosed herein can be of any isotype. The monoclonal antibody can be, for example, an IgM or an IgG antibody, such as IgG₁, IgG₂, IgG₃ or an IgG₄. However, in other embodiments, the disclosed monoclonal antibodies are not an IgG. The class of an antibody that specifically binds ALK can be switched with another (for example, IgG can be switched to IgM), according to well-known procedures. For example, a nucleic acid molecule encoding the V_{L} or V_{H} of a disclosed antibody can be operatively linked to a nucleic acid sequence encoding a C_{L} or C_{H} from a different class of immunoglobulin molecule. This can be achieved using a vector or nucleic acid molecule that comprises a C_{L} or C_{H} chain, as known in the art. For example, an antibody that specifically binds ALK, that was originally IgG may be class switched to an IgM. Class switching can be used to convert one IgG subclass to another, such as from IgG₁ to IgG_{2,} IgG_{3,} or IgG₄.

In some examples, the disclosed antibodies are oligomers of antibodies, such as dimers, trimers, tetramers, pentamers, hexamers, septamers, octomers and so on. In some examples, the antibodies are pentamers.

In several embodiments, the constant region of the antibody includes one or more amino acid substitutions to optimize *in vivo* half-life of the antibody. The serum half-life of IgG Abs is regulated by the neonatal Fc receptor (FcRn). Thus, in several embodiments, the antibody includes an amino acid substitution that increases binding to the FcRn. Several such substitutions are known to the person of ordinary skill in the art, such as substitutions at IgG constant regions T250Q and M428L (see, e.g., Hinton et al., J Immunol., 176:346-356, 2006); M428L and N434S (see, e.g., Zalevsky, et al., Nature Biotechnology, 28:157-159, 2010); N434A (see, e.g., Petkova et al., Int. Immunol., 18:1759-1769, 2006); T307A, E380A, and N434A (see, e.g., Petkova et al., Int. Immunol., 18:1759-1769, 2006); and M252Y, S254T, and T256E (see, e.g., Dall'Acqua et al., J. Biol. Chem., 281:23514-23524, 2006).

In some embodiments, the constant region of the antibody includes one of more amino acid substitutions to optimize Antibody-dependent cell-mediated cytotoxicity (ADCC). ADCC is mediated primarily through a set of closely related Fcy receptors. In some embodiments, the antibody includes one or more amino acid substitutions that increase binding to FcγRIIIa. Several such substitutions are known to the person of ordinary skill in the art, such as substitutions at IgG constant regions S239D and I332E (see, e.g., Lazar et al., Proc. Natl., Acad. Sci. U.S.A., 103:4005-4010, 2006); and S239D, A330L, and I332E (see, e.g., Lazar et al., Proc. Natl., Acad. Sci. U.S.A., 103:4005-4010, 2006).

Combinations of the above substitutions are also included, to generate an IgG constant region with increased binding to FcRn and FcyRIIIa. The combinations increase antibody half-life and ADCC.

Antigen binding fragments of the antibodies that specifically bind to ALK are also encompassed by the present disclosure, such as single-domain antibodies (for example, VH domain antibodies), Fab, F(ab')₂, and Fv. These antigen binding fragments retain the ability to specifically bind ALK. These fragments include:
(1) Fab, the fragment which contains a monovalent antigen-binding fragment of an antibody molecule, can be produced by digestion of whole antibody with the enzyme papain to yield an intact light chain and a portion of one heavy chain;
(2) Fab', the fragment of an antibody molecule can be obtained by treating whole antibody with pepsin, followed by reduction, to yield an intact light chain and a portion of the heavy chain; two Fab' fragments are obtained per antibody molecule;
(3) (Fab')₂, the fragment of the antibody that can be obtained by treating whole antibody with the enzyme pepsin without subsequent reduction; F(ab')₂ is a dimer of two Fab' fragments held together by two disulfide bonds;
(4) Fv, a genetically engineered fragment containing the variable region of the light chain and the variable region of the heavy chain expressed as two chains;
(5) Single chain antibody (such as scFv), a genetically engineered molecule containing the variable region of the light chain, the variable region of the heavy chain, linked by a suitable polypeptide linker as a genetically fused single chain molecule;
(6) A dimer of a single chain antibody (scFV₂), defined as a dimer of a scFV (also known as a "mini-antibody"); and
(7) VH single-domain antibody, an antigen binding fragment consisting of the heavy chain variable domain.

Methods of making these fragments are known in the art (see for example, Harlow and Lane, Antibodies: A Laboratory Manual, Cold Spring Harbor Laboratory, New York, 1988).

In some embodiments, the antigen binding fragments are Fv antibodies, which are typically about 25 kDa and contain a complete antigen-binding site with three CDRs per each heavy chain and each light chain. To produce these antibodies, the V_{H} and the V_{L} can be expressed from two individual nucleic acid constructs in a host cell. If the V_{H} and the V_{L} are expressed non-contiguously, the chains of the Fv antibody are typically held together by noncovalent interactions. However, these chains tend to dissociate upon dilution, so methods have been developed to crosslink the chains through glutaraldehyde, intermolecular disulfides, or a peptide linker. Thus, in one example, the Fv can be a disulfide stabilized Fv (dsFv), wherein the heavy chain variable region and the light chain variable region are chemically linked by disulfide bonds.

In an additional example, the Fv fragments include V_{H} and V_{L} chains connected by a peptide linker. These single-chain antigen binding proteins (scFv) are prepared by constructing a structural gene including DNA sequences encoding the V_{H} and V_{L} domains connected by an oligonucleotide. The structural gene is inserted into an expression vector, which is subsequently introduced into a host cell such as *E. coli.* The recombinant host cells synthesize a single polypeptide chain with a linker peptide bridging the two V domains. Methods for producing scFvs are known in the art (see Whitlow et al., Methods: a Companion to Methods in Enzymology, Vol. 2, page 97, 1991; Bird et al., Science 242:423, 1988; U.S. Patent No. 4,946,778; Pack et al., Bio/Technology 11:1271, 1993; and Sandhu, *supra).* Dimers of a single chain antibody (scFV₂), are also contemplated.

Antigen binding fragments can be prepared by proteolytic hydrolysis of the antibody or by expression in *E. coli* of DNA encoding the fragment. Antigen binding fragments can be obtained by pepsin or papain digestion of whole antibodies by conventional methods. For example, antigen binding fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly (see U.S. Patent No. 4,036,945 and U.S. Patent No. 4,331,647, and references contained therein; Nisonhoff et al., Arch. Biochem. Biophys. 89:230, 1960; Porter, Biochem. J. 73:119, 1959; Edelman et al., Methods in Enzymology, Vol. 1, page 422, Academic Press, 1967; and Coligan *et al.* at sections 2.8.1-2.8.10 and 2.10.1-2.10.4).

Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

In some cases, antigen binding fragments can be prepared by proteolytic hydrolysis of the antibody or by expression in a host cell (such as *E. coli*) of DNA encoding the fragment. Antigen binding fragments can be obtained by pepsin or papain digestion of whole antibodies by conventional methods. For example, antigen binding fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using pepsin produces two monovalent Fab' fragments and an Fc fragment directly (see U.S. Patent No. 4,036,945 and U.S. Patent No. 4,331,647).

Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

One of skill will realize that conservative variants of the antibodies can be produced. Such conservative variants employed in antigen binding fragments, such as dsFv fragments or in scFv fragments, will retain critical amino acid residues necessary for correct folding and stabilizing between the V_{H} and the V_{L} regions, and will retain the charge characteristics of the residues in order to preserve the low pI and low toxicity of the molecules. Amino acid substitutions (such as at most one, at most two, at most three, at most four, or at most five amino acid substitutions) can be made in the V_{H} or the V_{L} regions to increase yield. In particular examples, the V_{H} sequence is one of SEQ ID NO: 1, 3, 5, or 7. In other examples, the V_{L} sequence is one of SEQ ID NO: 2, 4, 7, or 8. Conservative amino acid substitution tables providing functionally similar amino acids are well known to one of ordinary skill in the art. The following six groups are examples of amino acids that are considered to be conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Also included are antibodies that bind to the same epitope on ALK to which the ALK specific antibodies provided herein bind. Antibodies that bind to such an epitope can be identified based on their ability to cross-compete (for example, to competitively inhibit the binding of, in a statistically significant manner) with the ALK specific antibodies provided herein in ALK binding assays (such as those described in the Examples). An antibody "competes" for binding when the competing antibody inhibits ALK binding of an antibody of the invention by more than 50%, in the presence of competing antibody concentrations higher than 10⁶ x K_{D} of the competing antibody. In a certain embodiment, the antibody that binds to the same epitope on ALK as the antibodies of the present invention is a human monoclonal antibody. Such human monoclonal antibodies can be prepared and isolated as described herein.

Additionally, to increase binding affinity of the antibody, the V_{L} and V_{H} segments can be randomly mutated, such as within H-CDR3 region or the L-CDR3 region, in a process analogous to the *in vivo* somatic mutation process responsible for affinity maturation of antibodies during a natural immune response. Thus *in vitro* affinity maturation can be accomplished by amplifying V_{H} and V_{L} regions using PCR primers complementary to the H-CDR3 or L-CDR3, respectively. In this process, the primers have been "spiked" with a random mixture of the four nucleotide bases at certain positions such that the resultant PCR products encode V_{H} and V_{L} segments into which random mutations have been introduced into the V_{H} and/or V_{L} CDR3 regions. These randomly mutated V_{H} and V_{L} segments can be tested to determine the binding affinity for ALK. In particular examples, the V_{H} amino acid sequence is one of SEQ ID NOs: 1, 3, 5, or 7. In other examples, the V_{L} amino acid sequence is SEQ ID NOs: 2, 4, 6, or 8.

### C. Conjugates

Monoclonal antibodies specific for ALK, or antigen binding fragments thereof, can be conjugated to an agent, such as an effector molecule or detectable marker, using any number of means known to those of skill in the art. Both covalent and noncovalent attachment means may be used. Conjugates include, but are not limited to, molecules in which there is a covalent linkage of an effector molecule or a detectable marker to an antibody or antigen binding fragment that specifically binds ALK. One of skill in the art will appreciate that various effector molecules and detectable markers can be used, including (but not limited to) chemotherapeutic agents, anti-angiogenic agents, toxins, radioactive agents such as ¹²⁵I, ³²P, ¹⁴C, ³H and ³⁵S and other labels, target moieties and ligands, etc.

The choice of a particular effector molecule or detectable marker depends on the particular target molecule or cell, and the desired biological effect. Thus, for example, the effector molecule can be a cytotoxin that is used to bring about the death of a particular target cell (such as a tumor cell).

The procedure for attaching an effector molecule or detectable marker to an antibody or antigen binding fragment varies according to the chemical structure of the effector. Polypeptides typically contain a variety of functional groups; such as carboxylic acid (COOH), free amine (-NH₂) or sulfhydryl (-SH) groups, which are available for reaction with a suitable functional group on an antibody to result in the binding of the effector molecule or detectable marker. Alternatively, the antibody or antigen binding fragment is derivatized to expose or attach additional reactive functional groups. The derivatization may involve attachment of any of a number of known linker molecules such as those available from Pierce Chemical Company, Rockford, IL. The linker can be any molecule used to join the antibody or antigen binding fragment to the effector molecule or detectable marker. The linker is capable of forming covalent bonds to both the antibody or antigen binding fragment and to the effector molecule or detectable marker. Suitable linkers are well known to those of skill in the art and include, but are not limited to, straight or branched-chain carbon linkers, heterocyclic carbon linkers, or peptide linkers. Where the antibody or antigen binding fragment and the effector molecule or detectable marker are polypeptides, the linkers may be joined to the constituent amino acids through their side groups (such as through a disulfide linkage to cysteine) or to the alpha carbon amino and carboxyl groups of the terminal amino acids.

In several embodiments, the linker can include a spacer element, which, when present, increases the size of the linker such that the distance between the effector molecule or the detectable marker and the antibody or antigen binding fragment is increased. Exemplary spacers are known to the person of ordinary skill, and include those listed in U.S. Pat. Nos. 7,964,5667, 498,298, 6,884,869, 6,323,315, 6,239,104, 6,034,065, 5,780,588, 5,665,860, 5,663,149, 5,635,483, 5,599,902, 5,554,725, 5,530,097, 5,521,284, 5,504,191, 5,410,024, 5,138,036, 5,076,973, 4,986,988, 4,978,744, 4,879,278, 4,816,444, and 4,486,414, as well as U.S. Pat. Pub. Nos. 20110212088 and 20110070248.

In some embodiments, the linker is cleavable under intracellular conditions, such that cleavage of the linker releases the effector molecule or detectable marker from the antibody or antigen binding fragment in the intracellular environment. In yet other embodiments, the linker is not cleavable and the effector molecule or detectable marker is released, for example, by antibody degradation. In some embodiments, the linker is cleavable by a cleaving agent that is present in the intracellular environment (for example, within a lysosome or endosome or caveolea). The linker can be, for example, a peptide linker that is cleaved by an intracellular peptidase or protease enzyme, including, but not limited to, a lysosomal or endosomal protease. In some embodiments, the peptide linker is at least two amino acids long or at least three amino acids long. However, the linker can be 4, 5, 6, 7, 8, 9, 10, 11, 12, 13, 14 or 15 amino acids long, such as 1-2, 1-3, 2-5, 3-10, 3-15, 1-5, 1-10, 1-15, amino acids long. Proteases can include cathepsins B and D and plasmin, all of which are known to hydrolyze dipeptide drug derivatives resulting in the release of active drug inside target cells (see, for example, Dubowchik and Walker, 1999, Pharm. Therapeutics 83:67-123). For example, a peptide linker that is cleavable by the thiol-dependent protease cathepsin-B, can be used (for example, a Phenylalanine -Leucine or a Glycine- Phenylalanine -Leucine-Glycine linker). Other examples of such linkers are described, for example, in U.S. Pat. No. 6,214,345. In a specific embodiment, the peptide linker cleavable by an intracellular protease is a Valine-Citruline linker or a Phenylalanine-Lysine linker (see, for example, U.S. Pat. No. 6,214,345, which describes the synthesis of doxorubicin with the Valine-Citruline linker).

In other embodiments, the cleavable linker is pH-sensitive, i.e., sensitive to hydrolysis at certain pH values. Typically, the pH-sensitive linker is hydrolyzable under acidic conditions. For example, an acid-labile linker that is hydrolyzable in the lysosome (for example, a hydrazone, semicarbazone, thiosemicarbazone, cis-aconitic amide, orthoester, acetal, ketal, or the like) can be used. (See, for example, U.S. Pat. Nos. 5,122,368; 5,824,805; 5,622,929; Dubowchik and Walker, 1999, Pharm. Therapeutics 83:67-123; Neville et al., 1989, Biol. Chem. 264:14653-14661.) Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, for example, a thioether attached to the therapeutic agent via an acylhydrazone bond (see, for example, U.S. Pat. No. 5,622,929).

In other embodiments, the linker is cleavable under reducing conditions (for example, a disulfide linker). A variety of disulfide linkers are known in the art, including, for example, those that can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate) and SMPT (N-succinimidyl-oxycarbonyl-alpha-methyl-alpha-(2-pyridyl-dithio)toluene)-, SPDB and SMPT. (See, for example, Thorpe et al., 1987, Cancer Res. 47:5924-5931; Wawrzynczak et al., In Immunoconjugates: Antibody Conjugates in Radioimagery and Therapy of Cancer (C. W. Vogel ed., Oxford U. Press, 1987); Phillips et al., Cancer Res. 68:92809290, 2008). See also U.S. Pat. No. 4,880,935.)

In yet other specific embodiments, the linker is a malonate linker (Johnson et al., 1995, Anticancer Res. 15:1387-93), a maleimidobenzoyl linker (Lau et al., 1995, Bioorg-Med-Chem. 3(10):1299-1304), or a 3'-N-amide analog (Lau et al., 1995, Bioorg-Med-Chem. 3(10):1305-12).

In yet other embodiments, the linker is not cleavable and the effector molecule or detectable marker is released by antibody degradation. (See U.S. Publication No. 2005/0238649).

In several embodiments, the linker is resistant to cleavage in an extracellular environment. For example, no more than about 20%, no more than about 15%, no more than about 10%, no more than about 5%, no more than about 3%, or no more than about 1% of the linkers, in a sample of conjugate, are cleaved when the conjugate is present in an extracellular environment (for example, in plasma). Whether or not a linker is resistant to cleavage in an extracellular environment can be determined, for example, by incubating the conjugate containing the linker of interest with plasma for a predetermined time period (for example, 2, 4, 8, 16, or 24 hours) and then quantitating the amount of free effector molecule or detectable marker present in the plasma. A variety of exemplary linkers that can be used in conjugates are described in WO 2004-010957, U.S. Publication No. 2006/0074008, U.S. Publication No. 20050238649, and U.S. Publication No. 2006/0024317. In several embodiments, conjugates of an antibody or antigen binding fragment and one or more small molecule toxins, such as a calicheamicin, maytansinoids, dolastatins, auristatins, a trichothecene, and CC1065, and the derivatives of these toxins that have toxin activity, are provided.

Maytansine compounds suitable for use as maytansinoid toxin moieties are well known in the art, and can be isolated from natural sources according to known methods, produced using genetic engineering techniques (see Yu et al (2002) PNAS 99:7968-7973), or maytansinol and maytansinol analogues prepared synthetically according to known methods. Maytansinoids are mitototic inhibitors which act by inhibiting tubulin polymerization. Maytansine was first isolated from the east African shrub Maytenus serrata (U.S. Pat. No. 3,896,111). Subsequently, it was discovered that certain microbes also produce maytansinoids, such as maytansinol and C-3 maytansinol esters (U.S. Pat. No. 4,151,042). Synthetic maytansinol and derivatives and analogues thereof are disclosed, for example, in U.S. Pat. Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533. Conjugates containing maytansinoids, methods of making same, and their therapeutic use are disclosed, for example, in U.S. Pat. Nos. 5,208,020; 5,416,064; 6,441,163 and European Patent EP 0 425 235 B1.

In one example, the conjugate includes a monoclonal antibody that specifically binds ALK (or antigen binding fragment thereof), a non-reducible thioester linker and the maytansinoid toxin DM1; for example the conjugate can include the structure set forth as (wherein "mAb" refers to the monoclonal antibody or antigen binding fragment thereof):

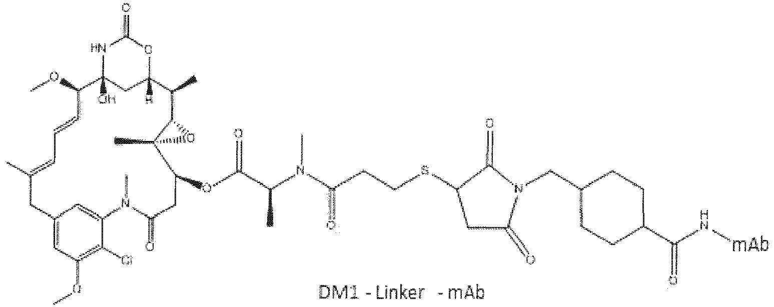

In some embodiments, the effector molecule is an auristatin, such as auristatin E (also known in the art as a derivative of dolastatin-10) or a derivative thereof. The auristatin can be, for example, an ester formed between auristatin E and a keto acid. For example, auristatin E can be reacted with paraacetyl benzoic acid or benzoylvaleric acid to produce AEB and AEVB, respectively. Other exemplary auristatins include AFP, MMAF, and MMAE. The synthesis and structure of exemplary auristatins are described in U.S. Patent Application Publication No. 2003/0083263; International Patent Publication No. WO 04/010957, International Patent Publication No. WO 02/088172, and U.S. Pat. Nos. 7, 498,298, 6,884,869, 6,323,315; 6,239,104; 6,034,065; 5,780,588; 5,665,860; 5,663,149; 5,635,483; 5,599,902; 5,554,725; 5,530,097; 5,521,284; 5,504,191; 5,410,024; 5,138,036; 5,076,973; 4,986,988; 4,978,744; 4,879,278; 4,816,444; and 4,486,414. Auristatins have been shown to interfere with microtubule dynamics and nuclear and cellular division and have anticancer activity. Auristatins bind tubulin and can exert a cytotoxic or cytostatic effect on cells. There are a number of different assays, known in the art, which can be used for determining whether an auristatin or resultant conjugate exerts a cytostatic or cytotoxic effect on a desired cell line.

In one example, the conjugate includes a monoclonal antibody that specifically binds ALK (or antigen binding fragment thereof), a cleavable linker including a Valine-Citruline peptide cleavage site, a spacer, and the toxin MMAE; for example the conjugate can include the structure set forth as (wherein "mAb" refers to the monoclonal antibody or antigen binding fragment thereof):

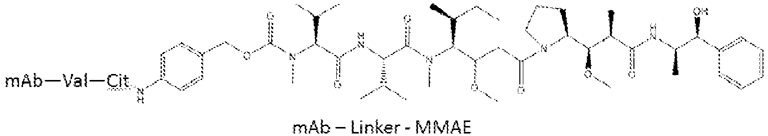

Additional toxins can be employed with antibodies that specifically bind ALK, and antigen binding fragment of these antibodies. Exemplary toxins include Pseudomonas exotoxin (PE), ricin, abrin, diphtheria toxin and subunits thereof, ribotoxin, ribonuclease, saporin, and calicheamicin, as well as botulinum toxins A through F. These toxins are well known in the art and many are readily available from commercial sources (for example, Sigma Chemical Company, St. Louis, MO). Contemplated toxins also include variants of the toxins (see, for example, see, U.S. Patent Nos. 5,079,163 and 4,689,401). In some embodiments, these conjugates are of use for the treatment of a tumor, such as a neuroblastoma.

Saporin is a toxin derived from Saponaria officinalis that disrupts protein synthesis by inactivating the 60S portion of the ribosomal complex (Stirpe et al., Bio/Technology, 10:405-412, 1992). However, the toxin has no mechanism for specific entry into cells, and therefore requires conjugation to an antibody or antigen binding fragment that recognizes a cell-surface protein that is internalized in order to be efficiently taken up by cells.

Diphtheria toxin is isolated from Corynebacterium diphtheriae. Typically, diphtheria toxin for use in immunotoxins is mutated to reduce or to eliminate non-specific toxicity. A mutant known as CRM107, which has full enzymatic activity but markedly reduced non-specific toxicity, has been known since the 1970's (Laird and Groman, J. Virol. 19:220, 1976), and has been used in human clinical trials. See, U.S. Patent No. 5,792,458 and U.S. Patent No. 5,208,021.

Ricin is the lectin RCA60 from Ricinus communis (Castor bean). For examples of ricin, see, U.S. Patent No. 5,079,163 and U.S. Patent No. 4,689,401. Ricinus communis agglutinin (RCA) occurs in two forms designated RCA₆₀ and RCA₁₂₀ according to their molecular weights of approximately 65 and 120 kD, respectively (Nicholson & Blaustein, J. Biochim. Biophys. Acta 266:543, 1972). The A chain is responsible for inactivating protein synthesis and killing cells. The B chain binds ricin to cell-surface galactose residues and facilitates transport of the A chain into the cytosol (Olsnes et al., Nature 249:627-631, 1974 and U.S. Patent No. 3,060,165).

Ribonucleases have also been conjugated to targeting molecules for use as immunotoxins (see Suzuki et al., Nat. Biotech. 17:265-70, 1999). Exemplary ribotoxins such as α-sarcin and restrictocin are discussed in, for example Rathore et al., Gene 190:31-5, 1997; and Goyal and Batra, Biochem. 345 Pt 2:247-54, 2000. Calicheamicins were first isolated from Micromonospora echinospora and are members of the enediyne antitumor antibiotic family that cause double strand breaks in DNA that lead to apoptosis (see, for example Lee et al., J. Antibiot. 42:1070-87,1989). The drug is the toxic moiety of an immunotoxin in clinical trials (see, for example, Gillespie et al., Ann. Oncol. 11:735-41, 2000).

Abrin includes toxic lectins from Abrus precatorius. The toxic principles, abrin a, b, c, and d, have a molecular weight of from about 63 and 67 kD and are composed of two disulfide-linked polypeptide chains A and B. The A chain inhibits protein synthesis; the B chain (abrin-b) binds to D-galactose residues (see, Funatsu et al., Agr. Biol. Chem. 52:1095, 1988; and Olsnes, Methods Enzymol. 50:330-335, 1978).

In one embodiment, the toxin is Pseudomonas exotoxin (PE) (U.S. Patent No. 5,602,095). As used herein, PE includes full-length native (naturally occurring) PE or a PE that has been modified. Such modifications can include, but are not limited to, elimination of domain Ia, various amino acid deletions in domains Ib, II and III, single amino acid substitutions and the addition of one or more sequences at the carboxyl terminus (for example, see Siegall et al., J. Biol. Chem. 264:14256-14261, 1989). PE employed with the provided antibodies can include the native sequence, cytotoxic fragments of the native sequence, and conservatively modified variants of native PE and its cytotoxic fragments. Cytotoxic fragments of PE include those which are cytotoxic with or without subsequent proteolytic or other processing in the target cell. Cytotoxic fragments of PE include PE40, PE38, and PE35. For additional description of PE and variants thereof, see for example, U.S. Patent Nos. 4,892,827; 5,512,658; 5,602,095; 5,608,039; 5,821,238; and 5,854,044; PCT Publication No. WO 99/51643; Pai et al., Proc. Natl. Acad. Sci. USA, 88:3358-3362, 1991; Kondo et al., J. Biol. Chem., 263:9470-9475, 1988; Pastan et al., Biochim. Biophys. Acta, 1333:C1-C6, 1997.

Also contemplated herein are protease-resistant PE variants and PE variants with reduced immunogenicity, such as, but not limited to PE-LR, PE-6X, PE-8X, PE-LR/6X and PE-LR/8X (see, for example, Weldon et al., Blood 113(16):3792-3800, 2009; Onda et al., Proc. Natl. Acad. Sci. USA, 105(32):11311-11316, 2008; and PCT Publication Nos. WO 2007/016150, WO 2009/032954 and WO 2011/032022.

In some examples, the PE is a variant that is resistant to lysosomal degradation, such as PE-LR (Weldon et al., Blood 113(16):3792-3800, 2009; PCT Publication No. WO 2009/032954). In other examples, the PE is a variant designated PE-LR/6X (PCT Publication No. WO 2011/032022). In other examples, the PE is a variant designated PE-LR/8M (PCT Publication No. WO 2011/032022).

A monoclonal antibody that specifically binds ALK (or antigen binding fragment thereof) can also be conjugated with a detectable marker; for example, a detectable marker capable of detection by ELISA, spectrophotometry, flow cytometry, microscopy or diagnostic imaging techniques (such as computed tomography (CT), computed axial tomography (CAT) scans, magnetic resonance imaging (MRI), nuclear magnetic resonance imaging NMRI), magnetic resonance tomography (MTR), ultrasound, fiberoptic examination, and laparoscopic examination). Specific, non-limiting examples of detectable markers include fluorophores, chemiluminescent agents, enzymatic linkages, radioactive isotopes and heavy metals or compounds (for example super paramagnetic iron oxide nanocrystals for detection by MRI). For example, useful detectable markers include fluorescent compounds, including fluorescein, fluorescein isothiocyanate, rhodamine, 5-dimethylamine-1-napthalenesulfonyl chloride, phycoerythrin, lanthanide phosphors and the like. Bioluminescent markers are also of use, such as luciferase, Green fluorescent protein (GFP), Yellow fluorescent protein (YFP). An antibody or antigen binding fragment can also be conjugated with enzymes that are useful for detection, such as horseradish peroxidase, β- galactosidase, luciferase, alkaline phosphatase, glucose oxidase and the like. When an antibody or antigen binding fragment is conjugated with a detectable enzyme, it can be detected by adding additional reagents that the enzyme uses to produce a reaction product that can be discerned. For example, when the agent horseradish peroxidase is present the addition of hydrogen peroxide and diaminobenzidine leads to a colored reaction product, which is visually detectable. An antibody or antigen binding fragment may also be conjugated with biotin, and detected through indirect measurement of avidin or streptavidin binding. It should be noted that the avidin itself can be conjugated with an enzyme or a fluorescent label.

An antibody or antigen binding fragment may be conjugated with a paramagnetic agent, such as gadolinium. Paramagnetic agents such as superparamagnetic iron oxide are also of use as labels. Antibodies can also be conjugated with lanthanides (such as europium and dysprosium), and manganese. An antibody or antigen binding fragment may also be labeled with a predetermined polypeptide epitopes recognized by a secondary reporter (such as leucine zipper pair sequences, binding sites for secondary antibodies, metal binding domains, epitope tags).

An antibody or antigen binding fragment can also be conjugated with a radiolabeled amino acid. The radiolabel may be used for both diagnostic and therapeutic purposes. For instance, the radiolabel may be used to detect ALK and ALK expressing cells by x-ray, emission spectra, or other diagnostic techniques. Further, the radiolabel may be used therapeutically as a toxin for treatment of tumors in a subject, for example for treatment of a neuroblastoma. Examples of labels for polypeptides include, but are not limited to, the following radioisotopes or radionucleotides: ³H, ¹⁴C, ¹⁵N, ³⁵S, ⁹⁰Y, ⁹⁹Tc, ¹¹¹In, ¹²⁵I, ¹³¹I.

Means of detecting such detectable markers are well known to those of skill in the art. Thus, for example, radiolabels may be detected using photographic film or scintillation counters, fluorescent markers may be detected using a photodetector to detect emitted illumination. Enzymatic labels are typically detected by providing the enzyme with a substrate and detecting the reaction product produced by the action of the enzyme on the substrate, and colorimetric labels are detected by simply visualizing the colored label.

The average number of effector molecule or detectable marker moieties per antibody or antigen binding fragment in a conjugate can range, for example, from 1 to 20 moieties per antibody or antigen binding fragment. In certain embodiments, the average number of effector molecule or detectable marker moieties per antibody or antigen binding fragment in a conjugate range from 1 to about 8; from about 2 to about 6; from about 3 to about 5; from about 3 to about 4; from about 3.1 to about 3.9; from about 3.2 to about 3.8; from about 3.2 to about 3.7; from about 3.2 to about 3.6; from about 3.3 to about 3.8; or from about 3.3 to about 3.7. See, for example, U.S. Pat. No. 7,498,298.

The loading (for example, effector molecule/antibody ratio) of an conjugate may be controlled in different ways, for example, by: (i) limiting the molar excess of effector molecule-linker intermediate or linker reagent relative to antibody, (ii) limiting the conjugation reaction time or temperature, (iii) partial or limiting reductive conditions for cysteine thiol modification, (iv) engineering by recombinant techniques the amino acid sequence of the antibody such that the number and position of cysteine residues is modified for control of the number or position of linker-effector molecule attachments (such as thioMab or thioFab prepared as disclosed in WO2006/03448.

### D. Nucleotides, Expression, Vectors, and Host Cells

Nucleic acids encoding the amino acid sequences of antibodies, antibody binding fragments, conjugates, and CARs that specifically bind ALK are provided. Nucleic acids encoding these molecules can readily be produced by one of skill in the art, using the amino acid sequences provided herein (such as the CDR sequences, heavy chain and light chain sequences), sequences available in the art (such as framework sequences), and the genetic code. One of skill in the art can readily use the genetic code to construct a variety of functionally equivalent nucleic acids, such as nucleic acids which differ in sequence but which encode the same antibody sequence, or encode a conjugate or fusion protein including the V_{L} and/or V_{H} nucleic acid sequence.

Nucleic acid sequences encoding the of antibodies, antibody binding fragments, conjugates, and CARs that specifically bind ALK can be prepared by any suitable method including, for example, cloning of appropriate sequences or by direct chemical synthesis by methods such as the phosphotriester method of Narang et al., Meth. Enzymol. 68:90-99, 1979; the phosphodiester method of Brown et al., Meth. Enzymol. 68:109-151, 1979; the diethylphosphoramidite method of Beaucage et al., Tetra. Lett. 22:1859-1862, 1981; the solid phase phosphoramidite triester method described by Beaucage & Caruthers, Tetra. Letts. 22(20):1859-1862, 1981, for example, using an automated synthesizer as described in, for example, Needham-VanDevanter et al., Nucl. Acids Res. 12:6159-6168, 1984; and, the solid support method of U.S. Patent No. 4,458,066. Chemical synthesis produces a single stranded oligonucleotide. This can be converted into double stranded DNA by hybridization with a complementary sequence or by polymerization with a DNA polymerase using the single strand as a template. One of skill would recognize that while chemical synthesis of DNA is generally limited to sequences of about 100 bases, longer sequences may be obtained by the ligation of shorter sequences.

Exemplary nucleic acids can be prepared by cloning techniques. Examples of appropriate cloning and sequencing techniques, and instructions sufficient to direct persons of skill through many cloning exercises are known (see, e.g., Sambrook et al. (Molecular Cloning: A Laboratory Manual, 4th ed, Cold Spring Harbor, New York, 2012) and Ausubel et al. (In Current Protocols in Molecular Biology, John Wiley & Sons, New York, through supplement 104, 2013). Product information from manufacturers of biological reagents and experimental equipment also provide useful information. Such manufacturers include the SIGMA Chemical Company (Saint Louis, MO), R&D Systems (Minneapolis, MN), Pharmacia Amersham (Piscataway, NJ), CLONTECH Laboratories, Inc. (Palo Alto, CA), Chem Genes Corp., Aldrich Chemical Company (Milwaukee, WI), Glen Research, Inc., GIBCO BRL Life Technologies, Inc. (Gaithersburg, MD), Fluka Chemica-Biochemika Analytika (Fluka Chemie AG, Buchs, Switzerland), Invitrogen (Carlsbad, CA), and Applied Biosystems (Foster City, CA), as well as many other commercial sources known to one of skill.

Nucleic acids can also be prepared by amplification methods. Amplification methods include polymerase chain reaction (PCR), the ligase chain reaction (LCR), the transcription-based amplification system (TAS), the self-sustained sequence replication system (3SR). A wide variety of cloning methods, host cells, and *in vitro* amplification methodologies are well known to persons of skill.

In some embodiments, the nucleic acid molecule encodes a CAR as provided herein for expression in a T cell to generate a chimeric antigen receptor T cell. The nucleic acid molecule encoding the chimeric antigen binding receptor can be included in a vector (such as a lentiviral vector) for expression in a host cell, such as a T cell. Exemplary cells include a T cell, a Natural Killer (NK) cell, a cytotoxic T lymphocyte (CTL), and a regulatory T cell. Methods of generating nucleic acid molecules encoding chimeric antigen receptors and T cells including such receptors are known in the art (see, e.g., Brentjens et al., 2010, Molecular Therapy, 18:4, 666-668; Morgan et al., 2010, Molecular Therapy, published online February 23, 2010, pages 1 -9; Till et al., 2008, Blood, 1 12:2261 -2271; Park et al., Trends Biotechnol., 29:550-557, 2011; Grupp et al., N Engl J Med., 368:1509-1518, 2013; Han et al., J. Hematol Oncol., 6:47, 2013; PCT Pub. WO2012/079000, WO2013/126726; and U.S. Pub. 2012/0213783.

The nucleic acid molecules can be expressed in a recombinantly engineered cell such as bacteria, plant, yeast, insect and mammalian cells. The antibodies, antigen binding fragments, and conjugates can be expressed as individual V_{H} and/or V_{L} chain (linked to an effector molecule or detectable marker as needed), or can be expressed as a fusion protein. Methods of expressing and purifying antibodies and antigen binding fragments are known and further described herein (see, e.g., Al-Rubeai (ed), Antibody Expression and Production, Springer Press, 2011). An immunoadhesin can also be expressed. Thus, in some examples, nucleic acids encoding a V_{H} and V_{L}, and immunoadhesin are provided. The nucleic acid sequences can optionally encode a leader sequence.

To create a scFv the V_{H}- and V_{L}-encoding DNA fragments can be operatively linked to another fragment encoding a flexible linker, e.g., encoding the amino acid sequence (Gly₄-Ser)₃, such that the V_{H} and V_{L} sequences can be expressed as a contiguous single-chain protein, with the V_{L} and V_{H} domains joined by the flexible linker (see, *e.g.,* Bird et al., Science 242:423-426, 1988; Huston et al., Proc. Natl. Acad. Sci. USA 85:5879-5883, 1988; McCafferty et al., Nature 348:552-554, 1990; Kontermann and Dubel (Ed), Antibody Engineering, Vols. 1-2, 2nd Ed., Springer Press, 2010; Harlow and Lane, Antibodies: A Lab oratoiy Manual, 2nd, Cold Spring Harbor Laboratory, New York, 2013,). Optionally, a cleavage site can be included in a linker, such as a furin cleavage site.

The nucleic acid encoding a V_{H} and/or the V_{L} optionally can encode an Fc domain (immunoadhesin). The Fc domain can be an IgA, IgM or IgG Fc domain. The Fc domain can be an optimized Fc domain, as described in U.S. Published Patent Application No. 20100/093979. In one example, the immunoadhesin is an IgG₁ Fc.

The single chain antibody may be monovalent, if only a single V_{H} and V_{L} are used, bivalent, if two V_{H} and V_{L} are used, or polyvalent, if more than two V_{H} and V_{L} are used. Bispecific or polyvalent antibodies may be generated that bind specifically to ALK and another antigen, such as, but not limited to CD3. The encoded V_{H} and V_{L} optionally can include a furin cleavage site between the V_{H} and V_{L} domains.

One or more DNA sequences encoding the antibodies, antibody binding fragments, conjugates, and CARs can be expressed *in vitro* by DNA transfer into a suitable host cell. The cell may be prokaryotic or eukaryotic. The term also includes any progeny of the subject host cell. It is understood that all progeny may not be identical to the parental cell since there may be mutations that occur during replication. Methods of stable transfer, meaning that the foreign DNA is continuously maintained in the host, are known in the art. Hybridomas expressing the antibodies of interest are also encompassed by this disclosure.

Polynucleotide sequences encoding the amino acid sequences of CARs, antibodies, antibody binding fragments, and conjugates that specifically bind ALK can be operatively linked to expression control sequences. For example, the expression of nucleic acids encoding the proteins described herein can be achieved by operably linking the DNA or cDNA to a promoter (which is either constitutive or inducible), followed by incorporation into an expression cassette. The promoter can be any promoter of interest, including a cytomegalovirus promoter and a human T cell lymphotrophic virus promoter (HTLV)-1. Optionally, an enhancer, such as a cytomegalovirus enhancer, is included in the construct. The cassettes can be suitable for replication and integration in either prokaryotes or eukaryotes. Typical expression cassettes contain specific sequences useful for regulation of the expression of the DNA encoding the protein. For example, the expression cassettes can include appropriate promoters, enhancers, transcription and translation terminators, initiation sequences, a start codon (*i.e*., ATG) in front of a protein-encoding gene, splicing signal for introns, sequences for the maintenance of the correct reading frame of that gene to permit proper translation of mRNA, and stop codons. The vector can encode a selectable marker, such as a marker encoding drug resistance (for example, ampicillin or tetracycline resistance).

To obtain high level expression of a cloned gene, it is desirable to construct expression cassettes which contain, at the minimum, a strong promoter to direct transcription, a ribosome binding site for translational initiation (internal ribosomal binding sequences), and a transcription/translation terminator. For *E. coli,* this includes a promoter such as the T7, trp, lac, or lambda promoters, a ribosome binding site, and preferably a transcription termination signal. For eukaryotic cells, the control sequences can include a promoter and/or an enhancer derived from, for example, an immunoglobulin gene, HTLV, SV40 or cytomegalovirus, and a polyadenylation sequence, and can further include splice donor and/or acceptor sequences (for example, CMV and/or HTLV splice acceptor and donor sequences). The cassettes can be transferred into the chosen host cell by well-known methods such as transformation or electroporation for *E. coli* and calcium phosphate treatment, electroporation or lipofection for mammalian cells. Cells transformed by the cassettes can be selected by resistance to antibiotics conferred by genes contained in the cassettes, such as the amp, gpt, neo and hyg genes.

For purposes of producing a recombinant CAR, the host cell may be a mammalian cell. The host cell may be a human cell. In some embodiments, the host cell may be a peripheral blood lymphocyte (PBL) or a peripheral blood mononuclear cell (PBMC), or a T cell. The T cell can be any T cell, such as a cultured T cell, e.g., a primary T cell, or a T cell from a cultured T cell line, e.g., Jurkat, SupTl, etc., or a T cell obtained from a mammal. If obtained from a mammal, the T cell can be obtained from numerous sources, including but not limited to blood, bone marrow, lymph node, the thymus, or other tissues or fluids. T cells can also be enriched for or purified. The T cell may be a human T cell. The T cell may be a T cell isolated from a human. The T cell can be any type of T cell and can be of any developmental stage, including but not limited to, CD4⁺/CD8⁺ double positive T cells, CD4⁺ helper T cells, e.g., Th₁ and Th₂ cells, CD8⁺ T cells (e.g., cytotoxic T cells), tumor infiltrating cells, memory T cells, naive T cells, and the like. The T cell may be a CD8⁺ T cell or a CD4⁺ T cell.

Also provided is a population of cells comprising at least one host cell described herein. The population of cells can be a heterogeneous population comprising the host cell comprising any of the recombinant expression vectors described, in addition to at least one other cell, e.g., a host cell (e.g., a T cell), which does not comprise any of the recombinant expression vectors, or a cell other than a T cell, e.g., a B cell, a macrophage, a neutrophil, an erythrocyte, a hepatocyte, an endothelial cell, an epithelial cell, a muscle cell, a brain cell, etc. Alternatively, the population of cells can be a substantially homogeneous population, in which the population comprises mainly host cells (e.g., consisting essentially of) comprising the recombinant expression vector. The population also can be a clonal population of cells, in which all cells of the population are clones of a single host cell comprising a recombinant expression vector, such that all cells of the population comprise the recombinant expression vector. In one embodiment of the invention, the population of cells is a clonal population comprising host cells comprising a recombinant expression vector as described herein.

Modifications can be made to a nucleic acid encoding a polypeptide described herein without diminishing its biological activity. Some modifications can be made to facilitate the cloning, expression, or incorporation of the targeting molecule into a fusion protein. Such modifications are well known to those of skill in the art and include, for example, termination codons, a methionine added at the amino terminus to provide an initiation, site, additional amino acids placed on either terminus to create conveniently located restriction sites, or additional amino acids (such as poly His) to aid in purification steps. In addition to recombinant methods, the immunoconjugates, effector moieties, and antibodies of the present disclosure can also be constructed in whole or in part using standard peptide synthesis well known in the art.

Once expressed, the antibodies, antigen binding fragments, and conjugates can be purified according to standard procedures in the art, including ammonium sulfate precipitation, affinity columns, column chromatography, and the like (see, generally, Simpson ed., Basic methods in Protein Purification and Analysis: A laboratory Manual, Cold Harbor Press, 2008). The antibodies, antigen binding fragment, and conjugates need not be 100% pure. Once purified, partially or to homogeneity as desired, if to be used therapeutically, the polypeptides should be substantially free of endotoxin.

Methods for expression of the antibodies, antigen binding fragments, and conjugates, and/or refolding to an appropriate active form, from mammalian cells, and bacteria such as *E. coli* have been described and are well-known and are applicable to the antibodies disclosed herein. See, e.g., Harlow and Lane, Antibodies: A Laboratory Manual, 2nd, Cold Spring Harbor Laboratory, New York, 2013, Simpson ed., Basic methods in Protein Purification and Analysis: A laboratory Manual, Cold Harbor Press, 2008, and Ward et al., Nature 341:544, 1989. Often, functional heterologous proteins from *E. coli* or other bacteria are isolated from inclusion bodies and require solubilization using strong denaturants, and subsequent refolding. During the solubilization step, as is well known in the art, a reducing agent must be present to separate disulfide bonds. An exemplary buffer with a reducing agent is: 0.1 M Tris pH 8, 6 M guanidine, 2 mM EDTA, 0.3 M DTE (dithioerythritol). Reoxidation of the disulfide bonds can occur in the presence of low molecular weight thiol reagents in reduced and oxidized form, as described in Saxena et al., Biochemistry 9: 5015-5021, 1970, and especially as described by Buchner *et al*., *supra.*

Isolation and purification of recombinantly expressed polypeptide can be carried out by conventional means including preparative chromatography and immunological separations. Once expressed, the conjugate, antibody, or antigen binding fragment thereof, can be purified according to standard procedures of the art, including ammonium sulfate precipitation, affinity columns, column chromatography, and the like (see, generally, R. Scopes, Protein Purification, Springer-Verlag, N.Y., 1982). Substantially pure compositions of at least about 90 to 95% homogeneity are disclosed herein, and 98 to 99% or more homogeneity can be used for pharmaceutical purposes. Once purified, partially or to homogeneity as desired, if to be used therapeutically, the polypeptides should be substantially free of endotoxin.

Methods for expression of single chain antibodies and refolding to an appropriate active form, including single chain antibodies, from bacteria such as *E. coli* have been described and are well-known and are applicable to the antibodies disclosed herein. See, Buchner et al., Anal. Biochem. 205:263-270, 1992; Pluckthun, Biotechnology 9:545, 1991; Huse et al., Science 246:1275, 1989 and Ward et al., Nature 341:544, 1989. Often, functional heterologous proteins from *E. coli* or other bacteria are isolated from inclusion bodies and require solubilization using strong denaturants, and subsequent refolding. During the solubilization step, as is well known in the art, a reducing agent must be present to separate disulfide bonds. An exemplary buffer with a reducing agent is: 0.1 M Tris pH 8, 6 M guanidine, 2 mM EDTA, 0.3 M DTE (dithioerythritol). Reoxidation of the disulfide bonds can occur in the presence of low molecular weight thiol reagents in reduced and oxidized form, as described in Saxena et al., Biochemistry, 9: 5015-5021, 1970 and especially as described by Buchner *et al*., *supra.* Renaturation is typically accomplished by dilution (for example, 100-fold) of the denatured and reduced protein into refolding buffer. An exemplary buffer is 0.1 M Tris, pH 8.0, 0.5 M_{L}-arginine, 8 mM oxidized glutathione (GSSG), and 2 mM EDTA.

As a modification to the two chain antibody purification protocol, the heavy and light chain regions are separately solubilized and reduced and then combined in the refolding solution. An exemplary yield is obtained when these two proteins are mixed in a molar ratio such that a 5 fold molar excess of one protein over the other is not exceeded. Excess oxidized glutathione or other oxidizing low molecular weight compounds can be added to the refolding solution after the redox-shuffling is completed.

In addition to recombinant methods, the antibodies, antigen binding fragments, and/or conjugates can also be constructed in whole or in part using standard peptide synthesis. Solid phase synthesis of the polypeptides can be accomplished by attaching the C-terminal amino acid of the sequence to an insoluble support followed by sequential addition of the remaining amino acids in the sequence. Techniques for solid phase synthesis are described by Barany & Merrifield, The Peptides: Analysis, Synthesis, Biology. Vol. 2: Special Methods in Peptide Synthesis, Part A. pp. 3-284; Merrifield et al., J. Am. Chem. Soc. 85:2149-2156, 1963, and Stewart et al., Solid Phase Peptide Synthesis, 2nd ed., Pierce Chem. Co., Rockford, I11., 1984. Proteins of greater length may be synthesized by condensation of the amino and carboxyl termini of shorter fragments. Methods of forming peptide bonds by activation of a carboxyl terminal end (such as by the use of the coupling reagent N, N'-dicylohexylcarbodimide) are well known in the art.

### E. Methods of Treatment

A therapeutically effective amount of a disclosed antibody, antigen binding fragment, conjugate, CAR or T cell expressing a CAR can be administered to a subject to treat a tumor in the subject. A subject can be selected for treatment that has, is suspected of having or is at risk of developing a tumor, such as a neuroblastoma, a rhabdomyosarcoma, or a glioblastoma. Subjects that can benefit from the disclosed methods include human and veterinary subjects.

In some examples, a disclosed antibody, antigen binding fragment, conjugate, CAR or T cell expressing a CAR disclosed herein can be administered to a subject to slow or inhibit the growth or metastasis of a tumor. In these applications, a therapeutically effective amount of a disclosed antibody, antigen binding fragment, conjugate, CAR or T cell expressing a CAR or composition is administered to a subject in an amount and under conditions sufficient to form an immune complex with ALK, thereby slowing or inhibiting the growth or the metastasis of a tumor, or to inhibit a sign or a symptom of a tumor. Examples of suitable subjects include those diagnosed with or suspecting of having cancer (for example, a subject having a tumor), for example a subject having a neuroblastoma.

The therapeutically effective amount will depend upon the severity of the disease and the general state of the patient's health. A therapeutically effective amount is that which provides either subjective relief of a symptom(s) or an objectively identifiable improvement as noted by the clinician or other qualified observer. In one embodiment, a therapeutically effective amount is the amount necessary to inhibit tumor growth (such as growth of a neuroblastoma), or the amount that is effective at reducing a sign or a symptom of the tumor. The therapeutically effective amount of the agents administered can vary depending upon the desired effects and the subject to be treated. In some examples, therapeutic amounts are amounts which eliminate or reduce the patient's tumor burden, or which prevent or reduce the proliferation of metastatic cells.

In some non-limiting embodiments, a therapeutically effective amount of T cells expressing one or more ALK-specific chimeric antigen receptors as described herein can be administered to a subject in need thereof, for example a subject with an ALK-positive tumor. The therapeutically effective amount of the CAR T cells administered to the subject will depend upon the severity of the disease and the general state of the patient's health. In some embodiments, the subject is administered from 1 x 10⁵ to 1 x 10⁷ (such as from 1 x 10⁵ to 1 x 10⁶, from 1 x 10⁶ to 1 x 10⁷, from 5 x 10⁵ to 5 x 10⁶, from 5 x 10⁵ to 1 x 10⁶, from 7 x 10⁵ to 3 x 10⁶, from 8 x 10⁵ to 2 x 10⁶, or about 5 x 10⁵, 6 x 10⁵, 7 x 10⁵, 8 x 10⁵, 9 x 10⁵, 1 x 10⁶, 2 x 10⁶, 3 x 10⁶, 4 x 10⁶, or 5 x 10⁶) CART cells/kg in a single dose, in multiple doses (e.g., 2, 3, or 4, doses) or spread over multiple doses (e.g., 2, 3, or 4 doses). The T cells can be autologous T cells that have been obtained from the subject and transduced or transformed with a vector (such as a lentiviral vector) or nucleic acid molecule encoding the ALK-specific CAR. Methods of making such T cells are known and disclosed herein.

Methods of generating chimeric antigen receptors, T cells including such receptors, and their use (e.g., for treatment of cancer) are known in the art and further described herein (see, e.g., Brentjens et al., 2010, Molecular Therapy, 18:4, 666-668; Morgan et al., 2010, Molecular Therapy, published online February 23, 2010, pages 1 -9; Till et al., 2008, Blood, 1 12:2261 -2271; Park et al., Trends Biotechnol., 29:550-557,2011; Grupp et al., N Engl J Med., 368:1509-1518, 2013; Han et al., J. Hematol Oncol., 6:47, 2013; Tumaini et al., Cytotherapy, 15, 1406-1417, 2013; Haso et al., (2013) Blood, 121, 1165-1174; PCT Pubs. WO2012/079000, WO2013/126726; and U.S. Pub. 2012/0213783.

In some embodiments, the disclosed methods include isolating T cells from a subject, transducing the T cells with an expression vector (such as a lentiviral vector) encoding the chimeric antigen receptor, and administering the CAR-expressing T cells to the subject for treatment, for example for treatment of an ALK-positive tumor in the subject.

Subjects can be screened prior to initiating the disclosed therapies, for example to determine whether the subject has a tumor. The presence of a tumor indicates that the tumor can be treated using the methods provided herein. In some embodiments, a subject with an ALK-positive tumor is selected for treatment, for example, by detecting ALK expression and/or activity in a biological sample obtained from the subject. In some embodiments, cell surface expression of ALK is detected to identify an ALK positive tumor. For example ALK nucleic acids (such as an ALK gene, cDNA, or mRNA), ALK proteins, or ALK kinase activity, can be detected, and in some examples quantified. The detected ALK in the biological sample is compared to a control (such as a normal, non-melanoma sample, for example a normal skin sample). An increase in the amount of expressed ALK (such as ALK nucleic acids (for example an ALK gene, cDNA, or mRNA), ALK proteins, or ALK kinase activity in the biological sample relative to the control indicates the presence of an ALK positive tumor, and can be used to select a subject for treatment with one or more of the agents disclosed herein. For example, an increase in the test sample of at least 10%, at least 20%, at least 30%, at least 50%, at least 75%, at least 80%, at least 90%, at least 100%, at least 200% or even greater than 500%, relative to the control, indicates the subject (such as a human subject) is likely to respond favorably to treatment with one or more of the agents disclosed herein. Suitable methods for detecting and/or monitoring an ALK-positive tumor in a subject (such as an ALK-positive neuroblastoma) cane be selected by a treating physician. In one embodiment, a sample is obtained from a subject, and the presence of a cell that expresses ALK is assessed *in vitro.* In another embodiment, the antibodies disclosed herein can be used to detect cells that express ALK *in vivo.* In some examples, *in vivo* detection of a cell that expresses ALK detects a tumor in the subject.

Any method of administration can be used for the disclosed therapeutic agents, including local and systemic administration. For example topical, oral, intravascular such as intravenous, intramuscular, intraperitoneal, intranasal, intradermal, intrathecal and subcutaneous administration can be used. The particular mode of administration and the dosage regimen will be selected by the attending clinician, taking into account the particulars of the case (for example the subject, the disease, the disease state involved, and whether the treatment is prophylactic). In cases in which more than one agent or composition is being administered, one or more routes of administration may be used; for example, a chemotherapeutic agent may be administered orally and an antibody or antigen binding fragment or conjugate or composition may be administered intravenously. Methods of administration include injection for which the conjugates, antibodies, antigen binding fragments, or compositions are provided in a nontoxic pharmaceutically acceptable carrier such as water, saline, Ringer's solution, dextrose solution, 5% human serum albumin, fixed oils, ethyl oleate, or liposomes. In some embodiments, local administration of the disclosed compounds can be used, for instance by applying the antibody or antigen binding fragment to a region of tissue from which a tumor has been removed, or a region suspected of being prone to tumor development. In some embodiments, sustained intra-tumoral (or near-tumoral) release of the pharmaceutical preparation that includes a therapeutically effective amount of the antibody or antigen binding fragment may be beneficial. In other examples, the conjugate is applied as an eye drop topically to the cornea, or intravitreally into the eye.

The disclosed therapeutic agents can be formulated in unit dosage form suitable for individual administration of precise dosages. In addition, the disclosed therapeutic agents may be administered in a single dose or in a multiple dose schedule. A multiple dose schedule is one in which a primary course of treatment may be with more than one separate dose, for instance 1-10 doses, followed by other doses given at subsequent time intervals as needed to maintain or reinforce the action of the compositions. Treatment can involve daily or multi-daily doses of compound(s) over a period of a few days to months, or even years. Thus, the dosage regime will also, at least in part, be determined based on the particular needs of the subject to be treated and will be dependent upon the judgment of the administering practitioner.

Typical dosages of the antibodies or conjugates can range from about 0.01 to about 30 mg/kg, such as from about 0.1 to about 10 mg/kg.

In particular examples, the subject is administered a therapeutic composition that includes one or more of the conjugates, antibodies, compositions, CAR T cells or additional agents, on a multiple daily dosing schedule, such as at least two consecutive days, 10 consecutive days, and so forth, for example for a period of weeks, months, or years. In one example, the subject is administered the conjugates, antibodies, compositions or additional agents for a period of at least 30 days, such as at least 2 months, at least 4 months, at least 6 months, at least 12 months, at least 24 months, or at least 36 months.

In some embodiments, the disclosed methods include providing surgery, radiation therapy, and/or chemotherapeutics to the subject in combination with a disclosed antibody, antigen binding fragment, conjugate, CAR or T cell expressing a CAR (for example, sequentially, substantially simultaneously, or simultaneously). Methods and therapeutic dosages of such agents and treatments are known to those skilled in the art, and can be determined by a skilled clinician. Preparation and dosing schedules for the additional agent may be used according to manufacturer's instructions or as determined empirically by the skilled practitioner. Preparation and dosing schedules for such chemotherapy are also described in Chemotherapy Service, (1992) Ed., M. C. Perry, Williams & Wilkins, Baltimore, Md.

In some embodiments, the combination therapy can include administration of a therapeutically effective amount of an additional ALK inhibitor to a subject (such as a subject having ALK-positive tumor). The ALK inhibitor can be is a small molecule inhibitor, such as crizotinib (Pfizer, New York, NY), AP26113 (Ariad Pharmaceuticals, Cambridge, MA), CH5424802 (Chugai Pharmaceutical, Tokyo, Japan), LDK378 (Novartis, Basel, Switzerland), ASP3026 (Astellas Pharma, Northbrook, IL), X-396 (Xcovery, West Palm Beach, FL), or retaspimycin (Infinity Pharmaceuticals, Cambridge, MA). Additional ALK inhibitors include 3-39 (Novartis), GSK1838705A (GlaxoSmithKline, Boston, MA), and CEP-28122 (Cephalon, Frazer, PA). In another example, an ALK inhibitor is an anti-ALK antibody, such as a humanized anti-ALK antibody.

Methods and therapeutic dosages of such agents and treatments are known to those of ordinary skill in the art, and for example, can be determined by a skilled clinician. In a non-limiting example, a therapeutically effective amount of crizotinib is administered to a subject having a tumor that is identified as ALK-positive. In some examples, a therapeutically effective amount of crizotinib can be about 50-2000 mg/day (such as about 50, 100, 150, 200, 250, 300, 400, 500, 600, 700 ,800, 900, 1000, 1100, 1200, 1300, 1400, 1500, 1600, 1700, 1800, 1900, 2000 mg/day), administered orally in one or two doses per day. In some examples, the methods include orally administering 200 mg of crizotinib to the subject once or twice per day if the sample from the subject is scored as ALK-positive. In other examples, the methods include orally administering 250 mg of crizotinib to the subject once or twice per day if the sample from the subject is scored as ALK-positive. Dosages and dosing schedules of crizotinib for a subject can be determined by a skilled clinician, taking into account additional factors such as tumor site, tumor stage, tumor grade, patient treatment history, patient performance and nutritional status, concomitant health problems, social and logistic factors, previous primary tumors, and patient preference. Crizotinib may be administered on a continuous dosing schedule or administered for one or more cycles (for example, one or more cycles of 21-28 days). Treatment may repeat every 21-28 days if administered in cycles.

Non-limiting examples of additional therapeutic agents that can be used with the combination therapy include microtubule binding agents, DNA intercalators or cross-linkers, DNA synthesis inhibitors, DNA and RNA transcription inhibitors, antibodies, enzymes, enzyme inhibitors, gene regulators, and angiogenesis inhibitors. These agents (which are administered at a therapeutically effective amount) and treatments can be used alone or in combination. For example, any suitable anti-cancer or anti-angiogenic agent can be administered in combination with the antibodies, conjugates disclosed herein. Methods and therapeutic dosages of such agents are known to those skilled in the art, and can be determined by a skilled clinician.

Additional chemotherapeutic agents include, but are not limited to alkylating agents, such as nitrogen mustards (for example, chlorambucil, chlormethine, cyclophosphamide, ifosfamide, and melphalan), nitrosoureas (for example, carmustine, fotemustine, lomustine, and streptozocin), platinum compounds (for example, carboplatin, cisplatin, oxaliplatin, and BBR3464), busulfan, dacarbazine, mechlorethamine, procarbazine, temozolomide, thiotepa, and uramustine; antimetabolites, such as folic acid (for example, methotrexate, pemetrexed, and raltitrexed), purine (for example, cladribine, clofarabine, fludarabine, mercaptopurine, and tioguanine), pyrimidine (for example, capecitabine), cytarabine, fluorouracil, and gemcitabine; plant alkaloids, such as podophyllum (for example, etoposide, and teniposide), taxane (for example, docetaxel and paclitaxel), vinca (for example, vinblastine, vincristine, vindesine, and vinorelbine); cytotoxic/antitumor antibiotics, such as anthracycline family members (for example, daunorubicin, doxorubicin, epirubicin, idarubicin, mitoxantrone, and valrubicin), bleomycin, rifampicin, hydroxyurea, and mitomycin; topoisomerase inhibitors, such as topotecan and irinotecan; monoclonal antibodies, such as alemtuzumab, bevacizumab, cetuximab, gemtuzumab, rituximab, panitumumab, pertuzumab, and trastuzumab; photosensitizers, such as aminolevulinic acid, methyl aminolevulinate, porfimer sodium, and verteporfin; and other agents , such as alitretinoin, altretamine, amsacrine, anagrelide, arsenic trioxide, asparaginase, axitinib, bexarotene, bevacizumab, bortezomib, celecoxib, denileukin diftitox, erlotinib, estramustine, gefitinib, hydroxycarbamide, imatinib, lapatinib, pazopanib, pentostatin, masoprocol, mitotane, pegaspargase, tamoxifen, sorafenib, sunitinib, vemurafinib, vandetanib, and tretinoin. Selection and therapeutic dosages of such agents are known to those skilled in the art, and can be determined by a skilled clinician.

The combination therapy may provide synergy and prove synergistic, that is, the effect achieved when the active ingredients used together is greater than the sum of the effects that results from using the compounds separately. A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation, a synergistic effect may be attained when the compounds are administered or delivered sequentially, for example by different injections in separate syringes. In general, during alternation, an effective dosage of each active ingredient is administered sequentially, i.e. serially, whereas in combination therapy, effective dosages of two or more active ingredients are administered together.

In one embodiment, an effective amount of an antibody or antigen binding fragment that specifically binds to ALK or a conjugate thereof is administered to a subject having an ALK positive tumor following anti-cancer treatment. After a sufficient amount of time has elapsed to allow for the administered antibody or antigen binding fragment or conjugate to form an immune complex with ALK on an endothelial cell, the immune complex is detected. The presence (or absence) of the immune complex indicates the effectiveness of the treatment. For example, an increase in the immune complex compared to a control taken prior to the treatment indicates that the treatment is not effective, whereas a decrease in the immune complex compared to a control taken prior to the treatment indicates that the treatment is effective.

### F. Compositions

Compositions are provided that include one or more of the disclosed antibodies, antigen binding fragments, conjugates, CARs, or T cells expressing a CAR that specifically bind to ALK, in a carrier (such as a pharmaceutically acceptable carrier). The compositions can be prepared in unit dosage forms for administration to a subject. The amount and timing of administration are at the discretion of the treating clinician to achieve the desired outcome. The compositions can be formulated for systemic (such as intravenus) or local (such as intra-tumor) administration. In one example, a disclosed antibody, antigen binding fragment, conjugate, CAR or T cell expressing a CAR, is formulated for parenteral administration, such as intravenous administration. Compositions including a conjugate, antibody or antigen binding fragment as disclosed herein are of use, for example, for the treatment and detection of a tumor, for a neuroblastoma. In some examples, the compositions are useful for the treatment or detection of a carcinoma. The compositions including a conjugate, antibody or antigen binding fragment as disclosed herein are also of use, for example, for the detection of pathological angiogenesis.

The compositions for administration can include a solution of the conjugate, antibody or antigen binding fragment dissolved in a pharmaceutically acceptable carrier, such as an aqueous carrier. A variety of aqueous carriers can be used, for example, buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of antibody or antigen binding fragment or conjugate in these formulations can vary widely, and will be selected primarily based on fluid volumes, viscosities, body weight and the like in accordance with the particular mode of administration selected and the subject's needs. Actual methods of preparing such dosage forms are known, or will be apparent, to those skilled in the art.

A typical composition for intravenous administration includes about 0.01 to about 30 mg/kg of antibody or antigen binding fragment or conjugate per subject per day (or the corresponding dose of a conjugate including the antibody or antigen binding fragment). Actual methods for preparing administrable compositions will be known or apparent to those skilled in the art and are described in more detail in such publications as Remington's Pharmaceutical Science, 19th ed., Mack Publishing Company, Easton, PA (1995).

Antibodies, antigen binding fragments, or conjugates may be provided in lyophilized form and rehydrated with sterile water before administration, although they are also provided in sterile solutions of known concentration. The antibody or antigen binding fragment or conjugate solution is then added to an infusion bag containing 0.9% sodium chloride, USP, and in some cases administered at a dosage of from 0.5 to 15 mg/kg of body weight. Considerable experience is available in the art in the administration of antibody or antigen binding fragment and conjugate drugs; for example, antibody drugs have been marketed in the U.S. since the approval of RITUXAN® in 1997. Antibodies, antigen binding fragments and conjugates can be administered by slow infusion, rather than in an intravenous push or bolus. In one example, a higher loading dose is administered, with subsequent, maintenance doses being administered at a lower level. For example, an initial loading dose of 4 mg/kg antibody or antigen binding fragment (or the corresponding dose of a conjugate including the antibody or antigen binding fragment) may be infused over a period of some 90 minutes, followed by weekly maintenance doses for 4-8 weeks of 2 mg/kg infused over a 30 minute period if the previous dose was well tolerated.

Controlled release parenteral formulations can be made as implants, oily injections, or as particulate systems. For a broad overview of protein delivery systems see, Banga, A.J., Therapeutic Peptides and Proteins: Formulation, Processing, and Delivery Systems, Technomic Publishing Company, Inc., Lancaster, PA, (1995). Particulate systems include microspheres, microparticles, microcapsules, nanocapsules, nanospheres, and nanoparticles. Microcapsules contain the therapeutic protein, such as a cytotoxin or a drug, as a central core. In microspheres the therapeutic is dispersed throughout the particle. Particles, microspheres, and microcapsules smaller than about 1 µm are generally referred to as nanoparticles, nanospheres, and nanocapsules, respectively. Capillaries have a diameter of approximately 5 µm so that only nanoparticles are administered intravenously. Microparticles are typically around 100 µm in diameter and are administered subcutaneously or intramuscularly. See, for example, Kreuter, J., Colloidal Drug Delivery Systems, J. Kreuter, ed., Marcel Dekker, Inc., New York, NY, pp. 219-342 (1994); and Tice & Tabibi, Treatise on Controlled Drug Delivery, A. Kydonieus, ed., Marcel Dekker, Inc. New York, NY, pp. 315-339, (1992).

Polymers can be used for ion-controlled release of the antibody or antigen binding fragment or conjugate compositions disclosed herein. Various degradable and nondegradable polymeric matrices for use in controlled drug delivery are known in the art (Langer, Accounts Chem. Res. 26:537-542, 1993). For example, the block copolymer, polaxamer 407, exists as a viscous yet mobile liquid at low temperatures but forms a semisolid gel at body temperature. It has been shown to be an effective vehicle for formulation and sustained delivery of recombinant interleukin-2 and urease (Johnston et al., Pharm. Res. 9:425-434, 1992; and Pec et al., J. Parent. Sci. Tech. 44(2):58-65, 1990). Alternatively, hydroxyapatite has been used as a microcarrier for controlled release of proteins (Ijntema et al., Int. J. Pharm.112:215-224, 1994). In yet another aspect, liposomes are used for controlled release as well as drug targeting of the lipid-capsulated drug (Betageri et al., Liposome Drug Delivery Systems, Technomic Publishing Co., Inc., Lancaster, PA (1993)). Numerous additional systems for controlled delivery of therapeutic proteins are known (see U.S. Patent No. 5,055,303; U.S. Patent No. 5,188,837; U.S. Patent No. 4,235,871; U.S. Patent No. 4,501,728; U.S. Patent No. 4,837,028; U.S. Patent No. 4,957,735; U.S. Patent No. 5,019,369; U.S. Patent No. 5,055,303; U.S. Patent No. 5,514,670; U.S. Patent No. 5,413,797; U.S. Patent No. 5,268,164; U.S. Patent No. 5,004,697; U.S. Patent No. 4,902,505; U.S. Patent No. 5,506,206; U.S. Patent No. 5,271,961; U.S. Patent No. 5,254,342 and U.S. Patent No. 5,534,496).

### G. Kits

Kits are also provided. For example, kits for treating a tumor in a subject, or making a CAR T cell that expresses one or more of the CARs disclosed herein. The kits will typically include a disclosed antibody, antigen binding fragment, conjugate, nucleic acid molecule, CAR or T cell expressing a CAR as disclosed herein. More than one of the disclosed antibodies, antigen binding fragments, conjugates, nucleic acid molecules, CARs or T cells expressing a CAR can be included in the kit.

The kit can include a container and a label or package insert on or associated with the container. Suitable containers include, for example, bottles, vials, syringes, etc. The containers may be formed from a variety of materials such as glass or plastic. The container typically holds a composition including one or more of the disclosed antibodies, antigen binding fragments, conjugates, nucleic acid molecules, CARs or T cells expressing a CAR. In several embodiments the container may have a sterile access port (for example the container may be an intravenous solution bag or a vial having a stopper pierceable by a hypodermic injection needle). A label or package insert indicates that the composition is used for treating the particular condition.

The label or package insert typically will further include instructions for use of a disclosed antibodies, antigen binding fragments, conjugates, nucleic acid molecules, CARs or T cells expressing a CAR, for example, in a method of treating or preventing a tumor or of making a CAR T cell. The package insert typically includes instructions customarily included in commercial packages of therapeutic products that contain information about the indications, usage, dosage, administration, contraindications and/or warnings concerning the use of such therapeutic products. The instructional materials may be written, in an electronic form (such as a computer diskette or compact disk) or may be visual (such as video files). The kits may also include additional components to facilitate the particular application for which the kit is designed. Thus, for example, the kit may additionally contain means of detecting a label (such as enzyme substrates for enzymatic labels, filter sets to detect fluorescent labels, appropriate secondary labels such as a secondary antibody, or the like). The kits may additionally include buffers and other reagents routinely used for the practice of a particular method. Such kits and appropriate contents are well known to those of skill in the art.

### EXAMPLES

The following examples are provided to illustrate particular features of certain embodiments, but the scope of the claims should not be limited to those features exemplified.

### Example 1

### Targeting Cell-Surface ALK with CARs Derived from Anti-ALK Antibodies

The identification of unique or over-expressed cell-surface proteins on tumor cells that are absent on normal tissues, has been challenging for pediatric malignancies. The cell surface tyrosine kinase ALK (CD246, anaplastic lymphoma kinase) is unique in that it is expressed in native, mutated, or over-expressed forms on the plasma membrane surface of neuroblastoma. Antibodies that bind to ALK were identified, their variable regions sequenced, and used to construct chimeric antigen receptors (CARs). The ability to successfully transduce primary T lymphocytes with retroviral gene vectors expressing a series of ALK-specific CARs is disclosed below. T lymphocytes transduced with ALK-specific CARs were demonstrated to mediate cytolytic activity against ALK-expressing tumors as well as to produce cytokines. In exploring different iterations of CAR protein domain structure it was found that the antibody-derived ALK binding sequences were quite robust. The synthetic scFv domains created from the heavy and light variable domain sequences of immunoglobulin could be interchanged with respect to their orientation in the context of CAR tertiary protein structure. Moreover, ALK-specific scFv functioned whether expressed in a short format (as a single domain proximal to the T cell membrane) or in long format (extended away from the plasma membrane using an IgG1-derived spacer domain composed of CH2 and CH3). ALK-specific CARs may serve as a new means to treat pediatric solid tumors.

### Introduction:

The developmentally-regulated cell surface receptor tyrosine kinase ALK (anaplastic lymphoma kinase) is expressed as a tumor-associated antigen in either a full-length from or as a fusion protein resulting from a chromosomal translocation. Full-length ALK plays a role in mesodermal differentiation in *drosophila,* eye development in *c*. *elegans,* neural-crest derived iridiphore development in zebrafish, and in the developing nervous system in mammals (Palmer et al., (2009) Biochem J, 420, 345-361). Cancer-associated ALK was first described as a 2;5 chromosomal translocation associated with nucleophosmin (NPM) in anaplastic large cell leukemia (ALCL; Morris et al., (1994) Science, 263, 1281-1284). The fusion protein was composed of the intracellular domain of NPM and the intracellular kinase domain of ALK. ALK is expressed on neuroblastoma as an intact protein, an amplified protein, or as a mutated protein that continues to signal through its kinase domain. ALK can also be found as a fusion partner to EML4 in up 9% of non-small cell lung carcinomas (NSCLC; Sasaki et al., (2010) Eur J Cancer, 46, 1773-1780). The range of ALK-associated translocations has been recently reviewed (Chiarle et al., (2008) Nat Rev Cancer, 8, 11-23). Activating mutations of full-length ALK are found in 8-12% of primary neuroblastoma cases (Mosse et al., (2008) Nature, 455, 930-935; George et al., (2008) Nature, 455, 975-9785). Importantly, ALK has also been show to account for the long sought after "second-hit" in familial neuroblastoma using whole genome scans of patient pedigrees (Mosse et al., (2008) Nature, 455, 930-935).

Chimeric antigen receptors are an example of synthetic biology, wherein a protein not encoded by the genome, is designed in the laboratory and is expressed in normal human tissues for a therapeutic effect by adoptive immunotherapy of cancer. When Eschar et al., demonstrated that mature human T lymphocytes from the peripheral blood could be activated by synthetic chimeric antigen receptor molecules, the era of designer cytotoxic T lymphocytes began (Eshhar et al., (1990) Br J Cancer Suppl, 10, 27-29). There are a number of CAR constricts in clinical trials, but most of the activity has been in hematologic malignancies, most notably in B cell leukemias (Lee et al., (2012) Clin Cancer Res, 18, 2780-2790; Sadelain et al., (2013) Cancer Discov, 3, 388-398). This is due in part to the acceptable safety profile of B cell antigen-specific CAR-modified T cells. Elimination of B cell leukemia by infused CAR-modified T cells that target B cell antigens is also been accompanied by a subsequent B cell aplasia. More caution is warranted in targeting solid tumors, in that cross-reactive tissue antigen reactivity may have a much more severe outcome.

Chimeric antigen receptors are composed of an extracellular binding domain, spacer domain, transmembrane domains, and intracellular T cell signaling domains (Long et al., (2013) *Oncoimmunology, 2,* e23621). In addition to these possible variations in structural design elements, how these elements are linked to one another by joining domains introduced another level of variability. The impact of including or not including an IgG derived spacer domain, the constant heavy chain regions 2 and 3 (CH2HC3), was explored. Initial inclusion of this domain allows for rapid assessment of T cell transduction of CAR retroviral expression vectors, as not all CARs bind to protein L. CARs are classified according to the number of signaling domains they encode. First-generation CARs include only the CD3 zeta chain-derived cytoplasmic signaling domain. Second generation CARs include CD28 or CD137-derived signaling domains. Third generation CARs encode three signaling domains and may also include sequences derived from CD137, OX40, or GITR. An overarching rule for the assemble of CAR domains into a functional chimeric receptor has yet to be developed, and thus, starting with hybridomas encoding ALK-specific antibodies, the variable regions were sequenced, synthetic scFv domains designed, and linked to CAR structural domains to create a series of CARs specific for ALK. It was found that these synthetic scFv domain were functional with or without CH2CH3 spacers, and that the order of the heavy and light chains were interchangeable in the construct tested. The cytolytic activity of anti-ALK CAR-modified T cells was encouraging and in vivo analysis of CAR activity is currently underway. ALK-specific CARs have the potential to enter the clinical arena as a new generation of adoptive immunotherapeutic approaches for pediatric solid tumors.

### Materials and Methods

*Cell Lines and Antibodies.* Cell lines used in these studies were: Sy5Y, LAN5, K562, Rh18, IMR32, KCNR. Cells were cultured in RPMI-1640 supplemented with 2 mM lgln, 10 mM HEPES, Pen/Strep, (Gibco, Life Technologies, Grand Island, NY) and 10% FBS (Omega Scientific, Tarzana, CA). Anti-ALK antibodies were produced as described previously: ALK15A (IgG2b, weak agonist, membrane proximal binding), ALK48B (IgG2a, agonist, membrane proximal binding), ALK53A, ALK58A (binds to a membrane distal epitope) (Mazot et al., Oncogene, 30, 2017-2025, 2011; Moog-Lutz et al., J Biol. Chem., 280, 26039-26048, 2005). Staining for ALK expression on tumor lines was performed as follows: 2.5µL primary antibody (1 mg/ml conc.) per 1 million cells was added to cells on ice in FACS buffer, FB, (PBS, 0.5%BSA, 0.02% NaN3) for 20 minutes, cells were washed twice, then stained with 5µL FITC-F(ab')2 Fragment Goat Anti-Mouse IgG + IgM (H+L) secondary antibody (Jackson ImmunoResearch, 1 mg/ml) for 20 minutes, washed, then analyzed by flow cytometry.

CAR expression on transduced T cells was also measured by flow cytometry. CARs containing an IgG1 CH2CH3 domain were detected with PE-F(ab') 2 goat antihuman Fc fragment-specific antibody (Jackson ImmunoResearch, Inc., West Grove, PA), 0.5 ug per million cells in FB, stained for 20 minutes on ice. All CAR structural formats could be stained with protein L, as previously described (Zheng et al., (2012) J Transl Med, 10, 29). 500 ng Biotin-Protein L (Thermo Fisher) per million cells in FACS buffer were incubated on ice for 20 min., washed twice, then stained with 250 ng SA-PE (BD Biosciences), 10 minutes in ice. All incubation were in a 0.1 ml volume.

*CAR construct synthesis and vector production.* To sequence the variable regions of the heavy and light chain, PCR primers were used to amplify hybridoma cell line derived cDNA. PCR primer sequences specific for murine IgG were from Kettleborough, *et al*. (Kettleborough et al., (1993) Eur J Immunol, 23, 206-211). Frozen hybridoma cell pellets were resuspended in RLY buffer, passed over QIAShredder columns, and total RNA isolated on RNAeasy spin columns (Qiagen GmbH, Hilden, Germany) as per manufacturer's protocol. PCR products were coned into a TopoTA vector (Invitrogen), and 10 independent bacterial colonies DNA sequenced. Sequences were aligned and once consensus was reached, cognate chains were linked with a (GGGGS)x3 sequence. ALK scFv encoding plasmids were sequence optimized and synthesized by DNA2.0 (Menlo Park, CA). Engineered restriction sites were used to introduce scFv sequences into retroviral expression vectors that included various structural elements as described previously (Haso et al., (2013) Blood, 121, 1165-1174.

*T Cell Activation and Transduction.* De-identified PBMC were obtained from the NIH Clinical Center, Department of Transfusion Medicine, under approved protocol. PBMC were activated by culture for 3 days in the presence of 40 U/ml IL-2 and anti- CD3/CD28 beads (Dynabeads, Human T-Activator CD3/CD28, Life Technologies, Grand Island, NY) in AIM-V media (Life Technologies) supplemented with 2 mM lgln, 10 mM HEPES, Pen/Strep, (Gibco, Life Technologies, Grand Island, NY) and 5% FBS (Omega Scientific, Tarzana, CA). Cells exposed to RV containing supernatants on day 3 and 4 in media containing 300 U/ml rIL-2, beads magnetically removed on day 5, and cells were expanded for 5 more days in media containing 300 U/ml IL-2, then analyzed for transduction.

*T Cell Functional Assays*. T cell cytolytic function was assayed in standard 51 Crrelease assays as described elsewhere (Haso et al., (2013) Blood, 121, 1165-1174). Cytokine release was assayed by co-incubating 25,000 T cells with 25,000 tumor cell targets in cRPMI. At 24 hours culture media was collected and cytokines measured (Human TH1/TH2 multiplex, Meso Scale Discovery, Rockville, MD).

*Xenograft Assays.* NSG mice were injected s.c. on day 0 with 5x10⁶ human neuroblastoma cells (SY5Y *in vivo* passaged and re-cultured as a new line, SY5Y.P1). On day 6, 5.1x10⁶ ALK48-28z CAR(+)ve T cells were injected r.o. (34% positive/15 million total). CAR-treated mice were given a cocktail of (0.9 µg IL-7 + 6.6 µg M25 anti-IL7 mAb) i.p. every 2-3 days, for the time period indicated above. Survival statistics were calculated using Log-rank (Mantel-Cox) analysis (Prism Software, Inc.). On day 13, 3.2x10⁶ ALK48SH-trasnduced T cells were given. The experiment was ended on day 64 due to graft vs host disease-like symptoms, standardly encountered in the NSG model system.

### Results

*Expression of ALK on Tumor Cell Lines.* As reported previously, ALK MAbs 7, 48, 53, and 58 were able to detected ALK expression on tumor cell lines by flow cytometry. The ALK-specific MAb was used to define the expression of ALK on a series of neuroblastoma call lines, and a rhabdomyosarcoma cell line. FIG. 1 illustrates that the neuroblastoma cell lines SY5Y, LAN5, KCNR, IMR32, all express cell surface ALK, as does the rhabdomyosarcoma cell line Rh18. The differences in expression were quite varied with the SY5Y staining the brightest for ALK expression. This was true when the other MAbs specific for ALK were tested as well.

*Creation of ALK CAR.* CAR expression on activated primary human T lymphocytes can be efficiently induced by transduction with retroviral gene vectors. A series of CAR expression vectors was constructed in order to test if the heavy and light immunoglobulin chains of anti-ALK monoclonal antibodies could be used to construct synthetic scFv, and then to link these synthetic scFV to CAR structural and T cell signaling domains, FIG. 2. In some constructs a CH2CH3 structural domain was included. This domain extends the scFV away from the plasma membrane extracellular surface, and also allows for the efficient detection of transduced T cells with anti-IgG Fc-specific antibody. "SH" (or "short") indicates that the extracellular domain of the CAR does not include a CH2CH3 spacer domain.

*Expression of ALK CAR on transduced T cells.* Transduced PBMC were analyzed for the CAR expression by flow cytometry. PBMC were activated with OKT3/CD28 beads in the presence of IL-2 low lever IL-2, exposed to retroviral vector (RV)-containing supernatant for two consecutive days and then expand for 5-6 more days in high level (300 u/ml) IL-2. Cells were then assessed for CAR expression by direct staining with anti-Fc antibody if the construct contained a CH2CH3 domain, or by indirect staining with protein L, FIG. 3. All ALK-specific CARs generated were able to bind protein L.

The ALK48 and ALK58-derived CARs showed the best overall cell surface expression levels, either in the long or short formats. The order of the heavy and light chains was switched in a short CAR format for ALK58. No difference in expression level at the surface was seen when the order of the heavy and light variable domains were switched in the synthetic CAR construct, FIG. 3F. Also, no significant differences were seen in expression level of the short versus the long CAR for either clone 58 or 48, indicating that the scFv domain itself may drive overall expression levels. This was certainly true for the ALK53 CAR, which showed only 9% transduction, although it was generated under identical conditions, and included the exact same structural domains as ALK48 and ALK58.

*Lytic Activity of ALK CARs.* The in vitro biological activity of anti-ALK CARs was assessed using 51Cr-release assays for cytolysis. Tumor cell targets were radiolabeled and then incubated with CAR-transduced T cells. Both the neuroblastoma cell line LAN5 and the rhabdomyosarcoma cell line Rh18 were lysed by all three CAR constructs tested in the CH2CH3-containing format, FIG. 4. The control cell leukemia cell line K562, which is not ALK positive and is included as a control for NK cell activity, was not lysed. The effector to target ratio was normalized such that the E:T target ratio reflected the number of CAR positive T cells in the assay. The strong lytic activity of ALK53 at lower E:T ratios is intriguing, but higher E:T ratios could not be tested due to very low levels of transduction. As with analysis of expression level, the shorter format ALK CAR constructs were equally able to lyse tumor lines, FIG. 5. Even when the heavy and light chains were reversed in order, ALK59LH, efficient lysis was seen, FIG. 5B.

*Cytokine Data.* T cells transduced with ALK48, ALK53, and ALK58 were also tested for the production of cytokine upon 24-hour co-culture with tumor cell lines, FIG. 6. Strong IFN-gamma production was noted with lower levels of IL-2 and IFNalpha. The ability to produce interferon as well as the ability to lyse tumor cell lines is a strong indicator that the synthetically constructed scFv are active as CARs upon interaction with tumor cell lines expressing cell-surface ALK.

*Xenograft Data.* NOD *scid* gamma (NSG) mice were inoculated with ALK+ human neuroblastoma cells (SY5Y cells). On day 6, the mice were treated with human T cells transduced to express the murine ALK specific ALK48-28z CAR (SEQ ID NO: 49, long form CAR with CH2CH3 spacer domain) or mock treated T cells. The ALK48-28z vector-transduced T cells showed significant disease control (p<0.005) when compared to the control. In a separate set of mice, on day 13 post inoculation (and without any treatment on day 6), the mice were treated with ALK48SH-28z (SEQ ID NO: 50, short form CAR without spacer domain) transduced T cells. In these mice, there was no effect on survival, likely due to the delayed time of infusion and low numbers.

The mice also received biweekly injections of IL-7 complexed to anti-IL7 antibody to promote T cell persistence. The experiment was terminated at day 66 due to graft-versus host disease arising in the ALK48L-28z treated mice, none of which were sacrificed due to tumor growth. The other two groups all died of tumor. The onset on graft-versus host disease is commonly seen in xenograft models with human T cells, and may be exacerbated by the addition of IL-7 to the system.

### Discussion

The extracellular aspect of the ALK tyrosine kinase provides a ready target for the adoptive immunotherapy of cancer with chimeric antigen receptor (CAR)-modified T cells. In glioblastoma stem cell lines, ALK was shown to be a critical factor for both self-renewal and tumorigenic capacity (Koyama-Nasu et al., (2013) Oncogene, May 20). ALK expression induced gene expression signatures similar to those seen in both ESC and myc-driven signaling pathways. Moreover overexpression of pleiotrophin (an ALK ligand) was show to be driven by SOX2 expression in cancer stem cells, and thus may serve as an autocrine stimulatory factor. In rhabdomyosarcoma, frequent ALK expression as well as ALK amplification has been detected at the genomic level (Nishimura et al., (2013) Cancer Sci, 104, 856-864*).* Interestingly, that ability of patients to produce antibodies to ALK may correlate with better outcomes in ALCL (Ait-Tahar et al., (2010) Blood, 115, 3314-3319). If this is a measure of a patient's general immune capacity, or a specific ALK-targeted effect is not clear, but it may indicate that in postnatal individuals ALK can be safely targeted by antibody. In rat studies, ALK expression appears to peak just at birth in the dorsal root ganglia (DRG), and to recede thereafter (Chiarle et al., (2008) Nat Rev Cancer, 8, 11-23, Degoutin et al., (2009) Eur J Neurosci, 29, 275-286). ALK is expressed on a subset of neurons in the DRG that co-express TrkA and ret. In a detailed analysis of embryonic development in mice, ALK expression is seen in numerous tissues, but near term becomes progressively restricted to the CNS (Vernersson et al., (2006) Gene Expr Patterns, 6, 448-461).

In 2005, Moog-Lutz, et al., described the production of monoclonal antibodies (mAb) against the extracellular domain of ALK (Moog-Lutz et al., J Biol. Chem., 280, 26039-26048, 2005). These studies led to the description of ALK as both a 220 kD and 140 kDa cleaved form at the cell surface, and two of the antibodies created bound with nM affinity and were able to induce differentiation and activation of both PC12 and HEK293 cells transfected with ALK. These mAbs are the basis of the CARs reported here. The two clones worked with most were derived from antibody 58 which binds to only the 220 KDa form of ALK, and antibody 48, which binds to both. From this data it can be inferred clone 48 binds closer to the cell surface membrane, while clone 58 binds to the more distal cleaved region (Moog-Lutz et al., (2012) PLoS One, 7, e33581). Both mAb 48 and 58 induce phosphorylation of the receptor upon binding, and are thus considered to be activating (Mazot et al., (2011) Oncogene, 30, 2017-2025).

The description of point mutations in ALK has led to detailed analysis of the intracellular versus intracellular residence of the protein. In general, mutations in ALK appear to increase the number of intracellular ALK molecules. However, even wild-type ALK can be detected both within and on the surface of neuroblastoma cell lines (Mazot, et al., (2012) PLoS One, 7, e3358121).

The binding moiety of CARs can be derived from a B-cell derived scFv expression libraries, or can be assembled synthetically from monoclonal antibody producing hybridoma cDNA. A number of CARs specific for ALK were generated using hybridoma derived cDNA as the starting material, FIG. 2. The expression levels of these CARs on transduced T cells varied widely. In some constructs a distinct and bright population was seen, as in ALK58, in others a weaker and less distinct expression was seen, as in ALK48, while in others, T cells with high levels of transduction were not generated at all, as in ALK53 (FIG. 2). This indicates that the synthetic scFv sequences themselves have a bearing on the expression level of the CAR. In the case of the hybridoma-derived CARs, it was demonstrated that the addition of the CH2CH3 domain did not affect lytic activity, FIG. 5. In fact all the CARs tested had strong levels of cytolysis against tumor cell lines, as well as cytokine production once data was normalized for transduction efficiency, FIGs. 4 and 6. In conclusion, a series of highly active CARs was generated that may serve as a platform for the pre-clinical testing of ALK specific adoptive immunotherapy of malignancies expressing cell-surface ALK.

### Example 2

### Exemplary CAR Sequences

This example illustrated exemplary amino acid and nucleic acid sequences of ALK-specific CARs.

All the sequences have an N-terminal signal peptide (SP; SEQ ID NO: 26). All the sequences have a scFv sequence, a transmembrane (TM) sequence and a CD3 zeta signaling sequence. Sequences that refer to "SH" *do not* have a CH2CH3 spacer domain.
*For the CAR extracellular domain, the following nomenclature is used:*
"short" CAR extracellular domain, without a CH2CH3 spacer

| | |
|---|---|
| **ALK15SH** | SP - murine ALK15 scFv |
| **ALK48SH** | SP - murine ALK48 scFv |
| **ALK5SH** | SP - murine ALK53 scFv |
| **ALK58SH** | SP - murine ALK58 scFv |
| **hALK15SH** | SP - humanized ALK15 scFv |
| **hALK48SH** | SP - humanized ALK48 scFv |
| **hALK53SH** | SP - humanized ALK53 scFv |
| **hALK58SH** | SP - humanized ALK58 scFv |

"long" CAR extracellular domain, with a CH2CH3 spacer

| | |
|---|---|
| **ALK15** | SP - murine ALK15 scFv - CH2CH3 spacer |
| **ALK48** | SP - murine ALK48 scFv - CH2CH3 spacer |
| **ALK53** | SP - murine ALK53 scFv - CH2CH3 spacer |
| **ALK58** | SP - murine ALK58 scFv - CH2CH3 spacer |
| **hALK15** | SP - humanized ALK15 scFv - CH2CH3 spacer |
| **hALK48** | SP - humanized ALK48 scFv - CH2CH3 spacer |
| **hALK53** | SP - humanized ALK53 scFv - CH2CH3 spacer |
| **hALK58** | SP - humanized ALK58 scFv - CH2CH3 spacer |

*For the CAR transmembrane domain and intracellular domains, the following nomenclature is used:*

| | |
|---|---|
| **28z** | CD28 transmembrane - CD28 signaling - CD3 zeta signaling |
| **BBz** | CD8 transmembrane - 4-1BB/CD137 signaling - CD3 zeta signaling |
| **28BBz** | CD8 transmembrane - CD28 signaling - 4-1BB/CD137 signaling - CD3 zeta signaling |

**ALK15-28z (SEQ ID NO: 43):**
**ALK15SH-28z (SEQ ID NO: 44):**
**ALK15-BBz (SEQ ID NO: 45):**
**ALK15SH-BBz (SEQ ID NO: 46):**
**ALK15-28BBz (SEQ ID NO: 47):**
**ALK15SH-28BBz (SEQ ID NO: 48):**
**ALK48-28z (SEQ ID NO: 49):**
**ALK48SH-28z (SEQ ID NO: 50):**
**ALK48-BBz (SEQ ID NO: 51):**
**ALK48SH-BBz (SEQ ID NO: 52):**
**ALK48-28BBz (SEQ ID NO: 53):**
**ALK48SH-28BBz (SEQ ID NO: 54):**
**ALK53-28z (SEQ ID NO: 55):**
**ALK53SH-28z (SEQ ID NO: 56):**
**ALK53-BBz (SEQ ID NO: 57):**
**ALK53SH-BBz (SEQ ID NO: 58):**
**ALK53-28BBz (SEQ ID NO: 59):**
**ALK53SH-28BBz (SEQ ID NO: 60):**
**ALK58-28z (SEQ ID NO: 61):**
**ALK58SH-28z (SEQ ID NO: 62):**
**ALK58-BBz (SEQ ID NO: 63):**
**ALK58SH-BBz (SEQ ID NO: 64):**
**ALK58-28BBz (SEQ ID NO: 65):**
**ALK58SH-28BBz (SEQ ID NO: 66):**
**hALK15-28z (SEQ ID NO: 67):**
**hALK15SH-28z (SEQ ID NO: 68):**
**hALK15-BBz (SEQ ID NO: 69):**
**hALK15SH-BBz (SEQ ID NO: 70):**
**hALK15-28BBz (SEQ ID NO: 71):**
**hALK15SH-28BBz (SEQ ID NO: 72):**
**hALK48-28z (SEQ ID NO: 73):**
**hALK48SH-28z (SEQ ID NO: 74):**
**hALK48-BBz (SEQ ID NO: 75):**
**hALK48SH-BBz (SEQ ID NO: 76):**
**hALK48-28BBz (SEQ ID NO: 77):**
**hALK48SH-28BBz (SEQ ID NO: 78):**
**hALK53-28z (SEQ ID NO: 79):**
**hALK53SH-28z (SEQ ID NO: 80):**
**hALK53-BBz (SEQ ID NO: 81):**
**hALK53SH-BBz (SEQ ID NO: 82):**
**hALK53-28BBz (SEQ ID NO: 83):**
**hALK53SH-28BBz (SEQ ID NO: 84):**
**hALK58-28z (SEQ ID NO: 85):**
**hALK58SH-28z (SEQ ID NO: 86):**
**hALK58-BBz (SEQ ID NO: 87):**
**hALK58SH-BBz (SEQ ID NO: 88):**
**hALK58-28BBz (SEQ ID NO: 89):**
**hALK58SH-28BBz (SEQ ID NO: 90):**

### Exemplary DNA Sequences of CARs

**ALK15-28z (SEQ ID NO: 91):**
**ALK15SH-28z (SEQ ID NO: 92):**
**ALK15-BBz (SEQ ID NO: 93):**
**ALK15SH-BBz (SEQ ID NO: 94):**
**ALK15-28BBz (SEQ ID NO: 95):**
**ALK15SH-28BBz (SEQ ID NO: 96):**
**ALK48-28z (SEQ ID NO: 97):**
**ALK48SH-28z (SEQ ID NO: 98):**
**ALK48-BBz (SEQ ID NO: 99):**
**ALK48SH-BBz (SEQ ID NO: 100):**
**ALK48-28BBz (SEQ ID NO: 101):**
**ALK48SH-28BBz (SEQ ID NO: 102):**
**ALK53-28z (SEQ ID NO: 103):**
**ALK53SH-28z (SEQ ID NO: 104):**
**ALK53-BBz (SEQ ID NO: 105):**
**ALK53SH-BBz (SEQ ID NO: 106):**
**ALK53-28BBz (SEQ ID NO: 107):**
**ALK53SH-28BBz (SEQ ID NO: 108):**
**ALK58-28z (SEQ ID NO: 109):**
**ALK58SH-28z (SEQ ID NO: 110):**
**ALK58-BBz (SEQ ID NO: 111):**
**ALK58SH-BBz (SEQ ID NO: 112):**
**ALK58-28BBz (SEQ ID NO: 113):**
**ALK58SH-28BBz (SEQ ID NO: 114):**

In view of the many possible embodiments to which the principles of the disclosed embodiments may be applied, it should be recognized that the illustrated embodiments are only preferred examples of the invention and should not be taken as limiting. All that comes within the scope of the following claims is claimed.

## Claims

1. A nucleic acid molecule encoding a chimeric antigen receptor, the chimeric antigen receptor comprising:
an antigen binding domain, a transmembrane domain, and at least one intracellular T-cell signaling domain, wherein the antigen binding domain comprises a heavy chain variable region and a light chain variable region comprising:
a H-CDR1, a H-CDR2, and a H-CDR3 of the heavy chain variable region sequence set forth as SEQ ID NO: 3, and a L-CDR1, a L-CDR2, and a L-CDR3 of the light chain variable region sequence set forth as SEQ ID NO: 4;
and wherein the chimeric antigen receptor specifically binds to an extracellular domain of anaplastic lymphoma kinase,
wherein the H-CDR1, H-CDR2, and H-CDR3 comprise amino acids 26-33, 51-58, and 96-110 of SEQ ID NO: 3, respectively, and the L-CDR1, L-CDR2, and L-CDR3 comprise amino acids 27-36, 54-56, and 92-102 of SEQ ID NO: 4, respectively, as identified by the IMGT numbering scheme, or
the H-CDR1, H-CDR2, and H-CDR3 comprise amino acids 31-35, 50-66, and 99-109 of SEQ ID NO: 3, respectively, and the L-CDR1, L-CDR2, and L-CDR3 comprise amino acids 24-38, 54-60, and 93-101 of SEQ ID NO: 4, respectively, as identified by the Kabat numbering scheme.

2. The nucleic acid molecule of claim 1, wherein:
(a) the heavy chain variable region comprises or consists of the amino acid sequence set forth as SEQ ID NO: 3 or SEQ ID NO: 11; or
(b) the light chain variable region comprises or consists of the amino acid sequence set forth as SEQ ID NO: 4 or SEQ ID NO: 12.

3. The nucleic acid molecule of any one of the preceding claims, wherein the heavy and light chain variable regions comprise or consist of the amino acid sequences set forth as:
(a) SEQ ID NO: 3 and SEQ ID NO: 4, respectively; or
(b) SEQ ID NO: 11 and SEQ ID NO: 12, respectively.

4. The nucleic acid molecule of any one of the preceding claims, wherein the antigen binding domain is a scFv,
particularly wherein a) the scFv comprises or consists of the amino acid sequence set forth as SEQ ID NO: 18, or SEQ ID NO: 22, and/or b) wherein the transmembrane domain comprises or consists of the amino acid sequence set forth as SEQ ID NO: 27 or SEQ ID NO: 30.

5. The nucleic acid molecule of any of the preceding claims, wherein the T-cell signaling domain comprises or consists of a CD3 zeta signaling domain, and/or
wherein the chimeric antigen receptor comprises
(a) a CD8 signaling domain;
(b) a CD28 signaling domain;
(c) a CD27 signaling domain
(d) a CD154 signaling domain
(e) a GITR (TNFRSF18) signaling domain
(f) a OX40 (CD134) signaling domain;
(g) a CD137 (4-1BB) signaling domain; or
(h) a combination of two or more of (a) -(g).

6. The nucleic acid molecule of any one of the preceding claims, wherein the at least one T-cell signaling domain comprises, from N-terminus to C-terminus,
(a) a CD3 zeta signaling domain;
(b) a CD28 signaling domain and a CD3 zeta signaling domain;
(c) a CD137 (4-1BB) signaling domain and a CD3 zeta signaling domain;
(d) an OX40 signaling domain and a CD3 zeta signaling domain;
(e) a CD28 signaling domain, a CD137 (4-1BB) signaling domain, and a CD3 zeta signaling domain; or
(f) a CD28 signaling domain, an OX40 (CD134) signaling domain, and a CD3 zeta signaling domain.

7. The nucleic acid molecule of claim 6 wherein
(a) the CD3 zeta signaling domain comprises or consists of the amino acid sequence set forth as SEQ ID NO: 34
(b) the CD8 signaling domain comprises or consists of the amino acid sequence set forth as SEQ ID NO: 31
(c) the CD28 signaling domain comprises or consists of the amino acid sequence set forth as SEQ ID NO: 28; or
(d) the CD137 signaling domain comprises or consists of the amino acid sequence set forth as SEQ ID NO: 32 or SEQ ID NO: 33.

8. The nucleic acid molecule of any of the preceding claims, wherein the transmembrane domain comprises or consists of the amino acid sequence set forth as SEQ ID NO: 27, or
wherein the transmembrane domain comprises or consists of the amino acid sequence set forth as SEQ ID NO: 30.

9. The nucleic acid molecule of any of the preceding claims, wherein the chimeric antigen receptor comprises, from N-terminus to C-terminus, the antigen binding domain, the transmembrane domain, and the at least one intracellular T-cell signaling domain; and/or
wherein the chimeric antigen receptor further comprises a spacer domain C-terminal to the antigen binding domain and N-terminal to the transmembrane domain,
particularly wherein the spacer domain comprises an immunoglobulin domain, optionally wherein the immunoglobulin domain comprises a CH2CH3 domain,
particularly wherein the immunoglobulin domain comprises the amino acid sequence set forth as SEQ ID NO: 35; and/or
wherein the chimeric antigen receptor further comprises a signal peptide N-terminal to the antigen binding domain.

10. The nucleic acid molecule of claim 1, wherein the chimeric antigen receptor comprises or consists of the amino acid sequence set forth as SEQ ID NO: 49, SEQ ID NO: 50, SEQ ID NO: 51, SEQ ID NO: 52, SEQ ID NO: 53, SEQ ID NO: 54, SEQ ID NO: 73, SEQ ID NO: 74, SEQ ID NO: 75, SEQ ID NO: 76, SEQ ID NO: 77, or SEQ ID NO: 78, or comprises or consists of the nucleic acid sequence set forth as SEQ ID NO: 97, SEQ ID NO: 98, SEQ ID NO: 99, SEQ ID NO: 100, SEQ ID NO: 101, or SEQ ID NO: 102.

11. The nucleic acid molecule of any of the preceding claims, codon optimized for expression in a human T cell, and/or operably linked to an expression control sequence.

12. A vector comprising the nucleic acid molecule of any one of the preceding claims , particularly wherein the vector is a recombinant DNA expression vector, or
wherein the vector is a viral vector,
particularly wherein the viral vector is a lentiviral vector,
particularly wherein the vector is for use in making a chimeric antigen receptor T-cell.

13. A polypeptide comprising the chimeric antigen receptor encoded by the nucleic acid molecule of any one of the preceding claims.

14. A host cell, comprising the nucleic acid molecule or vector of any one of claims 1-12, particularly wherein the host cell is a T cell.

15. A composition, comprising a pharmaceutically effective amount of the nucleic acid molecule, vector, polypeptide, or host cell of any one of the preceding claims, and a pharmaceutically acceptable carrier.

16. A method of making a chimeric antigen receptor T-cell comprising:
transducing a T cell with the vector of claim 12, thereby making the chimeric antigen receptor T cell.

17. A chimeric antigen receptor encoded by the nucleic acid molecule of any of claims 1-11 for use in a method of treating a subject with a tumor, wherein the tumor comprises cell surface expression of anaplastic lymphoma kinase, the method comprising:
administering to the subject a therapeutically effective amount of T-cells expressing the chimeric antigen receptor, under conditions sufficient to form an immune complex of the antigen binding domain on the chimeric antigen receptor and the extracellular domain of anaplastic lymphoma kinase in the subject,
particularly wherein the T-cells are T cells from the subject that have been transformed with the nucleic acid molecule of any one of claims 1-11 encoding the chimeric antigen receptor or transduced with a vector comprising the nucleic acid molecule.

18. The chimeric antigen receptor for use of claim 17, wherein the method further comprises the steps of:
obtaining the T cells from the subject, and
transforming the T cells with the nucleic acid molecule encoding the chimeric antigen receptor or transducing the T cells with a vector comprising the nucleic acid molecule, and/or
wherein the tumor does not comprise an anaplastic lymphoma kinase fusion protein, and/or where in the method further comprises administering to the subject a therapeutically effective amount of a chemotherapeutic agent, particularly
wherein the chemotherapeutic agent comprises an anaplastic lymphoma kinase inhibitor, particularly wherein the anaplastic lymphoma kinase inhibitor comprises crizotinib, and/or
wherein treating the tumor comprises a reduction in tumor burden.

19. The chimeric antigen receptor for use of claim 17 or 18, wherein the tumor is a neuroblastoma, a rhabdomyosarcoma, or a glioblastoma.

20. The chimeric antigen receptor for use of claim 17, wherein the method further comprises selecting the subject by detecting cell-surface expression of anaplastic lymphoma kinase on the tumor.

21. A kit for making a chimeric antigen receptor T-cell or treating a tumor in a subject wherein the tumor comprises cell surface expression of anaplastic lymphoma kinase, comprising a container comprising the nucleic acid molecule, vector, host cell, or composition of any of claims 1-12, 14 or 15, and instructions for using the kit.

22. The nucleic acid molecule, vector, host cell, or composition of any of claims 1,-12, 14 or 15, for use in treating a tumor in a subject, wherein the tumor comprises cell surface expression of anaplastic lymphoma kinase, wherein the host cell is a T-cell.

23. Use of the nucleic acid molecule or vector of any of claims 1-12, to make a chimeric antigen receptor T cell.

## Patentansprüche

1. Nukleinsäuremolekül, kodierend für einen chimären Antigenrezeptor, wobei der chimäre Antigenrezeptor Folgendes umfasst:
eine Antigenbindungsdomäne, eine Transmembrandomäne und mindestens eine intrazelluläre T-Zell-Signalisierungsdomäne, wobei die Antigenbindungsdomäne eine variable Region der schweren Kette und eine variable Region der leichten Kette umfasst, die umfassen:
eine H-CDR1, eine H-CDR2 und eine H-CDR3 der Sequenz der variablen Region der schweren Kette, dargelegt als SEQ ID NO: 3, und eine L-CDR1, eine L-CDR2 und eine L-CDR3 der Sequenz der variablen Region der leichten Kette, dargelegt als SEQ ID NO: 4;
und wobei der chimäre Antigenrezeptor spezifisch an eine extrazelluläre Domäne der anaplastischen Lymphomkinase bindet, wobei die H-CDR1, H-CDR2 und H-CDR3 die Aminosäuren 26-33, 51-58 bzw. 96-110 von SEQ ID NO: 3 umfassen und die L-CDR1, L-CDR2 und L-CDR3 die Aminosäuren 27-36, 54-56 bzw. 92-102 von SEQ ID NO: 4 umfassen, wie durch das IMGT-Nummerierungsschema identifiziert, oder
wobei die H-CDR1, H-CDR2 und H-CDR3 die Aminosäuren 31-35, 50-66 bzw. 99-109 von SEQ ID NO: 3 umfassen und die L-CDR1, L-CDR2 und L-CDR3 die Aminosäuren 24-38, 54-60 bzw. 93-101 von SEQ ID NO: 4 umfassen, wie durch das Kabat-Nummerierungsschema identifiziert.

2. Nukleinsäuremolekül nach Anspruch 1, wobei:
(a) die variable Region der schweren Kette die als SEQ ID NO: 3 oder SEQ ID NO: 11 dargelegte Aminosäuresequenz umfasst oder aus ihr besteht; oder
(b) die variable Region der leichten Kette die als SEQ ID NO: 4 oder SEQ ID NO: 12 dargelegte Aminosäuresequenz umfasst oder aus ihr besteht.

3. Nukleinsäuremolekül nach einem der vorhergehenden Ansprüche, wobei die variablen Regionen der schweren und leichten Kette die wie folgt dargelegten Aminosäuresequenzen umfassen oder aus ihnen bestehen:
(a) SEQ ID NO: 3 bzw. SEQ ID NO 4; oder
(b) SEQ ID NO: 11 bzw. SEQ ID NO 12.

4. Nukleinsäuremolekül nach einem der vorhergehenden Ansprüche, wobei die Antigenbindungsdomäne ein scFv ist, insbesondere wobei a) das scFv die als SEQ ID NO: 18 oder SEQ ID NO: 22 dargelegte Aminosäuresequenz umfasst oder aus ihr besteht, und/oder b) wobei die Transmembrandomäne die als SEQ ID NO: 27 oder SEQ ID NO: 30 dargestellte Aminosäuresequenz umfasst oder aus ihr besteht.

5. Nukleinsäuremolekül nach einem der vorhergehenden Ansprüche, wobei die T-Zell-Signalisierungsdomäne eine CD3-Zeta-Signalisierungsdomäne umfasst oder aus ihr besteht, und/oder wobei der chimäre Antigenrezeptor
(a) eine CD8-Signalisierungsdomäne;
(b) eine CD28-Signalisierungsdomäne;
(c) eine CD27-Signalisierungsdomäne;
(d) eine CD154-Signalisierungsdomäne;
(e) eine GITR(TNFRSF18)-Signalisierungsdomäne;
(f) eine OX40 (CD134)-Signalisierungsdomäne;
(g) eine CD137(4-1BB)-Signalisierungsdomäne; oder
(h) eine Kombination aus zwei oder mehr von (a)-(g) umfasst.

6. Nukleinsäuremolekül nach einem der vorhergehenden Ansprüche, wobei die mindestens eine T-Zell-Signalisierungsdomäne vom N-Terminus bis zum C-Terminus,
(a) eine CD3-Zeta-Signalisierungsdomäne;
(b) eine CD28-Signalisierungsdomäne und eine CD3-Zeta-Signalisierungsdomäne;
(c) eine CD137(4-1BB)-Signalisierungsdomäne und eine CD3-Zeta-Signalisierungsdomäne;
(d) eine OX40-Signalisierungsdomäne und eine CD3-Zeta-Signalisierungsdomäne;
(e) eine CD28-Signalisierungsdomäne, eine CD137(4-1BB)-Signalisierungsdomäne und eine CD3-Zeta-Signalisierungsdomäne; oder
(f) eine CD28-Signalisierungsdomäne, eine OX40 (CD134)-Signalisierungsdomäne und eine CD3-Zeta-Signalisierungsdomäne umfasst.

7. Nukleinsäuremolekül nach Anspruch 6, wobei
(a) die CD3-Zeta-Signalisierungsdomäne die als SEQ ID NO: 34 dargelegte Aminosäuresequenz umfasst oder aus ihr besteht
(b) die CD8-Signalisierungsdomäne die als SEQ ID NO: 31 dargelegte Aminosäuresequenz umfasst oder aus ihr besteht
(c) die CD28-Signalisierungsdomäne die als SEQ ID NO: 28 dargelegte Aminosäuresequenz umfasst oder aus ihr besteht; oder
(d) die CD137-Signalisierungsdomäne die als SEQ ID NO: 32 oder SEQ ID NO: 33 dargelegte Aminosäuresequenz umfasst oder aus ihr besteht.

8. Nukleinsäuremolekül nach einem der vorhergehenden Ansprüche, wobei die Transmembrandomäne die als SEQ ID NO: 27 dargelegte Aminosäuresequenz umfasst oder aus ihr besteht; oder
wobei die Transmembrandomäne die als SEQ ID NO: 30 dargelegte Aminosäuresequenz umfasst oder aus ihr besteht.

9. Nukleinsäuremolekül nach einem der vorhergehenden Ansprüche, wobei der chimäre Antigenrezeptor vom N-Terminus bis zum C-Terminus die Antigenbindungsdomäne, die Transmembrandomäne und die mindestens eine intrazelluläre T-Zell-Signaldomäne umfasst; und/oder
wobei der chimäre Antigenrezeptor des Weiteren eine Spacer-Domäne C-terminal zur Antigenbindungsdomäne und N-terminal zur Transmembrandomäne umfasst,
insbesondere wobei die Spacer-Domäne eine Immunglobulindomäne umfasst, gegebenenfalls wobei die Immunglobulindomäne eine CH2CH3-Domäne umfasst,
insbesondere wobei die Immunglobulindomäne die als SEQ ID NO: 35 dargelegte Aminosäuresequenz umfasst; und/oder wobei der chimäre Antigenrezeptor des Weiteren ein Signalpeptid N-terminal zur Antigenbindungsdomäne umfasst.

10. Nukleinsäuremolekül nach Anspruch 1, wobei der chimäre Antigenrezeptor die als SEQ ID NO: 49, SEQ ID NO: 50, SEQ ID NO: 51, SEQ ID NO: 52, SEQ ID NO: 53, SEQ ID NO: 54, SEQ ID NO: 73, SEQ ID NO: 74, SEQ ID NO: 75, SEQ ID NO: 76, SEQ ID NO: 77 oder SEQ ID NO: 78 dargelegte Aminosäuresequenz umfasst oder aus ihr besteht oder die als SEQ ID NO: 97, SEQ ID NO: 98, SEQ ID NO: 99, SEQ ID NO: 100, SEQ ID NO: 101 oder SEQ ID NO: 102 dargelegte Nukleinsäuresequenz umfasst oder daraus besteht.

11. Nukleinsäuremolekül nach einem der vorhergehenden Ansprüche, codonoptimiert für die Expression in einer humanen T-Zelle und/oder funktionell verbunden mit einer Expressionskontrollsequenz.

12. Vektor, umfassend das Nukleinsäuremolekül nach einem der vorhergehenden Ansprüche, insbesondere wobei der Vektor ein rekombinanter DNA-Expressionsvektor ist, oder
wobei der Vektor ein viraler Vektor ist,
insbesondere wobei der virale Vektor ein Lentivirus-Vektor ist, insbesondere wobei der Vektor zur Verwendung zur Herstellung einer chimären Antigenrezeptor-T-Zelle vorgesehen ist.

13. Polypeptid, umfassend den chimären Antigenrezeptor, der durch das Nukleinsäuremolekül nach einem der vorhergehenden Ansprüche kodiert wird.

14. Wirtszelle, umfassend das Nukleinsäuremolekül oder den Vektor nach einem der Ansprüche 1-12, insbesondere wobei die Wirtszelle eine T-Zelle ist.

15. Zusammensetzung, umfassend eine pharmazeutisch wirksame Menge des Nukleinsäuremoleküls, Vektors, Polypeptids oder der Wirtszelle nach einem der vorhergehenden Ansprüche und einen pharmazeutisch unbedenklichen Träger.

16. Verfahren zur Herstellung einer chimären Antigenrezeptor-T-Zelle, umfassend:
Transduzieren einer T-Zelle mit dem Vektor nach Anspruch 12, wodurch die chimäre Antigenrezeptor-T-Zelle hergestellt wird.

17. Chimärer Antigenrezeptor, der durch das Nukleinsäuremolekül nach einem der Ansprüche 1-11 kodiert ist, zur Verwendung in einem Verfahren zum Behandeln eines Subjekts mit einem Tumor, wobei der Tumor die Zelloberflächenexpression von anaplastischer Lymphomkinase umfasst, wobei das Verfahren umfasst:
Verabreichen einer therapeutisch wirksamen Menge von T-Zellen, die den chimären Antigenrezeptor exprimieren, an das Subjekt unter Bedingungen, die ausreichen, um einen Immunkomplex der Antigenbindungsdomäne auf dem chimären Antigenrezeptor und der extrazellulären Domäne der anaplastischen Lymphomkinase in dem Subjekt zu bilden,
insbesondere wobei die T-Zellen T-Zellen von dem Subjekt sind, die mit dem für den chimären Antigenrezeptor kodierenden Nukleinsäuremolekül nach einem der Ansprüche 1-11 transformiert oder mit einem das Nukleinsäuremolekül umfassenden Vektor transduziert wurden.

18. Chimärer Antigenrezeptor zur Verwendung nach Anspruch 17, wobei das Verfahren des Weiteren die folgenden Schritte umfasst:
Erhalten der T-Zellen von dem Subjekt und
Transformieren der T-Zellen mit dem für den chimären Antigenrezeptor kodierenden Nukleinsäuremolekül oder Transduzieren der T-Zellen mit einem das Nukleinsäuremolekül umfassenden Vektor, und/oder
wobei der Tumor kein anaplastisches Lymphomkinase-Fusionsprotein umfasst, und/oder wobei das Verfahren des Weiteren das Verabreichen einer therapeutisch wirksamen Menge eines chemotherapeutischen Mittels an das Subjekt umfasst, insbesondere
wobei das chemotherapeutische Mittel einen Inhibitor der anaplastischen Lymphomkinase umfasst, insbesondere wobei der Inhibitor der anaplastischen Lymphomkinase Crizotinib umfasst, und/oder
wobei das Behandeln des Tumors eine Verringerung der Tumorlast umfasst.

19. Chimärer Antigenrezeptor zur Verwendung nach Anspruch 17 oder 18, wobei der Tumor ein Neuroblastom, ein Rhabdomyosarkom oder ein Glioblastom ist.

20. Chimärer Antigenrezeptor zur Verwendung nach Anspruch 17, wobei das Verfahren des Weiteren das Auswählen des Subjekts durch Nachweisen der Zelloberflächenexpression der anaplastischen Lymphomkinase auf dem Tumor umfasst.

21. Kit zur Herstellung einer chimären Antigenrezeptor-T-Zelle oder zum Behandeln eines Tumors in einem Subjekt, wobei der Tumor die Zelloberflächenexpression einer anaplastischen Lymphomkinase umfasst, umfassend einen Behälter, der das Nukleinsäuremolekül, den Vektor, die Wirtszelle oder die Zusammensetzung nach einem der Ansprüche 1-12, 14 oder 15 umfasst, und Anweisungen zur Verwendung des Kits.

22. Nukleinsäuremolekül, Vektor, Wirtszelle oder Zusammensetzung nach einem der Ansprüche 1-12, 14 oder 15 zur Verwendung beim Behandeln eines Tumors in einem Subjekt, wobei der Tumor die Zelloberflächenexpression einer anaplastischen Lymphomkinase umfasst, wobei die Wirtszelle eine T-Zelle ist.

23. Verwendung des Nukleinsäuremoleküls oder Vektors nach einem der Ansprüche 1-12, um eine chimäre Antigenrezeptor-T-Zelle herzustellen.

## Revendications

1. Molécule d'acide nucléique codant pour un récepteur antigénique chimérique, le récepteur antigénique chimérique comprenant :
un domaine de liaison à l'antigène, un domaine transmembranaire et au moins un domaine de signalisation intracellulaire de lymphocytes T, dans lequel le domaine de liaison à l'antigène comprend une région variable de chaîne lourde et une région variable de chaîne légère comprenant :
une H-CDR1, une H-CDR2 et une H-CDR3 de la séquence de la région variable de chaîne lourde définie par SEQ ID NO:3 et une L-CDR1, une L-CDR2 et une L-CDR3 de la séquence de région variable de chaîne légère définie par SEQ ID NO:4 ;
et dans laquelle le récepteur antigénique chimérique se lie spécifiquement à un domaine extracellulaire de la kinase du lymphome anaplasique,
dans laquelle les H-CDR1, H-CDR2 et H-CDR3 comprennent les acides aminés 26 à 33, 51 à 58 et 96 à 110 de SEQ ID NO:3, respectivement, et les L-CDR1, L-CDR2 et L-CDR3 comprennent les acides aminés 27 à 36, 54 à 56 et 92 à 102 de SEQ ID NO:4, respectivement, tels qu'identifiés par le schéma de numérotation IMGT, ou
les H-CDR1, H-CDR2 et H-CDR3 comprennent les acides aminés 31 à 35, 50 à 66 et 99 à 109 de SEQ ID NO:3, respectivement, et les L-CDR1, L-CDR2 et L-CDR3 comprennent les acides aminés 24 à 38, 54 à 60 et 93 à 101 de SEQ ID NO:4, respectivement, tels qu'identifiés par le schéma de numérotation de Kabat.

2. Molécule d'acide nucléique selon la revendication 1, dans laquelle :
(a) la région variable de chaîne lourde comprend ou consiste en la séquence d'acides aminés définie par SEQ ID NO:3 ou SEQ ID NO:11, ou
(b) la région variable de chaîne légère comprend ou consiste en la séquence d'acides aminés définie par SEQ ID NO:4 ou SEQ ID NO:12.

3. Molécule d'acide nucléique selon l'une quelconque des revendications précédentes, dans laquelle les régions variables des chaînes lourde et légère comprennent ou consistent en les séquence d'acides aminés définies par :
(a) SEQ ID NO:3 et SEQ ID NO:4, respectivement ; ou
(b) SEQ ID NO:11 et SEQ ID NO:12, respectivement.

4. Molécule d'acide nucléique selon l'une quelconque des revendications précédentes, dans laquelle le domaine de liaison à l'antigène est un scFv,
en particulier dans laquelle a) le scFv comprend ou consiste en la séquence d'acides aminé définie par SEQ ID NO:18 ou SEQ ID NO:22 et/ou b) dans laquelle le domaine transmembranaire comprend ou consiste en la séquence d'acides aminés définie par SEQ ID NO:27 ou SEQ ID NO:30.

5. Molécule d'acide nucléique selon l'une quelconque des revendications précédentes, dans laquelle le domaine de signalisation de lymphocytes T comprend ou consiste en un domaine de signalisation CD3 zêta et/ou
dans laquelle le récepteur antigénique chimérique comprend
(a) un domaine de signalisation CD8 ;
(b) un domaine de signalisation CD28 ;
(c) un domaine de signalisation CD27 ;
(d) un domaine de signalisation CD154 ;
(e) un domaine de signalisation GITR (TNFRSF18) ;
(f) un domaine de signalisation OX40 (CD134) ;
(g) un domaine de signalisation CD137 (4-1BB) ; ou
(h) une combinaison d'au moins deux de (a)-(g).

6. Molécule d'acide nucléique selon l'une quelconque des revendications précédentes, dans laquelle le au moins un domaine de signalisation de lymphocytes T comprend, de l'extrémité N-terminale à l'extrémité C-terminale,
(a) un domaine de signalisation CD3 zêta ;
(b) un domaine de signalisation CD28 et un domaine de signalisation CD3 zêta ;
(c) un domaine de signalisation CD137 (4-1BB) et un domaine de signalisation CD3 zêta ;
(d) un domaine de signalisation OX40 (CD134) et un domaine de signalisation CD3 zêta ;
(e) un domaine de signalisation CD28 , un domaine de signalisation CD137 (4-1BB) et un domaine de signalisation CD3 zêta ;
ou
(f) un domaine de signalisation CD28 , un domaine de signalisation OX40 (CD134) et un domaine de signalisation CD3 zêta.

7. Molécule d'acide nucléique selon la revendication 6, dans laquelle :
(a) le domaine de signalisation CD3 zêta comprend ou consiste en la séquence d'acides aminés définie par SEQ ID NO:34 ;
(b) le domaine de signalisation CD8 comprend ou consiste en la séquence d'acides aminés définie par SEQ ID NO:31 ;
(c) le domaine de signalisation CD28 comprend ou consiste en la séquence d'acides aminés définie par SEQ ID NO:28 ; ou
(d) le domaine de signalisation CD137 comprend ou consiste en la séquence d'acides aminés définie par SEQ ID NO:32 ou SEQ ID NO:33.

8. Molécule d'acide nucléique selon l'une quelconque des revendications précédentes, dans laquelle le domaine transmembranaire comprend ou consiste en la séquence d'acides aminés définie par SEQ ID NO:27, ou
dans laquelle le domaine transmembranaire comprend ou consiste en la séquence d'acides aminés définie par SEQ ID NO:30.

9. Molécule d'acide nucléique selon l'une quelconque des revendications précédentes, dans laquelle le récepteur antigénique chimérique comprend, de l'extrémité N-terminale à l'extrémité C-terminale, le domaine de liaison à l'antigène, le domaine transmembranaire et le au moins un domaine de signalisation intracellulaire de lymphocytes T ; et/ou
dans laquelle le récepteur antigénique chimérique comprend en outre un domaine espaceur C-terminal par rapport au domaine de liaison à l'antigène et N-terminal par rapport au domaine transmembranaire,
en particulier dans laquelle le domaine espaceur comprend un domaine immunoglobuline, facultativement dans laquelle le domaine immunoglobuline comprend un domaine CH2CH3,
en particulier dans laquelle le domaine immunoglobuline comprend la séquence d'acides aminés définie par SEQ ID NO:35 ; et/ou
dans laquelle le récepteur antigénique chimérique comprend en outre un peptide signal N-terminal par rapport au domaine de liaison à l'antigène.

10. Molécule d'acide nucléique selon la revendication 1, dans laquelle le récepteur antigénique chimérique comprend ou consiste en la séquence d'acides aminés définie par SEQ ID NO:49, SEQ ID NO:50, SEQ ID NO:51, SEQ ID NO:52, SEQ ID NO:53, SEQ ID NO:54, SEQ ID NO:73, SEQ ID NO:74, SEQ ID NO:75, SEQ ID NO:76, SEQ ID NO:77 ou SEQ ID NO:78, ou comprend ou consiste en la séquence d'acide nucléique définie par SEQ ID NO:97, SEQ ID NO:98, SEQ ID NO:99, SEQ ID NO:100, SEQ ID NO:101 ou SEQ ID NO:102.

11. Molécule d'acide nucléique selon l'une quelconque des revendications précédentes, codon optimisée pour l'expression dans un lymphocyte T humain et/ou liée de façon fonctionnelle à une séquence de contrôle de l'expression.

12. Vecteur comprenant la molécule d'acide nucléique selon l'une quelconque des revendications précédentes, en particulier dans lequel le vecteur est un vecteur d'expression d'ADN recombinant, ou
dans lequel le vecteur est un vecteur viral,
en particulier dans lequel le vecteur viral est un vecteur lentiviral, en particulier dans lequel le vecteur est destiné à l'utilisation dans la production de lymphocyte T à récepteur antigénique chimérique.

13. Polypeptide comprenant le récepteur antigénique chimérique codé par la molécule d'acide nucléique selon l'une quelconque des revendications précédentes.

14. Cellule hôte, comprenant la molécule d'acide nucléique ou le vecteur selon l'une quelconque des revendications 1 à 12, en particulier dans laquelle la cellule hôte est un lymphocyte T.

15. Composition, comprenant une quantité pharmaceutiquement efficace de la molécule d'acide nucléique, du vecteur, du polypeptide ou de la cellule hôte selon l'une quelconque des revendications précédentes, et un support pharmaceutiquement acceptable.

16. Procédé de production d'un lymphocyte T à récepteur antigénique chimérique comprenant :
transduire un lymphocyte T avec le vecteur selon la revendication 12, produisant ainsi le lymphocyte T à récepteur antigénique chimérique.

17. Récepteur antigénique chimérique codé par la molécule d'acide nucléique selon l'une quelconque des revendications 1 à 11 pour une utilisation dans une méthode de traitement d'un sujet ayant une tumeur, dans lequel la tumeur comprend une expression à la surface des cellules de la kinase du lymphome anaplasique, la méthode comprenant :
Administrer au sujet une quantité thérapeutiquement efficace de lymphocytes T exprimant le récepteur antigénique chimérique, dans des conditions suffisantes pour former un complexe immun du domaine de liaison à l'antigène sur le récepteur antigénique chimérique et du domaine extracellulaire de la kinase du lymphome anaplasique chez le sujet,
en particulier dans lequel les lymphocytes T sont des lymphocytes T provenant du sujet qui ont été transformés avec la molécule d'acide nucléique selon l'une quelconque des revendications 1 à 11 codant pour le récepteur antigénique chimérique ou transduits avec un vecteur comprenant la molécule d'acide nucléique.

18. Récepteur antigénique chimérique pour l'utilisation selon la revendication 17, dans lequel la méthode comprend en outre les étapes de :
obtenir les lymphocytes T à partir du sujet, et
transformer les lymphocytes T avec la molécule d'acide nucléique codant pour le récepteur antigénique chimérique ou transduire les lymphocytes T avec un vecteur comprenant la molécule d'acide nucléique, et/ou
dans lequel la tumeur ne comprend pas une protéine de fusion de la kinase du lymphome anaplasique et/ou dans lequel la méthode comprend en outre administrer au sujet une quantité thérapeutiquement efficace d'un agent chimiothérapeutique, en particulier
dans lequel l'agent chimiothérapeutique comprend un inhibiteur de la kinase du lymphome anaplasique, en particulier dans lequel l'inhibiteur de la kinase du lymphome anaplasique comprend le crizotinib, et/ou
dans lequel traiter la tumeur comprend une réduction de la charge tumorale.

19. Récepteur antigénique chimérique pour l'utilisation selon l'une des revendications 17 ou 18, dans lequel la tumeur est un neuroblastome, un rhabdomyosarcome ou un glioblastome.

20. Récepteur antigénique chimérique pour l'utilisation selon la revendication 17, dans lequel la méthode comprend en outre sélectionner le sujet par la détection de l'expression à la surface des cellules de la kinase du lymphome anaplasique sur la tumeur.

21. Kit pour produire un lymphocyte T à récepteur antigénique chimérique ou pour traiter une tumeur chez un sujet, dans lequel la tumeur comprend une expression à la surface des cellules de la kinase du lymphome anaplasique, comprenant un récipient comprenant la molécule d'acide nucléique, le vecteur, la cellule hôte ou la composition selon l'une quelconque des revendications 1 à 12, 14 ou 15, et des instructions pour utiliser le kit.

22. Molécule d'acide nucléique, vecteur, cellule hôte ou composition selon l'une quelconque des revendications 1 à 12, 14 ou 15, pour une utilisation dans le traitement d'une tumeur chez un sujet, dans lequel ou laquelle la tumeur comprend une expression à la surface des cellules de la kinase du lymphome anaplasique, la cellule hôte étant un lymphocyte T.

23. Utilisation de la molécule d'acide nucléique ou du vecteur selon l'une quelconque des revendications 1 à 12, pour produire un lymphocyte T à récepteur antigénique chimérique.


### SEQUENCE LISTING

<110> The United States of America, as Represented by the Secretary, Department of Health and human Services MacKall, Crystal Orentas, Rimas
<120> ALK ANTIBODIES, CONJUGATES, AND CHIMERIC ANTIGEN RECEPTORS, AND THEIR USE
<130> 4239-92075-02
<150> 61/900,806
   <151> 2013-11-06
<160> 116
<170> PatentIn version 3.5
<210> 1
   <211> 119
   <212> PRT
   <213> Artificial sequence
<220>
   <223> Antibody variable domain sequence
<400> 1
   Asp Val Lys Leu Gln Glu Ser Gly Pro Gly Leu Val Ala Pro Ser Gln
   1 5 10 15
   Ser Leu Ser Ile Thr Cys Thr Val Ser Gly Phe Ser Leu Thr Ser Tyr
   20 25 30
   Ala Val Ser Trp Val Arg Gln Pro Pro Gly Lys Gly Leu Glu Trp Leu
   35 40 45
   Gly Ile Ile Trp Ser Gly Gly Ala Thr Asn Tyr Asn Ser Ala Leu Lys
   50 55 60
   Ser Arg Leu Ser Ile Ser Lys Asp Asn Ser Lys Ser Gln Val Phe Leu
   65 70 75 80
   Lys Met Asn Gly Leu Gln Thr Asp Asp Thr Ala Arg Tyr Tyr Cys Ala
   85 90 95
   Arg Glu His Tyr Tyr Gly Ser Ser Ala Met Asp Tyr Trp Gly Gln Gly
   100 105 110
   Ala Ser Ile Thr Val Ser Ser
   115
<210> 2
   <211> 112
   <212> PRT
   <213> Artificial sequence
<220>
   <223> Antibody variable domain sequence
<400> 2
   Gly Ile Val Met Thr Gln Ser Pro Leu Ser Leu Pro Val Ser Leu Gly
   1               5                   10                  15
   Asp Gln Ala Ser Ile Ser Cys Arg Ser Ser Gln Ser Ile Val His Ser
               20                  25                  30
   Tyr Gly Asn Thr Tyr Leu Phe Trp Tyr Leu Gln Lys Pro Gly Gln Ser
           35                  40                  45
   Pro Lys Leu Leu Ile Tyr Arg Val Ser Asn Arg Phe Ser Gly Val Pro
       50                  55                  60
   Asp Arg Phe Ser Gly Ser Gly Ser Gly Thr Asn Phe Thr Leu Lys Ile
   65                  70                  75                  80
   Ser Arg Val Glu Ala Glu Asp Met Gly Val Tyr Tyr Cys Phe Gln Gly
                   85                  90                  95
   Thr His Val Pro Tyr Thr Phe Gly Gly Gly Thr Lys Leu Glu Ile Lys
               100                 105                 110
<210> 3
   <211> 120
   <212> PRT
   <213> Artificial sequence
<220>
   <223> Antibody variable domain sequence
<400> 3
   Gln Val Gln Leu Gln Gln Ser Gly Ala Glu Leu Val Lys Pro Gly Ala
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Lys Gly Leu Glu Trp Ile
           35                  40                  45
   Gly Gln Ile Tyr Pro Gly Asp Gly Asp Thr Thr Tyr Asn Gly Lys Phe
       50                  55                  60
   Lys Gly Lys Ala Thr Leu Thr Ala Asp Lys Ser Ser Ser Thr Val Tyr
   65                  70                  75                  80
   Met Gln Leu Asn Ser Leu Thr Ser Glu Asp Ser Ala Val Tyr Phe Cys
                   85                  90                  95
   Val Arg Tyr Tyr Tyr Gly Ser Ser Gly Tyr Phe Asp Tyr Trp Gly Gln
               100                 105                 110
   Gly Thr Thr Leu Thr Val Ser Ser
           115                 120
<210> 4
   <211> 111
   <212> PRT
   <213> Artificial sequence
<220>
   <223> Antibody variable domain sequence
<400> 4
   Asp Val Gln Met Ile Gln Thr Pro Asp Ser Leu Ala Val Ser Leu Gly
   1               5                   10                  15
   Gln Arg Ala Thr Ile Ser Cys Arg Ala Ser Glu Ser Val Asp Asn Tyr
               20                  25                  30
   Gly Ile Ser Phe Met His Trp Tyr Gln Gln Lys Pro Gly Gln Ser Pro
           35                  40                  45
   Lys Leu Leu Ile Tyr Arg Ala Ser Asn Leu Glu Ser Gly Ile Pro Ala
       50                  55                  60
   Arg Phe Ser Gly Ser Gly Ser Arg Thr Asp Phe Thr Leu Thr Ile Asn
   65                  70                  75                  80
   Pro Val Glu Thr Asp Asp Val Ala Thr Tyr Tyr Cys Gln Gln Asn Asn
                   85                  90                  95
   Lys Asp Pro Pro Thr Phe Gly Gly Gly Thr Lys Leu Glu Ile Lys
               100                 105                 110
<210> 5
   <211> 118
   <212> PRT
   <213> Artificial sequence
<220>
   <223> Antibody variable domain sequence
<400> 5
   Asp Val Gln Leu Gln Glu Ser Gly Pro Val Leu Val Lys Thr Gly Ala
   1               5                   10                  15
   Ser Val Lys Met Ser Cys Thr Ala Ser Gly Tyr Thr Phe Thr Asp His
               20                  25                  30
   Phe Met Asp Trp Val Lys Gln Ser His Gly Lys Ser Leu Glu Trp Ile
           35                  40                  45
   Gly Ser Leu Asn Pro Tyr Ser Gly Gly Thr Ser Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Asn Ser Leu Thr Ser Val Asp Ser Ala Val Tyr Tyr Cys
                   85                  90                  95
   Ala Arg His Asn Trp Gly Ala Tyr Phe Asp Tyr Trp Gly Gln Gly Thr
               100                 105                 110
   Thr Leu Thr Val Ser Ser
           115
<210> 6
   <211> 112
   <212> PRT
   <213> Artificial sequence
<220>
   <223> Antibody variable domain sequence
<400> 6
   Asp Ile Val Met Thr Gln Ala Ala Pro Ser Val Pro Val Thr Pro Gly
   1               5                   10                  15
   Glu Ser Val Ser Ile Ser Cys Arg Ser Ser Lys Ser Leu Leu His Ser
               20                  25                  30
   Asn Gly Asn Thr Tyr Leu Tyr Trp Phe Leu Gln Arg Pro Gly Gln Ser
           35                  40                  45
   Pro Gln Arg Leu Ile Tyr Tyr Met Ser Asn Leu Ala Ser Gly Val Pro
       50                  55                  60
   Asp Arg Phe Ser Gly Arg Gly Ser Gly Thr Asp Phe Thr Leu Arg Ile
   65                  70                  75                  80
   Ser Arg Val Glu Ala Glu Asp Val Gly Val Tyr Tyr Cys Met Gln Gly
                   85                  90                  95
   Leu Glu Asp Pro Tyr Thr Phe Gly Gly Gly Thr Lys Leu Glu Ile Lys
               100                 105                 110
<210> 7
   <211> 120
   <212> PRT
   <213> Artificial sequence
<220>
   <223> Antibody variable domain sequence
<400> 7
   Ala Leu Gln Leu Gln Gln Ser Gly Ala Glu Leu Val Arg Pro Gly Ala
   1               5                   10                  15
   Ser Val Thr Leu Ser Cys Lys Ala Ser Gly Tyr Thr Phe Thr Asp Tyr
               20                  25                  30
   Glu Met His Trp Val Lys Gln Thr Pro Val His Gly Leu Glu Trp Ile
           35                  40                  45
   Gly Ala Ile Asp Pro Glu Thr Gly Gly Thr Ala Tyr Asn Gln Lys Phe
       50                  55                  60
   Glu Gly Lys Ala Ile Leu Thr Ala Asp Lys Ser Ser Ser Thr Ala Tyr
   65                  70                  75                  80
   Met Glu Leu Arg Ser Leu Thr Ser Glu Asp Ser Pro Val Tyr Tyr Cys
                   85                  90                  95
   Ala Arg Arg Arg Tyr Tyr Gly Ser Ser Ser Phe Asp Tyr Trp Gly Gln
               100                 105                 110
   Gly Thr Thr Leu Thr Val Ser Ser
           115                 120
<210> 8
   <211> 107
   <212> PRT
   <213> Artificial sequence
<220>
   <223> Antibody variable domain sequence
<400> 8
   Asp Val Gln Met Ile Gln Thr Pro Ser Ser Leu Ser Ala Ser Leu Gly
   1               5                   10                  15
   Asp Arg Val Thr Ile Ser Cys Arg Ala Ser Gln Asp Ile Gly Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Arg Leu His Ser Gly Val Pro Ser Arg Phe Ser Gly
       50                  55                  60
   Ser Gly Ser Gly Thr Glu Tyr Ser Leu Thr Ile Ser Asn Leu Glu Gln
   65                  70                  75                  80
   Glu Asp Ile Ala Thr Tyr Phe Cys Gln Gln Gly Ser Ala Leu Pro Pro
                   85                  90                  95
   Thr Phe Gly Gly Gly Thr Lys Leu Glu Ile Asn
               100                 105
<210> 9
   <211> 119
   <212> PRT
   <213> Artificial sequence
<220>
   <223> Antibody variable domain sequence
<400> 9
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Phe Ser Leu Thr Ser Tyr
               20                  25                  30
   Ala Ile Ser Trp Val Arg Gln Ala Pro Gly Gln Gly Leu Glu Trp Met
           35                  40                  45
   Gly Gly Ile Ile Trp Ser Gly Gly Ala Thr Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Glu His Tyr Tyr Gly Ser Ser Ala Met Asp Tyr Trp Trp Gly
               100                 105                 110
   Gln Gly Thr Met Val Thr Val
   115
<210> 10
   <211> 112
   <212> PRT
   <213> Artificial sequence
<220>
   <223> Antibody variable domain sequence
<400> 10
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Ser Ile Val His Ser
               20                  25                  30
   Tyr Gly Asn Thr Tyr Ala Trp Tyr Gln Gln Lys Pro Gly Gln Ala Pro
           35                  40                  45
   Arg Leu Leu Ile Tyr Arg Val Ser Arg Ala Thr Gly Ile Pro Ala Arg
       50                  55                  60
   Phe Ser Gly Ser Gly Ser Gly Thr Asp Phe Thr Leu Thr Ile Ser Ser
   65                  70                  75                  80
   Leu Glu Pro Glu Asp Phe Ala Val Tyr Tyr Cys Phe Gln Gly Thr His
                   85                  90                  95
   Val Pro Tyr Thr Phe Phe Gly Gln Gly Thr Lys Leu Glu Ile Lys Arg
               100                 105                 110
<210> 11
   <211> 119
   <212> PRT
   <213> Artificial sequence
<220>
   <223> Antibody variable domain sequence
<400> 11
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Ala Phe Ser Ser Tyr
               20                  25                  30
   Ile Ser Trp Val Arg Gln Ala Pro Gly Gln Gly Leu Glu Trp Met Gly
           35                  40                  45
   Gly Gln Ile Tyr Pro Gly Asp Gly Asp Thr Asn Tyr Ala Gln Lys Phe
   50 55 60
   Gln Gly Arg Val Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Tyr Cys
   85 90 95
   Val Arg Tyr Tyr Tyr Gly Ser Ser Gly Tyr Phe Asp Tyr Trp Trp Gly
   100 105 110
   Gln Gly Thr Met Val Thr Val
   115
<210> 12
   <211> 111
   <212> PRT
   <213> Artificial sequence
<220>
   <223> Antibody variable domain sequence
<400> 12
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Glu Ser Val Asp Asn Tyr
   20 25 30
   Gly Ile Ser Phe Ala Trp Tyr Gln Gln Lys Pro Gly Gln Ala Pro Arg
   35 40 45
   Leu Leu Ile Tyr Arg Ala Ser Arg Ala Thr Gly Ile Pro Ala Arg Phe
   50 55 60
   Ser Gly Ser Gly Ser Gly Thr Asp Phe Thr Leu Thr Ile Ser Ser Leu
   65 70 75 80
   Glu Pro Glu Asp Phe Ala Val Tyr Tyr Cys Gln Gln Asn Asn Lys Asp
   85 90 95
   Pro Pro Thr Phe Phe Gly Gln Gly Thr Lys Leu Glu Ile Lys Arg
   100 105 110
<210> 13
   <211> 117
   <212> PRT
   <213> Artificial sequence
<220>
   <223> Antibody variable domain sequence
<400> 13
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asp His
               20                  25                  30
   Phe Ile Ser Trp Val Arg Gln Ala Pro Gly Gln Gly Leu Glu Trp Met
           35                  40                  45
   Gly Gly Leu Asn Pro Tyr Ser Gly Gly Thr Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg His Asn Trp Gly Ala Tyr Phe Asp Tyr Trp Trp Gly Gln Gly
               100                 105                 110
   Thr Met Val Thr Val
           115
<210> 14
   <211> 112
   <212> PRT
   <213> Artificial sequence
<220>
   <223> Antibody variable domain sequence
<400> 14
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Lys Ser Leu Leu His Ser
               20                  25                  30
   Asn Gly Asn Thr Tyr Ala Trp Tyr Gln Gln Lys Pro Gly Gln Ala Pro
           35                  40                  45
   Arg Leu Leu Ile Tyr Tyr Met Ser Arg Ala Thr Gly Ile Pro Ala Arg
       50                  55                  60
   Phe Ser Gly Ser Gly Ser Gly Thr Asp Phe Thr Leu Thr Ile Ser Ser
   65                  70                  75                  80
   Leu Glu Pro Glu Asp Phe Ala Val Tyr Tyr Cys Met Gln Gly Leu Glu
                   85                  90                  95
   Asp Pro Tyr Thr Phe Phe Gly Gln Gly Thr Lys Leu Glu Ile Lys Arg
               100                 105                 110
<210> 15
   <211> 119
   <212> PRT
   <213> Artificial sequence
<220>
   <223> Antibody variable domain sequence
<400> 15
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asp Tyr
               20                  25                  30
   Glu Ile Ser Trp Val Arg Gln Ala Pro Gly Gln Gly Leu Glu Trp Met
           35                  40                  45
   Gly Gly Ile Asp Pro Glu Thr Gly Gly Thr Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Arg Tyr Tyr Gly Ser Ser Ser Phe Asp Tyr Trp Trp Gly
               100                 105                 110
   Gln Gly Thr Met Val Thr Val
           115
<210> 16
   <211> 107
   <212> PRT
   <213> Artificial sequence
<220>
   <223> Antibody variable domain sequence
<400> 16
   Glu Ile Val Leu Thr Gln Ser Pro Ala Thr Leu Ser Leu Ser Pro Gly
   1               5                   10                  15
   Glu Arg Ala Thr Leu Ser Cys Arg Ala Ser Gln Asp Ile Gly Asn Tyr
               20                  25                  30
   Ala Trp Tyr Gln Gln Lys Pro Gly Gln Ala Pro Arg Leu Leu Ile Tyr
           35                  40                  45
   Tyr Thr Ser Arg Ala Thr Gly Ile Pro Ala Arg Phe Ser Gly Ser Gly
       50                  55                  60
   Ser Gly Thr Asp Phe Thr Leu Thr Ile Ser Ser Leu Glu Pro Glu Asp
   65                  70                  75                  80
   Phe Ala Val Tyr Tyr Cys Gln Gln Gly Ser Ala Leu Pro Pro Thr Phe
                   85                  90                  95
   Phe Gly Gln Gly Thr Lys Leu Glu Ile Lys Arg
               100                 105
<210> 17
   <211> 246
   <212> PRT
   <213> Artificial sequence
<220>
   <223> scFv sequence
<400> 17
   Asp Val Lys Leu Gln Glu Ser Gly Pro Gly Leu Val Ala Pro Ser Gln
   1               5                   10                  15
   Ser Leu Ser Ile Thr Cys Thr Val Ser Gly Phe Ser Leu Thr Ser Tyr
               20                  25                  30
   Ala Val Ser Trp Val Arg Gln Pro Pro Gly Lys Gly Leu Glu Trp Leu
           35                  40                  45
   Gly Ile Ile Trp Ser Gly Gly Ala Thr Asn Tyr Asn Ser Ala Leu Lys
       50                  55                  60
   Ser Arg Leu Ser Ile Ser Lys Asp Asn Ser Lys Ser Gln Val Phe Leu
   65                  70                  75                  80
   Lys Met Asn Gly Leu Gln Thr Asp Asp Thr Ala Arg Tyr Tyr Cys Ala
                   85                  90                  95
   Arg Glu His Tyr Tyr Gly Ser Ser Ala Met Asp Tyr Trp Gly Gln Gly
               100                 105                 110
   Ala Ser Ile Thr Val Ser Ser Gly Gly Gly Gly Ser Gly Gly Gly Gly
           115                 120                 125
   Ser Gly Gly Gly Gly Ser Gly Ile Val Met Thr Gln Ser Pro Leu Ser
       130                 135                 140
   Leu Pro Val Ser Leu Gly Asp Gln Ala Ser Ile Ser Cys Arg Ser Ser
   145                 150                 155                 160
   Gln Ser Ile Val His Ser Tyr Gly Asn Thr Tyr Leu Phe Trp Tyr Leu
                   165                 170                 175
   Gln Lys Pro Gly Gln Ser Pro Lys Leu Leu Ile Tyr Arg Val Ser Asn
               180                 185                 190
   Arg Phe Ser Gly Val Pro Asp Arg Phe Ser Gly Ser Gly Ser Gly Thr
           195                 200                 205
   Asn Phe Thr Leu Lys Ile Ser Arg Val Glu Ala Glu Asp Met Gly Val
       210                 215                 220
   Tyr Tyr Cys Phe Gln Gly Thr His Val Pro Tyr Thr Phe Gly Gly Gly
   225                 230                 235                 240
   Thr Lys Leu Glu Ile Lys
                   245
<210> 18
   <211> 246
   <212> PRT
   <213> Artificial sequence
<220>
   <223> scFv sequence
<400> 18
   Gln Val Gln Leu Gln Gln Ser Gly Ala Glu Leu Val Lys Pro Gly Ala
   1               5                   10                  15
   Ser Val Lys Ile Ser Cys Lys Ala Ser Gly Tyr Ala Phe Ser Ser Tyr
               20                  25                  30
   Trp Met Asn Trp Val Lys Gln Arg Pro Gly Lys Gly Leu Glu Trp Ile
           35                  40                  45
   Gly Gln Ile Tyr Pro Gly Asp Gly Asp Thr Thr Tyr Asn Gly Lys Phe
           50                  55                  60
   Lys Gly Lys Ala Thr Leu Thr Ala Asp Lys Ser Ser Ser Thr Val Tyr
   65                  70                  75                  80
   Met Gln Leu Asn Ser Leu Thr Ser Glu Asp Ser Ala Val Tyr Phe Cys
                   85                  90                  95
   Val Arg Tyr Tyr Tyr Gly Ser Ser Gly Tyr Phe Asp Tyr Trp Gly Gln
               100                 105                 110
   Gly Thr Thr Leu Thr Val Ser Ser Gly Gly Gly Gly Ser Gly Gly Gly
           115                 120                 125
   Gly Ser Gly Gly Gly Gly Ser Asp Val Gln Met Ile Gln Thr Pro Asp
       130                 135                 140
   Ser Leu Ala Val Ser Leu Gly Gln Arg Ala Thr Ile Ser Cys Arg Ala
   145                 150                 155                 160
   Ser Glu Ser Val Asp Asn Tyr Gly Ile Ser Phe Met His Trp Tyr Gln
                   165                 170                 175
   Gln Lys Pro Gly Gln Ser Pro Lys Leu Leu Ile Tyr Arg Ala Ser Asn
               180                 185                 190
   Leu Glu Ser Gly Ile Pro Ala Arg Phe Ser Gly Ser Gly Ser Arg Thr
           195                 200                 205
   Asp Phe Thr Leu Thr Ile Asn Pro Val Glu Thr Asp Asp Val Ala Thr
       210                 215                 220
   Tyr Tyr Cys Gln Gln Asn Asn Lys Asp Pro Pro Thr Phe Gly Gly Gly
   225                 230                 235                 240
   Thr Lys Leu Glu Ile Lys
                   245
<210> 19
   <211> 245
   <212> PRT
   <213> Artificial sequence
<220>
   <223> scFv sequence
<400> 19
   Asp Val Gln Leu Gln Glu Ser Gly Pro Val Leu Val Lys Thr Gly Ala
   1               5                   10                  15
   Ser Val Lys Met Ser Cys Thr Ala Ser Gly Tyr Thr Phe Thr Asp His
               20                  25                  30
   Phe Met Asp Trp Val Lys Gln Ser His Gly Lys Ser Leu Glu Trp Ile
           35                  40                  45
   Gly Ser Leu Asn Pro Tyr Ser Gly Gly Thr Ser Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Asn Ser Leu Thr Ser Val Asp Ser Ala Val Tyr Tyr Cys
                   85                  90                  95
   Ala Arg His Asn Trp Gly Ala Tyr Phe Asp Tyr Trp Gly Gln Gly Thr
               100                 105                 110
   Thr Leu Thr Val Ser Ser Gly Gly Gly Gly Ser Gly Gly Gly Gly Ser
           115                 120                 125
   Gly Gly Gly Gly Ser Asp Ile Val Met Thr Gln Ala Ala Pro Ser Val
       130                 135                 140
   Pro Val Thr Pro Gly Glu Ser Val Ser Ile Ser Cys Arg Ser Ser Lys
   145                 150                 155                 160
   Ser Leu Leu His Ser Asn Gly Asn Thr Tyr Leu Tyr Trp Phe Leu Gln
                   165                 170                 175
   Arg Pro Gly Gln Ser Pro Gln Arg Leu Ile Tyr Tyr Met Ser Asn Leu
               180                 185                 190
   Ala Ser Gly Val Pro Asp Arg Phe Ser Gly Arg Gly Ser Gly Thr Asp
           195                 200                 205
   Phe Thr Leu Arg Ile Ser Arg Val Glu Ala Glu Asp Val Gly Val Tyr
       210                 215                 220
   Tyr Cys Met Gln Gly Leu Glu Asp Pro Tyr Thr Phe Gly Gly Gly Thr
   225                 230                 235                 240
   Lys Leu Glu Ile Lys
                   245
<210> 20
   <211> 242
   <212> PRT
   <213> Artificial sequence
<220>
   <223> scFv sequence
<400> 20
   Ala Leu Gln Leu Gln Gln Ser Gly Ala Glu Leu Val Arg Pro Gly Ala
   1               5                   10                  15
   Ser Val Thr Leu Ser Cys Lys Ala Ser Gly Tyr Thr Phe Thr Asp Tyr
               20                  25                  30
   Glu Met His Trp Val Lys Gln Thr Pro Val His Gly Leu Glu Trp Ile
           35                  40                  45
   Gly Ala Ile Asp Pro Glu Thr Gly Gly Thr Ala Tyr Asn Gln Lys Phe
       50                  55                  60
   Glu Gly Lys Ala Ile Leu Thr Ala Asp Lys Ser Ser Ser Thr Ala Tyr
   65                  70                  75                  80
   Met Glu Leu Arg Ser Leu Thr Ser Glu Asp Ser Pro Val Tyr Tyr Cys
                   85                  90                  95
   Ala Arg Arg Arg Tyr Tyr Gly Ser Ser Ser Phe Asp Tyr Trp Gly Gln
               100                 105                 110
   Gly Thr Thr Leu Thr Val Ser Ser Gly Gly Gly Gly Ser Gly Gly Gly
           115                 120                 125
   Gly Ser Gly Gly Gly Gly Ser Asp Val Gln Met Ile Gln Thr Pro Ser
       130                 135                 140
   Ser Leu Ser Ala Ser Leu Gly Asp Arg Val Thr Ile Ser Cys Arg Ala
   145                 150                 155                 160
   Ser Gln Asp Ile Gly Asn Tyr Leu Asn Trp Tyr Gln Gln Lys Pro Asp
                   165                 170                 175
   Gly Thr Val Lys Leu Leu Ile Tyr Tyr Thr Ser Arg Leu His Ser Gly
               180                 185                 190
   Val Pro Ser Arg Phe Ser Gly Ser Gly Ser Gly Thr Glu Tyr Ser Leu
           195                 200                 205
   Thr Ile Ser Asn Leu Glu Gln Glu Asp Ile Ala Thr Tyr Phe Cys Gln
       210                 215                 220
   Gln Gly Ser Ala Leu Pro Pro Thr Phe Gly Gly Gly Thr Lys Leu Glu
   225                 230                 235                 240
   Ile Asn
<210> 21
   <211> 248
   <212> PRT
   <213> Artificial sequence
<220>
   <223> scFv sequence
<400> 21
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Phe Ser Leu Thr Ser Tyr
               20                  25                  30
   Ala Ile Ser Trp Val Arg Gln Ala Pro Gly Gln Gly Leu Glu Trp Met
           35                  40                  45
   Gly Gly Ile Ile Trp Ser Gly Gly Ala Thr Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Glu His Tyr Tyr Gly Ser Ser Ala Met Asp Tyr Trp Trp Gly
               100                 105                 110
   Gln Gly Thr Met Val Thr Val Ser Ser Gly Gly Gly Gly Ser Gly Gly
           115                 120                 125
   Gly Gly Ser Gly Gly Gly Gly Ser Glu Ile Val Leu Thr Gln Ser Pro
       130                 135                 140
   Ala Thr Leu Ser Leu Ser Pro Gly Glu Arg Ala Thr Leu Ser Cys Arg
   145                 150                 155                 160
   Ala Ser Gln Ser Ile Val His Ser Tyr Gly Asn Thr Tyr Ala Trp Tyr
                   165                 170                 175
   Gln Gln Lys Pro Gly Gln Ala Pro Arg Leu Leu Ile Tyr Arg Val Ser
   180 185 190
   Arg Ala Thr Gly Ile Pro Ala Arg Phe Ser Gly Ser Gly Ser Gly Thr
   195 200 205
   Asp Phe Thr Leu Thr Ile Ser Ser Leu Glu Pro Glu Asp Phe Ala Val
   210 215 220
   Tyr Tyr Cys Phe Gln Gly Thr His Val Pro Tyr Thr Phe Phe Gly Gln
   225 230 235 240
   Gly Thr Lys Leu Glu Ile Lys Arg
   245
<210> 22
   <211> 247
   <212> PRT
   <213> Artificial sequence
<220>
   <223> scFv sequence
<400> 22
   Gln Val Gln Leu Gln Gln Ser Gly Ala Glu Val Lys Lys Pro Gly Ser
   1 5 10 15
   Ser Val Lys Val Ser Cys Lys Ala Ser Gly Tyr Ala Phe Ser Ser Tyr
   20 25 30
   Ile Ser Trp Val Arg Gln Ala Pro Gly Gln Gly Leu Glu Trp Met Gly
   35 40 45
   Gly Gln Ile Tyr Pro Gly Asp Gly Asp Thr Asn Tyr Ala Gln Lys Phe
   50 55 60
   Gln Gly Arg Val Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Tyr Cys
   85 90 95
   Val Arg Tyr Tyr Tyr Gly Ser Ser Gly Tyr Phe Asp Tyr Trp Trp Gly
   100 105 110
   Gln Gly Thr Met Val Thr Val Ser Ser Gly Gly Gly Gly Ser Gly Gly
   115 120 125
   Gly Gly Ser Gly Gly Gly Gly Ser Glu Ile Val Leu Thr Gln Ser Pro
       130                 135                 140
   Ala Thr Leu Ser Leu Ser Pro Gly Glu Arg Ala Thr Leu Ser Cys Arg
   145                 150                 155                 160
   Ala Ser Glu Ser Val Asp Asn Tyr Gly Ile Ser Phe Ala Trp Tyr Gln
                   165                 170                 175
   Gln Lys Pro Gly Gln Ala Pro Arg Leu Leu Ile Tyr Arg Ala Ser Arg
               180                 185                 190
   Ala Thr Gly Ile Pro Ala Arg Phe Ser Gly Ser Gly Ser Gly Thr Asp
           195                 200                 205
   Phe Thr Leu Thr Ile Ser Ser Leu Glu Pro Glu Asp Phe Ala Val Tyr
       210                 215                 220
   Tyr Cys Gln Gln Asn Asn Lys Asp Pro Pro Thr Phe Phe Gly Gln Gly
   225                 230                 235                 240
   Thr Lys Leu Glu Ile Lys Arg
                   245
<210> 23
   <211> 246
   <212> PRT
   <213> Artificial sequence
<220>
   <223> scFv sequence
<400> 23
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asp His
               20                  25                  30
   Phe Ile Ser Trp Val Arg Gln Ala Pro Gly Gln Gly Leu Glu Trp Met
           35                  40                  45
   Gly Gly Leu Asn Pro Tyr Ser Gly Gly Thr Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
   85 90 95
   Ala Arg His Asn Trp Gly Ala Tyr Phe Asp Tyr Trp Trp Gly Gln Gly
   100 105 110
   Thr Met Val Thr Val Ser Ser Gly Gly Gly Gly Ser Gly Gly Gly Gly
   115 120 125
   Ser Gly Gly Gly Gly Ser Glu Ile Val Leu Thr Gln Ser Pro Ala Thr
   130 135 140
   Leu Ser Leu Ser Pro Gly Glu Arg Ala Thr Leu Ser Cys Arg Ala Ser
   145 150 155 160
   Lys Ser Leu Leu His Ser Asn Gly Asn Thr Tyr Ala Trp Tyr Gln Gln
   165 170 175
   Lys Pro Gly Gln Ala Pro Arg Leu Leu Ile Tyr Tyr Met Ser Arg Ala
   180 185 190
   Thr Gly Ile Pro Ala Arg Phe Ser Gly Ser Gly Ser Gly Thr Asp Phe
   195 200 205
   Thr Leu Thr Ile Ser Ser Leu Glu Pro Glu Asp Phe Ala Val Tyr Tyr
   210 215 220
   Cys Met Gln Gly Leu Glu Asp Pro Tyr Thr Phe Phe Gly Gln Gly Thr
   225 230 235 240
   Lys Leu Glu Ile Lys Arg
   245
<210> 24
   <211> 243
   <212> PRT
   <213> Artificial sequence
<220>
   <223> scFv sequence
<400> 24
   Gln Val Gln Leu Gln Gln Ser Gly Ala Glu Val Lys Lys Pro Gly Ser
   1               5                   10                  15
   Ser Val Lys Val Ser Cys Lys Ala Ser Gly Tyr Thr Phe Thr Asp Tyr
               20                  25                  30
   Glu Ile Ser Trp Val Arg Gln Ala Pro Gly Gln Gly Leu Glu Trp Met
           35                  40                  45
   Gly Gly Ile Asp Pro Glu Thr Gly Gly Thr Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Arg Tyr Tyr Gly Ser Ser Ser Phe Asp Tyr Trp Trp Gly
               100                 105                 110
   Gln Gly Thr Met Val Thr Val Ser Ser Gly Gly Gly Gly Ser Gly Gly
           115                 120                 125
   Gly Gly Ser Gly Gly Gly Gly Ser Glu Ile Val Leu Thr Gln Ser Pro
       130                 135                 140
   Ala Thr Leu Ser Leu Ser Pro Gly Glu Arg Ala Thr Leu Ser Cys Arg
   145                 150                 155                 160
   Ala Ser Gln Asp Ile Gly Asn Tyr Ala Trp Tyr Gln Gln Lys Pro Gly
                   165                 170                 175
   Gln Ala Pro Arg Leu Leu Ile Tyr Tyr Thr Ser Arg Ala Thr Gly Ile
               180                 185                 190
   Pro Ala Arg Phe Ser Gly Ser Gly Ser Gly Thr Asp Phe Thr Leu Thr
           195                 200                 205
   Ile Ser Ser Leu Glu Pro Glu Asp Phe Ala Val Tyr Tyr Cys Gln Gln
       210                 215                 220
   Gly Ser Ala Leu Pro Pro Thr Phe Phe Gly Gln Gly Thr Lys Leu Glu
   225                 230                 235                 240
   Ile Lys Arg
<210> 25
   <211> 15
   <212> PRT
   <213> Artificial sequence
<220>
   <223> Peptide linker
<400> 25
   Gly Gly Gly Gly Ser Gly Gly Gly Gly Ser Gly Gly Gly Gly Ser
   1               5                   10                  15
<210> 26
   <211> 22
   <212> PRT
   <213> homo sapiens
<400> 26
   Leu Leu Val Thr Ser Leu Leu Leu Cys Glu Leu Pro His Pro Ala Phe
   1               5                   10                  15
   Leu Leu Ile Pro Asp Thr
               20
<210> 27
   <211> 67
   <212> PRT
   <213> homo sapiens
<400> 27
   Ile Glu Val Met Tyr Pro Pro Pro Tyr Leu Asp Asn Glu Lys Ser Asn
   1               5                   10                  15
   Gly Thr Ile Ile His Val Lys Gly Lys His Leu Cys Pro Ser Pro Leu
               20                  25                  30
   Phe Pro Gly Pro Ser Lys Pro Phe Trp Val Leu Val Val Val Gly Gly
           35                  40                  45
   Val Leu Ala Cys Tyr Ser Leu Leu Val Thr Val Ala Phe Ile Ile Phe
       50                  55                  60
   Trp Val Arg
   65
<210> 28
   <211> 40
   <212> PRT
   <213> homo sapiens
<400> 28
   Ser Lys Arg Ser Arg Leu Leu His Ser Asp Tyr Met Asn Met Thr Pro
   1               5                   10                  15
   Arg Arg Pro Gly Pro Thr Arg Lys His Tyr Gln Pro Tyr Ala Pro Pro
               20                  25                  30
   Arg Asp Phe Ala Ala Tyr Arg Ser
           35                  40
<210> 29
   <211> 107
   <212> PRT
   <213> homo sapiens
<400> 29
   Ile Glu Val Met Tyr Pro Pro Pro Tyr Leu Asp Asn Glu Lys Ser Asn
   1               5                   10                  15
   Gly Thr Ile Ile His Val Lys Gly Lys His Leu Cys Pro Ser Pro Leu
               20                  25                  30
   Phe Pro Gly Pro Ser Lys Pro Phe Trp Val Leu Val Val Val Gly Gly
           35                  40                  45
   Val Leu Ala Cys Tyr Ser Leu Leu Val Thr Val Ala Phe Ile Ile Phe
       50                  55                  60
   Trp Val Arg Ser Lys Arg Ser Arg Leu Leu His Ser Asp Tyr Met Asn
   65                  70                  75                  80
   Met Thr Pro Arg Arg Pro Gly Pro Thr Arg Lys His Tyr Gln Pro Tyr
                   85                  90                  95
   Ala Pro Pro Arg Asp Phe Ala Ala Tyr Arg Ser
               100                 105
<210> 30
   <211> 69
   <212> PRT
   <213> homo sapiens
<400> 30
   Thr Thr Thr Pro Ala Pro Arg Pro Pro Thr Pro Ala Pro Thr Ile Ala
   1 5 10 15
   Ser Gln Pro Leu Ser Leu Arg Pro Glu Ala Cys Arg Pro Ala Ala Gly
   20 25 30
   Gly Ala Val His Thr Arg Gly Leu Asp Phe Ala Cys Asp Ile Tyr Ile
   35 40 45
   Trp Ala Pro Leu Ala Gly Thr Cys Gly Val Leu Leu Leu Ser Leu Val
   50 55 60
   Ile Thr Leu Tyr Cys
   65
<210> 31
   <211> 84
   <212> PRT
   <213> homo sapiens
<400> 31
   Phe Val Pro Val Phe Leu Pro Ala Lys Pro Thr Thr Thr Pro Ala Pro
   1 5 10 15
   Arg Pro Pro Thr Pro Ala Pro Thr Ile Ala Ser Gln Pro Leu Ser Leu
   20 25 30
   Arg Pro Glu Ala Cys Arg Pro Ala Ala Gly Gly Ala Val His Thr Arg
   35 40 45
   Gly Leu Asp Phe Ala Cys Asp Ile Tyr Ile Trp Ala Pro Leu Ala Gly
   50 55 60
   Thr Cys Gly Val Leu Leu Leu Ser Leu Val Ile Thr Leu Tyr Cys Asn
   65 70 75 80
   His Arg Asn Arg
<210> 32
   <211> 42
   <212> PRT
   <213> homo sapiens
<400> 32
   Lys Arg Gly Arg Lys Lys Leu Leu Tyr Ile Phe Lys Gln Pro Phe Met
   1 5 10 15
   Arg Pro Val Gln Thr Thr Gln Glu Glu Asp Gly Cys Ser Cys Arg Phe
   20 25 30
   Pro Glu Glu Glu Glu Gly Gly Cys Glu Leu
   35 40
<210> 33
   <211> 47
   <212> PRT
   <213> homo sapiens
<400> 33
   Arg Phe Ser Val Val Lys Arg Gly Arg Lys Lys Leu Leu Tyr Ile Phe
   1 5 10 15
   Lys Gln Pro Phe Met Arg Pro Val Gln Thr Thr Gln Glu Glu Asp Gly
   20 25 30
   Cys Ser Cys Arg Phe Pro Glu Glu Glu Glu Gly Gly Cys Glu Leu
   35 40 45
<210> 34
   <211> 112
   <212> PRT
   <213> homo sapiens
<400> 34
   Arg Val Lys Phe Ser Arg Ser Ala Asp Ala Pro Ala Tyr Gln Gln Gly
   1               5                   10                  15
   Gln Asn Gln Leu Tyr Asn Glu Leu Asn Leu Gly Arg Arg Glu Glu Tyr
               20                  25                  30
   Asp Val Leu Asp Lys Arg Arg Gly Arg Asp Pro Glu Met Gly Gly Lys
           35                  40                  45
   Pro Arg Arg Lys Asn Pro Gln Glu Gly Leu Tyr Asn Glu Leu Gln Lys
       50                  55                  60
   Asp Lys Met Ala Glu Ala Tyr Ser Glu Ile Gly Met Lys Gly Glu Arg
   65                  70                  75                  80
   Arg Arg Gly Lys Gly His Asp Gly Leu Tyr Gln Gly Leu Ser Thr Ala
                   85                  90                  95
   Thr Lys Asp Thr Tyr Asp Ala Leu His Met Gln Ala Leu Pro Pro Arg
               100                 105                 110
<210> 35
   <211> 236
   <212> PRT
   <213> homo sapiens
<400> 35
   Glu Pro Lys Ser Cys Asp Lys Thr His Thr Cys Pro Pro Cys Pro Ala
   1               5                   10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
           50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys Lys Asp Pro Lys
   225                 230                 235
<210> 36
   <211> 708
   <212> DNA
   <213> homo sapiens
<400> 36
   gaacccaagt catgcgataa gacccacact tgtccaccct gtccagcccc tgaactgctc      60
   ggaggtccgt cagtgtttct tttcccgcca aagcctaagg acactctgat gatctctcgg     120
   acccctgaag tgacttgcgt cgtcgtggac gtgtcacacg aggatcccga ggtgaagttc     180
   aactggtatg tggacggggt ggaagtgcat aatgctaaga ccaagcccag ggaggaacaa     240
   tacaactcaa cctaccgcgt ggtgtccgtg ctcaccgtcc ttcatcaaga ctggctgaac     300
   ggaaaagagt ataagtgcaa agtctccaat aaggctctgc cagcccctat cgaaaagacc     360
   atttcaaagg ccaaggggca gcctagagag ccccaagtgt acacccttcc tccctcaaga     420
   gatgagctca ctaagaatca ggtcagcctg acttgtcttg tgaaaggctt ctatcccagc     480
   gatattgccg tcgaatggga aagcaatgga caaccagaga acaactacaa gaccaccccg     540
   cctgtgctgg actccgacgg ctctttcttc ctttactcaa agctgaccgt cgataagagc     600
   cggtggcaac aggggaatgt gttcagctgc tccgtcatgc acgaggctct ccataaccac     660
   tacacccaga aaagcctgtc tctttctccg ggcaaaaagg acccaaag                   708
<210> 37
   <211> 222
   <212> PRT
   <213> Artificial Sequence
<220>
   <223> CAR TM and instracellular sequence
<400> 37
   Ala Ala Ala Ile Glu Val Met Tyr Pro Pro Pro Tyr Leu Asp Asn Glu
   1               5                   10                  15
   Lys Ser Asn Gly Thr Ile Ile His Val Lys Gly Lys His Leu Cys Pro
               20                  25                  30
   Ser Pro Leu Phe Pro Gly Pro Ser Lys Pro Phe Trp Val Leu Val Val
           35                  40                  45
   Val Gly Gly Val Leu Ala Cys Tyr Ser Leu Leu Val Thr Val Ala Phe
       50                  55                  60
   Ile Ile Phe Trp Val Arg Ser Lys Arg Ser Arg Leu Leu His Ser Asp
   65                  70                  75                  80
   Tyr Met Asn Met Thr Pro Arg Arg Pro Gly Pro Thr Arg Lys His Tyr
                   85                  90                  95
   Gln Pro Tyr Ala Pro Pro Arg Asp Phe Ala Ala Tyr Arg Ser Arg Val
               100                 105                 110
   Lys Phe Ser Arg Ser Ala Asp Ala Pro Ala Tyr Gln Gln Gly Gln Asn
           115                 120                 125
   Gln Leu Tyr Asn Glu Leu Asn Leu Gly Arg Arg Glu Glu Tyr Asp Val
       130                 135                 140
   Leu Asp Lys Arg Arg Gly Arg Asp Pro Glu Met Gly Gly Lys Pro Arg
   145                 150                 155                 160
   Arg Lys Asn Pro Gln Glu Gly Leu Tyr Asn Glu Leu Gln Lys Asp Lys
                   165                 170                 175
   Met Ala Glu Ala Tyr Ser Glu Ile Gly Met Lys Gly Glu Arg Arg Arg
               180                 185                 190
   Gly Lys Gly His Asp Gly Leu Tyr Gln Gly Leu Ser Thr Ala Thr Lys
           195                 200                 205
   Asp Thr Tyr Asp Ala Leu His Met Gln Ala Leu Pro Pro Arg
       210                 215                 220
<210> 38
   <211> 669
   <212> DNA
   <213> Artificial Sequence
<220>
   <223> CAR TM and instracellular sequence
<400> 38
   gcggccgcaa ttgaagttat gtatcctcct ccttacctag acaatgagaa gagcaatgga 60
   accattatcc atgtgaaagg gaaacacctt tgtccaagtc ccctatttcc cggaccttct 120
   aagccctttt gggtgctggt ggtggttggg ggagtcctgg cttgctatag cttgctagta 180
   acagtggcct ttattatttt ctgggtgagg agtaagagga gcaggctcct gcacagtgac 240
   tacatgaaca tgactccccg ccgccccggg cccacccgca agcattacca gccctatgcc 300
   ccaccacgcg acttcgcagc ctatcgctcc agagtgaagt tcagcaggag cgcagacgcc 360
   cccgcgtacc agcagggcca gaaccagctc tataacgagc tcaatctagg acgaagagag 420
   gagtacgatg ttttggacaa gagacgtggc cgggaccctg agatgggggg aaagccgaga 480
   aggaagaacc ctcaggaagg cctgtacaat gaactgcaga aagataagat ggcggaggcc 540
   tacagtgaga ttgggatgaa aggcgagcgc cggaggggca aggggcacga tggcctttac 600
   cagggtctca gtacagccac caaggacacc tacgacgccc ttcacatgca ggccctgccc 660
   cctcgctaa 669
<210> 39
   <211> 226
   <212> PRT
   <213> Artificial Sequence
<220>
   <223> CAR TM and instracellular sequence
<400> 39
   Ala Ala Ala Thr Thr Thr Pro Ala Pro Arg Pro Pro Thr Pro Ala Pro
   1               5                   10                  15
   Thr Ile Ala Ser Gln Pro Leu Ser Leu Arg Pro Glu Ala Cys Arg Pro
               20                  25                  30
   Ala Ala Gly Gly Ala Val His Thr Arg Gly Leu Asp Phe Ala Cys Asp
           35                  40                  45
   Ile Tyr Ile Trp Ala Pro Leu Ala Gly Thr Cys Gly Val Leu Leu Leu
       50                  55                  60
   Ser Leu Val Ile Thr Leu Tyr Cys Lys Arg Gly Arg Lys Lys Leu Leu
   65                  70                  75                  80
   Tyr Ile Phe Lys Gln Pro Phe Met Arg Pro Val Gln Thr Thr Gln Glu
                   85                  90                  95
   Glu Asp Gly Cys Ser Cys Arg Phe Pro Glu Glu Glu Glu Gly Gly Cys
               100                 105                 110
   Glu Leu Arg Val Lys Phe Ser Arg Ser Ala Asp Ala Pro Ala Tyr Lys
           115                 120                 125
   Gln Gly Gln Asn Gln Leu Tyr Asn Glu Leu Asn Leu Gly Arg Arg Glu
       130                 135                 140
   Glu Tyr Asp Val Leu Asp Lys Arg Arg Gly Arg Asp Pro Glu Met Gly
   145                 150                 155                 160
   Gly Lys Pro Arg Arg Lys Asn Pro Gln Glu Gly Leu Tyr Asn Glu Leu
                   165                 170                 175
   Gln Lys Asp Lys Met Ala Glu Ala Tyr Ser Glu Ile Gly Met Lys Gly
               180                 185                 190
   Glu Arg Arg Arg Gly Lys Gly His Asp Gly Leu Tyr Gln Gly Leu Ser
           195                 200                 205
   Thr Ala Thr Lys Asp Thr Tyr Asp Ala Leu His Met Gln Ala Leu Pro
       210                 215                 220
   Pro Arg
   225
<210> 40
   <211> 681
   <212> DNA
   <213> Artificial Sequence
<220>
   <223> CAR TM and instracellular sequence
<400> 40
   gcggccgcaa ccacgacgcc agcgccgcga ccaccaacac cggcgcccac catcgcgtcg      60
   cagcccctgt ccctgcgccc agaggcgtgc cggccagcgg cggggggcgc agtgcacacg     120
   agggggctgg acttcgcctg tgatatctac atctgggcgc ccttggccgg gacttgtggg     180
   gtccttctcc tgtcactggt tatcaccctt tactgcaaac ggggcagaaa gaaactcctg     240
   tatatattca aacaaccatt tatgagacca gtacaaacta ctcaagagga agatggctgt     300
   agctgccgat ttccagaaga agaagaagga ggatgtgaac tgagagtgaa gttcagcagg     360
   agcgcagacg cccccgcgta caagcagggc cagaaccagc tctataacga gctcaatcta     420
   ggacgaagag aggagtacga tgttttggac aagagacgtg gccgggaccc tgagatgggg     480
   ggaaagccga gaaggaagaa ccctcaggaa ggcctgtaca atgaactgca gaaagataag     540
   atggcggagg cctacagtga gattgggatg aaaggcgagc gccggagggg caaggggcac     600
   gatggccttt accagggtct cagtacagcc accaaggaca cctacgacgc ccttcacatg     660
   caggccctgc cccctcgcta a                                               681
<210> 41
   <211> 286
   <212> PRT
   <213> Artificial Sequence
<220>
   <223> CAR TM and instracellular sequence
<400> 41
   Ala Ala Ala Phe Val Pro Val Phe Leu Pro Ala Lys Pro Thr Thr Thr
   1               5                   10                  15
   Pro Ala Pro Arg Pro Pro Thr Pro Ala Pro Thr Ile Ala Ser Gln Pro
               20                  25                  30
   Leu Ser Leu Arg Pro Glu Ala Cys Arg Pro Ala Ala Gly Gly Ala Val
           35                  40                  45
   His Thr Arg Gly Leu Asp Phe Ala Cys Asp Ile Tyr Ile Trp Ala Pro
       50                  55                  60
   Leu Ala Gly Thr Cys Gly Val Leu Leu Leu Ser Leu Val Ile Thr Leu
   65                  70                  75                  80
   Tyr Cys Asn His Arg Asn Arg Ser Lys Arg Ser Arg Leu Leu His Ser
                   85                  90                  95
   Asp Tyr Met Asn Met Thr Pro Arg Arg Pro Gly Pro Thr Arg Lys His
               100                 105                 110
   Tyr Gln Pro Tyr Ala Pro Pro Arg Asp Phe Ala Ala Tyr Arg Ser Arg
           115                 120                 125
   Phe Ser Val Val Lys Arg Gly Arg Lys Lys Leu Leu Tyr Ile Phe Lys
       130                 135                 140
   Gln Pro Phe Met Arg Pro Val Gln Thr Thr Gln Glu Glu Asp Gly Cys
   145                 150                 155                 160
   Ser Cys Arg Phe Pro Glu Glu Glu Glu Gly Gly Cys Glu Leu Arg Val
                   165                 170                 175
   Lys Phe Ser Arg Ser Ala Asp Ala Pro Ala Tyr Gln Gln Gly Gln Asn
               180                 185                 190
   Gln Leu Tyr Asn Glu Leu Asn Leu Gly Arg Arg Glu Glu Tyr Asp Val
           195                 200                 205
   Leu Asp Lys Arg Arg Gly Arg Asp Pro Glu Met Gly Gly Lys Pro Arg
       210                 215                 220
   Arg Lys Asn Pro Gln Glu Gly Leu Tyr Asn Glu Leu Gln Lys Asp Lys
   225                 230                 235                 240
   Met Ala Glu Ala Tyr Ser Glu Ile Gly Met Lys Gly Glu Arg Arg Arg
                   245                 250                 255
   Gly Lys Gly His Asp Gly Leu Tyr Gln Gly Leu Ser Thr Ala Thr Lys
               260                 265                 270
   Asp Thr Tyr Asp Ala Leu His Met Gln Ala Leu Pro Pro Arg
           275                 280                 285
<210> 42
   <211> 861
   <212> DNA
   <213> Artificial Sequence
<220>
   <223> CAR TM and instracellular sequence
<400> 42
   gcggccgcat tcgtgccggt cttcctgcca gcgaagccca ccacgacgcc agcgccgcga      60
   ccaccaacac cggcgcccac catcgcgtcg cagcccctgt ccctgcgccc agaggcgtgc     120
   cggccagcgg cggggggcgc agtgcacacg agggggctgg acttcgcctg tgatatctac     180
   atctgggcgc ccttggccgg gacttgtggg gtccttctcc tgtcactggt tatcaccctt     240
   tactgcaacc acaggaacag gagtaagagg agcaggctcc tgcacagtga ctacatgaac     300
   atgactcccc gccgccccgg gcccacccgc aagcattacc agccctatgc cccaccacgc     360
   gacttcgcag cctatcgctc ccgtttctct gttgttaaac ggggcagaaa gaagctcctg     420
   tatatattca aacaaccatt tatgagacca gtacaaacta ctcaagagga agatggctgt     480
   agctgccgat ttccagaaga agaagaagga ggatgtgaac tgagagtgaa gttcagcagg     540
   agcgcagacg cccccgcgta ccagcagggc cagaaccagc tctataacga gctcaatcta     600
   ggacgaagag aggagtacga tgttttggac aagagacgtg gccgggaccc tgagatgggg     660
   ggaaagccga gaaggaagaa ccctcaggaa ggcctgtaca atgaactgca gaaagataag     720
   atggcggagg cctacagtga gattgggatg aaaggcgagc gccggagggg caaggggcac     780
   gatggccttt accagggtct cagtacagcc accaaggaca cctacgacgc ccttcacatg     840
   caggccctgc cccctcgcta a                                               861
<210> 43
   <211> 726
   <212> PRT
   <213> Artificial Sequence
<220>
   <223> chimeric antigen receptor sequence
<400> 43
   Leu Leu Val Thr Ser Leu Leu Leu Cys Glu Leu Pro His Pro Ala Phe
   1               5                   10                  15
   Leu Leu Ile Pro Asp Thr Asp Val Lys Leu Gln Glu Ser Gly Pro Gly
               20                  25                  30
   Leu Val Ala Pro Ser Gln Ser Leu Ser Ile Thr Cys Thr Val Ser Gly
           35                  40                  45
   Phe Ser Leu Thr Ser Tyr Ala Val Ser Trp Val Arg Gln Pro Pro Gly
       50                  55                  60
   Lys Gly Leu Glu Trp Leu Gly Ile Ile Trp Ser Gly Gly Ala Thr Asn
   65                  70                  75                  80
   Tyr Asn Ser Ala Leu Lys Ser Arg Leu Ser Ile Ser Lys Asp Asn Ser
                   85                  90                  95
   Lys Ser Gln Val Phe Leu Lys Met Asn Gly Leu Gln Thr Asp Asp Thr
               100                 105                 110
   Ala Arg Tyr Tyr Cys Ala Arg Glu His Tyr Tyr Gly Ser Ser Ala Met
           115                 120                 125
   Asp Tyr Trp Gly Gln Gly Ala Ser Ile Thr Val Ser Ser Gly Gly Gly
       130                 135                 140
   Gly Ser Gly Gly Gly Gly Ser Gly Gly Gly Gly Ser Gly Ile Val Met
   145                 150                 155                 160
   Thr Gln Ser Pro Leu Ser Leu Pro Val Ser Leu Gly Asp Gln Ala Ser
                   165                 170                 175
   Ile Ser Cys Arg Ser Ser Gln Ser Ile Val His Ser Tyr Gly Asn Thr
               180                 185                 190
   Tyr Leu Phe Trp Tyr Leu Gln Lys Pro Gly Gln Ser Pro Lys Leu Leu
           195                 200                 205
   Ile Tyr Arg Val Ser Asn Arg Phe Ser Gly Val Pro Asp Arg Phe Ser
       210                 215                 220
   Gly Ser Gly Ser Gly Thr Asn Phe Thr Leu Lys Ile Ser Arg Val Glu
   225                 230                 235                 240
   Ala Glu Asp Met Gly Val Tyr Tyr Cys Phe Gln Gly Thr His Val Pro
                   245                 250                 255
   Tyr Thr Phe Gly Gly Gly Thr Lys Leu Glu Ile Lys Glu Pro Lys Ser
               260                 265                 270
   Cys Asp Lys Thr His Thr Cys Pro Pro Cys Pro Ala Pro Glu Leu Leu
           275                 280                 285
   Gly Gly Pro Ser Val Phe Leu Phe Pro Pro Lys Pro Lys Asp Thr Leu
       290                 295                 300
   Met Ile Ser Arg Thr Pro Glu Val Thr Cys Val Val Val Asp Val Ser
   305                 310                 315                 320
   His Glu Asp Pro Glu Val Lys Phe Asn Trp Tyr Val Asp Gly Val Glu
                   325                 330                 335
   Val His Asn Ala Lys Thr Lys Pro Arg Glu Glu Gln Tyr Asn Ser Thr
               340                 345                 350
   Tyr Arg Val Val Ser Val Leu Thr Val Leu His Gln Asp Trp Leu Asn
           355                 360                 365
   Gly Lys Glu Tyr Lys Cys Lys Val Ser Asn Lys Ala Leu Pro Ala Pro
       370                 375                 380
   Ile Glu Lys Thr Ile Ser Lys Ala Lys Gly Gln Pro Arg Glu Pro Gln
   385                 390                 395                 400
   Val Tyr Thr Leu Pro Pro Ser Arg Asp Glu Leu Thr Lys Asn Gln Val
                   405                 410                 415
   Ser Leu Thr Cys Leu Val Lys Gly Phe Tyr Pro Ser Asp Ile Ala Val
               420                 425                 430
   Glu Trp Glu Ser Asn Gly Gln Pro Glu Asn Asn Tyr Lys Thr Thr Pro
           435                 440                 445
   Pro Val Leu Asp Ser Asp Gly Ser Phe Phe Leu Tyr Ser Lys Leu Thr
       450                 455                 460
   Val Asp Lys Ser Arg Trp Gln Gln Gly Asn Val Phe Ser Cys Ser Val
   465                 470                 475                 480
   Met His Glu Ala Leu His Asn His Tyr Thr Gln Lys Ser Leu Ser Leu
                   485                 490                 495
   Ser Pro Gly Lys Lys Asp Pro Lys Ala Ala Ala Ile Glu Val Met Tyr
               500                 505                 510
   Pro Pro Pro Tyr Leu Asp Asn Glu Lys Ser Asn Gly Thr Ile Ile His
           515                 520                 525
   Val Lys Gly Lys His Leu Cys Pro Ser Pro Leu Phe Pro Gly Pro Ser
       530                 535                 540
   Lys Pro Phe Trp Val Leu Val Val Val Gly Gly Val Leu Ala Cys Tyr
   545                 550                 555                 560
   Ser Leu Leu Val Thr Val Ala Phe Ile Ile Phe Trp Val Arg Ser Lys
                   565                 570                 575
   Arg Ser Arg Leu Leu His Ser Asp Tyr Met Asn Met Thr Pro Arg Arg
               580                 585                 590
   Pro Gly Pro Thr Arg Lys His Tyr Gln Pro Tyr Ala Pro Pro Arg Asp
           595                 600                 605
   Phe Ala Ala Tyr Arg Ser Arg Val Lys Phe Ser Arg Ser Ala Asp Ala
       610                 615                 620
   Pro Ala Tyr Gln Gln Gly Gln Asn Gln Leu Tyr Asn Glu Leu Asn Leu
   625                 630                 635                 640
   Gly Arg Arg Glu Glu Tyr Asp Val Leu Asp Lys Arg Arg Gly Arg Asp
                   645                 650                 655
   Pro Glu Met Gly Gly Lys Pro Arg Arg Lys Asn Pro Gln Glu Gly Leu
               660                 665                 670
   Tyr Asn Glu Leu Gln Lys Asp Lys Met Ala Glu Ala Tyr Ser Glu Ile
           675                 680                 685
   Gly Met Lys Gly Glu Arg Arg Arg Gly Lys Gly His Asp Gly Leu Tyr
       690                 695                 700
   Gln Gly Leu Ser Thr Ala Thr Lys Asp Thr Tyr Asp Ala Leu His Met
   705                 710                 715                 720
   Gln Ala Leu Pro Pro Arg
                   725
<210> 44
   <211> 490
   <212> PRT
   <213> Artificial Sequence
<220>
   <223> chimeric antigen receptor sequence
<400> 44
   Leu Leu Val Thr Ser Leu Leu Leu Cys Glu Leu Pro His Pro Ala Phe
   1               5                   10                  15
   Leu Leu Ile Pro Asp Thr Asp Val Lys Leu Gln Glu Ser Gly Pro Gly
               20                  25                  30
   Leu Val Ala Pro Ser Gln Ser Leu Ser Ile Thr Cys Thr Val Ser Gly
           35                  40                  45
   Phe Ser Leu Thr Ser Tyr Ala Val Ser Trp Val Arg Gln Pro Pro Gly
       50                  55                  60
   Lys Gly Leu Glu Trp Leu Gly Ile Ile Trp Ser Gly Gly Ala Thr Asn
   65                  70                  75                  80
   Tyr Asn Ser Ala Leu Lys Ser Arg Leu Ser Ile Ser Lys Asp Asn Ser
                   85                  90                  95
   Lys Ser Gln Val Phe Leu Lys Met Asn Gly Leu Gln Thr Asp Asp Thr
               100                 105                 110
   Ala Arg Tyr Tyr Cys Ala Arg Glu His Tyr Tyr Gly Ser Ser Ala Met
           115                 120                 125
   Asp Tyr Trp Gly Gln Gly Ala Ser Ile Thr Val Ser Ser Gly Gly Gly
       130                 135                 140
   Gly Ser Gly Gly Gly Gly Ser Gly Gly Gly Gly Ser Gly Ile Val Met
   145                 150                 155                 160
   Thr Gln Ser Pro Leu Ser Leu Pro Val Ser Leu Gly Asp Gln Ala Ser
                   165                 170                 175
   Ile Ser Cys Arg Ser Ser Gln Ser Ile Val His Ser Tyr Gly Asn Thr
               180                 185                 190
   Tyr Leu Phe Trp Tyr Leu Gln Lys Pro Gly Gln Ser Pro Lys Leu Leu
           195                 200                 205
   Ile Tyr Arg Val Ser Asn Arg Phe Ser Gly Val Pro Asp Arg Phe Ser
       210                 215                 220
   Gly Ser Gly Ser Gly Thr Asn Phe Thr Leu Lys Ile Ser Arg Val Glu
   225                 230                 235                 240
   Ala Glu Asp Met Gly Val Tyr Tyr Cys Phe Gln Gly Thr His Val Pro
                   245                 250                 255
   Tyr Thr Phe Gly Gly Gly Thr Lys Leu Glu Ile Lys Ala Ala Ala Ile
               260                 265                 270
   Glu Val Met Tyr Pro Pro Pro Tyr Leu Asp Asn Glu Lys Ser Asn Gly
           275                 280                 285
   Thr Ile Ile His Val Lys Gly Lys His Leu Cys Pro Ser Pro Leu Phe
       290                 295                 300
   Pro Gly Pro Ser Lys Pro Phe Trp Val Leu Val Val Val Gly Gly Val
   305                 310                 315                 320
   Leu Ala Cys Tyr Ser Leu Leu Val Thr Val Ala Phe Ile Ile Phe Trp
                   325                 330                 335
   Val Arg Ser Lys Arg Ser Arg Leu Leu His Ser Asp Tyr Met Asn Met
                   340                 345                 350
   Thr Pro Arg Arg Pro Gly Pro Thr Arg Lys His Tyr Gln Pro Tyr Ala
           355                 360                 365
   Pro Pro Arg Asp Phe Ala Ala Tyr Arg Ser Arg Val Lys Phe Ser Arg
       370                 375                 380
   Ser Ala Asp Ala Pro Ala Tyr Gln Gln Gly Gln Asn Gln Leu Tyr Asn
   385                 390                 395                 400
   Glu Leu Asn Leu Gly Arg Arg Glu Glu Tyr Asp Val Leu Asp Lys Arg
                   405                 410                 415
   Arg Gly Arg Asp Pro Glu Met Gly Gly Lys Pro Arg Arg Lys Asn Pro
               420                 425                 430
   Gln Glu Gly Leu Tyr Asn Glu Leu Gln Lys Asp Lys Met Ala Glu Ala
           435                 440                 445
   Tyr Ser Glu Ile Gly Met Lys Gly Glu Arg Arg Arg Gly Lys Gly His
       450                 455                 460
   Asp Gly Leu Tyr Gln Gly Leu Ser Thr Ala Thr Lys Asp Thr Tyr Asp
   465                 470                 475                 480
   Ala Leu His Met Gln Ala Leu Pro Pro Arg
                   485                 490
<210> 45
   <211> 730
   <212> PRT
   <213> Artificial Sequence
<220>
   <223> chimeric antigen receptor sequence
<400> 45
   Leu Leu Val Thr Ser Leu Leu Leu Cys Glu Leu Pro His Pro Ala Phe
   1               5                   10                  15
   Leu Leu Ile Pro Asp Thr Asp Val Lys Leu Gln Glu Ser Gly Pro Gly
               20                  25                  30
   Leu Val Ala Pro Ser Gln Ser Leu Ser Ile Thr Cys Thr Val Ser Gly
           35                  40                  45
   Phe Ser Leu Thr Ser Tyr Ala Val Ser Trp Val Arg Gln Pro Pro Gly
       50                  55                  60
   Lys Gly Leu Glu Trp Leu Gly Ile Ile Trp Ser Gly Gly Ala Thr Asn
   65 70 75 80
   Tyr Asn Ser Ala Leu Lys Ser Arg Leu Ser Ile Ser Lys Asp Asn Ser
   85 90 95
   Lys Ser Gln Val Phe Leu Lys Met Asn Gly Leu Gln Thr Asp Asp Thr
   100 105 110
   Ala Arg Tyr Tyr Cys Ala Arg Glu His Tyr Tyr Gly Ser Ser Ala Met
   115 120 125
   Asp Tyr Trp Gly Gln Gly Ala Ser Ile Thr Val Ser Ser Gly Gly Gly
   130 135 140
   Gly Ser Gly Gly Gly Gly Ser Gly Gly Gly Gly Ser Gly Ile Val Met
   145 150 155 160
   Thr Gln Ser Pro Leu Ser Leu Pro Val Ser Leu Gly Asp Gln Ala Ser
   165 170 175
   Ile Ser Cys Arg Ser Ser Gln Ser Ile Val His Ser Tyr Gly Asn Thr
   180 185 190
   Tyr Leu Phe Trp Tyr Leu Gln Lys Pro Gly Gln Ser Pro Lys Leu Leu
   195 200 205
   Ile Tyr Arg Val Ser Asn Arg Phe Ser Gly Val Pro Asp Arg Phe Ser
   210 215 220
   Gly Ser Gly Ser Gly Thr Asn Phe Thr Leu Lys Ile Ser Arg Val Glu
   225 230 235 240
   Ala Glu Asp Met Gly Val Tyr Tyr Cys Phe Gln Gly Thr His Val Pro
   245 250 255
   Tyr Thr Phe Gly Gly Gly Thr Lys Leu Glu Ile Lys Glu Pro Lys Ser
   260 265 270
   Cys Asp Lys Thr His Thr Cys Pro Pro Cys Pro Ala Pro Glu Leu Leu
   275 280 285
   Gly Gly Pro Ser Val Phe Leu Phe Pro Pro Lys Pro Lys Asp Thr Leu
   290 295 300
   Met Ile Ser Arg Thr Pro Glu Val Thr Cys Val Val Val Asp Val Ser
   305 310 315 320
   His Glu Asp Pro Glu Val Lys Phe Asn Trp Tyr Val Asp Gly Val Glu
   325 330 335
   Val His Asn Ala Lys Thr Lys Pro Arg Glu Glu Gln Tyr Asn Ser Thr
   340 345 350
   Tyr Arg Val Val Ser Val Leu Thr Val Leu His Gln Asp Trp Leu Asn
   355 360 365
   Gly Lys Glu Tyr Lys Cys Lys Val Ser Asn Lys Ala Leu Pro Ala Pro
   370 375 380
   Ile Glu Lys Thr Ile Ser Lys Ala Lys Gly Gln Pro Arg Glu Pro Gln
   385 390 395 400
   Val Tyr Thr Leu Pro Pro Ser Arg Asp Glu Leu Thr Lys Asn Gln Val
   405 410 415
   Ser Leu Thr Cys Leu Val Lys Gly Phe Tyr Pro Ser Asp Ile Ala Val
   420 425 430
   Glu Trp Glu Ser Asn Gly Gln Pro Glu Asn Asn Tyr Lys Thr Thr Pro
   435 440 445
   Pro Val Leu Asp Ser Asp Gly Ser Phe Phe Leu Tyr Ser Lys Leu Thr
   450 455 460
   Val Asp Lys Ser Arg Trp Gln Gln Gly Asn Val Phe Ser Cys Ser Val
   465 470 475 480
   Met His Glu Ala Leu His Asn His Tyr Thr Gln Lys Ser Leu Ser Leu
   485 490 495
   Ser Pro Gly Lys Lys Asp Pro Lys Ala Ala Ala Thr Thr Thr Pro Ala
   500 505 510
   Pro Arg Pro Pro Thr Pro Ala Pro Thr Ile Ala Ser Gln Pro Leu Ser
   515 520 525
   Leu Arg Pro Glu Ala Cys Arg Pro Ala Ala Gly Gly Ala Val His Thr
   530 535 540
   Arg Gly Leu Asp Phe Ala Cys Asp Ile Tyr Ile Trp Ala Pro Leu Ala
   545 550 555 560
   Gly Thr Cys Gly Val Leu Leu Leu Ser Leu Val Ile Thr Leu Tyr Cys
   565 570 575
   Lys Arg Gly Arg Lys Lys Leu Leu Tyr Ile Phe Lys Gln Pro Phe Met
   580 585 590
   Arg Pro Val Gln Thr Thr Gln Glu Glu Asp Gly Cys Ser Cys Arg Phe
   595 600 605
   Pro Glu Glu Glu Glu Gly Gly Cys Glu Leu Arg Val Lys Phe Ser Arg
   610 615 620
   Ser Ala Asp Ala Pro Ala Tyr Lys Gln Gly Gln Asn Gln Leu Tyr Asn
   625 630 635 640
   Glu Leu Asn Leu Gly Arg Arg Glu Glu Tyr Asp Val Leu Asp Lys Arg
   645 650 655
   Arg Gly Arg Asp Pro Glu Met Gly Gly Lys Pro Arg Arg Lys Asn Pro
   660 665 670
   Gln Glu Gly Leu Tyr Asn Glu Leu Gln Lys Asp Lys Met Ala Glu Ala
   675 680 685
   Tyr Ser Glu Ile Gly Met Lys Gly Glu Arg Arg Arg Gly Lys Gly His
   690 695 700
   Asp Gly Leu Tyr Gln Gly Leu Ser Thr Ala Thr Lys Asp Thr Tyr Asp
   705 710 715 720
   Ala Leu His Met Gln Ala Leu Pro Pro Arg
   725 730
<210> 46
   <211> 494
   <212> PRT
   <213> Artificial Sequence
<220>
   <223> chimeric antigen receptor sequence
<400> 46
   Leu Leu Val Thr Ser Leu Leu Leu Cys Glu Leu Pro His Pro Ala Phe
   1               5                   10                  15
   Leu Leu Ile Pro Asp Thr Asp Val Lys Leu Gln Glu Ser Gly Pro Gly
               20                  25                  30
   Leu Val Ala Pro Ser Gln Ser Leu Ser Ile Thr Cys Thr Val Ser Gly
   35 40 45
   Phe Ser Leu Thr Ser Tyr Ala Val Ser Trp Val Arg Gln Pro Pro Gly
   50 55 60
   Lys Gly Leu Glu Trp Leu Gly Ile Ile Trp Ser Gly Gly Ala Thr Asn
   65 70 75 80
   Tyr Asn Ser Ala Leu Lys Ser Arg Leu Ser Ile Ser Lys Asp Asn Ser
   85 90 95
   Lys Ser Gln Val Phe Leu Lys Met Asn Gly Leu Gln Thr Asp Asp Thr
   100 105 110
   Ala Arg Tyr Tyr Cys Ala Arg Glu His Tyr Tyr Gly Ser Ser Ala Met
   115 120 125
   Asp Tyr Trp Gly Gln Gly Ala Ser Ile Thr Val Ser Ser Gly Gly Gly
   130 135 140
   Gly Ser Gly Gly Gly Gly Ser Gly Gly Gly Gly Ser Gly Ile Val Met
   145 150 155 160
   Thr Gln Ser Pro Leu Ser Leu Pro Val Ser Leu Gly Asp Gln Ala Ser
   165 170 175
   Ile Ser Cys Arg Ser Ser Gln Ser Ile Val His Ser Tyr Gly Asn Thr
   180 185 190
   Tyr Leu Phe Trp Tyr Leu Gln Lys Pro Gly Gln Ser Pro Lys Leu Leu
   195 200 205
   Ile Tyr Arg Val Ser Asn Arg Phe Ser Gly Val Pro Asp Arg Phe Ser
   210 215 220
   Gly Ser Gly Ser Gly Thr Asn Phe Thr Leu Lys Ile Ser Arg Val Glu
   225 230 235 240
   Ala Glu Asp Met Gly Val Tyr Tyr Cys Phe Gln Gly Thr His Val Pro
   245 250 255
   Tyr Thr Phe Gly Gly Gly Thr Lys Leu Glu Ile Lys Ala Ala Ala Thr
   260 265 270
   Thr Thr Pro Ala Pro Arg Pro Pro Thr Pro Ala Pro Thr Ile Ala Ser
   275 280 285
   Gln Pro Leu Ser Leu Arg Pro Glu Ala Cys Arg Pro Ala Ala Gly Gly
       290                 295                 300
   Ala Val His Thr Arg Gly Leu Asp Phe Ala Cys Asp Ile Tyr Ile Trp
   305                 310                 315                 320
   Ala Pro Leu Ala Gly Thr Cys Gly Val Leu Leu Leu Ser Leu Val Ile
                   325                 330                 335
   Thr Leu Tyr Cys Lys Arg Gly Arg Lys Lys Leu Leu Tyr Ile Phe Lys
               340                 345                 350
   Gln Pro Phe Met Arg Pro Val Gln Thr Thr Gln Glu Glu Asp Gly Cys
           355                 360                 365
   Ser Cys Arg Phe Pro Glu Glu Glu Glu Gly Gly Cys Glu Leu Arg Val
       370                 375                 380
   Lys Phe Ser Arg Ser Ala Asp Ala Pro Ala Tyr Lys Gln Gly Gln Asn
   385                 390                 395                 400
   Gln Leu Tyr Asn Glu Leu Asn Leu Gly Arg Arg Glu Glu Tyr Asp Val
                   405                 410                 415
   Leu Asp Lys Arg Arg Gly Arg Asp Pro Glu Met Gly Gly Lys Pro Arg
               420                 425                 430
   Arg Lys Asn Pro Gln Glu Gly Leu Tyr Asn Glu Leu Gln Lys Asp Lys
           435                 440                 445
   Met Ala Glu Ala Tyr Ser Glu Ile Gly Met Lys Gly Glu Arg Arg Arg
       450                 455                 460
   Gly Lys Gly His Asp Gly Leu Tyr Gln Gly Leu Ser Thr Ala Thr Lys
   465                 470                 475                 480
   Asp Thr Tyr Asp Ala Leu His Met Gln Ala Leu Pro Pro Arg
                   485                 490
<210> 47
   <211> 790
   <212> PRT
   <213> Artificial Sequence
<220>
   <223> chimeric antigen receptor sequence
<400> 47
   Leu Leu Val Thr Ser Leu Leu Leu Cys Glu Leu Pro His Pro Ala Phe
   1               5                   10                  15
   Leu Leu Ile Pro Asp Thr Asp Val Lys Leu Gln Glu Ser Gly Pro Gly
               20                  25                  30
   Leu Val Ala Pro Ser Gln Ser Leu Ser Ile Thr Cys Thr Val Ser Gly
           35                  40                  45
   Phe Ser Leu Thr Ser Tyr Ala Val Ser Trp Val Arg Gln Pro Pro Gly
       50                  55                  60
   Lys Gly Leu Glu Trp Leu Gly Ile Ile Trp Ser Gly Gly Ala Thr Asn
   65                  70                  75                  80
   Tyr Asn Ser Ala Leu Lys Ser Arg Leu Ser Ile Ser Lys Asp Asn Ser
                   85                  90                  95
   Lys Ser Gln Val Phe Leu Lys Met Asn Gly Leu Gln Thr Asp Asp Thr
               100                 105                 110
   Ala Arg Tyr Tyr Cys Ala Arg Glu His Tyr Tyr Gly Ser Ser Ala Met
           115                 120                 125
   Asp Tyr Trp Gly Gln Gly Ala Ser Ile Thr Val Ser Ser Gly Gly Gly
       130                 135                 140
   Gly Ser Gly Gly Gly Gly Ser Gly Gly Gly Gly Ser Gly Ile Val Met
   145                 150                 155                 160
   Thr Gln Ser Pro Leu Ser Leu Pro Val Ser Leu Gly Asp Gln Ala Ser
                   165                 170                 175
   Ile Ser Cys Arg Ser Ser Gln Ser Ile Val His Ser Tyr Gly Asn Thr
               180                 185                 190
   Tyr Leu Phe Trp Tyr Leu Gln Lys Pro Gly Gln Ser Pro Lys Leu Leu
           195                 200                 205
   Ile Tyr Arg Val Ser Asn Arg Phe Ser Gly Val Pro Asp Arg Phe Ser
       210                 215                 220
   Gly Ser Gly Ser Gly Thr Asn Phe Thr Leu Lys Ile Ser Arg Val Glu
   225                 230                 235                 240
   Ala Glu Asp Met Gly Val Tyr Tyr Cys Phe Gln Gly Thr His Val Pro
                   245                 250                 255
   Tyr Thr Phe Gly Gly Gly Thr Lys Leu Glu Ile Lys Glu Pro Lys Ser
               260                 265                 270
   Cys Asp Lys Thr His Thr Cys Pro Pro Cys Pro Ala Pro Glu Leu Leu
           275                 280                 285
   Gly Gly Pro Ser Val Phe Leu Phe Pro Pro Lys Pro Lys Asp Thr Leu
       290                 295                 300
   Met Ile Ser Arg Thr Pro Glu Val Thr Cys Val Val Val Asp Val Ser
   305                 310                 315                 320
   His Glu Asp Pro Glu Val Lys Phe Asn Trp Tyr Val Asp Gly Val Glu
                   325                 330                 335
   Val His Asn Ala Lys Thr Lys Pro Arg Glu Glu Gln Tyr Asn Ser Thr
               340                 345                 350
   Tyr Arg Val Val Ser Val Leu Thr Val Leu His Gln Asp Trp Leu Asn
           355                 360                 365
   Gly Lys Glu Tyr Lys Cys Lys Val Ser Asn Lys Ala Leu Pro Ala Pro
       370                 375                 380
   Ile Glu Lys Thr Ile Ser Lys Ala Lys Gly Gln Pro Arg Glu Pro Gln
   385                 390                 395                 400
   Val Tyr Thr Leu Pro Pro Ser Arg Asp Glu Leu Thr Lys Asn Gln Val
                   405                 410                 415
   Ser Leu Thr Cys Leu Val Lys Gly Phe Tyr Pro Ser Asp Ile Ala Val
               420                 425                 430
   Glu Trp Glu Ser Asn Gly Gln Pro Glu Asn Asn Tyr Lys Thr Thr Pro
           435                 440                 445
   Pro Val Leu Asp Ser Asp Gly Ser Phe Phe Leu Tyr Ser Lys Leu Thr
       450                 455                 460
   Val Asp Lys Ser Arg Trp Gln Gln Gly Asn Val Phe Ser Cys Ser Val
   465                 470                 475                 480
   Met His Glu Ala Leu His Asn His Tyr Thr Gln Lys Ser Leu Ser Leu
                   485                 490                 495
   Ser Pro Gly Lys Lys Asp Pro Lys Ala Ala Ala Phe Val Pro Val Phe
               500                 505                 510
   Leu Pro Ala Lys Pro Thr Thr Thr Pro Ala Pro Arg Pro Pro Thr Pro
           515                 520                 525
   Ala Pro Thr Ile Ala Ser Gln Pro Leu Ser Leu Arg Pro Glu Ala Cys
       530                 535                 540
   Arg Pro Ala Ala Gly Gly Ala Val His Thr Arg Gly Leu Asp Phe Ala
   545                 550                 555                 560
   Cys Asp Ile Tyr Ile Trp Ala Pro Leu Ala Gly Thr Cys Gly Val Leu
                   565                 570                 575
   Leu Leu Ser Leu Val Ile Thr Leu Tyr Cys Asn His Arg Asn Arg Ser
               580                 585                 590
   Lys Arg Ser Arg Leu Leu His Ser Asp Tyr Met Asn Met Thr Pro Arg
           595                 600                 605
   Arg Pro Gly Pro Thr Arg Lys His Tyr Gln Pro Tyr Ala Pro Pro Arg
       610                 615                 620
   Asp Phe Ala Ala Tyr Arg Ser Arg Phe Ser Val Val Lys Arg Gly Arg
   625                 630                 635                 640
   Lys Lys Leu Leu Tyr Ile Phe Lys Gln Pro Phe Met Arg Pro Val Gln
                   645                 650                 655
   Thr Thr Gln Glu Glu Asp Gly Cys Ser Cys Arg Phe Pro Glu Glu Glu
               660                 665                 670
   Glu Gly Gly Cys Glu Leu Arg Val Lys Phe Ser Arg Ser Ala Asp Ala
           675                 680                 685
   Pro Ala Tyr Gln Gln Gly Gln Asn Gln Leu Tyr Asn Glu Leu Asn Leu
       690                 695                 700
   Gly Arg Arg Glu Glu Tyr Asp Val Leu Asp Lys Arg Arg Gly Arg Asp
   705                 710                 715                 720
   Pro Glu Met Gly Gly Lys Pro Arg Arg Lys Asn Pro Gln Glu Gly Leu
                   725                 730                 735
   Tyr Asn Glu Leu Gln Lys Asp Lys Met Ala Glu Ala Tyr Ser Glu Ile
               740                 745                 750
   Gly Met Lys Gly Glu Arg Arg Arg Gly Lys Gly His Asp Gly Leu Tyr
           755                 760                 765
   Gln Gly Leu Ser Thr Ala Thr Lys Asp Thr Tyr Asp Ala Leu His Met
   770 775 780
   Gln Ala Leu Pro Pro Arg
   785 790
<210> 48
   <211> 554
   <212> PRT
   <213> Artificial Sequence
<220>
   <223> chimeric antigen receptor sequence
<400> 48
   Leu Leu Val Thr Ser Leu Leu Leu Cys Glu Leu Pro His Pro Ala Phe
   1 5 10 15
   Leu Leu Ile Pro Asp Thr Asp Val Lys Leu Gln Glu Ser Gly Pro Gly
   20 25 30
   Leu Val Ala Pro Ser Gln Ser Leu Ser Ile Thr Cys Thr Val Ser Gly
   35 40 45
   Phe Ser Leu Thr Ser Tyr Ala Val Ser Trp Val Arg Gln Pro Pro Gly
   50 55 60
   Lys Gly Leu Glu Trp Leu Gly Ile Ile Trp Ser Gly Gly Ala Thr Asn
   65 70 75 80
   Tyr Asn Ser Ala Leu Lys Ser Arg Leu Ser Ile Ser Lys Asp Asn Ser
   85 90 95
   Lys Ser Gln Val Phe Leu Lys Met Asn Gly Leu Gln Thr Asp Asp Thr
   100 105 110
   Ala Arg Tyr Tyr Cys Ala Arg Glu His Tyr Tyr Gly Ser Ser Ala Met
   115 120 125
   Asp Tyr Trp Gly Gln Gly Ala Ser Ile Thr Val Ser Ser Gly Gly Gly
   130 135 140
   Gly Ser Gly Gly Gly Gly Ser Gly Gly Gly Gly Ser Gly Ile Val Met
   145 150 155 160
   Thr Gln Ser Pro Leu Ser Leu Pro Val Ser Leu Gly Asp Gln Ala Ser
   165 170 175
   Ile Ser Cys Arg Ser Ser Gln Ser Ile Val His Ser Tyr Gly Asn Thr
               180                 185                 190
   Tyr Leu Phe Trp Tyr Leu Gln Lys Pro Gly Gln Ser Pro Lys Leu Leu
           195                 200                 205
   Ile Tyr Arg Val Ser Asn Arg Phe Ser Gly Val Pro Asp Arg Phe Ser
       210                 215                 220
   Gly Ser Gly Ser Gly Thr Asn Phe Thr Leu Lys Ile Ser Arg Val Glu
   225                 230                 235                 240
   Ala Glu Asp Met Gly Val Tyr Tyr Cys Phe Gln Gly Thr His Val Pro
                   245                 250                 255
   Tyr Thr Phe Gly Gly Gly Thr Lys Leu Glu Ile Lys Ala Ala Ala Phe
               260                 265                 270
   Val Pro Val Phe Leu Pro Ala Lys Pro Thr Thr Thr Pro Ala Pro Arg
           275                 280                 285
   Pro Pro Thr Pro Ala Pro Thr Ile Ala Ser Gln Pro Leu Ser Leu Arg
       290                 295                 300
   Pro Glu Ala Cys Arg Pro Ala Ala Gly Gly Ala Val His Thr Arg Gly
   305                 310                 315                 320
   Leu Asp Phe Ala Cys Asp Ile Tyr Ile Trp Ala Pro Leu Ala Gly Thr
                   325                 330                 335
   Cys Gly Val Leu Leu Leu Ser Leu Val Ile Thr Leu Tyr Cys Asn His
               340                 345                 350
   Arg Asn Arg Ser Lys Arg Ser Arg Leu Leu His Ser Asp Tyr Met Asn
           355                 360                 365
   Met Thr Pro Arg Arg Pro Gly Pro Thr Arg Lys His Tyr Gln Pro Tyr
       370                 375                 380
   Ala Pro Pro Arg Asp Phe Ala Ala Tyr Arg Ser Arg Phe Ser Val Val
   385                 390                 395                 400
   Lys Arg Gly Arg Lys Lys Leu Leu Tyr Ile Phe Lys Gln Pro Phe Met
                   405                 410                 415
   Arg Pro Val Gln Thr Thr Gln Glu Glu Asp Gly Cys Ser Cys Arg Phe
               420                 425                 430
   Pro Glu Glu Glu Glu Gly Gly Cys Glu Leu Arg Val Lys Phe Ser Arg
           435                 440                 445
   Ser Ala Asp Ala Pro Ala Tyr Gln Gln Gly Gln Asn Gln Leu Tyr Asn
       450                 455                 460
   Glu Leu Asn Leu Gly Arg Arg Glu Glu Tyr Asp Val Leu Asp Lys Arg
   465                 470                 475                 480
   Arg Gly Arg Asp Pro Glu Met Gly Gly Lys Pro Arg Arg Lys Asn Pro
                   485                 490                 495
   Gln Glu Gly Leu Tyr Asn Glu Leu Gln Lys Asp Lys Met Ala Glu Ala
               500                 505                 510
   Tyr Ser Glu Ile Gly Met Lys Gly Glu Arg Arg Arg Gly Lys Gly His
           515                 520                 525
   Asp Gly Leu Tyr Gln Gly Leu Ser Thr Ala Thr Lys Asp Thr Tyr Asp
       530                 535                 540
   Ala Leu His Met Gln Ala Leu Pro Pro Arg
   545                 550
<210> 49
   <211> 728
   <212> PRT
   <213> Artificial Sequence
<220>
   <223> chimeric antigen receptor sequence
<400> 49
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Leu Val Lys Pro Gly Ala Ser Val Lys Ile Ser Cys Lys Ala Ser Gly
           35                  40                  45
   Tyr Ala Phe Ser Ser Tyr Trp Met Asn Trp Val Lys Gln Arg Pro Gly
       50                  55                  60
   Lys Gly Leu Glu Trp Ile Gly Gln Ile Tyr Pro Gly Asp Gly Asp Thr
   65                  70                  75                  80
   Thr Tyr Asn Gly Lys Phe Lys Gly Lys Ala Thr Leu Thr Ala Asp Lys
                   85                  90                  95
   Ser Ser Ser Thr Val Tyr Met Gln Leu Asn Ser Leu Thr Ser Glu Asp
               100                 105                 110
   Ser Ala Val Tyr Phe Cys Val Arg Tyr Tyr Tyr Gly Ser Ser Gly Tyr
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Asp Ser Leu Ala Val Ser Leu Gly Gln Arg Ala
                   165                 170                 175
   Thr Ile Ser Cys Arg Ala Ser Glu Ser Val Asp Asn Tyr Gly Ile Ser
               180                 185                 190
   Phe Met His Trp Tyr Gln Gln Lys Pro Gly Gln Ser Pro Lys Leu Leu
           195                 200                 205
   Ile Tyr Arg Ala Ser Asn Leu Glu Ser Gly Ile Pro Ala Arg Phe Ser
       210                 215                 220
   Gly Ser Gly Ser Arg Thr Asp Phe Thr Leu Thr Ile Asn Pro Val Glu
   225                 230                 235                 240
   Thr Asp Asp Val Ala Thr Tyr Tyr Cys Gln Gln Asn Asn Lys Asp Pro
                   245                 250                 255
   Pro Thr Phe Gly Gly Gly Thr Lys Leu Glu Ile Lys Arg Ala Glu Pro
               260                 265                 270
   Lys Ser Cys Asp Lys Thr His Thr Cys Pro Pro Cys Pro Ala Pro Glu
           275                 280                 285
   Leu Leu Gly Gly Pro Ser Val Phe Leu Phe Pro Pro Lys Pro Lys Asp
       290                 295                 300
   Thr Leu Met Ile Ser Arg Thr Pro Glu Val Thr Cys Val Val Val Asp
   305                 310                 315                 320
   Val Ser His Glu Asp Pro Glu Val Lys Phe Asn Trp Tyr Val Asp Gly
                   325                 330                 335
   Val Glu Val His Asn Ala Lys Thr Lys Pro Arg Glu Glu Gln Tyr Asn
               340                 345                 350
   Ser Thr Tyr Arg Val Val Ser Val Leu Thr Val Leu His Gln Asp Trp
           355                 360                 365
   Leu Asn Gly Lys Glu Tyr Lys Cys Lys Val Ser Asn Lys Ala Leu Pro
       370                 375                 380
   Ala Pro Ile Glu Lys Thr Ile Ser Lys Ala Lys Gly Gln Pro Arg Glu
   385                 390                 395                 400
   Pro Gln Val Tyr Thr Leu Pro Pro Ser Arg Asp Glu Leu Thr Lys Asn
                   405                 410                 415
   Gln Val Ser Leu Thr Cys Leu Val Lys Gly Phe Tyr Pro Ser Asp Ile
               420                 425                 430
   Ala Val Glu Trp Glu Ser Asn Gly Gln Pro Glu Asn Asn Tyr Lys Thr
           435                 440                 445
   Thr Pro Pro Val Leu Asp Ser Asp Gly Ser Phe Phe Leu Tyr Ser Lys
       450                 455                 460
   Leu Thr Val Asp Lys Ser Arg Trp Gln Gln Gly Asn Val Phe Ser Cys
   465                 470                 475                 480
   Ser Val Met His Glu Ala Leu His Asn His Tyr Thr Gln Lys Ser Leu
                   485                 490                 495
   Ser Leu Ser Pro Gly Lys Lys Asp Pro Lys Ala Ala Ala Ile Glu Val
               500                 505                 510
   Met Tyr Pro Pro Pro Tyr Leu Asp Asn Glu Lys Ser Asn Gly Thr Ile
           515                 520                 525
   Ile His Val Lys Gly Lys His Leu Cys Pro Ser Pro Leu Phe Pro Gly
       530                 535                 540
   Pro Ser Lys Pro Phe Trp Val Leu Val Val Val Gly Gly Val Leu Ala
   545                 550                 555                 560
   Cys Tyr Ser Leu Leu Val Thr Val Ala Phe Ile Ile Phe Trp Val Arg
                   565                 570                 575
   Ser Lys Arg Ser Arg Leu Leu His Ser Asp Tyr Met Asn Met Thr Pro
   580 585 590
   Arg Arg Pro Gly Pro Thr Arg Lys His Tyr Gln Pro Tyr Ala Pro Pro
   595 600 605
   Arg Asp Phe Ala Ala Tyr Arg Ser Arg Val Lys Phe Ser Arg Ser Ala
   610 615 620
   Asp Ala Pro Ala Tyr Gln Gln Gly Gln Asn Gln Leu Tyr Asn Glu Leu
   625 630 635 640
   Asn Leu Gly Arg Arg Glu Glu Tyr Asp Val Leu Asp Lys Arg Arg Gly
   645 650 655
   Arg Asp Pro Glu Met Gly Gly Lys Pro Arg Arg Lys Asn Pro Gln Glu
   660 665 670
   Gly Leu Tyr Asn Glu Leu Gln Lys Asp Lys Met Ala Glu Ala Tyr Ser
   675 680 685
   Glu Ile Gly Met Lys Gly Glu Arg Arg Arg Gly Lys Gly His Asp Gly
   690 695 700
   Leu Tyr Gln Gly Leu Ser Thr Ala Thr Lys Asp Thr Tyr Asp Ala Leu
   705 710 715 720
   His Met Gln Ala Leu Pro Pro Arg
   725
<210> 50
   <211> 491
   <212> PRT
   <213> Artificial Sequence
<220>
   <223> chimeric antigen receptor sequence
<400> 50
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Leu Val Lys Pro Gly Ala Ser Val Lys Ile Ser Cys Lys Ala Ser Gly
           35                  40                  45
   Tyr Ala Phe Ser Ser Tyr Trp Met Asn Trp Val Lys Gln Arg Pro Gly
       50                  55                  60
   Lys Gly Leu Glu Trp Ile Gly Gln Ile Tyr Pro Gly Asp Gly Asp Thr
   65                  70                  75                  80
   Thr Tyr Asn Gly Lys Phe Lys Gly Lys Ala Thr Leu Thr Ala Asp Lys
                   85                  90                  95
   Ser Ser Ser Thr Val Tyr Met Gln Leu Asn Ser Leu Thr Ser Glu Asp
               100                 105                 110
   Ser Ala Val Tyr Phe Cys Val Arg Tyr Tyr Tyr Gly Ser Ser Gly Tyr
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Asp Ser Leu Ala Val Ser Leu Gly Gln Arg Ala
                   165                 170                 175
   Thr Ile Ser Cys Arg Ala Ser Glu Ser Val Asp Asn Tyr Gly Ile Ser
               180                 185                 190
   Phe Met His Trp Tyr Gln Gln Lys Pro Gly Gln Ser Pro Lys Leu Leu
           195                 200                 205
   Ile Tyr Arg Ala Ser Asn Leu Glu Ser Gly Ile Pro Ala Arg Phe Ser
       210                 215                 220
   Gly Ser Gly Ser Arg Thr Asp Phe Thr Leu Thr Ile Asn Pro Val Glu
   225                 230                 235                 240
   Thr Asp Asp Val Ala Thr Tyr Tyr Cys Gln Gln Asn Asn Lys Asp Pro
                   245                 250                 255
   Pro Thr Phe Gly Gly Gly Thr Lys Leu Glu Ile Lys Arg Ala Ala Ala
               260                 265                 270
   Ile Glu Val Met Tyr Pro Pro Pro Tyr Leu Asp Asn Glu Lys Ser Asn
           275                 280                 285
   Gly Thr Ile Ile His Val Lys Gly Lys His Leu Cys Pro Ser Pro Leu
       290                 295                 300
   Phe Pro Gly Pro Ser Lys Pro Phe Trp Val Leu Val Val Val Gly Gly
   305 310 315 320
   Val Leu Ala Cys Tyr Ser Leu Leu Val Thr Val Ala Phe Ile Ile Phe
   325 330 335
   Trp Val Arg Ser Lys Arg Ser Arg Leu Leu His Ser Asp Tyr Met Asn
   340 345 350
   Met Thr Pro Arg Arg Pro Gly Pro Thr Arg Lys His Tyr Gln Pro Tyr
   355 360 365
   Ala Pro Pro Arg Asp Phe Ala Ala Tyr Arg Ser Arg Val Lys Phe Ser
   370 375 380
   Arg Ser Ala Asp Ala Pro Ala Tyr Gln Gln Gly Gln Asn Gln Leu Tyr
   385 390 395 400
   Asn Glu Leu Asn Leu Gly Arg Arg Glu Glu Tyr Asp Val Leu Asp Lys
   405 410 415
   Arg Arg Gly Arg Asp Pro Glu Met Gly Gly Lys Pro Arg Arg Lys Asn
   420 425 430
   Pro Gln Glu Gly Leu Tyr Asn Glu Leu Gln Lys Asp Lys Met Ala Glu
   435 440 445
   Ala Tyr Ser Glu Ile Gly Met Lys Gly Glu Arg Arg Arg Gly Lys Gly
   450 455 460
   His Asp Gly Leu Tyr Gln Gly Leu Ser Thr Ala Thr Lys Asp Thr Tyr
   465 470 475 480
   Asp Ala Leu His Met Gln Ala Leu Pro Pro Arg
   485 490
<210> 51
   <211> 732
   <212> PRT
   <213> Artificial Sequence
<220>
   <223> chimeric antigen receptor sequence
<400> 51
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Leu Val Lys Pro Gly Ala Ser Val Lys Ile Ser Cys Lys Ala Ser Gly
   35 40 45
   Tyr Ala Phe Ser Ser Tyr Trp Met Asn Trp Val Lys Gln Arg Pro Gly
   50 55 60
   Lys Gly Leu Glu Trp Ile Gly Gln Ile Tyr Pro Gly Asp Gly Asp Thr
   65 70 75 80
   Thr Tyr Asn Gly Lys Phe Lys Gly Lys Ala Thr Leu Thr Ala Asp Lys
   85 90 95
   Ser Ser Ser Thr Val Tyr Met Gln Leu Asn Ser Leu Thr Ser Glu Asp
   100 105 110
   Ser Ala Val Tyr Phe Cys Val Arg Tyr Tyr Tyr Gly Ser Ser Gly Tyr
   115 120 125
   Phe Asp Tyr Trp Gly Gln Gly Thr Thr Leu Thr Val Ser Ser Gly Gly
   130 135 140
   Gly Gly Ser Gly Gly Gly Gly Ser Gly Gly Gly Gly Ser Asp Val Gln
   145 150 155 160
   Met Ile Gln Thr Pro Asp Ser Leu Ala Val Ser Leu Gly Gln Arg Ala
   165 170 175
   Thr Ile Ser Cys Arg Ala Ser Glu Ser Val Asp Asn Tyr Gly Ile Ser
   180 185 190
   Phe Met His Trp Tyr Gln Gln Lys Pro Gly Gln Ser Pro Lys Leu Leu
   195 200 205
   Ile Tyr Arg Ala Ser Asn Leu Glu Ser Gly Ile Pro Ala Arg Phe Ser
   210 215 220
   Gly Ser Gly Ser Arg Thr Asp Phe Thr Leu Thr Ile Asn Pro Val Glu
   225 230 235 240
   Thr Asp Asp Val Ala Thr Tyr Tyr Cys Gln Gln Asn Asn Lys Asp Pro
   245 250 255
   Pro Thr Phe Gly Gly Gly Thr Lys Leu Glu Ile Lys Arg Ala Glu Pro
   260 265 270
   Lys Ser Cys Asp Lys Thr His Thr Cys Pro Pro Cys Pro Ala Pro Glu
   275 280 285
   Leu Leu Gly Gly Pro Ser Val Phe Leu Phe Pro Pro Lys Pro Lys Asp
   290 295 300
   Thr Leu Met Ile Ser Arg Thr Pro Glu Val Thr Cys Val Val Val Asp
   305 310 315 320
   Val Ser His Glu Asp Pro Glu Val Lys Phe Asn Trp Tyr Val Asp Gly
   325 330 335
   Val Glu Val His Asn Ala Lys Thr Lys Pro Arg Glu Glu Gln Tyr Asn
   340 345 350
   Ser Thr Tyr Arg Val Val Ser Val Leu Thr Val Leu His Gln Asp Trp
   355 360 365
   Leu Asn Gly Lys Glu Tyr Lys Cys Lys Val Ser Asn Lys Ala Leu Pro
   370 375 380
   Ala Pro Ile Glu Lys Thr Ile Ser Lys Ala Lys Gly Gln Pro Arg Glu
   385 390 395 400
   Pro Gln Val Tyr Thr Leu Pro Pro Ser Arg Asp Glu Leu Thr Lys Asn
   405 410 415
   Gln Val Ser Leu Thr Cys Leu Val Lys Gly Phe Tyr Pro Ser Asp Ile
   420 425 430
   Ala Val Glu Trp Glu Ser Asn Gly Gln Pro Glu Asn Asn Tyr Lys Thr
   435 440 445
   Thr Pro Pro Val Leu Asp Ser Asp Gly Ser Phe Phe Leu Tyr Ser Lys
   450 455 460
   Leu Thr Val Asp Lys Ser Arg Trp Gln Gln Gly Asn Val Phe Ser Cys
   465 470 475 480
   Ser Val Met His Glu Ala Leu His Asn His Tyr Thr Gln Lys Ser Leu
   485 490 495
   Ser Leu Ser Pro Gly Lys Lys Asp Pro Lys Ala Ala Ala Thr Thr Thr
   500 505 510
   Pro Ala Pro Arg Pro Pro Thr Pro Ala Pro Thr Ile Ala Ser Gln Pro
   515 520 525
   Leu Ser Leu Arg Pro Glu Ala Cys Arg Pro Ala Ala Gly Gly Ala Val
   530 535 540
   His Thr Arg Gly Leu Asp Phe Ala Cys Asp Ile Tyr Ile Trp Ala Pro
   545 550 555 560
   Leu Ala Gly Thr Cys Gly Val Leu Leu Leu Ser Leu Val Ile Thr Leu
   565 570 575
   Tyr Cys Lys Arg Gly Arg Lys Lys Leu Leu Tyr Ile Phe Lys Gln Pro
   580 585 590
   Phe Met Arg Pro Val Gln Thr Thr Gln Glu Glu Asp Gly Cys Ser Cys
   595 600 605
   Arg Phe Pro Glu Glu Glu Glu Gly Gly Cys Glu Leu Arg Val Lys Phe
   610 615 620
   Ser Arg Ser Ala Asp Ala Pro Ala Tyr Lys Gln Gly Gln Asn Gln Leu
   625 630 635 640
   Tyr Asn Glu Leu Asn Leu Gly Arg Arg Glu Glu Tyr Asp Val Leu Asp
   645 650 655
   Lys Arg Arg Gly Arg Asp Pro Glu Met Gly Gly Lys Pro Arg Arg Lys
   660 665 670
   Asn Pro Gln Glu Gly Leu Tyr Asn Glu Leu Gln Lys Asp Lys Met Ala
   675 680 685
   Glu Ala Tyr Ser Glu Ile Gly Met Lys Gly Glu Arg Arg Arg Gly Lys
   690 695 700
   Gly His Asp Gly Leu Tyr Gln Gly Leu Ser Thr Ala Thr Lys Asp Thr
   705 710 715 720
   Tyr Asp Ala Leu His Met Gln Ala Leu Pro Pro Arg
   725 730
<210> 52
   <211> 495
   <212> PRT
   <213> Artificial Sequence
<220>
   <223> chimeric antigen receptor sequence
<400> 52
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Leu Val Lys Pro Gly Ala Ser Val Lys Ile Ser Cys Lys Ala Ser Gly
   35 40 45
   Tyr Ala Phe Ser Ser Tyr Trp Met Asn Trp Val Lys Gln Arg Pro Gly
   50 55 60
   Lys Gly Leu Glu Trp Ile Gly Gln Ile Tyr Pro Gly Asp Gly Asp Thr
   65 70 75 80
   Thr Tyr Asn Gly Lys Phe Lys Gly Lys Ala Thr Leu Thr Ala Asp Lys
   85 90 95
   Ser Ser Ser Thr Val Tyr Met Gln Leu Asn Ser Leu Thr Ser Glu Asp
   100 105 110
   Ser Ala Val Tyr Phe Cys Val Arg Tyr Tyr Tyr Gly Ser Ser Gly Tyr
   115 120 125
   Phe Asp Tyr Trp Gly Gln Gly Thr Thr Leu Thr Val Ser Ser Gly Gly
   130 135 140
   Gly Gly Ser Gly Gly Gly Gly Ser Gly Gly Gly Gly Ser Asp Val Gln
   145 150 155 160
   Met Ile Gln Thr Pro Asp Ser Leu Ala Val Ser Leu Gly Gln Arg Ala
   165 170 175
   Thr Ile Ser Cys Arg Ala Ser Glu Ser Val Asp Asn Tyr Gly Ile Ser
   180 185 190
   Phe Met His Trp Tyr Gln Gln Lys Pro Gly Gln Ser Pro Lys Leu Leu
   195 200 205
   Ile Tyr Arg Ala Ser Asn Leu Glu Ser Gly Ile Pro Ala Arg Phe Ser
   210 215 220
   Gly Ser Gly Ser Arg Thr Asp Phe Thr Leu Thr Ile Asn Pro Val Glu
   225 230 235 240
   Thr Asp Asp Val Ala Thr Tyr Tyr Cys Gln Gln Asn Asn Lys Asp Pro
   245 250 255
   Pro Thr Phe Gly Gly Gly Thr Lys Leu Glu Ile Lys Arg Ala Ala Ala
               260                 265                 270
   Thr Thr Thr Pro Ala Pro Arg Pro Pro Thr Pro Ala Pro Thr Ile Ala
           275                 280                 285
   Ser Gln Pro Leu Ser Leu Arg Pro Glu Ala Cys Arg Pro Ala Ala Gly
       290                 295                 300
   Gly Ala Val His Thr Arg Gly Leu Asp Phe Ala Cys Asp Ile Tyr Ile
   305                 310                 315                 320
   Trp Ala Pro Leu Ala Gly Thr Cys Gly Val Leu Leu Leu Ser Leu Val
                   325                 330                 335
   Ile Thr Leu Tyr Cys Lys Arg Gly Arg Lys Lys Leu Leu Tyr Ile Phe
               340                 345                 350
   Lys Gln Pro Phe Met Arg Pro Val Gln Thr Thr Gln Glu Glu Asp Gly
           355                 360                 365
   Cys Ser Cys Arg Phe Pro Glu Glu Glu Glu Gly Gly Cys Glu Leu Arg
       370                 375                 380
   Val Lys Phe Ser Arg Ser Ala Asp Ala Pro Ala Tyr Lys Gln Gly Gln
   385                 390                 395                 400
   Asn Gln Leu Tyr Asn Glu Leu Asn Leu Gly Arg Arg Glu Glu Tyr Asp
                   405                 410                 415
   Val Leu Asp Lys Arg Arg Gly Arg Asp Pro Glu Met Gly Gly Lys Pro
               420                 425                 430
   Arg Arg Lys Asn Pro Gln Glu Gly Leu Tyr Asn Glu Leu Gln Lys Asp
           435                 440                 445
   Lys Met Ala Glu Ala Tyr Ser Glu Ile Gly Met Lys Gly Glu Arg Arg
       450                 455                 460
   Arg Gly Lys Gly His Asp Gly Leu Tyr Gln Gly Leu Ser Thr Ala Thr
   465                 470                 475                 480
   Lys Asp Thr Tyr Asp Ala Leu His Met Gln Ala Leu Pro Pro Arg
                   485                 490                 495
<210> 53
   <211> 792
   <212> PRT
   <213> Artificial Sequence
<220>
   <223> chimeric antigen receptor sequence
<400> 53
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Leu Val Lys Pro Gly Ala Ser Val Lys Ile Ser Cys Lys Ala Ser Gly
           35                  40                  45
   Tyr Ala Phe Ser Ser Tyr Trp Met Asn Trp Val Lys Gln Arg Pro Gly
       50                  55                  60
   Lys Gly Leu Glu Trp Ile Gly Gln Ile Tyr Pro Gly Asp Gly Asp Thr
   65                  70                  75                  80
   Thr Tyr Asn Gly Lys Phe Lys Gly Lys Ala Thr Leu Thr Ala Asp Lys
                   85                  90                  95
   Ser Ser Ser Thr Val Tyr Met Gln Leu Asn Ser Leu Thr Ser Glu Asp
               100                 105                 110
   Ser Ala Val Tyr Phe Cys Val Arg Tyr Tyr Tyr Gly Ser Ser Gly Tyr
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Asp Ser Leu Ala Val Ser Leu Gly Gln Arg Ala
                   165                 170                 175
   Thr Ile Ser Cys Arg Ala Ser Glu Ser Val Asp Asn Tyr Gly Ile Ser
               180                 185                 190
   Phe Met His Trp Tyr Gln Gln Lys Pro Gly Gln Ser Pro Lys Leu Leu
           195                 200                 205
   Ile Tyr Arg Ala Ser Asn Leu Glu Ser Gly Ile Pro Ala Arg Phe Ser
       210                 215                 220
   Gly Ser Gly Ser Arg Thr Asp Phe Thr Leu Thr Ile Asn Pro Val Glu
   225 230 235 240
   Thr Asp Asp Val Ala Thr Tyr Tyr Cys Gln Gln Asn Asn Lys Asp Pro
   245 250 255
   Pro Thr Phe Gly Gly Gly Thr Lys Leu Glu Ile Lys Arg Ala Glu Pro
   260 265 270
   Lys Ser Cys Asp Lys Thr His Thr Cys Pro Pro Cys Pro Ala Pro Glu
   275 280 285
   Leu Leu Gly Gly Pro Ser Val Phe Leu Phe Pro Pro Lys Pro Lys Asp
   290 295 300
   Thr Leu Met Ile Ser Arg Thr Pro Glu Val Thr Cys Val Val Val Asp
   305 310 315 320
   Val Ser His Glu Asp Pro Glu Val Lys Phe Asn Trp Tyr Val Asp Gly
   325 330 335
   Val Glu Val His Asn Ala Lys Thr Lys Pro Arg Glu Glu Gln Tyr Asn
   340 345 350
   Ser Thr Tyr Arg Val Val Ser Val Leu Thr Val Leu His Gln Asp Trp
   355 360 365
   Leu Asn Gly Lys Glu Tyr Lys Cys Lys Val Ser Asn Lys Ala Leu Pro
   370 375 380
   Ala Pro Ile Glu Lys Thr Ile Ser Lys Ala Lys Gly Gln Pro Arg Glu
   385 390 395 400
   Pro Gln Val Tyr Thr Leu Pro Pro Ser Arg Asp Glu Leu Thr Lys Asn
   405 410 415
   Gln Val Ser Leu Thr Cys Leu Val Lys Gly Phe Tyr Pro Ser Asp Ile
   420 425 430
   Ala Val Glu Trp Glu Ser Asn Gly Gln Pro Glu Asn Asn Tyr Lys Thr
   435 440 445
   Thr Pro Pro Val Leu Asp Ser Asp Gly Ser Phe Phe Leu Tyr Ser Lys
   450 455 460
   Leu Thr Val Asp Lys Ser Arg Trp Gln Gln Gly Asn Val Phe Ser Cys
   465 470 475 480
   Ser Val Met His Glu Ala Leu His Asn His Tyr Thr Gln Lys Ser Leu
                   485                 490                 495
   Ser Leu Ser Pro Gly Lys Lys Asp Pro Lys Ala Ala Ala Phe Val Pro
               500                 505                 510
   Val Phe Leu Pro Ala Lys Pro Thr Thr Thr Pro Ala Pro Arg Pro Pro
           515                 520                 525
   Thr Pro Ala Pro Thr Ile Ala Ser Gln Pro Leu Ser Leu Arg Pro Glu
       530                 535                 540
   Ala Cys Arg Pro Ala Ala Gly Gly Ala Val His Thr Arg Gly Leu Asp
   545                 550                 555                 560
   Phe Ala Cys Asp Ile Tyr Ile Trp Ala Pro Leu Ala Gly Thr Cys Gly
                   565                 570                 575
   Val Leu Leu Leu Ser Leu Val Ile Thr Leu Tyr Cys Asn His Arg Asn
               580                 585                 590
   Arg Ser Lys Arg Ser Arg Leu Leu His Ser Asp Tyr Met Asn Met Thr
           595                 600                 605
   Pro Arg Arg Pro Gly Pro Thr Arg Lys His Tyr Gln Pro Tyr Ala Pro
       610                 615                 620
   Pro Arg Asp Phe Ala Ala Tyr Arg Ser Arg Phe Ser Val Val Lys Arg
   625                 630                 635                 640
   Gly Arg Lys Lys Leu Leu Tyr Ile Phe Lys Gln Pro Phe Met Arg Pro
                   645                 650                 655
   Val Gln Thr Thr Gln Glu Glu Asp Gly Cys Ser Cys Arg Phe Pro Glu
               660                 665                 670
   Glu Glu Glu Gly Gly Cys Glu Leu Arg Val Lys Phe Ser Arg Ser Ala
           675                 680                 685
   Asp Ala Pro Ala Tyr Gln Gln Gly Gln Asn Gln Leu Tyr Asn Glu Leu
       690                 695                 700
   Asn Leu Gly Arg Arg Glu Glu Tyr Asp Val Leu Asp Lys Arg Arg Gly
   705                 710                 715                 720
   Arg Asp Pro Glu Met Gly Gly Lys Pro Arg Arg Lys Asn Pro Gln Glu
                   725                 730                 735
   Gly Leu Tyr Asn Glu Leu Gln Lys Asp Lys Met Ala Glu Ala Tyr Ser
   740 745 750
   Glu Ile Gly Met Lys Gly Glu Arg Arg Arg Gly Lys Gly His Asp Gly
   755 760 765
   Leu Tyr Gln Gly Leu Ser Thr Ala Thr Lys Asp Thr Tyr Asp Ala Leu
   770 775 780
   His Met Gln Ala Leu Pro Pro Arg
   785 790
<210> 54
   <211> 555
   <212> PRT
   <213> Artificial Sequence
<220>
   <223> chimeric antigen receptor sequence
<400> 54
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Leu Val Lys Pro Gly Ala Ser Val Lys Ile Ser Cys Lys Ala Ser Gly
   35 40 45
   Tyr Ala Phe Ser Ser Tyr Trp Met Asn Trp Val Lys Gln Arg Pro Gly
   50 55 60
   Lys Gly Leu Glu Trp Ile Gly Gln Ile Tyr Pro Gly Asp Gly Asp Thr
   65 70 75 80
   Thr Tyr Asn Gly Lys Phe Lys Gly Lys Ala Thr Leu Thr Ala Asp Lys
   85 90 95
   Ser Ser Ser Thr Val Tyr Met Gln Leu Asn Ser Leu Thr Ser Glu Asp
   100 105 110
   Ser Ala Val Tyr Phe Cys Val Arg Tyr Tyr Tyr Gly Ser Ser Gly Tyr
   115 120 125
   Phe Asp Tyr Trp Gly Gln Gly Thr Thr Leu Thr Val Ser Ser Gly Gly
   130 135 140
   Gly Gly Ser Gly Gly Gly Gly Ser Gly Gly Gly Gly Ser Asp Val Gln
   145                 150                 155                 160
   Met Ile Gln Thr Pro Asp Ser Leu Ala Val Ser Leu Gly Gln Arg Ala
                   165                 170                 175
   Thr Ile Ser Cys Arg Ala Ser Glu Ser Val Asp Asn Tyr Gly Ile Ser
               180                 185                 190
   Phe Met His Trp Tyr Gln Gln Lys Pro Gly Gln Ser Pro Lys Leu Leu
           195                 200                 205
   Ile Tyr Arg Ala Ser Asn Leu Glu Ser Gly Ile Pro Ala Arg Phe Ser
       210                 215                 220
   Gly Ser Gly Ser Arg Thr Asp Phe Thr Leu Thr Ile Asn Pro Val Glu
   225                 230                 235                 240
   Thr Asp Asp Val Ala Thr Tyr Tyr Cys Gln Gln Asn Asn Lys Asp Pro
                   245                 250                 255
   Pro Thr Phe Gly Gly Gly Thr Lys Leu Glu Ile Lys Arg Ala Ala Ala
               260                 265                 270
   Phe Val Pro Val Phe Leu Pro Ala Lys Pro Thr Thr Thr Pro Ala Pro
           275                 280                 285
   Arg Pro Pro Thr Pro Ala Pro Thr Ile Ala Ser Gln Pro Leu Ser Leu
       290                 295                 300
   Arg Pro Glu Ala Cys Arg Pro Ala Ala Gly Gly Ala Val His Thr Arg
   305                 310                 315                 320
   Gly Leu Asp Phe Ala Cys Asp Ile Tyr Ile Trp Ala Pro Leu Ala Gly
                   325                 330                 335
   Thr Cys Gly Val Leu Leu Leu Ser Leu Val Ile Thr Leu Tyr Cys Asn
               340                 345                 350
   His Arg Asn Arg Ser Lys Arg Ser Arg Leu Leu His Ser Asp Tyr Met
           355                 360                 365
   Asn Met Thr Pro Arg Arg Pro Gly Pro Thr Arg Lys His Tyr Gln Pro
       370                 375                 380
   Tyr Ala Pro Pro Arg Asp Phe Ala Ala Tyr Arg Ser Arg Phe Ser Val
   385                 390                 395                 400
   Val Lys Arg Gly Arg Lys Lys Leu Leu Tyr Ile Phe Lys Gln Pro Phe
   405 410 415
   Met Arg Pro Val Gln Thr Thr Gln Glu Glu Asp Gly Cys Ser Cys Arg
   420 425 430
   Phe Pro Glu Glu Glu Glu Gly Gly Cys Glu Leu Arg Val Lys Phe Ser
   435 440 445
   Arg Ser Ala Asp Ala Pro Ala Tyr Gln Gln Gly Gln Asn Gln Leu Tyr
   450 455 460
   Asn Glu Leu Asn Leu Gly Arg Arg Glu Glu Tyr Asp Val Leu Asp Lys
   465 470 475 480
   Arg Arg Gly Arg Asp Pro Glu Met Gly Gly Lys Pro Arg Arg Lys Asn
   485 490 495
   Pro Gln Glu Gly Leu Tyr Asn Glu Leu Gln Lys Asp Lys Met Ala Glu
   500 505 510
   Ala Tyr Ser Glu Ile Gly Met Lys Gly Glu Arg Arg Arg Gly Lys Gly
   515 520 525
   His Asp Gly Leu Tyr Gln Gly Leu Ser Thr Ala Thr Lys Asp Thr Tyr
   530 535 540
   Asp Ala Leu His Met Gln Ala Leu Pro Pro Arg
   545 550 555
<210> 55
   <211> 725
   <212> PRT
   <213> Artificial Sequence
<220>
   <223> chimeric antigen receptor sequence
<400> 55
   Leu Leu Val Thr Ser Leu Leu Leu Cys Glu Leu Pro His Pro Ala Phe
   1 5 10 15
   Leu Leu Ile Pro Asp Thr Asp Val Gln Leu Gln Glu Ser Gly Pro Val
   20 25 30
   Leu Val Lys Thr Gly Ala Ser Val Lys Met Ser Cys Thr Ala Ser Gly
   35 40 45
   Tyr Thr Phe Thr Asp His Phe Met Asp Trp Val Lys Gln Ser His Gly
   50 55 60
   Lys Ser Leu Glu Trp Ile Gly Ser Leu Asn Pro Tyr Ser Gly Gly Thr
   65 70 75 80
   Ser Tyr Asn Gln Lys Phe Lys Gly Lys Ala Thr Leu Thr Val Asp Lys
   85 90 95
   Ser Ser Ser Thr Ala Tyr Met Glu Leu Asn Ser Leu Thr Ser Val Asp
   100 105 110
   Ser Ala Val Tyr Tyr Cys Ala Arg His Asn Trp Gly Ala Tyr Phe Asp
   115 120 125
   Tyr Trp Gly Gln Gly Thr Thr Leu Thr Val Ser Ser Gly Gly Gly Gly
   130 135 140
   Ser Gly Gly Gly Gly Ser Gly Gly Gly Gly Ser Asp Ile Val Met Thr
   145 150 155 160
   Gln Ala Ala Pro Ser Val Pro Val Thr Pro Gly Glu Ser Val Ser Ile
   165 170 175
   Ser Cys Arg Ser Ser Lys Ser Leu Leu His Ser Asn Gly Asn Thr Tyr
   180 185 190
   Leu Tyr Trp Phe Leu Gln Arg Pro Gly Gln Ser Pro Gln Arg Leu Ile
   195 200 205
   Tyr Tyr Met Ser Asn Leu Ala Ser Gly Val Pro Asp Arg Phe Ser Gly
   210 215 220
   Arg Gly Ser Gly Thr Asp Phe Thr Leu Arg Ile Ser Arg Val Glu Ala
   225 230 235 240
   Glu Asp Val Gly Val Tyr Tyr Cys Met Gln Gly Leu Glu Asp Pro Tyr
   245 250 255
   Thr Phe Gly Gly Gly Thr Lys Leu Glu Ile Lys Glu Pro Lys Ser Cys
   260 265 270
   Asp Lys Thr His Thr Cys Pro Pro Cys Pro Ala Pro Glu Leu Leu Gly
   275 280 285
   Gly Pro Ser Val Phe Leu Phe Pro Pro Lys Pro Lys Asp Thr Leu Met
   290 295 300
   Ile Ser Arg Thr Pro Glu Val Thr Cys Val Val Val Asp Val Ser His
   305 310 315 320
   Glu Asp Pro Glu Val Lys Phe Asn Trp Tyr Val Asp Gly Val Glu Val
   325 330 335
   His Asn Ala Lys Thr Lys Pro Arg Glu Glu Gln Tyr Asn Ser Thr Tyr
   340 345 350
   Arg Val Val Ser Val Leu Thr Val Leu His Gln Asp Trp Leu Asn Gly
   355 360 365
   Lys Glu Tyr Lys Cys Lys Val Ser Asn Lys Ala Leu Pro Ala Pro Ile
   370 375 380
   Glu Lys Thr Ile Ser Lys Ala Lys Gly Gln Pro Arg Glu Pro Gln Val
   385 390 395 400
   Tyr Thr Leu Pro Pro Ser Arg Asp Glu Leu Thr Lys Asn Gln Val Ser
   405 410 415
   Leu Thr Cys Leu Val Lys Gly Phe Tyr Pro Ser Asp Ile Ala Val Glu
   420 425 430
   Trp Glu Ser Asn Gly Gln Pro Glu Asn Asn Tyr Lys Thr Thr Pro Pro
   435 440 445
   Val Leu Asp Ser Asp Gly Ser Phe Phe Leu Tyr Ser Lys Leu Thr Val
   450 455 460
   Asp Lys Ser Arg Trp Gln Gln Gly Asn Val Phe Ser Cys Ser Val Met
   465 470 475 480
   His Glu Ala Leu His Asn His Tyr Thr Gln Lys Ser Leu Ser Leu Ser
   485 490 495
   Pro Gly Lys Lys Asp Pro Lys Ala Ala Ala Ile Glu Val Met Tyr Pro
   500 505 510
   Pro Pro Tyr Leu Asp Asn Glu Lys Ser Asn Gly Thr Ile Ile His Val
   515 520 525
   Lys Gly Lys His Leu Cys Pro Ser Pro Leu Phe Pro Gly Pro Ser Lys
   530 535 540
   Pro Phe Trp Val Leu Val Val Val Gly Gly Val Leu Ala Cys Tyr Ser
   545 550 555 560
   Leu Leu Val Thr Val Ala Phe Ile Ile Phe Trp Val Arg Ser Lys Arg
   565 570 575
   Ser Arg Leu Leu His Ser Asp Tyr Met Asn Met Thr Pro Arg Arg Pro
   580 585 590
   Gly Pro Thr Arg Lys His Tyr Gln Pro Tyr Ala Pro Pro Arg Asp Phe
   595 600 605
   Ala Ala Tyr Arg Ser Arg Val Lys Phe Ser Arg Ser Ala Asp Ala Pro
   610 615 620
   Ala Tyr Gln Gln Gly Gln Asn Gln Leu Tyr Asn Glu Leu Asn Leu Gly
   625 630 635 640
   Arg Arg Glu Glu Tyr Asp Val Leu Asp Lys Arg Arg Gly Arg Asp Pro
   645 650 655
   Glu Met Gly Gly Lys Pro Arg Arg Lys Asn Pro Gln Glu Gly Leu Tyr
   660 665 670
   Asn Glu Leu Gln Lys Asp Lys Met Ala Glu Ala Tyr Ser Glu Ile Gly
   675 680 685
   Met Lys Gly Glu Arg Arg Arg Gly Lys Gly His Asp Gly Leu Tyr Gln
   690 695 700
   Gly Leu Ser Thr Ala Thr Lys Asp Thr Tyr Asp Ala Leu His Met Gln
   705 710 715 720
   Ala Leu Pro Pro Arg
   725
<210> 56
   <211> 489
   <212> PRT
   <213> Artificial Sequence
<220>
   <223> chimeric antigen receptor sequence
<400> 56
   Leu Leu Val Thr Ser Leu Leu Leu Cys Glu Leu Pro His Pro Ala Phe
   1               5                   10                  15
   Leu Leu Ile Pro Asp Thr Asp Val Gln Leu Gln Glu Ser Gly Pro Val
               20                  25                  30
   Leu Val Lys Thr Gly Ala Ser Val Lys Met Ser Cys Thr Ala Ser Gly
   35 40 45
   Tyr Thr Phe Thr Asp His Phe Met Asp Trp Val Lys Gln Ser His Gly
   50 55 60
   Lys Ser Leu Glu Trp Ile Gly Ser Leu Asn Pro Tyr Ser Gly Gly Thr
   65 70 75 80
   Ser Tyr Asn Gln Lys Phe Lys Gly Lys Ala Thr Leu Thr Val Asp Lys
   85 90 95
   Ser Ser Ser Thr Ala Tyr Met Glu Leu Asn Ser Leu Thr Ser Val Asp
   100 105 110
   Ser Ala Val Tyr Tyr Cys Ala Arg His Asn Trp Gly Ala Tyr Phe Asp
   115 120 125
   Tyr Trp Gly Gln Gly Thr Thr Leu Thr Val Ser Ser Gly Gly Gly Gly
   130 135 140
   Ser Gly Gly Gly Gly Ser Gly Gly Gly Gly Ser Asp Ile Val Met Thr
   145 150 155 160
   Gln Ala Ala Pro Ser Val Pro Val Thr Pro Gly Glu Ser Val Ser Ile
   165 170 175
   Ser Cys Arg Ser Ser Lys Ser Leu Leu His Ser Asn Gly Asn Thr Tyr
   180 185 190
   Leu Tyr Trp Phe Leu Gln Arg Pro Gly Gln Ser Pro Gln Arg Leu Ile
   195 200 205
   Tyr Tyr Met Ser Asn Leu Ala Ser Gly Val Pro Asp Arg Phe Ser Gly
   210 215 220
   Arg Gly Ser Gly Thr Asp Phe Thr Leu Arg Ile Ser Arg Val Glu Ala
   225 230 235 240
   Glu Asp Val Gly Val Tyr Tyr Cys Met Gln Gly Leu Glu Asp Pro Tyr
   245 250 255
   Thr Phe Gly Gly Gly Thr Lys Leu Glu Ile Lys Ala Ala Ala Ile Glu
   260 265 270
   Val Met Tyr Pro Pro Pro Tyr Leu Asp Asn Glu Lys Ser Asn Gly Thr
   275 280 285
   Ile Ile His Val Lys Gly Lys His Leu Cys Pro Ser Pro Leu Phe Pro
   290 295 300
   Gly Pro Ser Lys Pro Phe Trp Val Leu Val Val Val Gly Gly Val Leu
   305 310 315 320
   Ala Cys Tyr Ser Leu Leu Val Thr Val Ala Phe Ile Ile Phe Trp Val
   325 330 335
   Arg Ser Lys Arg Ser Arg Leu Leu His Ser Asp Tyr Met Asn Met Thr
   340 345 350
   Pro Arg Arg Pro Gly Pro Thr Arg Lys His Tyr Gln Pro Tyr Ala Pro
   355 360 365
   Pro Arg Asp Phe Ala Ala Tyr Arg Ser Arg Val Lys Phe Ser Arg Ser
   370 375 380
   Ala Asp Ala Pro Ala Tyr Gln Gln Gly Gln Asn Gln Leu Tyr Asn Glu
   385 390 395 400
   Leu Asn Leu Gly Arg Arg Glu Glu Tyr Asp Val Leu Asp Lys Arg Arg
   405 410 415
   Gly Arg Asp Pro Glu Met Gly Gly Lys Pro Arg Arg Lys Asn Pro Gln
   420 425 430
   Glu Gly Leu Tyr Asn Glu Leu Gln Lys Asp Lys Met Ala Glu Ala Tyr
   435 440 445
   Ser Glu Ile Gly Met Lys Gly Glu Arg Arg Arg Gly Lys Gly His Asp
   450 455 460
   Gly Leu Tyr Gln Gly Leu Ser Thr Ala Thr Lys Asp Thr Tyr Asp Ala
   465 470 475 480
   Leu His Met Gln Ala Leu Pro Pro Arg
   485
<210> 57
   <211> 729
   <212> PRT
   <213> Artificial Sequence
<220>
   <223> chimeric antigen receptor sequence
<400> 57
   Leu Leu Val Thr Ser Leu Leu Leu Cys Glu Leu Pro His Pro Ala Phe
   1               5                   10                  15
   Leu Leu Ile Pro Asp Thr Asp Val Gln Leu Gln Glu Ser Gly Pro Val
               20                  25                  30
   Leu Val Lys Thr Gly Ala Ser Val Lys Met Ser Cys Thr Ala Ser Gly
           35                  40                  45
   Tyr Thr Phe Thr Asp His Phe Met Asp Trp Val Lys Gln Ser His Gly
       50                  55                  60
   Lys Ser Leu Glu Trp Ile Gly Ser Leu Asn Pro Tyr Ser Gly Gly Thr
   65                  70                  75                  80
   Ser Tyr Asn Gln Lys Phe Lys Gly Lys Ala Thr Leu Thr Val Asp Lys
                   85                  90                  95
   Ser Ser Ser Thr Ala Tyr Met Glu Leu Asn Ser Leu Thr Ser Val Asp
               100                 105                 110
   Ser Ala Val Tyr Tyr Cys Ala Arg His Asn Trp Gly Ala Tyr Phe Asp
           115                 120                 125
   Tyr Trp Gly Gln Gly Thr Thr Leu Thr Val Ser Ser Gly Gly Gly Gly
       130                 135                 140
   Ser Gly Gly Gly Gly Ser Gly Gly Gly Gly Ser Asp Ile Val Met Thr
   145                 150                 155                 160
   Gln Ala Ala Pro Ser Val Pro Val Thr Pro Gly Glu Ser Val Ser Ile
                   165                 170                 175
   Ser Cys Arg Ser Ser Lys Ser Leu Leu His Ser Asn Gly Asn Thr Tyr
               180                 185                 190
   Leu Tyr Trp Phe Leu Gln Arg Pro Gly Gln Ser Pro Gln Arg Leu Ile
           195                 200                 205
   Tyr Tyr Met Ser Asn Leu Ala Ser Gly Val Pro Asp Arg Phe Ser Gly
       210                 215                 220
   Arg Gly Ser Gly Thr Asp Phe Thr Leu Arg Ile Ser Arg Val Glu Ala
   225                 230                 235                 240
   Glu Asp Val Gly Val Tyr Tyr Cys Met Gln Gly Leu Glu Asp Pro Tyr
                   245                 250                 255
   Thr Phe Gly Gly Gly Thr Lys Leu Glu Ile Lys Glu Pro Lys Ser Cys
               260                 265                 270
   Asp Lys Thr His Thr Cys Pro Pro Cys Pro Ala Pro Glu Leu Leu Gly
           275                 280                 285
   Gly Pro Ser Val Phe Leu Phe Pro Pro Lys Pro Lys Asp Thr Leu Met
       290                 295                 300
   Ile Ser Arg Thr Pro Glu Val Thr Cys Val Val Val Asp Val Ser His
   305                 310                 315                 320
   Glu Asp Pro Glu Val Lys Phe Asn Trp Tyr Val Asp Gly Val Glu Val
                   325                 330                 335
   His Asn Ala Lys Thr Lys Pro Arg Glu Glu Gln Tyr Asn Ser Thr Tyr
               340                 345                 350
   Arg Val Val Ser Val Leu Thr Val Leu His Gln Asp Trp Leu Asn Gly
           355                 360                 365
   Lys Glu Tyr Lys Cys Lys Val Ser Asn Lys Ala Leu Pro Ala Pro Ile
       370                 375                 380
   Glu Lys Thr Ile Ser Lys Ala Lys Gly Gln Pro Arg Glu Pro Gln Val
   385                 390                 395                 400
   Tyr Thr Leu Pro Pro Ser Arg Asp Glu Leu Thr Lys Asn Gln Val Ser
                   405                 410                 415
   Leu Thr Cys Leu Val Lys Gly Phe Tyr Pro Ser Asp Ile Ala Val Glu
               420                 425                 430
   Trp Glu Ser Asn Gly Gln Pro Glu Asn Asn Tyr Lys Thr Thr Pro Pro
           435                 440                 445
   Val Leu Asp Ser Asp Gly Ser Phe Phe Leu Tyr Ser Lys Leu Thr Val
       450                 455                 460
   Asp Lys Ser Arg Trp Gln Gln Gly Asn Val Phe Ser Cys Ser Val Met
   465                 470                 475                 480
   His Glu Ala Leu His Asn His Tyr Thr Gln Lys Ser Leu Ser Leu Ser
                   485                 490                 495
   Pro Gly Lys Lys Asp Pro Lys Ala Ala Ala Thr Thr Thr Pro Ala Pro
               500                 505                 510
   Arg Pro Pro Thr Pro Ala Pro Thr Ile Ala Ser Gln Pro Leu Ser Leu
           515                 520                 525
   Arg Pro Glu Ala Cys Arg Pro Ala Ala Gly Gly Ala Val His Thr Arg
       530                 535                 540
   Gly Leu Asp Phe Ala Cys Asp Ile Tyr Ile Trp Ala Pro Leu Ala Gly
   545                 550                 555                 560
   Thr Cys Gly Val Leu Leu Leu Ser Leu Val Ile Thr Leu Tyr Cys Lys
                   565                 570                 575
   Arg Gly Arg Lys Lys Leu Leu Tyr Ile Phe Lys Gln Pro Phe Met Arg
               580                 585                 590
   Pro Val Gln Thr Thr Gln Glu Glu Asp Gly Cys Ser Cys Arg Phe Pro
           595                 600                 605
   Glu Glu Glu Glu Gly Gly Cys Glu Leu Arg Val Lys Phe Ser Arg Ser
       610                 615                 620
   Ala Asp Ala Pro Ala Tyr Lys Gln Gly Gln Asn Gln Leu Tyr Asn Glu
   625                 630                 635                 640
   Leu Asn Leu Gly Arg Arg Glu Glu Tyr Asp Val Leu Asp Lys Arg Arg
                   645                 650                 655
   Gly Arg Asp Pro Glu Met Gly Gly Lys Pro Arg Arg Lys Asn Pro Gln
               660                 665                 670
   Glu Gly Leu Tyr Asn Glu Leu Gln Lys Asp Lys Met Ala Glu Ala Tyr
           675                 680                 685
   Ser Glu Ile Gly Met Lys Gly Glu Arg Arg Arg Gly Lys Gly His Asp
       690                 695                 700
   Gly Leu Tyr Gln Gly Leu Ser Thr Ala Thr Lys Asp Thr Tyr Asp Ala
   705                 710                 715                 720
   Leu His Met Gln Ala Leu Pro Pro Arg
                   725
<210> 58
   <211> 493
   <212> PRT
   <213> Artificial Sequence
<220>
   <223> chimeric antigen receptor sequence
<400> 58
   Leu Leu Val Thr Ser Leu Leu Leu Cys Glu Leu Pro His Pro Ala Phe
   1               5                   10                  15
   Leu Leu Ile Pro Asp Thr Asp Val Gln Leu Gln Glu Ser Gly Pro Val
               20                  25                  30
   Leu Val Lys Thr Gly Ala Ser Val Lys Met Ser Cys Thr Ala Ser Gly
           35                  40                  45
   Tyr Thr Phe Thr Asp His Phe Met Asp Trp Val Lys Gln Ser His Gly
       50                  55                  60
   Lys Ser Leu Glu Trp Ile Gly Ser Leu Asn Pro Tyr Ser Gly Gly Thr
   65                  70                  75                  80
   Ser Tyr Asn Gln Lys Phe Lys Gly Lys Ala Thr Leu Thr Val Asp Lys
                   85                  90                  95
   Ser Ser Ser Thr Ala Tyr Met Glu Leu Asn Ser Leu Thr Ser Val Asp
               100                 105                 110
   Ser Ala Val Tyr Tyr Cys Ala Arg His Asn Trp Gly Ala Tyr Phe Asp
           115                 120                 125
   Tyr Trp Gly Gln Gly Thr Thr Leu Thr Val Ser Ser Gly Gly Gly Gly
       130                 135                 140
   Ser Gly Gly Gly Gly Ser Gly Gly Gly Gly Ser Asp Ile Val Met Thr
   145                 150                 155                 160
   Gln Ala Ala Pro Ser Val Pro Val Thr Pro Gly Glu Ser Val Ser Ile
                   165                 170                 175
   Ser Cys Arg Ser Ser Lys Ser Leu Leu His Ser Asn Gly Asn Thr Tyr
               180                 185                 190
   Leu Tyr Trp Phe Leu Gln Arg Pro Gly Gln Ser Pro Gln Arg Leu Ile
           195                 200                 205
   Tyr Tyr Met Ser Asn Leu Ala Ser Gly Val Pro Asp Arg Phe Ser Gly
       210                 215                 220
   Arg Gly Ser Gly Thr Asp Phe Thr Leu Arg Ile Ser Arg Val Glu Ala
   225                 230                 235                 240
   Glu Asp Val Gly Val Tyr Tyr Cys Met Gln Gly Leu Glu Asp Pro Tyr
                   245                 250                 255
   Thr Phe Gly Gly Gly Thr Lys Leu Glu Ile Lys Ala Ala Ala Thr Thr
               260                 265                 270
   Thr Pro Ala Pro Arg Pro Pro Thr Pro Ala Pro Thr Ile Ala Ser Gln
           275                 280                 285
   Pro Leu Ser Leu Arg Pro Glu Ala Cys Arg Pro Ala Ala Gly Gly Ala
       290                 295                 300
   Val His Thr Arg Gly Leu Asp Phe Ala Cys Asp Ile Tyr Ile Trp Ala
   305                 310                 315                 320
   Pro Leu Ala Gly Thr Cys Gly Val Leu Leu Leu Ser Leu Val Ile Thr
                   325                 330                 335
   Leu Tyr Cys Lys Arg Gly Arg Lys Lys Leu Leu Tyr Ile Phe Lys Gln
               340                 345                 350
   Pro Phe Met Arg Pro Val Gln Thr Thr Gln Glu Glu Asp Gly Cys Ser
           355                 360                 365
   Cys Arg Phe Pro Glu Glu Glu Glu Gly Gly Cys Glu Leu Arg Val Lys
       370                 375                 380
   Phe Ser Arg Ser Ala Asp Ala Pro Ala Tyr Lys Gln Gly Gln Asn Gln
   385                 390                 395                 400
   Leu Tyr Asn Glu Leu Asn Leu Gly Arg Arg Glu Glu Tyr Asp Val Leu
                   405                 410                 415
   Asp Lys Arg Arg Gly Arg Asp Pro Glu Met Gly Gly Lys Pro Arg Arg
               420                 425                 430
   Lys Asn Pro Gln Glu Gly Leu Tyr Asn Glu Leu Gln Lys Asp Lys Met
           435                 440                 445
   Ala Glu Ala Tyr Ser Glu Ile Gly Met Lys Gly Glu Arg Arg Arg Gly
       450                 455                 460
   Lys Gly His Asp Gly Leu Tyr Gln Gly Leu Ser Thr Ala Thr Lys Asp
   465                 470                 475                 480
   Thr Tyr Asp Ala Leu His Met Gln Ala Leu Pro Pro Arg
   485 490
<210> 59
   <211> 789
   <212> PRT
   <213> Artificial Sequence
<220>
   <223> chimeric antigen receptor sequence
<400> 59
<210> 60
   <211> 553
   <212> PRT
   <213> Artificial Sequence
<220>
   <223> chimeric antigen receptor sequence
<400> 60
   Leu Leu Val Thr Ser Leu Leu Leu Cys Glu Leu Pro His Pro Ala Phe
   1 5 10 15
   Leu Leu Ile Pro Asp Thr Asp Val Gln Leu Gln Glu Ser Gly Pro Val
   20 25 30
   Leu Val Lys Thr Gly Ala Ser Val Lys Met Ser Cys Thr Ala Ser Gly
   35 40 45
   Tyr Thr Phe Thr Asp His Phe Met Asp Trp Val Lys Gln Ser His Gly
   50 55 60
   Lys Ser Leu Glu Trp Ile Gly Ser Leu Asn Pro Tyr Ser Gly Gly Thr
   65 70 75 80
   Ser Tyr Asn Gln Lys Phe Lys Gly Lys Ala Thr Leu Thr Val Asp Lys
   85 90 95
   Ser Ser Ser Thr Ala Tyr Met Glu Leu Asn Ser Leu Thr Ser Val Asp
   100 105 110
   Ser Ala Val Tyr Tyr Cys Ala Arg His Asn Trp Gly Ala Tyr Phe Asp
           115                 120                 125
   Tyr Trp Gly Gln Gly Thr Thr Leu Thr Val Ser Ser Gly Gly Gly Gly
       130                 135                 140
   Ser Gly Gly Gly Gly Ser Gly Gly Gly Gly Ser Asp Ile Val Met Thr
   145                 150                 155                 160
   Gln Ala Ala Pro Ser Val Pro Val Thr Pro Gly Glu Ser Val Ser Ile
                   165                 170                 175
   Ser Cys Arg Ser Ser Lys Ser Leu Leu His Ser Asn Gly Asn Thr Tyr
               180                 185                 190
   Leu Tyr Trp Phe Leu Gln Arg Pro Gly Gln Ser Pro Gln Arg Leu Ile
           195                 200                 205
   Tyr Tyr Met Ser Asn Leu Ala Ser Gly Val Pro Asp Arg Phe Ser Gly
       210                 215                 220
   Arg Gly Ser Gly Thr Asp Phe Thr Leu Arg Ile Ser Arg Val Glu Ala
   225                 230                 235                 240
   Glu Asp Val Gly Val Tyr Tyr Cys Met Gln Gly Leu Glu Asp Pro Tyr
                   245                 250                 255
   Thr Phe Gly Gly Gly Thr Lys Leu Glu Ile Lys Ala Ala Ala Phe Val
               260                 265                 270
   Pro Val Phe Leu Pro Ala Lys Pro Thr Thr Thr Pro Ala Pro Arg Pro
           275                 280                 285
   Pro Thr Pro Ala Pro Thr Ile Ala Ser Gln Pro Leu Ser Leu Arg Pro
       290                 295                 300
   Glu Ala Cys Arg Pro Ala Ala Gly Gly Ala Val His Thr Arg Gly Leu
   305                 310                 315                 320
   Asp Phe Ala Cys Asp Ile Tyr Ile Trp Ala Pro Leu Ala Gly Thr Cys
                   325                 330                 335
   Gly Val Leu Leu Leu Ser Leu Val Ile Thr Leu Tyr Cys Asn His Arg
               340                 345                 350
   Asn Arg Ser Lys Arg Ser Arg Leu Leu His Ser Asp Tyr Met Asn Met
           355                 360                 365
   Thr Pro Arg Arg Pro Gly Pro Thr Arg Lys His Tyr Gln Pro Tyr Ala
   370 375 380
   Pro Pro Arg Asp Phe Ala Ala Tyr Arg Ser Arg Phe Ser Val Val Lys
   385 390 395 400
   Arg Gly Arg Lys Lys Leu Leu Tyr Ile Phe Lys Gln Pro Phe Met Arg
   405 410 415
   Pro Val Gln Thr Thr Gln Glu Glu Asp Gly Cys Ser Cys Arg Phe Pro
   420 425 430
   Glu Glu Glu Glu Gly Gly Cys Glu Leu Arg Val Lys Phe Ser Arg Ser
   435 440 445
   Ala Asp Ala Pro Ala Tyr Gln Gln Gly Gln Asn Gln Leu Tyr Asn Glu
   450 455 460
   Leu Asn Leu Gly Arg Arg Glu Glu Tyr Asp Val Leu Asp Lys Arg Arg
   465 470 475 480
   Gly Arg Asp Pro Glu Met Gly Gly Lys Pro Arg Arg Lys Asn Pro Gln
   485 490 495
   Glu Gly Leu Tyr Asn Glu Leu Gln Lys Asp Lys Met Ala Glu Ala Tyr
   500 505 510
   Ser Glu Ile Gly Met Lys Gly Glu Arg Arg Arg Gly Lys Gly His Asp
   515 520 525
   Gly Leu Tyr Gln Gly Leu Ser Thr Ala Thr Lys Asp Thr Tyr Asp Ala
   530 535 540
   Leu His Met Gln Ala Leu Pro Pro Arg
   545 550
<210> 61
   <211> 724
   <212> PRT
   <213> Artificial Sequence
<220>
   <223> chimeric antigen receptor sequence
<400> 61
   Leu Leu Val Thr Ser Leu Leu Leu Cys Glu Leu Pro His Pro Ala Phe
   1 5 10 15
   Leu Leu Ile Pro Asp Thr Ala Leu Gln Leu Gln Gln Ser Gly Ala Glu
               20                  25                  30
   Leu Val Arg Pro Gly Ala Ser Val Thr Leu Ser Cys Lys Ala Ser Gly
           35                  40                  45
   Tyr Thr Phe Thr Asp Tyr Glu Met His Trp Val Lys Gln Thr Pro Val
       50                  55                  60
   His Gly Leu Glu Trp Ile Gly Ala Ile Asp Pro Glu Thr Gly Gly Thr
   65                  70                  75                  80
   Ala Tyr Asn Gln Lys Phe Glu Gly Lys Ala Ile Leu Thr Ala Asp Lys
                   85                  90                  95
   Ser Ser Ser Thr Ala Tyr Met Glu Leu Arg Ser Leu Thr Ser Glu Asp
               100                 105                 110
   Ser Pro Val Tyr Tyr Cys Ala Arg Arg Arg Tyr Tyr Gly Ser Ser Ser
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Ser Ser Leu Ser Ala Ser Leu Gly Asp Arg Val
                   165                 170                 175
   Thr Ile Ser Cys Arg Ala Ser Gln Asp Ile Gly Asn Tyr Leu Asn Trp
               180                 185                 190
   Tyr Gln Gln Lys Pro Asp Gly Thr Val Lys Leu Leu Ile Tyr Tyr Thr
           195                 200                 205
   Ser Arg Leu His Ser Gly Val Pro Ser Arg Phe Ser Gly Ser Gly Ser
       210                 215                 220
   Gly Thr Glu Tyr Ser Leu Thr Ile Ser Asn Leu Glu Gln Glu Asp Ile
   225                 230                 235                 240
   Ala Thr Tyr Phe Cys Gln Gln Gly Ser Ala Leu Pro Pro Thr Phe Gly
                   245                 250                 255
   Gly Gly Thr Lys Leu Glu Ile Asn Arg Ala Glu Pro Lys Ser Cys Asp
               260                 265                 270
   Lys Thr His Thr Cys Pro Pro Cys Pro Ala Pro Glu Leu Leu Gly Gly
           275                 280                 285
   Pro Ser Val Phe Leu Phe Pro Pro Lys Pro Lys Asp Thr Leu Met Ile
       290                 295                 300
   Ser Arg Thr Pro Glu Val Thr Cys Val Val Val Asp Val Ser His Glu
   305                 310                 315                 320
   Asp Pro Glu Val Lys Phe Asn Trp Tyr Val Asp Gly Val Glu Val His
                   325                 330                 335
   Asn Ala Lys Thr Lys Pro Arg Glu Glu Gln Tyr Asn Ser Thr Tyr Arg
               340                 345                 350
   Val Val Ser Val Leu Thr Val Leu His Gln Asp Trp Leu Asn Gly Lys
           355                 360                 365
   Glu Tyr Lys Cys Lys Val Ser Asn Lys Ala Leu Pro Ala Pro Ile Glu
       370                 375                 380
   Lys Thr Ile Ser Lys Ala Lys Gly Gln Pro Arg Glu Pro Gln Val Tyr
   385                 390                 395                 400
   Thr Leu Pro Pro Ser Arg Asp Glu Leu Thr Lys Asn Gln Val Ser Leu
                   405                 410                 415
   Thr Cys Leu Val Lys Gly Phe Tyr Pro Ser Asp Ile Ala Val Glu Trp
               420                 425                 430
   Glu Ser Asn Gly Gln Pro Glu Asn Asn Tyr Lys Thr Thr Pro Pro Val
           435                 440                 445
   Leu Asp Ser Asp Gly Ser Phe Phe Leu Tyr Ser Lys Leu Thr Val Asp
       450                 455                 460
   Lys Ser Arg Trp Gln Gln Gly Asn Val Phe Ser Cys Ser Val Met His
   465                 470                 475                 480
   Glu Ala Leu His Asn His Tyr Thr Gln Lys Ser Leu Ser Leu Ser Pro
                   485                 490                 495
   Gly Lys Lys Asp Pro Lys Ala Ala Ala Ile Glu Val Met Tyr Pro Pro
               500                 505                 510
   Pro Tyr Leu Asp Asn Glu Lys Ser Asn Gly Thr Ile Ile His Val Lys
               515                 520                 525
   Gly Lys His Leu Cys Pro Ser Pro Leu Phe Pro Gly Pro Ser Lys Pro
       530                 535                 540
   Phe Trp Val Leu Val Val Val Gly Gly Val Leu Ala Cys Tyr Ser Leu
   545                 550                 555                 560
   Leu Val Thr Val Ala Phe Ile Ile Phe Trp Val Arg Ser Lys Arg Ser
                   565                 570                 575
   Arg Leu Leu His Ser Asp Tyr Met Asn Met Thr Pro Arg Arg Pro Gly
               580                 585                 590
   Pro Thr Arg Lys His Tyr Gln Pro Tyr Ala Pro Pro Arg Asp Phe Ala
           595                 600                 605
   Ala Tyr Arg Ser Arg Val Lys Phe Ser Arg Ser Ala Asp Ala Pro Ala
       610                 615                 620
   Tyr Gln Gln Gly Gln Asn Gln Leu Tyr Asn Glu Leu Asn Leu Gly Arg
   625                 630                 635                 640
   Arg Glu Glu Tyr Asp Val Leu Asp Lys Arg Arg Gly Arg Asp Pro Glu
                   645                 650                 655
   Met Gly Gly Lys Pro Arg Arg Lys Asn Pro Gln Glu Gly Leu Tyr Asn
               660                 665                 670
   Glu Leu Gln Lys Asp Lys Met Ala Glu Ala Tyr Ser Glu Ile Gly Met
           675                 680                 685
   Lys Gly Glu Arg Arg Arg Gly Lys Gly His Asp Gly Leu Tyr Gln Gly
       690                 695                 700
   Leu Ser Thr Ala Thr Lys Asp Thr Tyr Asp Ala Leu His Met Gln Ala
   705                 710                 715                 720
   Leu Pro Pro Arg
<210> 62
   <211> 487
   <212> PRT
   <213> Artificial Sequence
<220>
   <223> chimeric antigen receptor sequence
<400> 62
   Leu Leu Val Thr Ser Leu Leu Leu Cys Glu Leu Pro His Pro Ala Phe
   1               5                   10                  15
   Leu Leu Ile Pro Asp Thr Ala Leu Gln Leu Gln Gln Ser Gly Ala Glu
               20                  25                  30
   Leu Val Arg Pro Gly Ala Ser Val Thr Leu Ser Cys Lys Ala Ser Gly
           35                  40                  45
   Tyr Thr Phe Thr Asp Tyr Glu Met His Trp Val Lys Gln Thr Pro Val
       50                  55                  60
   His Gly Leu Glu Trp Ile Gly Ala Ile Asp Pro Glu Thr Gly Gly Thr
   65                  70                  75                  80
   Ala Tyr Asn Gln Lys Phe Glu Gly Lys Ala Ile Leu Thr Ala Asp Lys
                   85                  90                  95
   Ser Ser Ser Thr Ala Tyr Met Glu Leu Arg Ser Leu Thr Ser Glu Asp
               100                 105                 110
   Ser Pro Val Tyr Tyr Cys Ala Arg Arg Arg Tyr Tyr Gly Ser Ser Ser
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Ser Ser Leu Ser Ala Ser Leu Gly Asp Arg Val
                   165                 170                 175
   Thr Ile Ser Cys Arg Ala Ser Gln Asp Ile Gly Asn Tyr Leu Asn Trp
               180                 185                 190
   Tyr Gln Gln Lys Pro Asp Gly Thr Val Lys Leu Leu Ile Tyr Tyr Thr
           195                 200                 205
   Ser Arg Leu His Ser Gly Val Pro Ser Arg Phe Ser Gly Ser Gly Ser
       210                 215                 220
   Gly Thr Glu Tyr Ser Leu Thr Ile Ser Asn Leu Glu Gln Glu Asp Ile
   225                 230                 235                 240
   Ala Thr Tyr Phe Cys Gln Gln Gly Ser Ala Leu Pro Pro Thr Phe Gly
   245 250 255
   Gly Gly Thr Lys Leu Glu Ile Asn Arg Ala Ala Ala Ile Glu Val Met
   260 265 270
   Tyr Pro Pro Pro Tyr Leu Asp Asn Glu Lys Ser Asn Gly Thr Ile Ile
   275 280 285
   His Val Lys Gly Lys His Leu Cys Pro Ser Pro Leu Phe Pro Gly Pro
   290 295 300
   Ser Lys Pro Phe Trp Val Leu Val Val Val Gly Gly Val Leu Ala Cys
   305 310 315 320
   Tyr Ser Leu Leu Val Thr Val Ala Phe Ile Ile Phe Trp Val Arg Ser
   325 330 335
   Lys Arg Ser Arg Leu Leu His Ser Asp Tyr Met Asn Met Thr Pro Arg
   340 345 350
   Arg Pro Gly Pro Thr Arg Lys His Tyr Gln Pro Tyr Ala Pro Pro Arg
   355 360 365
   Asp Phe Ala Ala Tyr Arg Ser Arg Val Lys Phe Ser Arg Ser Ala Asp
   370 375 380
   Ala Pro Ala Tyr Gln Gln Gly Gln Asn Gln Leu Tyr Asn Glu Leu Asn
   385 390 395 400
   Leu Gly Arg Arg Glu Glu Tyr Asp Val Leu Asp Lys Arg Arg Gly Arg
   405 410 415
   Asp Pro Glu Met Gly Gly Lys Pro Arg Arg Lys Asn Pro Gln Glu Gly
   420 425 430
   Leu Tyr Asn Glu Leu Gln Lys Asp Lys Met Ala Glu Ala Tyr Ser Glu
   435 440 445
   Ile Gly Met Lys Gly Glu Arg Arg Arg Gly Lys Gly His Asp Gly Leu
   450 455 460
   Tyr Gln Gly Leu Ser Thr Ala Thr Lys Asp Thr Tyr Asp Ala Leu His
   465 470 475 480
   Met Gln Ala Leu Pro Pro Arg
   485
<210> 63
   <211> 728
   <212> PRT
   <213> Artificial Sequence
<220>
   <223> chimeric antigen receptor sequence
<400> 63
   Leu Leu Val Thr Ser Leu Leu Leu Cys Glu Leu Pro His Pro Ala Phe
   1               5                   10                  15
   Leu Leu Ile Pro Asp Thr Ala Leu Gln Leu Gln Gln Ser Gly Ala Glu
               20                  25                  30
   Leu Val Arg Pro Gly Ala Ser Val Thr Leu Ser Cys Lys Ala Ser Gly
           35                  40                  45
   Tyr Thr Phe Thr Asp Tyr Glu Met His Trp Val Lys Gln Thr Pro Val
       50                  55                  60
   His Gly Leu Glu Trp Ile Gly Ala Ile Asp Pro Glu Thr Gly Gly Thr
   65                  70                  75                  80
   Ala Tyr Asn Gln Lys Phe Glu Gly Lys Ala Ile Leu Thr Ala Asp Lys
                   85                  90                  95
   Ser Ser Ser Thr Ala Tyr Met Glu Leu Arg Ser Leu Thr Ser Glu Asp
               100                 105                 110
   Ser Pro Val Tyr Tyr Cys Ala Arg Arg Arg Tyr Tyr Gly Ser Ser Ser
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Ser Ser Leu Ser Ala Ser Leu Gly Asp Arg Val
                   165                 170                 175
   Thr Ile Ser Cys Arg Ala Ser Gln Asp Ile Gly Asn Tyr Leu Asn Trp
               180                 185                 190
   Tyr Gln Gln Lys Pro Asp Gly Thr Val Lys Leu Leu Ile Tyr Tyr Thr
           195                 200                 205
   Ser Arg Leu His Ser Gly Val Pro Ser Arg Phe Ser Gly Ser Gly Ser
       210                 215                 220
   Gly Thr Glu Tyr Ser Leu Thr Ile Ser Asn Leu Glu Gln Glu Asp Ile
   225                 230                 235                 240
   Ala Thr Tyr Phe Cys Gln Gln Gly Ser Ala Leu Pro Pro Thr Phe Gly
                   245                 250                 255
   Gly Gly Thr Lys Leu Glu Ile Asn Arg Ala Glu Pro Lys Ser Cys Asp
               260                 265                 270
   Lys Thr His Thr Cys Pro Pro Cys Pro Ala Pro Glu Leu Leu Gly Gly
           275                 280                 285
   Pro Ser Val Phe Leu Phe Pro Pro Lys Pro Lys Asp Thr Leu Met Ile
       290                 295                 300
   Ser Arg Thr Pro Glu Val Thr Cys Val Val Val Asp Val Ser His Glu
   305                 310                 315                 320
   Asp Pro Glu Val Lys Phe Asn Trp Tyr Val Asp Gly Val Glu Val His
                   325                 330                 335
   Asn Ala Lys Thr Lys Pro Arg Glu Glu Gln Tyr Asn Ser Thr Tyr Arg
               340                 345                 350
   Val Val Ser Val Leu Thr Val Leu His Gln Asp Trp Leu Asn Gly Lys
           355                 360                 365
   Glu Tyr Lys Cys Lys Val Ser Asn Lys Ala Leu Pro Ala Pro Ile Glu
       370                 375                 380
   Lys Thr Ile Ser Lys Ala Lys Gly Gln Pro Arg Glu Pro Gln Val Tyr
   385                 390                 395                 400
   Thr Leu Pro Pro Ser Arg Asp Glu Leu Thr Lys Asn Gln Val Ser Leu
                   405                 410                 415
   Thr Cys Leu Val Lys Gly Phe Tyr Pro Ser Asp Ile Ala Val Glu Trp
               420                 425                 430
   Glu Ser Asn Gly Gln Pro Glu Asn Asn Tyr Lys Thr Thr Pro Pro Val
           435                 440                 445
   Leu Asp Ser Asp Gly Ser Phe Phe Leu Tyr Ser Lys Leu Thr Val Asp
       450                 455                 460
   Lys Ser Arg Trp Gln Gln Gly Asn Val Phe Ser Cys Ser Val Met His
   465 470 475 480
   Glu Ala Leu His Asn His Tyr Thr Gln Lys Ser Leu Ser Leu Ser Pro
   485 490 495
   Gly Lys Lys Asp Pro Lys Ala Ala Ala Thr Thr Thr Pro Ala Pro Arg
   500 505 510
   Pro Pro Thr Pro Ala Pro Thr Ile Ala Ser Gln Pro Leu Ser Leu Arg
   515 520 525
   Pro Glu Ala Cys Arg Pro Ala Ala Gly Gly Ala Val His Thr Arg Gly
   530 535 540
   Leu Asp Phe Ala Cys Asp Ile Tyr Ile Trp Ala Pro Leu Ala Gly Thr
   545 550 555 560
   Cys Gly Val Leu Leu Leu Ser Leu Val Ile Thr Leu Tyr Cys Lys Arg
   565 570 575
   Gly Arg Lys Lys Leu Leu Tyr Ile Phe Lys Gln Pro Phe Met Arg Pro
   580 585 590
   Val Gln Thr Thr Gln Glu Glu Asp Gly Cys Ser Cys Arg Phe Pro Glu
   595 600 605
   Glu Glu Glu Gly Gly Cys Glu Leu Arg Val Lys Phe Ser Arg Ser Ala
   610 615 620
   Asp Ala Pro Ala Tyr Lys Gln Gly Gln Asn Gln Leu Tyr Asn Glu Leu
   625 630 635 640
   Asn Leu Gly Arg Arg Glu Glu Tyr Asp Val Leu Asp Lys Arg Arg Gly
   645 650 655
   Arg Asp Pro Glu Met Gly Gly Lys Pro Arg Arg Lys Asn Pro Gln Glu
   660 665 670
   Gly Leu Tyr Asn Glu Leu Gln Lys Asp Lys Met Ala Glu Ala Tyr Ser
   675 680 685
   Glu Ile Gly Met Lys Gly Glu Arg Arg Arg Gly Lys Gly His Asp Gly
   690 695 700
   Leu Tyr Gln Gly Leu Ser Thr Ala Thr Lys Asp Thr Tyr Asp Ala Leu
   705 710 715 720
   His Met Gln Ala Leu Pro Pro Arg
                           725
<210> 64
   <211> 491
   <212> PRT
   <213> Artificial Sequence
<220>
   <223> chimeric antigen receptor sequence
<400> 64
   Leu Leu Val Thr Ser Leu Leu Leu Cys Glu Leu Pro His Pro Ala Phe
   1               5                   10                  15
   Leu Leu Ile Pro Asp Thr Ala Leu Gln Leu Gln Gln Ser Gly Ala Glu
               20                  25                  30
   Leu Val Arg Pro Gly Ala Ser Val Thr Leu Ser Cys Lys Ala Ser Gly
           35                  40                  45
   Tyr Thr Phe Thr Asp Tyr Glu Met His Trp Val Lys Gln Thr Pro Val
       50                  55                  60
   His Gly Leu Glu Trp Ile Gly Ala Ile Asp Pro Glu Thr Gly Gly Thr
   65                  70                  75                  80
   Ala Tyr Asn Gln Lys Phe Glu Gly Lys Ala Ile Leu Thr Ala Asp Lys
                   85                  90                  95
   Ser Ser Ser Thr Ala Tyr Met Glu Leu Arg Ser Leu Thr Ser Glu Asp
               100                 105                 110
   Ser Pro Val Tyr Tyr Cys Ala Arg Arg Arg Tyr Tyr Gly Ser Ser Ser
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Ser Ser Leu Ser Ala Ser Leu Gly Asp Arg Val
                   165                 170                 175
   Thr Ile Ser Cys Arg Ala Ser Gln Asp Ile Gly Asn Tyr Leu Asn Trp
               180                 185                 190
   Tyr Gln Gln Lys Pro Asp Gly Thr Val Lys Leu Leu Ile Tyr Tyr Thr
   195 200 205
   Ser Arg Leu His Ser Gly Val Pro Ser Arg Phe Ser Gly Ser Gly Ser
   210 215 220
   Gly Thr Glu Tyr Ser Leu Thr Ile Ser Asn Leu Glu Gln Glu Asp Ile
   225 230 235 240
   Ala Thr Tyr Phe Cys Gln Gln Gly Ser Ala Leu Pro Pro Thr Phe Gly
   245 250 255
   Gly Gly Thr Lys Leu Glu Ile Asn Arg Ala Ala Ala Thr Thr Thr Pro
   260 265 270
   Ala Pro Arg Pro Pro Thr Pro Ala Pro Thr Ile Ala Ser Gln Pro Leu
   275 280 285
   Ser Leu Arg Pro Glu Ala Cys Arg Pro Ala Ala Gly Gly Ala Val His
   290 295 300
   Thr Arg Gly Leu Asp Phe Ala Cys Asp Ile Tyr Ile Trp Ala Pro Leu
   305 310 315 320
   Ala Gly Thr Cys Gly Val Leu Leu Leu Ser Leu Val Ile Thr Leu Tyr
   325 330 335
   Cys Lys Arg Gly Arg Lys Lys Leu Leu Tyr Ile Phe Lys Gln Pro Phe
   340 345 350
   Met Arg Pro Val Gln Thr Thr Gln Glu Glu Asp Gly Cys Ser Cys Arg
   355 360 365
   Phe Pro Glu Glu Glu Glu Gly Gly Cys Glu Leu Arg Val Lys Phe Ser
   370 375 380
   Arg Ser Ala Asp Ala Pro Ala Tyr Lys Gln Gly Gln Asn Gln Leu Tyr
   385 390 395 400
   Asn Glu Leu Asn Leu Gly Arg Arg Glu Glu Tyr Asp Val Leu Asp Lys
   405 410 415
   Arg Arg Gly Arg Asp Pro Glu Met Gly Gly Lys Pro Arg Arg Lys Asn
   420 425 430
   Pro Gln Glu Gly Leu Tyr Asn Glu Leu Gln Lys Asp Lys Met Ala Glu
   435 440 445
   Ala Tyr Ser Glu Ile Gly Met Lys Gly Glu Arg Arg Arg Gly Lys Gly
       450                 455                 460
   His Asp Gly Leu Tyr Gln Gly Leu Ser Thr Ala Thr Lys Asp Thr Tyr
   465                 470                 475                 480
   Asp Ala Leu His Met Gln Ala Leu Pro Pro Arg
                   485                 490
<210> 65
   <211> 788
   <212> PRT
   <213> Artificial Sequence
<220>
   <223> chimeric antigen receptor sequence
<400> 65
   Leu Leu Val Thr Ser Leu Leu Leu Cys Glu Leu Pro His Pro Ala Phe
   1               5                   10                  15
   Leu Leu Ile Pro Asp Thr Ala Leu Gln Leu Gln Gln Ser Gly Ala Glu
               20                  25                  30
   Leu Val Arg Pro Gly Ala Ser Val Thr Leu Ser Cys Lys Ala Ser Gly
           35                  40                  45
   Tyr Thr Phe Thr Asp Tyr Glu Met His Trp Val Lys Gln Thr Pro Val
       50                  55                  60
   His Gly Leu Glu Trp Ile Gly Ala Ile Asp Pro Glu Thr Gly Gly Thr
   65                  70                  75                  80
   Ala Tyr Asn Gln Lys Phe Glu Gly Lys Ala Ile Leu Thr Ala Asp Lys
                   85                  90                  95
   Ser Ser Ser Thr Ala Tyr Met Glu Leu Arg Ser Leu Thr Ser Glu Asp
               100                 105                 110
   Ser Pro Val Tyr Tyr Cys Ala Arg Arg Arg Tyr Tyr Gly Ser Ser Ser
           115                 120                 125
   Phe Asp Tyr Trp Gly Gln Gly Thr Thr Leu Thr Val Ser Ser Gly Gly
       130                 135                 140
   Gly Gly Ser Gly Gly Gly Gly Ser Gly Gly Gly Gly Ser Asp Val Gln
   145                 150                 155                 160
   Met Ile Gln Thr Pro Ser Ser Leu Ser Ala Ser Leu Gly Asp Arg Val
                   165                 170                 175
   Thr Ile Ser Cys Arg Ala Ser Gln Asp Ile Gly Asn Tyr Leu Asn Trp
               180                 185                 190
   Tyr Gln Gln Lys Pro Asp Gly Thr Val Lys Leu Leu Ile Tyr Tyr Thr
           195                 200                 205
   Ser Arg Leu His Ser Gly Val Pro Ser Arg Phe Ser Gly Ser Gly Ser
       210                 215                 220
   Gly Thr Glu Tyr Ser Leu Thr Ile Ser Asn Leu Glu Gln Glu Asp Ile
   225                 230                 235                 240
   Ala Thr Tyr Phe Cys Gln Gln Gly Ser Ala Leu Pro Pro Thr Phe Gly
                   245                 250                 255
   Gly Gly Thr Lys Leu Glu Ile Asn Arg Ala Glu Pro Lys Ser Cys Asp
               260                 265                 270
   Lys Thr His Thr Cys Pro Pro Cys Pro Ala Pro Glu Leu Leu Gly Gly
           275                 280                 285
   Pro Ser Val Phe Leu Phe Pro Pro Lys Pro Lys Asp Thr Leu Met Ile
       290                 295                 300
   Ser Arg Thr Pro Glu Val Thr Cys Val Val Val Asp Val Ser His Glu
   305                 310                 315                 320
   Asp Pro Glu Val Lys Phe Asn Trp Tyr Val Asp Gly Val Glu Val His
                   325                 330                 335
   Asn Ala Lys Thr Lys Pro Arg Glu Glu Gln Tyr Asn Ser Thr Tyr Arg
               340                 345                 350
   Val Val Ser Val Leu Thr Val Leu His Gln Asp Trp Leu Asn Gly Lys
           355                 360                 365
   Glu Tyr Lys Cys Lys Val Ser Asn Lys Ala Leu Pro Ala Pro Ile Glu
       370                 375                 380
   Lys Thr Ile Ser Lys Ala Lys Gly Gln Pro Arg Glu Pro Gln Val Tyr
   385                 390                 395                 400
   Thr Leu Pro Pro Ser Arg Asp Glu Leu Thr Lys Asn Gln Val Ser Leu
                   405                 410                 415
   Thr Cys Leu Val Lys Gly Phe Tyr Pro Ser Asp Ile Ala Val Glu Trp
               420                 425                 430
   Glu Ser Asn Gly Gln Pro Glu Asn Asn Tyr Lys Thr Thr Pro Pro Val
           435                 440                 445
   Leu Asp Ser Asp Gly Ser Phe Phe Leu Tyr Ser Lys Leu Thr Val Asp
       450                 455                 460
   Lys Ser Arg Trp Gln Gln Gly Asn Val Phe Ser Cys Ser Val Met His
   465                 470                 475                 480
   Glu Ala Leu His Asn His Tyr Thr Gln Lys Ser Leu Ser Leu Ser Pro
                   485                 490                 495
   Gly Lys Lys Asp Pro Lys Ala Ala Ala Phe Val Pro Val Phe Leu Pro
               500                 505                 510
   Ala Lys Pro Thr Thr Thr Pro Ala Pro Arg Pro Pro Thr Pro Ala Pro
           515                 520                 525
   Thr Ile Ala Ser Gln Pro Leu Ser Leu Arg Pro Glu Ala Cys Arg Pro
       530                 535                 540
   Ala Ala Gly Gly Ala Val His Thr Arg Gly Leu Asp Phe Ala Cys Asp
   545                 550                 555                 560
   Ile Tyr Ile Trp Ala Pro Leu Ala Gly Thr Cys Gly Val Leu Leu Leu
                   565                 570                 575
   Ser Leu Val Ile Thr Leu Tyr Cys Asn His Arg Asn Arg Ser Lys Arg
               580                 585                 590
   Ser Arg Leu Leu His Ser Asp Tyr Met Asn Met Thr Pro Arg Arg Pro
           595                 600                 605
   Gly Pro Thr Arg Lys His Tyr Gln Pro Tyr Ala Pro Pro Arg Asp Phe
       610                 615                 620
   Ala Ala Tyr Arg Ser Arg Phe Ser Val Val Lys Arg Gly Arg Lys Lys
   625                 630                 635                 640
   Leu Leu Tyr Ile Phe Lys Gln Pro Phe Met Arg Pro Val Gln Thr Thr
                   645                 650                 655
   Gln Glu Glu Asp Gly Cys Ser Cys Arg Phe Pro Glu Glu Glu Glu Gly
               660                 665                 670
   Gly Cys Glu Leu Arg Val Lys Phe Ser Arg Ser Ala Asp Ala Pro Ala
   675 680 685
   Tyr Gln Gln Gly Gln Asn Gln Leu Tyr Asn Glu Leu Asn Leu Gly Arg
   690 695 700
   Arg Glu Glu Tyr Asp Val Leu Asp Lys Arg Arg Gly Arg Asp Pro Glu
   705 710 715 720
   Met Gly Gly Lys Pro Arg Arg Lys Asn Pro Gln Glu Gly Leu Tyr Asn
   725 730 735
   Glu Leu Gln Lys Asp Lys Met Ala Glu Ala Tyr Ser Glu Ile Gly Met
   740 745 750
   Lys Gly Glu Arg Arg Arg Gly Lys Gly His Asp Gly Leu Tyr Gln Gly
   755 760 765
   Leu Ser Thr Ala Thr Lys Asp Thr Tyr Asp Ala Leu His Met Gln Ala
   770 775 780
   Leu Pro Pro Arg
   785
<210> 66
   <211> 551
   <212> PRT
   <213> Artificial Sequence
<220>
   <223> chimeric antigen receptor sequence
<400> 66
   Leu Leu Val Thr Ser Leu Leu Leu Cys Glu Leu Pro His Pro Ala Phe
   1 5 10 15
   Leu Leu Ile Pro Asp Thr Ala Leu Gln Leu Gln Gln Ser Gly Ala Glu
   20 25 30
   Leu Val Arg Pro Gly Ala Ser Val Thr Leu Ser Cys Lys Ala Ser Gly
   35 40 45
   Tyr Thr Phe Thr Asp Tyr Glu Met His Trp Val Lys Gln Thr Pro Val
   50 55 60
   His Gly Leu Glu Trp Ile Gly Ala Ile Asp Pro Glu Thr Gly Gly Thr
   65 70 75 80
   Ala Tyr Asn Gln Lys Phe Glu Gly Lys Ala Ile Leu Thr Ala Asp Lys
   85 90 95
   Ser Ser Ser Thr Ala Tyr Met Glu Leu Arg Ser Leu Thr Ser Glu Asp
   100 105 110
   Ser Pro Val Tyr Tyr Cys Ala Arg Arg Arg Tyr Tyr Gly Ser Ser Ser
   115 120 125
   Phe Asp Tyr Trp Gly Gln Gly Thr Thr Leu Thr Val Ser Ser Gly Gly
   130 135 140
   Gly Gly Ser Gly Gly Gly Gly Ser Gly Gly Gly Gly Ser Asp Val Gln
   145 150 155 160
   Met Ile Gln Thr Pro Ser Ser Leu Ser Ala Ser Leu Gly Asp Arg Val
   165 170 175
   Thr Ile Ser Cys Arg Ala Ser Gln Asp Ile Gly Asn Tyr Leu Asn Trp
   180 185 190
   Tyr Gln Gln Lys Pro Asp Gly Thr Val Lys Leu Leu Ile Tyr Tyr Thr
   195 200 205
   Ser Arg Leu His Ser Gly Val Pro Ser Arg Phe Ser Gly Ser Gly Ser
   210 215 220
   Gly Thr Glu Tyr Ser Leu Thr Ile Ser Asn Leu Glu Gln Glu Asp Ile
   225 230 235 240
   Ala Thr Tyr Phe Cys Gln Gln Gly Ser Ala Leu Pro Pro Thr Phe Gly
   245 250 255
   Gly Gly Thr Lys Leu Glu Ile Asn Arg Ala Ala Ala Phe Val Pro Val
   260 265 270
   Phe Leu Pro Ala Lys Pro Thr Thr Thr Pro Ala Pro Arg Pro Pro Thr
   275 280 285
   Pro Ala Pro Thr Ile Ala Ser Gln Pro Leu Ser Leu Arg Pro Glu Ala
   290 295 300
   Cys Arg Pro Ala Ala Gly Gly Ala Val His Thr Arg Gly Leu Asp Phe
   305 310 315 320
   Ala Cys Asp Ile Tyr Ile Trp Ala Pro Leu Ala Gly Thr Cys Gly Val
   325 330 335
   Leu Leu Leu Ser Leu Val Ile Thr Leu Tyr Cys Asn His Arg Asn Arg
               340                 345                 350
   Ser Lys Arg Ser Arg Leu Leu His Ser Asp Tyr Met Asn Met Thr Pro
           355                 360                 365
   Arg Arg Pro Gly Pro Thr Arg Lys His Tyr Gln Pro Tyr Ala Pro Pro
       370                 375                 380
   Arg Asp Phe Ala Ala Tyr Arg Ser Arg Phe Ser Val Val Lys Arg Gly
   385                 390                 395                 400
   Arg Lys Lys Leu Leu Tyr Ile Phe Lys Gln Pro Phe Met Arg Pro Val
                   405                 410                 415
   Gln Thr Thr Gln Glu Glu Asp Gly Cys Ser Cys Arg Phe Pro Glu Glu
               420                 425                 430
   Glu Glu Gly Gly Cys Glu Leu Arg Val Lys Phe Ser Arg Ser Ala Asp
           435                 440                 445
   Ala Pro Ala Tyr Gln Gln Gly Gln Asn Gln Leu Tyr Asn Glu Leu Asn
       450                 455                 460
   Leu Gly Arg Arg Glu Glu Tyr Asp Val Leu Asp Lys Arg Arg Gly Arg
   465                 470                 475                 480
   Asp Pro Glu Met Gly Gly Lys Pro Arg Arg Lys Asn Pro Gln Glu Gly
                   485                 490                 495
   Leu Tyr Asn Glu Leu Gln Lys Asp Lys Met Ala Glu Ala Tyr Ser Glu
               500                 505                 510
   Ile Gly Met Lys Gly Glu Arg Arg Arg Gly Lys Gly His Asp Gly Leu
           515                 520                 525
   Tyr Gln Gly Leu Ser Thr Ala Thr Lys Asp Thr Tyr Asp Ala Leu His
       530                 535                 540
   Met Gln Ala Leu Pro Pro Arg
   545                 550
<210> 67
   <211> 728
   <212> PRT
   <213> Artificial Sequence
<220>
   <223> chimeric antigen receptor sequence
<400> 67
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Phe Ser Leu Thr Ser Tyr Ala Ile Ser Trp Val Arg Gln Ala Pro Gly
       50                  55                  60
   Gln Gly Leu Glu Trp Met Gly Gly Ile Ile Trp Ser Gly Gly Ala Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg Glu His Tyr Tyr Gly Ser Ser Ala
           115                 120                 125
   Met Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Gln Ser Ile Val His Ser Tyr Gly
               180                 185                 190
   Asn Thr Tyr Ala Trp Tyr Gln Gln Lys Pro Gly Gln Ala Pro Arg Leu
           195                 200                 205
   Leu Ile Tyr Arg Val Ser Arg Ala Thr Gly Ile Pro Ala Arg Phe Ser
       210                 215                 220
   Gly Ser Gly Ser Gly Thr Asp Phe Thr Leu Thr Ile Ser Ser Leu Glu
   225                 230                 235                 240
   Pro Glu Asp Phe Ala Val Tyr Tyr Cys Phe Gln Gly Thr His Val Pro
   245 250 255
   Tyr Thr Phe Phe Gly Gln Gly Thr Lys Leu Glu Ile Lys Arg Glu Pro
   260 265 270
   Lys Ser Cys Asp Lys Thr His Thr Cys Pro Pro Cys Pro Ala Pro Glu
   275 280 285
   Leu Leu Gly Gly Pro Ser Val Phe Leu Phe Pro Pro Lys Pro Lys Asp
   290 295 300
   Thr Leu Met Ile Ser Arg Thr Pro Glu Val Thr Cys Val Val Val Asp
   305 310 315 320
   Val Ser His Glu Asp Pro Glu Val Lys Phe Asn Trp Tyr Val Asp Gly
   325 330 335
   Val Glu Val His Asn Ala Lys Thr Lys Pro Arg Glu Glu Gln Tyr Asn
   340 345 350
   Ser Thr Tyr Arg Val Val Ser Val Leu Thr Val Leu His Gln Asp Trp
   355 360 365
   Leu Asn Gly Lys Glu Tyr Lys Cys Lys Val Ser Asn Lys Ala Leu Pro
   370 375 380
   Ala Pro Ile Glu Lys Thr Ile Ser Lys Ala Lys Gly Gln Pro Arg Glu
   385 390 395 400
   Pro Gln Val Tyr Thr Leu Pro Pro Ser Arg Asp Glu Leu Thr Lys Asn
   405 410 415
   Gln Val Ser Leu Thr Cys Leu Val Lys Gly Phe Tyr Pro Ser Asp Ile
   420 425 430
   Ala Val Glu Trp Glu Ser Asn Gly Gln Pro Glu Asn Asn Tyr Lys Thr
   435 440 445
   Thr Pro Pro Val Leu Asp Ser Asp Gly Ser Phe Phe Leu Tyr Ser Lys
   450 455 460
   Leu Thr Val Asp Lys Ser Arg Trp Gln Gln Gly Asn Val Phe Ser Cys
   465 470 475 480
   Ser Val Met His Glu Ala Leu His Asn His Tyr Thr Gln Lys Ser Leu
                   485                 490                 495
   Ser Leu Ser Pro Gly Lys Lys Asp Pro Lys Ala Ala Ala Ile Glu Val
               500                 505                 510
   Met Tyr Pro Pro Pro Tyr Leu Asp Asn Glu Lys Ser Asn Gly Thr Ile
           515                 520                 525
   Ile His Val Lys Gly Lys His Leu Cys Pro Ser Pro Leu Phe Pro Gly
       530                 535                 540
   Pro Ser Lys Pro Phe Trp Val Leu Val Val Val Gly Gly Val Leu Ala
   545                 550                 555                 560
   Cys Tyr Ser Leu Leu Val Thr Val Ala Phe Ile Ile Phe Trp Val Arg
                   565                 570                 575
   Ser Lys Arg Ser Arg Leu Leu His Ser Asp Tyr Met Asn Met Thr Pro
               580                 585                 590
   Arg Arg Pro Gly Pro Thr Arg Lys His Tyr Gln Pro Tyr Ala Pro Pro
           595                 600                 605
   Arg Asp Phe Ala Ala Tyr Arg Ser Arg Val Lys Phe Ser Arg Ser Ala
       610                 615                 620
   Asp Ala Pro Ala Tyr Gln Gln Gly Gln Asn Gln Leu Tyr Asn Glu Leu
   625                 630                 635                 640
   Asn Leu Gly Arg Arg Glu Glu Tyr Asp Val Leu Asp Lys Arg Arg Gly
                   645                 650                 655
   Arg Asp Pro Glu Met Gly Gly Lys Pro Arg Arg Lys Asn Pro Gln Glu
               660                 665                 670
   Gly Leu Tyr Asn Glu Leu Gln Lys Asp Lys Met Ala Glu Ala Tyr Ser
           675                 680                 685
   Glu Ile Gly Met Lys Gly Glu Arg Arg Arg Gly Lys Gly His Asp Gly
       690                 695                 700
   Leu Tyr Gln Gly Leu Ser Thr Ala Thr Lys Asp Thr Tyr Asp Ala Leu
   705                 710                 715                 720
   His Met Gln Ala Leu Pro Pro Arg
                   725
<210> 68
   <211> 492
   <212> PRT
   <213> Artificial Sequence
<220>
   <223> chimeric antigen receptor sequence
<400> 68
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Phe Ser Leu Thr Ser Tyr Ala Ile Ser Trp Val Arg Gln Ala Pro Gly
       50                  55                  60
   Gln Gly Leu Glu Trp Met Gly Gly Ile Ile Trp Ser Gly Gly Ala Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg Glu His Tyr Tyr Gly Ser Ser Ala
           115                 120                 125
   Met Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Gln Ser Ile Val His Ser Tyr Gly
               180                 185                 190
   Asn Thr Tyr Ala Trp Tyr Gln Gln Lys Pro Gly Gln Ala Pro Arg Leu
           195                 200                 205
   Leu Ile Tyr Arg Val Ser Arg Ala Thr Gly Ile Pro Ala Arg Phe Ser
   210 215 220
   Gly Ser Gly Ser Gly Thr Asp Phe Thr Leu Thr Ile Ser Ser Leu Glu
   225 230 235 240
   Pro Glu Asp Phe Ala Val Tyr Tyr Cys Phe Gln Gly Thr His Val Pro
   245 250 255
   Tyr Thr Phe Phe Gly Gln Gly Thr Lys Leu Glu Ile Lys Arg Ala Ala
   260 265 270
   Ala Ile Glu Val Met Tyr Pro Pro Pro Tyr Leu Asp Asn Glu Lys Ser
   275 280 285
   Asn Gly Thr Ile Ile His Val Lys Gly Lys His Leu Cys Pro Ser Pro
   290 295 300
   Leu Phe Pro Gly Pro Ser Lys Pro Phe Trp Val Leu Val Val Val Gly
   305 310 315 320
   Gly Val Leu Ala Cys Tyr Ser Leu Leu Val Thr Val Ala Phe Ile Ile
   325 330 335
   Phe Trp Val Arg Ser Lys Arg Ser Arg Leu Leu His Ser Asp Tyr Met
   340 345 350
   Asn Met Thr Pro Arg Arg Pro Gly Pro Thr Arg Lys His Tyr Gln Pro
   355 360 365
   Tyr Ala Pro Pro Arg Asp Phe Ala Ala Tyr Arg Ser Arg Val Lys Phe
   370 375 380
   Ser Arg Ser Ala Asp Ala Pro Ala Tyr Gln Gln Gly Gln Asn Gln Leu
   385 390 395 400
   Tyr Asn Glu Leu Asn Leu Gly Arg Arg Glu Glu Tyr Asp Val Leu Asp
   405 410 415
   Lys Arg Arg Gly Arg Asp Pro Glu Met Gly Gly Lys Pro Arg Arg Lys
   420 425 430
   Asn Pro Gln Glu Gly Leu Tyr Asn Glu Leu Gln Lys Asp Lys Met Ala
   435 440 445
   Glu Ala Tyr Ser Glu Ile Gly Met Lys Gly Glu Arg Arg Arg Gly Lys
   450 455 460
   Gly His Asp Gly Leu Tyr Gln Gly Leu Ser Thr Ala Thr Lys Asp Thr
   465 470 475 480
   Tyr Asp Ala Leu His Met Gln Ala Leu Pro Pro Arg
   485 490
<210> 69
   <211> 732
   <212> PRT
   <213> Artificial Sequence
<220>
   <223> chimeric antigen receptor sequence
<400> 69
<210> 70
   <211> 496
   <212> PRT
   <213> Artificial Sequence
<220>
   <223> chimeric antigen receptor sequence
<400> 70
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Val Lys Lys Pro Gly Ser Ser Val Lys Val Ser Cys Lys Ala Ser Gly
   35 40 45
   Phe Ser Leu Thr Ser Tyr Ala Ile Ser Trp Val Arg Gln Ala Pro Gly
   50 55 60
   Gln Gly Leu Glu Trp Met Gly Gly Ile Ile Trp Ser Gly Gly Ala Thr
   65 70 75 80
   Asn Tyr Ala Gln Lys Phe Gln Gly Arg Val Thr Ile Thr Ala Asp Glu
   85 90 95
   Ser Thr Ser Thr Ala Tyr Met Glu Leu Ser Ser Leu Arg Ser Glu Asp
   100 105 110
   Thr Ala Val Tyr Tyr Cys Ala Arg Glu His Tyr Tyr Gly Ser Ser Ala
   115 120 125
   Met Asp Tyr Trp Trp Gly Gln Gly Thr Met Val Thr Val Ser Ser Gly
   130 135 140
   Gly Gly Gly Ser Gly Gly Gly Gly Ser Gly Gly Gly Gly Ser Glu Ile
   145 150 155 160
   Val Leu Thr Gln Ser Pro Ala Thr Leu Ser Leu Ser Pro Gly Glu Arg
                   165                 170                 175
   Ala Thr Leu Ser Cys Arg Ala Ser Gln Ser Ile Val His Ser Tyr Gly
               180                 185                 190
   Asn Thr Tyr Ala Trp Tyr Gln Gln Lys Pro Gly Gln Ala Pro Arg Leu
           195                 200                 205
   Leu Ile Tyr Arg Val Ser Arg Ala Thr Gly Ile Pro Ala Arg Phe Ser
       210                 215                 220
   Gly Ser Gly Ser Gly Thr Asp Phe Thr Leu Thr Ile Ser Ser Leu Glu
   225                 230                 235                 240
   Pro Glu Asp Phe Ala Val Tyr Tyr Cys Phe Gln Gly Thr His Val Pro
                   245                 250                 255
   Tyr Thr Phe Phe Gly Gln Gly Thr Lys Leu Glu Ile Lys Arg Ala Ala
               260                 265                 270
   Ala Thr Thr Thr Pro Ala Pro Arg Pro Pro Thr Pro Ala Pro Thr Ile
           275                 280                 285
   Ala Ser Gln Pro Leu Ser Leu Arg Pro Glu Ala Cys Arg Pro Ala Ala
       290                 295                 300
   Gly Gly Ala Val His Thr Arg Gly Leu Asp Phe Ala Cys Asp Ile Tyr
   305                 310                 315                 320
   Ile Trp Ala Pro Leu Ala Gly Thr Cys Gly Val Leu Leu Leu Ser Leu
                   325                 330                 335
   Val Ile Thr Leu Tyr Cys Lys Arg Gly Arg Lys Lys Leu Leu Tyr Ile
               340                 345                 350
   Phe Lys Gln Pro Phe Met Arg Pro Val Gln Thr Thr Gln Glu Glu Asp
           355                 360                 365
   Gly Cys Ser Cys Arg Phe Pro Glu Glu Glu Glu Gly Gly Cys Glu Leu
       370                 375                 380
   Arg Val Lys Phe Ser Arg Ser Ala Asp Ala Pro Ala Tyr Lys Gln Gly
   385                 390                 395                 400
   Gln Asn Gln Leu Tyr Asn Glu Leu Asn Leu Gly Arg Arg Glu Glu Tyr
                   405                 410                 415
   Asp Val Leu Asp Lys Arg Arg Gly Arg Asp Pro Glu Met Gly Gly Lys
   420 425 430
   Pro Arg Arg Lys Asn Pro Gln Glu Gly Leu Tyr Asn Glu Leu Gln Lys
   435 440 445
   Asp Lys Met Ala Glu Ala Tyr Ser Glu Ile Gly Met Lys Gly Glu Arg
   450 455 460
   Arg Arg Gly Lys Gly His Asp Gly Leu Tyr Gln Gly Leu Ser Thr Ala
   465 470 475 480
   Thr Lys Asp Thr Tyr Asp Ala Leu His Met Gln Ala Leu Pro Pro Arg
   485 490 495
<210> 71
   <211> 792
   <212> PRT
   <213> Artificial Sequence
<220>
   <223> chimeric antigen receptor sequence
<400> 71
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Val Lys Lys Pro Gly Ser Ser Val Lys Val Ser Cys Lys Ala Ser Gly
   35 40 45
   Phe Ser Leu Thr Ser Tyr Ala Ile Ser Trp Val Arg Gln Ala Pro Gly
   50 55 60
   Gln Gly Leu Glu Trp Met Gly Gly Ile Ile Trp Ser Gly Gly Ala Thr
   65 70 75 80
   Asn Tyr Ala Gln Lys Phe Gln Gly Arg Val Thr Ile Thr Ala Asp Glu
   85 90 95
   Ser Thr Ser Thr Ala Tyr Met Glu Leu Ser Ser Leu Arg Ser Glu Asp
   100 105 110
   Thr Ala Val Tyr Tyr Cys Ala Arg Glu His Tyr Tyr Gly Ser Ser Ala
   115 120 125
   Met Asp Tyr Trp Trp Gly Gln Gly Thr Met Val Thr Val Ser Ser Gly
   130 135 140
   Gly Gly Gly Ser Gly Gly Gly Gly Ser Gly Gly Gly Gly Ser Glu Ile
   145 150 155 160
   Val Leu Thr Gln Ser Pro Ala Thr Leu Ser Leu Ser Pro Gly Glu Arg
   165 170 175
   Ala Thr Leu Ser Cys Arg Ala Ser Gln Ser Ile Val His Ser Tyr Gly
   180 185 190
   Asn Thr Tyr Ala Trp Tyr Gln Gln Lys Pro Gly Gln Ala Pro Arg Leu
   195 200 205
   Leu Ile Tyr Arg Val Ser Arg Ala Thr Gly Ile Pro Ala Arg Phe Ser
   210 215 220
   Gly Ser Gly Ser Gly Thr Asp Phe Thr Leu Thr Ile Ser Ser Leu Glu
   225 230 235 240
   Pro Glu Asp Phe Ala Val Tyr Tyr Cys Phe Gln Gly Thr His Val Pro
   245 250 255
   Tyr Thr Phe Phe Gly Gln Gly Thr Lys Leu Glu Ile Lys Arg Glu Pro
   260 265 270
   Lys Ser Cys Asp Lys Thr His Thr Cys Pro Pro Cys Pro Ala Pro Glu
   275 280 285
   Leu Leu Gly Gly Pro Ser Val Phe Leu Phe Pro Pro Lys Pro Lys Asp
   290 295 300
   Thr Leu Met Ile Ser Arg Thr Pro Glu Val Thr Cys Val Val Val Asp
   305 310 315 320
   Val Ser His Glu Asp Pro Glu Val Lys Phe Asn Trp Tyr Val Asp Gly
   325 330 335
   Val Glu Val His Asn Ala Lys Thr Lys Pro Arg Glu Glu Gln Tyr Asn
   340 345 350
   Ser Thr Tyr Arg Val Val Ser Val Leu Thr Val Leu His Gln Asp Trp
   355 360 365
   Leu Asn Gly Lys Glu Tyr Lys Cys Lys Val Ser Asn Lys Ala Leu Pro
   370 375 380
   Ala Pro Ile Glu Lys Thr Ile Ser Lys Ala Lys Gly Gln Pro Arg Glu
   385                 390                 395                 400
   Pro Gln Val Tyr Thr Leu Pro Pro Ser Arg Asp Glu Leu Thr Lys Asn
                   405                 410                 415
   Gln Val Ser Leu Thr Cys Leu Val Lys Gly Phe Tyr Pro Ser Asp Ile
               420                 425                 430
   Ala Val Glu Trp Glu Ser Asn Gly Gln Pro Glu Asn Asn Tyr Lys Thr
           435                 440                 445
   Thr Pro Pro Val Leu Asp Ser Asp Gly Ser Phe Phe Leu Tyr Ser Lys
       450                 455                 460
   Leu Thr Val Asp Lys Ser Arg Trp Gln Gln Gly Asn Val Phe Ser Cys
   465                 470                 475                 480
   Ser Val Met His Glu Ala Leu His Asn His Tyr Thr Gln Lys Ser Leu
                   485                 490                 495
   Ser Leu Ser Pro Gly Lys Lys Asp Pro Lys Ala Ala Ala Phe Val Pro
               500                 505                 510
   Val Phe Leu Pro Ala Lys Pro Thr Thr Thr Pro Ala Pro Arg Pro Pro
           515                 520                 525
   Thr Pro Ala Pro Thr Ile Ala Ser Gln Pro Leu Ser Leu Arg Pro Glu
       530                 535                 540
   Ala Cys Arg Pro Ala Ala Gly Gly Ala Val His Thr Arg Gly Leu Asp
   545                 550                 555                 560
   Phe Ala Cys Asp Ile Tyr Ile Trp Ala Pro Leu Ala Gly Thr Cys Gly
                   565                 570                 575
   Val Leu Leu Leu Ser Leu Val Ile Thr Leu Tyr Cys Asn His Arg Asn
               580                 585                 590
   Arg Ser Lys Arg Ser Arg Leu Leu His Ser Asp Tyr Met Asn Met Thr
           595                 600                 605
   Pro Arg Arg Pro Gly Pro Thr Arg Lys His Tyr Gln Pro Tyr Ala Pro
       610                 615                 620
   Pro Arg Asp Phe Ala Ala Tyr Arg Ser Arg Phe Ser Val Val Lys Arg
   625                 630                 635                 640
   Gly Arg Lys Lys Leu Leu Tyr Ile Phe Lys Gln Pro Phe Met Arg Pro
                   645                 650                 655
   Val Gln Thr Thr Gln Glu Glu Asp Gly Cys Ser Cys Arg Phe Pro Glu
               660                 665                 670
   Glu Glu Glu Gly Gly Cys Glu Leu Arg Val Lys Phe Ser Arg Ser Ala
           675                 680                 685
   Asp Ala Pro Ala Tyr Gln Gln Gly Gln Asn Gln Leu Tyr Asn Glu Leu
       690                 695                 700
   Asn Leu Gly Arg Arg Glu Glu Tyr Asp Val Leu Asp Lys Arg Arg Gly
   705                 710                 715                 720
   Arg Asp Pro Glu Met Gly Gly Lys Pro Arg Arg Lys Asn Pro Gln Glu
                   725                 730                 735
   Gly Leu Tyr Asn Glu Leu Gln Lys Asp Lys Met Ala Glu Ala Tyr Ser
               740                 745                 750
   Glu Ile Gly Met Lys Gly Glu Arg Arg Arg Gly Lys Gly His Asp Gly
           755                 760                 765
   Leu Tyr Gln Gly Leu Ser Thr Ala Thr Lys Asp Thr Tyr Asp Ala Leu
       770                 775                 780
   His Met Gln Ala Leu Pro Pro Arg
   785                 790
<210> 72
   <211> 556
   <212> PRT
   <213> Artificial Sequence
<220>
   <223> chimeric antigen receptor sequence
<400> 72
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Phe Ser Leu Thr Ser Tyr Ala Ile Ser Trp Val Arg Gln Ala Pro Gly
       50                  55                  60
   Gln Gly Leu Glu Trp Met Gly Gly Ile Ile Trp Ser Gly Gly Ala Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg Glu His Tyr Tyr Gly Ser Ser Ala
           115                 120                 125
   Met Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Gln Ser Ile Val His Ser Tyr Gly
               180                 185                 190
   Asn Thr Tyr Ala Trp Tyr Gln Gln Lys Pro Gly Gln Ala Pro Arg Leu
           195                 200                 205
   Leu Ile Tyr Arg Val Ser Arg Ala Thr Gly Ile Pro Ala Arg Phe Ser
       210                 215                 220
   Gly Ser Gly Ser Gly Thr Asp Phe Thr Leu Thr Ile Ser Ser Leu Glu
   225                 230                 235                 240
   Pro Glu Asp Phe Ala Val Tyr Tyr Cys Phe Gln Gly Thr His Val Pro
                   245                 250                 255
   Tyr Thr Phe Phe Gly Gln Gly Thr Lys Leu Glu Ile Lys Arg Ala Ala
               260                 265                 270
   Ala Phe Val Pro Val Phe Leu Pro Ala Lys Pro Thr Thr Thr Pro Ala
           275                 280                 285
   Pro Arg Pro Pro Thr Pro Ala Pro Thr Ile Ala Ser Gln Pro Leu Ser
       290                 295                 300
   Leu Arg Pro Glu Ala Cys Arg Pro Ala Ala Gly Gly Ala Val His Thr
   305                 310                 315                 320
   Arg Gly Leu Asp Phe Ala Cys Asp Ile Tyr Ile Trp Ala Pro Leu Ala
                   325                 330                 335
   Gly Thr Cys Gly Val Leu Leu Leu Ser Leu Val Ile Thr Leu Tyr Cys
               340                 345                 350
   Asn His Arg Asn Arg Ser Lys Arg Ser Arg Leu Leu His Ser Asp Tyr
           355                 360                 365
   Met Asn Met Thr Pro Arg Arg Pro Gly Pro Thr Arg Lys His Tyr Gln
       370                 375                 380
   Pro Tyr Ala Pro Pro Arg Asp Phe Ala Ala Tyr Arg Ser Arg Phe Ser
   385                 390                 395                 400
   Val Val Lys Arg Gly Arg Lys Lys Leu Leu Tyr Ile Phe Lys Gln Pro
                   405                 410                 415
   Phe Met Arg Pro Val Gln Thr Thr Gln Glu Glu Asp Gly Cys Ser Cys
               420                 425                 430
   Arg Phe Pro Glu Glu Glu Glu Gly Gly Cys Glu Leu Arg Val Lys Phe
           435                 440                 445
   Ser Arg Ser Ala Asp Ala Pro Ala Tyr Gln Gln Gly Gln Asn Gln Leu
       450                 455                 460
   Tyr Asn Glu Leu Asn Leu Gly Arg Arg Glu Glu Tyr Asp Val Leu Asp
   465                 470                 475                 480
   Lys Arg Arg Gly Arg Asp Pro Glu Met Gly Gly Lys Pro Arg Arg Lys
                   485                 490                 495
   Asn Pro Gln Glu Gly Leu Tyr Asn Glu Leu Gln Lys Asp Lys Met Ala
               500                 505                 510
   Glu Ala Tyr Ser Glu Ile Gly Met Lys Gly Glu Arg Arg Arg Gly Lys
           515                 520                 525
   Gly His Asp Gly Leu Tyr Gln Gly Leu Ser Thr Ala Thr Lys Asp Thr
       530                 535                 540
   Tyr Asp Ala Leu His Met Gln Ala Leu Pro Pro Arg
   545 550 555
<210> 73
   <211> 728
   <212> PRT
   <213> Artificial Sequence
<220>
   <223> chimeric antigen receptor sequence
<400> 73
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Ala Phe Ser Ser Tyr Ile Ser Trp Val Arg Gln Ala Pro Gly Gln
       50                  55                  60
   Gly Leu Glu Trp Met Gly Gly Gln Ile Tyr Pro Gly Asp Gly Asp Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Val Arg Tyr Tyr Tyr Gly Ser Ser Gly Tyr
           115                 120                 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Glu Ser Val Asp Asn Tyr Gly Ile
               180                 185                 190
   Ser Phe Ala Trp Tyr Gln Gln Lys Pro Gly Gln Ala Pro Arg Leu Leu
           195                 200                 205
   Ile Tyr Arg Ala Ser Arg Ala Thr Gly Ile Pro Ala Arg Phe Ser Gly
       210                 215                 220
   Ser Gly Ser Gly Thr Asp Phe Thr Leu Thr Ile Ser Ser Leu Glu Pro
   225                 230                 235                 240
   Glu Asp Phe Ala Val Tyr Tyr Cys Gln Gln Asn Asn Lys Asp Pro Pro
                   245                 250                 255
   Thr Phe Phe Gly Gln Gly Thr Lys Leu Glu Ile Lys Arg Ala Glu Pro
               260                 265                 270
   Lys Ser Cys Asp Lys Thr His Thr Cys Pro Pro Cys Pro Ala Pro Glu
           275                 280                 285
   Leu Leu Gly Gly Pro Ser Val Phe Leu Phe Pro Pro Lys Pro Lys Asp
       290                 295                 300
   Thr Leu Met Ile Ser Arg Thr Pro Glu Val Thr Cys Val Val Val Asp
   305                 310                 315                 320
   Val Ser His Glu Asp Pro Glu Val Lys Phe Asn Trp Tyr Val Asp Gly
                   325                 330                 335
   Val Glu Val His Asn Ala Lys Thr Lys Pro Arg Glu Glu Gln Tyr Asn
               340                 345                 350
   Ser Thr Tyr Arg Val Val Ser Val Leu Thr Val Leu His Gln Asp Trp
           355                 360                 365
   Leu Asn Gly Lys Glu Tyr Lys Cys Lys Val Ser Asn Lys Ala Leu Pro
       370                 375                 380
   Ala Pro Ile Glu Lys Thr Ile Ser Lys Ala Lys Gly Gln Pro Arg Glu
   385                 390                 395                 400
   Pro Gln Val Tyr Thr Leu Pro Pro Ser Arg Asp Glu Leu Thr Lys Asn
                   405                 410                 415
   Gln Val Ser Leu Thr Cys Leu Val Lys Gly Phe Tyr Pro Ser Asp Ile
               420                 425                 430
   Ala Val Glu Trp Glu Ser Asn Gly Gln Pro Glu Asn Asn Tyr Lys Thr
           435                 440                 445
   Thr Pro Pro Val Leu Asp Ser Asp Gly Ser Phe Phe Leu Tyr Ser Lys
           450                 455                 460
   Leu Thr Val Asp Lys Ser Arg Trp Gln Gln Gly Asn Val Phe Ser Cys
   465                 470                 475                 480
   Ser Val Met His Glu Ala Leu His Asn His Tyr Thr Gln Lys Ser Leu
                   485                 490                 495
   Ser Leu Ser Pro Gly Lys Lys Asp Pro Lys Ala Ala Ala Ile Glu Val
               500                 505                 510
   Met Tyr Pro Pro Pro Tyr Leu Asp Asn Glu Lys Ser Asn Gly Thr Ile
           515                 520                 525
   Ile His Val Lys Gly Lys His Leu Cys Pro Ser Pro Leu Phe Pro Gly
       530                 535                 540
   Pro Ser Lys Pro Phe Trp Val Leu Val Val Val Gly Gly Val Leu Ala
   545                 550                 555                 560
   Cys Tyr Ser Leu Leu Val Thr Val Ala Phe Ile Ile Phe Trp Val Arg
                   565                 570                 575
   Ser Lys Arg Ser Arg Leu Leu His Ser Asp Tyr Met Asn Met Thr Pro
               580                 585                 590
   Arg Arg Pro Gly Pro Thr Arg Lys His Tyr Gln Pro Tyr Ala Pro Pro
           595                 600                 605
   Arg Asp Phe Ala Ala Tyr Arg Ser Arg Val Lys Phe Ser Arg Ser Ala
       610                 615                 620
   Asp Ala Pro Ala Tyr Gln Gln Gly Gln Asn Gln Leu Tyr Asn Glu Leu
   625                 630                 635                 640
   Asn Leu Gly Arg Arg Glu Glu Tyr Asp Val Leu Asp Lys Arg Arg Gly
                   645                 650                 655
   Arg Asp Pro Glu Met Gly Gly Lys Pro Arg Arg Lys Asn Pro Gln Glu
               660                 665                 670
   Gly Leu Tyr Asn Glu Leu Gln Lys Asp Lys Met Ala Glu Ala Tyr Ser
           675                 680                 685
   Glu Ile Gly Met Lys Gly Glu Arg Arg Arg Gly Lys Gly His Asp Gly
       690                 695                 700
   Leu Tyr Gln Gly Leu Ser Thr Ala Thr Lys Asp Thr Tyr Asp Ala Leu
   705                 710                 715                 720
   His Met Gln Ala Leu Pro Pro Arg
                   725
<210> 74
   <211> 491
   <212> PRT
   <213> Artificial Sequence
<220>
   <223> chimeric antigen receptor sequence
<400> 74
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Ala Phe Ser Ser Tyr Ile Ser Trp Val Arg Gln Ala Pro Gly Gln
       50                  55                  60
   Gly Leu Glu Trp Met Gly Gly Gln Ile Tyr Pro Gly Asp Gly Asp Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Val Arg Tyr Tyr Tyr Gly Ser Ser Gly Tyr
           115                 120                 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Glu Ser Val Asp Asn Tyr Gly Ile
                   180                 185                 190
   Ser Phe Ala Trp Tyr Gln Gln Lys Pro Gly Gln Ala Pro Arg Leu Leu
               195                 200                 205
   Ile Tyr Arg Ala Ser Arg Ala Thr Gly Ile Pro Ala Arg Phe Ser Gly
       210                 215                 220
   Ser Gly Ser Gly Thr Asp Phe Thr Leu Thr Ile Ser Ser Leu Glu Pro
   225                 230                 235                 240
   Glu Asp Phe Ala Val Tyr Tyr Cys Gln Gln Asn Asn Lys Asp Pro Pro
                   245                 250                 255
   Thr Phe Phe Gly Gln Gly Thr Lys Leu Glu Ile Lys Arg Ala Ala Ala
               260                 265                 270
   Ile Glu Val Met Tyr Pro Pro Pro Tyr Leu Asp Asn Glu Lys Ser Asn
           275                 280                 285
   Gly Thr Ile Ile His Val Lys Gly Lys His Leu Cys Pro Ser Pro Leu
       290                 295                 300
   Phe Pro Gly Pro Ser Lys Pro Phe Trp Val Leu Val Val Val Gly Gly
   305                 310                 315                 320
   Val Leu Ala Cys Tyr Ser Leu Leu Val Thr Val Ala Phe Ile Ile Phe
                   325                 330                 335
   Trp Val Arg Ser Lys Arg Ser Arg Leu Leu His Ser Asp Tyr Met Asn
               340                 345                 350
   Met Thr Pro Arg Arg Pro Gly Pro Thr Arg Lys His Tyr Gln Pro Tyr
           355                 360                 365
   Ala Pro Pro Arg Asp Phe Ala Ala Tyr Arg Ser Arg Val Lys Phe Ser
       370                 375                 380
   Arg Ser Ala Asp Ala Pro Ala Tyr Gln Gln Gly Gln Asn Gln Leu Tyr
   385                 390                 395                 400
   Asn Glu Leu Asn Leu Gly Arg Arg Glu Glu Tyr Asp Val Leu Asp Lys
                   405                 410                 415
   Arg Arg Gly Arg Asp Pro Glu Met Gly Gly Lys Pro Arg Arg Lys Asn
               420                 425                 430
   Pro Gln Glu Gly Leu Tyr Asn Glu Leu Gln Lys Asp Lys Met Ala Glu
   435 440 445
   Ala Tyr Ser Glu Ile Gly Met Lys Gly Glu Arg Arg Arg Gly Lys Gly
   450 455 460
   His Asp Gly Leu Tyr Gln Gly Leu Ser Thr Ala Thr Lys Asp Thr Tyr
   465 470 475 480
   Asp Ala Leu His Met Gln Ala Leu Pro Pro Arg
   485 490
<210> 75
   <211> 732
   <212> PRT
   <213> Artificial Sequence
<220>
   <223> chimeric antigen receptor sequence
<400> 75
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Ala Phe Ser Ser Tyr Ile Ser Trp Val Arg Gln Ala Pro Gly Gln
       50                  55                  60
   Gly Leu Glu Trp Met Gly Gly Gln Ile Tyr Pro Gly Asp Gly Asp Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Val Arg Tyr Tyr Tyr Gly Ser Ser Gly Tyr
           115                 120                 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145 150 155 160
   Val Leu Thr Gln Ser Pro Ala Thr Leu Ser Leu Ser Pro Gly Glu Arg
   165 170 175
   Ala Thr Leu Ser Cys Arg Ala Ser Glu Ser Val Asp Asn Tyr Gly Ile
   180 185 190
   Ser Phe Ala Trp Tyr Gln Gln Lys Pro Gly Gln Ala Pro Arg Leu Leu
   195 200 205
   Ile Tyr Arg Ala Ser Arg Ala Thr Gly Ile Pro Ala Arg Phe Ser Gly
   210 215 220
   Ser Gly Ser Gly Thr Asp Phe Thr Leu Thr Ile Ser Ser Leu Glu Pro
   225 230 235 240
   Glu Asp Phe Ala Val Tyr Tyr Cys Gln Gln Asn Asn Lys Asp Pro Pro
   245 250 255
   Thr Phe Phe Gly Gln Gly Thr Lys Leu Glu Ile Lys Arg Ala Glu Pro
   260 265 270
   Lys Ser Cys Asp Lys Thr His Thr Cys Pro Pro Cys Pro Ala Pro Glu
   275 280 285
   Leu Leu Gly Gly Pro Ser Val Phe Leu Phe Pro Pro Lys Pro Lys Asp
   290 295 300
   Thr Leu Met Ile Ser Arg Thr Pro Glu Val Thr Cys Val Val Val Asp
   305 310 315 320
   Val Ser His Glu Asp Pro Glu Val Lys Phe Asn Trp Tyr Val Asp Gly
   325 330 335
   Val Glu Val His Asn Ala Lys Thr Lys Pro Arg Glu Glu Gln Tyr Asn
   340 345 350
   Ser Thr Tyr Arg Val Val Ser Val Leu Thr Val Leu His Gln Asp Trp
   355 360 365
   Leu Asn Gly Lys Glu Tyr Lys Cys Lys Val Ser Asn Lys Ala Leu Pro
   370 375 380
   Ala Pro Ile Glu Lys Thr Ile Ser Lys Ala Lys Gly Gln Pro Arg Glu
   385 390 395 400
   Pro Gln Val Tyr Thr Leu Pro Pro Ser Arg Asp Glu Leu Thr Lys Asn
   405 410 415
   Gln Val Ser Leu Thr Cys Leu Val Lys Gly Phe Tyr Pro Ser Asp Ile
   420 425 430
   Ala Val Glu Trp Glu Ser Asn Gly Gln Pro Glu Asn Asn Tyr Lys Thr
   435 440 445
   Thr Pro Pro Val Leu Asp Ser Asp Gly Ser Phe Phe Leu Tyr Ser Lys
   450 455 460
   Leu Thr Val Asp Lys Ser Arg Trp Gln Gln Gly Asn Val Phe Ser Cys
   465 470 475 480
   Ser Val Met His Glu Ala Leu His Asn His Tyr Thr Gln Lys Ser Leu
   485 490 495
   Ser Leu Ser Pro Gly Lys Lys Asp Pro Lys Ala Ala Ala Thr Thr Thr
   500 505 510
   Pro Ala Pro Arg Pro Pro Thr Pro Ala Pro Thr Ile Ala Ser Gln Pro
   515 520 525
   Leu Ser Leu Arg Pro Glu Ala Cys Arg Pro Ala Ala Gly Gly Ala Val
   530 535 540
   His Thr Arg Gly Leu Asp Phe Ala Cys Asp Ile Tyr Ile Trp Ala Pro
   545 550 555 560
   Leu Ala Gly Thr Cys Gly Val Leu Leu Leu Ser Leu Val Ile Thr Leu
   565 570 575
   Tyr Cys Lys Arg Gly Arg Lys Lys Leu Leu Tyr Ile Phe Lys Gln Pro
   580 585 590
   Phe Met Arg Pro Val Gln Thr Thr Gln Glu Glu Asp Gly Cys Ser Cys
   595 600 605
   Arg Phe Pro Glu Glu Glu Glu Gly Gly Cys Glu Leu Arg Val Lys Phe
   610 615 620
   Ser Arg Ser Ala Asp Ala Pro Ala Tyr Lys Gln Gly Gln Asn Gln Leu
   625 630 635 640
   Tyr Asn Glu Leu Asn Leu Gly Arg Arg Glu Glu Tyr Asp Val Leu Asp
   645 650 655
   Lys Arg Arg Gly Arg Asp Pro Glu Met Gly Gly Lys Pro Arg Arg Lys
   660 665 670
   Asn Pro Gln Glu Gly Leu Tyr Asn Glu Leu Gln Lys Asp Lys Met Ala
   675 680 685
   Glu Ala Tyr Ser Glu Ile Gly Met Lys Gly Glu Arg Arg Arg Gly Lys
   690 695 700
   Gly His Asp Gly Leu Tyr Gln Gly Leu Ser Thr Ala Thr Lys Asp Thr
   705 710 715 720
   Tyr Asp Ala Leu His Met Gln Ala Leu Pro Pro Arg
   725 730
<210> 76
   <211> 495
   <212> PRT
   <213> Artificial Sequence
<220>
   <223> chimeric antigen receptor sequence
<400> 76
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Val Lys Lys Pro Gly Ser Ser Val Lys Val Ser Cys Lys Ala Ser Gly
   35 40 45
   Tyr Ala Phe Ser Ser Tyr Ile Ser Trp Val Arg Gln Ala Pro Gly Gln
   50 55 60
   Gly Leu Glu Trp Met Gly Gly Gln Ile Tyr Pro Gly Asp Gly Asp Thr
   65 70 75 80
   Asn Tyr Ala Gln Lys Phe Gln Gly Arg Val Thr Ile Thr Ala Asp Glu
   85 90 95
   Ser Thr Ser Thr Ala Tyr Met Glu Leu Ser Ser Leu Arg Ser Glu Asp
   100 105 110
   Thr Ala Val Tyr Tyr Cys Val Arg Tyr Tyr Tyr Gly Ser Ser Gly Tyr
   115 120 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Glu Ser Val Asp Asn Tyr Gly Ile
               180                 185                 190
   Ser Phe Ala Trp Tyr Gln Gln Lys Pro Gly Gln Ala Pro Arg Leu Leu
           195                 200                 205
   Ile Tyr Arg Ala Ser Arg Ala Thr Gly Ile Pro Ala Arg Phe Ser Gly
       210                 215                 220
   Ser Gly Ser Gly Thr Asp Phe Thr Leu Thr Ile Ser Ser Leu Glu Pro
   225                 230                 235                 240
   Glu Asp Phe Ala Val Tyr Tyr Cys Gln Gln Asn Asn Lys Asp Pro Pro
                   245                 250                 255
   Thr Phe Phe Gly Gln Gly Thr Lys Leu Glu Ile Lys Arg Ala Ala Ala
               260                 265                 270
   Thr Thr Thr Pro Ala Pro Arg Pro Pro Thr Pro Ala Pro Thr Ile Ala
           275                 280                 285
   Ser Gln Pro Leu Ser Leu Arg Pro Glu Ala Cys Arg Pro Ala Ala Gly
       290                 295                 300
   Gly Ala Val His Thr Arg Gly Leu Asp Phe Ala Cys Asp Ile Tyr Ile
   305                 310                 315                 320
   Trp Ala Pro Leu Ala Gly Thr Cys Gly Val Leu Leu Leu Ser Leu Val
                   325                 330                 335
   Ile Thr Leu Tyr Cys Lys Arg Gly Arg Lys Lys Leu Leu Tyr Ile Phe
               340                 345                 350
   Lys Gln Pro Phe Met Arg Pro Val Gln Thr Thr Gln Glu Glu Asp Gly
           355                 360                 365
   Cys Ser Cys Arg Phe Pro Glu Glu Glu Glu Gly Gly Cys Glu Leu Arg
       370                 375                 380
   Val Lys Phe Ser Arg Ser Ala Asp Ala Pro Ala Tyr Lys Gln Gly Gln
   385 390 395 400
   Asn Gln Leu Tyr Asn Glu Leu Asn Leu Gly Arg Arg Glu Glu Tyr Asp
   405 410 415
   Val Leu Asp Lys Arg Arg Gly Arg Asp Pro Glu Met Gly Gly Lys Pro
   420 425 430
   Arg Arg Lys Asn Pro Gln Glu Gly Leu Tyr Asn Glu Leu Gln Lys Asp
   435 440 445
   Lys Met Ala Glu Ala Tyr Ser Glu Ile Gly Met Lys Gly Glu Arg Arg
   450 455 460
   Arg Gly Lys Gly His Asp Gly Leu Tyr Gln Gly Leu Ser Thr Ala Thr
   465 470 475 480
   Lys Asp Thr Tyr Asp Ala Leu His Met Gln Ala Leu Pro Pro Arg
   485 490 495
<210> 77
   <211> 792
   <212> PRT
   <213> Artificial Sequence
<220>
   <223> chimeric antigen receptor sequence
<400> 77
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Val Lys Lys Pro Gly Ser Ser Val Lys Val Ser Cys Lys Ala Ser Gly
   35 40 45
   Tyr Ala Phe Ser Ser Tyr Ile Ser Trp Val Arg Gln Ala Pro Gly Gln
   50 55 60
   Gly Leu Glu Trp Met Gly Gly Gln Ile Tyr Pro Gly Asp Gly Asp Thr
   65 70 75 80
   Asn Tyr Ala Gln Lys Phe Gln Gly Arg Val Thr Ile Thr Ala Asp Glu
   85 90 95
   Ser Thr Ser Thr Ala Tyr Met Glu Leu Ser Ser Leu Arg Ser Glu Asp
   100 105 110
   Thr Ala Val Tyr Tyr Cys Val Arg Tyr Tyr Tyr Gly Ser Ser Gly Tyr
   115 120 125
   Phe Asp Tyr Trp Trp Gly Gln Gly Thr Met Val Thr Val Ser Ser Gly
   130 135 140
   Gly Gly Gly Ser Gly Gly Gly Gly Ser Gly Gly Gly Gly Ser Glu Ile
   145 150 155 160
   Val Leu Thr Gln Ser Pro Ala Thr Leu Ser Leu Ser Pro Gly Glu Arg
   165 170 175
   Ala Thr Leu Ser Cys Arg Ala Ser Glu Ser Val Asp Asn Tyr Gly Ile
   180 185 190
   Ser Phe Ala Trp Tyr Gln Gln Lys Pro Gly Gln Ala Pro Arg Leu Leu
   195 200 205
   Ile Tyr Arg Ala Ser Arg Ala Thr Gly Ile Pro Ala Arg Phe Ser Gly
   210 215 220
   Ser Gly Ser Gly Thr Asp Phe Thr Leu Thr Ile Ser Ser Leu Glu Pro
   225 230 235 240
   Glu Asp Phe Ala Val Tyr Tyr Cys Gln Gln Asn Asn Lys Asp Pro Pro
   245 250 255
   Thr Phe Phe Gly Gln Gly Thr Lys Leu Glu Ile Lys Arg Ala Glu Pro
   260 265 270
   Lys Ser Cys Asp Lys Thr His Thr Cys Pro Pro Cys Pro Ala Pro Glu
   275 280 285
   Leu Leu Gly Gly Pro Ser Val Phe Leu Phe Pro Pro Lys Pro Lys Asp
   290 295 300
   Thr Leu Met Ile Ser Arg Thr Pro Glu Val Thr Cys Val Val Val Asp
   305 310 315 320
   Val Ser His Glu Asp Pro Glu Val Lys Phe Asn Trp Tyr Val Asp Gly
   325 330 335
   Val Glu Val His Asn Ala Lys Thr Lys Pro Arg Glu Glu Gln Tyr Asn
   340 345 350
   Ser Thr Tyr Arg Val Val Ser Val Leu Thr Val Leu His Gln Asp Trp
   355 360 365
   Leu Asn Gly Lys Glu Tyr Lys Cys Lys Val Ser Asn Lys Ala Leu Pro
   370 375 380
   Ala Pro Ile Glu Lys Thr Ile Ser Lys Ala Lys Gly Gln Pro Arg Glu
   385 390 395 400
   Pro Gln Val Tyr Thr Leu Pro Pro Ser Arg Asp Glu Leu Thr Lys Asn
   405 410 415
   Gln Val Ser Leu Thr Cys Leu Val Lys Gly Phe Tyr Pro Ser Asp Ile
   420 425 430
   Ala Val Glu Trp Glu Ser Asn Gly Gln Pro Glu Asn Asn Tyr Lys Thr
   435 440 445
   Thr Pro Pro Val Leu Asp Ser Asp Gly Ser Phe Phe Leu Tyr Ser Lys
   450 455 460
   Leu Thr Val Asp Lys Ser Arg Trp Gln Gln Gly Asn Val Phe Ser Cys
   465 470 475 480
   Ser Val Met His Glu Ala Leu His Asn His Tyr Thr Gln Lys Ser Leu
   485 490 495
   Ser Leu Ser Pro Gly Lys Lys Asp Pro Lys Ala Ala Ala Phe Val Pro
   500 505 510
   Val Phe Leu Pro Ala Lys Pro Thr Thr Thr Pro Ala Pro Arg Pro Pro
   515 520 525
   Thr Pro Ala Pro Thr Ile Ala Ser Gln Pro Leu Ser Leu Arg Pro Glu
   530 535 540
   Ala Cys Arg Pro Ala Ala Gly Gly Ala Val His Thr Arg Gly Leu Asp
   545 550 555 560
   Phe Ala Cys Asp Ile Tyr Ile Trp Ala Pro Leu Ala Gly Thr Cys Gly
   565 570 575
   Val Leu Leu Leu Ser Leu Val Ile Thr Leu Tyr Cys Asn His Arg Asn
   580 585 590
   Arg Ser Lys Arg Ser Arg Leu Leu His Ser Asp Tyr Met Asn Met Thr
   595 600 605
   Pro Arg Arg Pro Gly Pro Thr Arg Lys His Tyr Gln Pro Tyr Ala Pro
       610                 615                 620
   Pro Arg Asp Phe Ala Ala Tyr Arg Ser Arg Phe Ser Val Val Lys Arg
   625                 630                 635                 640
   Gly Arg Lys Lys Leu Leu Tyr Ile Phe Lys Gln Pro Phe Met Arg Pro
                   645                 650                 655
   Val Gln Thr Thr Gln Glu Glu Asp Gly Cys Ser Cys Arg Phe Pro Glu
               660                 665                 670
   Glu Glu Glu Gly Gly Cys Glu Leu Arg Val Lys Phe Ser Arg Ser Ala
           675                 680                 685
   Asp Ala Pro Ala Tyr Gln Gln Gly Gln Asn Gln Leu Tyr Asn Glu Leu
       690                 695                 700
   Asn Leu Gly Arg Arg Glu Glu Tyr Asp Val Leu Asp Lys Arg Arg Gly
   705                 710                 715                 720
   Arg Asp Pro Glu Met Gly Gly Lys Pro Arg Arg Lys Asn Pro Gln Glu
                   725                 730                 735
   Gly Leu Tyr Asn Glu Leu Gln Lys Asp Lys Met Ala Glu Ala Tyr Ser
               740                 745                 750
   Glu Ile Gly Met Lys Gly Glu Arg Arg Arg Gly Lys Gly His Asp Gly
           755                 760                 765
   Leu Tyr Gln Gly Leu Ser Thr Ala Thr Lys Asp Thr Tyr Asp Ala Leu
       770                 775                 780
   His Met Gln Ala Leu Pro Pro Arg
   785                 790
<210> 78
   <211> 555
   <212> PRT
   <213> Artificial Sequence
<220>
   <223> chimeric antigen receptor sequence
<400> 78
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Ala Phe Ser Ser Tyr Ile Ser Trp Val Arg Gln Ala Pro Gly Gln
       50                  55                  60
   Gly Leu Glu Trp Met Gly Gly Gln Ile Tyr Pro Gly Asp Gly Asp Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Val Arg Tyr Tyr Tyr Gly Ser Ser Gly Tyr
           115                 120                 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Glu Ser Val Asp Asn Tyr Gly Ile
               180                 185                 190
   Ser Phe Ala Trp Tyr Gln Gln Lys Pro Gly Gln Ala Pro Arg Leu Leu
           195                 200                 205
   Ile Tyr Arg Ala Ser Arg Ala Thr Gly Ile Pro Ala Arg Phe Ser Gly
       210                 215                 220
   Ser Gly Ser Gly Thr Asp Phe Thr Leu Thr Ile Ser Ser Leu Glu Pro
   225                 230                 235                 240
   Glu Asp Phe Ala Val Tyr Tyr Cys Gln Gln Asn Asn Lys Asp Pro Pro
                   245                 250                 255
   Thr Phe Phe Gly Gln Gly Thr Lys Leu Glu Ile Lys Arg Ala Ala Ala
               260                 265                 270
   Phe Val Pro Val Phe Leu Pro Ala Lys Pro Thr Thr Thr Pro Ala Pro
           275                 280                 285
   Arg Pro Pro Thr Pro Ala Pro Thr Ile Ala Ser Gln Pro Leu Ser Leu
       290                 295                 300
   Arg Pro Glu Ala Cys Arg Pro Ala Ala Gly Gly Ala Val His Thr Arg
   305                 310                 315                 320
   Gly Leu Asp Phe Ala Cys Asp Ile Tyr Ile Trp Ala Pro Leu Ala Gly
                   325                 330                 335
   Thr Cys Gly Val Leu Leu Leu Ser Leu Val Ile Thr Leu Tyr Cys Asn
               340                 345                 350
   His Arg Asn Arg Ser Lys Arg Ser Arg Leu Leu His Ser Asp Tyr Met
           355                 360                 365
   Asn Met Thr Pro Arg Arg Pro Gly Pro Thr Arg Lys His Tyr Gln Pro
       370                 375                 380
   Tyr Ala Pro Pro Arg Asp Phe Ala Ala Tyr Arg Ser Arg Phe Ser Val
   385                 390                 395                 400
   Val Lys Arg Gly Arg Lys Lys Leu Leu Tyr Ile Phe Lys Gln Pro Phe
                   405                 410                 415
   Met Arg Pro Val Gln Thr Thr Gln Glu Glu Asp Gly Cys Ser Cys Arg
               420                 425                 430
   Phe Pro Glu Glu Glu Glu Gly Gly Cys Glu Leu Arg Val Lys Phe Ser
           435                 440                 445
   Arg Ser Ala Asp Ala Pro Ala Tyr Gln Gln Gly Gln Asn Gln Leu Tyr
       450                 455                 460
   Asn Glu Leu Asn Leu Gly Arg Arg Glu Glu Tyr Asp Val Leu Asp Lys
   465                 470                 475                 480
   Arg Arg Gly Arg Asp Pro Glu Met Gly Gly Lys Pro Arg Arg Lys Asn
                   485                 490                 495
   Pro Gln Glu Gly Leu Tyr Asn Glu Leu Gln Lys Asp Lys Met Ala Glu
               500                 505                 510
   Ala Tyr Ser Glu Ile Gly Met Lys Gly Glu Arg Arg Arg Gly Lys Gly
   515 520 525
   His Asp Gly Leu Tyr Gln Gly Leu Ser Thr Ala Thr Lys Asp Thr Tyr
   530 535 540
   Asp Ala Leu His Met Gln Ala Leu Pro Pro Arg
   545 550 555
<210> 79
   <211> 726
   <212> PRT
   <213> Artificial Sequence
<220>
   <223> chimeric antigen receptor sequence
<400> 79
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp His Phe Ile Ser Trp Val Arg Gln Ala Pro Gly
       50                  55                  60
   Gln Gly Leu Glu Trp Met Gly Gly Leu Asn Pro Tyr Ser Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg His Asn Trp Gly Ala Tyr Phe Asp
           115                 120                 125
   Tyr Trp Trp Gly Gln Gly Thr Met Val Thr Val Ser Ser Gly Gly Gly
       130                 135                 140
   Gly Ser Gly Gly Gly Gly Ser Gly Gly Gly Gly Ser Glu Ile Val Leu
   145                 150                 155                 160
   Thr Gln Ser Pro Ala Thr Leu Ser Leu Ser Pro Gly Glu Arg Ala Thr
                   165                 170                 175
   Leu Ser Cys Arg Ala Ser Lys Ser Leu Leu His Ser Asn Gly Asn Thr
               180                 185                 190
   Tyr Ala Trp Tyr Gln Gln Lys Pro Gly Gln Ala Pro Arg Leu Leu Ile
           195                 200                 205
   Tyr Tyr Met Ser Arg Ala Thr Gly Ile Pro Ala Arg Phe Ser Gly Ser
       210                 215                 220
   Gly Ser Gly Thr Asp Phe Thr Leu Thr Ile Ser Ser Leu Glu Pro Glu
   225                 230                 235                 240
   Asp Phe Ala Val Tyr Tyr Cys Met Gln Gly Leu Glu Asp Pro Tyr Thr
                   245                 250                 255
   Phe Phe Gly Gln Gly Thr Lys Leu Glu Ile Lys Arg Glu Pro Lys Ser
               260                 265                 270
   Cys Asp Lys Thr His Thr Cys Pro Pro Cys Pro Ala Pro Glu Leu Leu
           275                 280                 285
   Gly Gly Pro Ser Val Phe Leu Phe Pro Pro Lys Pro Lys Asp Thr Leu
       290                 295                 300
   Met Ile Ser Arg Thr Pro Glu Val Thr Cys Val Val Val Asp Val Ser
   305                 310                 315                 320
   His Glu Asp Pro Glu Val Lys Phe Asn Trp Tyr Val Asp Gly Val Glu
                   325                 330                 335
   Val His Asn Ala Lys Thr Lys Pro Arg Glu Glu Gln Tyr Asn Ser Thr
               340                 345                 350
   Tyr Arg Val Val Ser Val Leu Thr Val Leu His Gln Asp Trp Leu Asn
           355                 360                 365
   Gly Lys Glu Tyr Lys Cys Lys Val Ser Asn Lys Ala Leu Pro Ala Pro
       370                 375                 380
   Ile Glu Lys Thr Ile Ser Lys Ala Lys Gly Gln Pro Arg Glu Pro Gln
   385                 390                 395                 400
   Val Tyr Thr Leu Pro Pro Ser Arg Asp Glu Leu Thr Lys Asn Gln Val
                   405                 410                 415
   Ser Leu Thr Cys Leu Val Lys Gly Phe Tyr Pro Ser Asp Ile Ala Val
               420                 425                 430
   Glu Trp Glu Ser Asn Gly Gln Pro Glu Asn Asn Tyr Lys Thr Thr Pro
           435                 440                 445
   Pro Val Leu Asp Ser Asp Gly Ser Phe Phe Leu Tyr Ser Lys Leu Thr
       450                 455                 460
   Val Asp Lys Ser Arg Trp Gln Gln Gly Asn Val Phe Ser Cys Ser Val
   465                 470                 475                 480
   Met His Glu Ala Leu His Asn His Tyr Thr Gln Lys Ser Leu Ser Leu
                   485                 490                 495
   Ser Pro Gly Lys Lys Asp Pro Lys Ala Ala Ala Ile Glu Val Met Tyr
               500                 505                 510
   Pro Pro Pro Tyr Leu Asp Asn Glu Lys Ser Asn Gly Thr Ile Ile His
           515                 520                 525
   Val Lys Gly Lys His Leu Cys Pro Ser Pro Leu Phe Pro Gly Pro Ser
       530                 535                 540
   Lys Pro Phe Trp Val Leu Val Val Val Gly Gly Val Leu Ala Cys Tyr
   545                 550                 555                 560
   Ser Leu Leu Val Thr Val Ala Phe Ile Ile Phe Trp Val Arg Ser Lys
                   565                 570                 575
   Arg Ser Arg Leu Leu His Ser Asp Tyr Met Asn Met Thr Pro Arg Arg
               580                 585                 590
   Pro Gly Pro Thr Arg Lys His Tyr Gln Pro Tyr Ala Pro Pro Arg Asp
           595                 600                 605
   Phe Ala Ala Tyr Arg Ser Arg Val Lys Phe Ser Arg Ser Ala Asp Ala
       610                 615                 620
   Pro Ala Tyr Gln Gln Gly Gln Asn Gln Leu Tyr Asn Glu Leu Asn Leu
   625                 630                 635                 640
   Gly Arg Arg Glu Glu Tyr Asp Val Leu Asp Lys Arg Arg Gly Arg Asp
                   645                 650                 655
   Pro Glu Met Gly Gly Lys Pro Arg Arg Lys Asn Pro Gln Glu Gly Leu
               660                 665                 670
   Tyr Asn Glu Leu Gln Lys Asp Lys Met Ala Glu Ala Tyr Ser Glu Ile
           675                 680                 685
   Gly Met Lys Gly Glu Arg Arg Arg Gly Lys Gly His Asp Gly Leu Tyr
       690                 695                 700
   Gln Gly Leu Ser Thr Ala Thr Lys Asp Thr Tyr Asp Ala Leu His Met
   705                 710                 715                 720
   Gln Ala Leu Pro Pro Arg
                   725
<210> 80
   <211> 490
   <212> PRT
   <213> Artificial Sequence
<220>
   <223> chimeric antigen receptor sequence
<400> 80
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp His Phe Ile Ser Trp Val Arg Gln Ala Pro Gly
       50                  55                  60
   Gln Gly Leu Glu Trp Met Gly Gly Leu Asn Pro Tyr Ser Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg His Asn Trp Gly Ala Tyr Phe Asp
           115                 120                 125
   Tyr Trp Trp Gly Gln Gly Thr Met Val Thr Val Ser Ser Gly Gly Gly
       130                 135                 140
   Gly Ser Gly Gly Gly Gly Ser Gly Gly Gly Gly Ser Glu Ile Val Leu
   145 150 155 160
   Thr Gln Ser Pro Ala Thr Leu Ser Leu Ser Pro Gly Glu Arg Ala Thr
   165 170 175
   Leu Ser Cys Arg Ala Ser Lys Ser Leu Leu His Ser Asn Gly Asn Thr
   180 185 190
   Tyr Ala Trp Tyr Gln Gln Lys Pro Gly Gln Ala Pro Arg Leu Leu Ile
   195 200 205
   Tyr Tyr Met Ser Arg Ala Thr Gly Ile Pro Ala Arg Phe Ser Gly Ser
   210 215 220
   Gly Ser Gly Thr Asp Phe Thr Leu Thr Ile Ser Ser Leu Glu Pro Glu
   225 230 235 240
   Asp Phe Ala Val Tyr Tyr Cys Met Gln Gly Leu Glu Asp Pro Tyr Thr
   245 250 255
   Phe Phe Gly Gln Gly Thr Lys Leu Glu Ile Lys Arg Ala Ala Ala Ile
   260 265 270
   Glu Val Met Tyr Pro Pro Pro Tyr Leu Asp Asn Glu Lys Ser Asn Gly
   275 280 285
   Thr Ile Ile His Val Lys Gly Lys His Leu Cys Pro Ser Pro Leu Phe
   290 295 300
   Pro Gly Pro Ser Lys Pro Phe Trp Val Leu Val Val Val Gly Gly Val
   305 310 315 320
   Leu Ala Cys Tyr Ser Leu Leu Val Thr Val Ala Phe Ile Ile Phe Trp
   325 330 335
   Val Arg Ser Lys Arg Ser Arg Leu Leu His Ser Asp Tyr Met Asn Met
   340 345 350
   Thr Pro Arg Arg Pro Gly Pro Thr Arg Lys His Tyr Gln Pro Tyr Ala
   355 360 365
   Pro Pro Arg Asp Phe Ala Ala Tyr Arg Ser Arg Val Lys Phe Ser Arg
   370 375 380
   Ser Ala Asp Ala Pro Ala Tyr Gln Gln Gly Gln Asn Gln Leu Tyr Asn
   385 390 395 400
   Glu Leu Asn Leu Gly Arg Arg Glu Glu Tyr Asp Val Leu Asp Lys Arg
   405 410 415
   Arg Gly Arg Asp Pro Glu Met Gly Gly Lys Pro Arg Arg Lys Asn Pro
   420 425 430
   Gln Glu Gly Leu Tyr Asn Glu Leu Gln Lys Asp Lys Met Ala Glu Ala
   435 440 445
   Tyr Ser Glu Ile Gly Met Lys Gly Glu Arg Arg Arg Gly Lys Gly His
   450 455 460
   Asp Gly Leu Tyr Gln Gly Leu Ser Thr Ala Thr Lys Asp Thr Tyr Asp
   465 470 475 480
   Ala Leu His Met Gln Ala Leu Pro Pro Arg
   485 490
<210> 81
   <211> 730
   <212> PRT
   <213> Artificial Sequence
<220>
   <223> chimeric antigen receptor sequence
<400> 81
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp His Phe Ile Ser Trp Val Arg Gln Ala Pro Gly
       50                  55                  60
   Gln Gly Leu Glu Trp Met Gly Gly Leu Asn Pro Tyr Ser Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg His Asn Trp Gly Ala Tyr Phe Asp
   115 120 125
   Tyr Trp Trp Gly Gln Gly Thr Met Val Thr Val Ser Ser Gly Gly Gly
   130 135 140
   Gly Ser Gly Gly Gly Gly Ser Gly Gly Gly Gly Ser Glu Ile Val Leu
   145 150 155 160
   Thr Gln Ser Pro Ala Thr Leu Ser Leu Ser Pro Gly Glu Arg Ala Thr
   165 170 175
   Leu Ser Cys Arg Ala Ser Lys Ser Leu Leu His Ser Asn Gly Asn Thr
   180 185 190
   Tyr Ala Trp Tyr Gln Gln Lys Pro Gly Gln Ala Pro Arg Leu Leu Ile
   195 200 205
   Tyr Tyr Met Ser Arg Ala Thr Gly Ile Pro Ala Arg Phe Ser Gly Ser
   210 215 220
   Gly Ser Gly Thr Asp Phe Thr Leu Thr Ile Ser Ser Leu Glu Pro Glu
   225 230 235 240
   Asp Phe Ala Val Tyr Tyr Cys Met Gln Gly Leu Glu Asp Pro Tyr Thr
   245 250 255
   Phe Phe Gly Gln Gly Thr Lys Leu Glu Ile Lys Arg Glu Pro Lys Ser
   260 265 270
   Cys Asp Lys Thr His Thr Cys Pro Pro Cys Pro Ala Pro Glu Leu Leu
   275 280 285
   Gly Gly Pro Ser Val Phe Leu Phe Pro Pro Lys Pro Lys Asp Thr Leu
   290 295 300
   Met Ile Ser Arg Thr Pro Glu Val Thr Cys Val Val Val Asp Val Ser
   305 310 315 320
   His Glu Asp Pro Glu Val Lys Phe Asn Trp Tyr Val Asp Gly Val Glu
   325 330 335
   Val His Asn Ala Lys Thr Lys Pro Arg Glu Glu Gln Tyr Asn Ser Thr
   340 345 350
   Tyr Arg Val Val Ser Val Leu Thr Val Leu His Gln Asp Trp Leu Asn
   355 360 365
   Gly Lys Glu Tyr Lys Cys Lys Val Ser Asn Lys Ala Leu Pro Ala Pro
       370                 375                 380
   Ile Glu Lys Thr Ile Ser Lys Ala Lys Gly Gln Pro Arg Glu Pro Gln
   385                 390                 395                 400
   Val Tyr Thr Leu Pro Pro Ser Arg Asp Glu Leu Thr Lys Asn Gln Val
                   405                 410                 415
   Ser Leu Thr Cys Leu Val Lys Gly Phe Tyr Pro Ser Asp Ile Ala Val
               420                 425                 430
   Glu Trp Glu Ser Asn Gly Gln Pro Glu Asn Asn Tyr Lys Thr Thr Pro
           435                 440                 445
   Pro Val Leu Asp Ser Asp Gly Ser Phe Phe Leu Tyr Ser Lys Leu Thr
       450                 455                 460
   Val Asp Lys Ser Arg Trp Gln Gln Gly Asn Val Phe Ser Cys Ser Val
   465                 470                 475                 480
   Met His Glu Ala Leu His Asn His Tyr Thr Gln Lys Ser Leu Ser Leu
                   485                 490                 495
   Ser Pro Gly Lys Lys Asp Pro Lys Ala Ala Ala Thr Thr Thr Pro Ala
               500                 505                 510
   Pro Arg Pro Pro Thr Pro Ala Pro Thr Ile Ala Ser Gln Pro Leu Ser
           515                 520                 525
   Leu Arg Pro Glu Ala Cys Arg Pro Ala Ala Gly Gly Ala Val His Thr
       530                 535                 540
   Arg Gly Leu Asp Phe Ala Cys Asp Ile Tyr Ile Trp Ala Pro Leu Ala
   545                 550                 555                 560
   Gly Thr Cys Gly Val Leu Leu Leu Ser Leu Val Ile Thr Leu Tyr Cys
                   565                 570                 575
   Lys Arg Gly Arg Lys Lys Leu Leu Tyr Ile Phe Lys Gln Pro Phe Met
               580                 585                 590
   Arg Pro Val Gln Thr Thr Gln Glu Glu Asp Gly Cys Ser Cys Arg Phe
           595                 600                 605
   Pro Glu Glu Glu Glu Gly Gly Cys Glu Leu Arg Val Lys Phe Ser Arg
       610                 615                 620
   Ser Ala Asp Ala Pro Ala Tyr Lys Gln Gly Gln Asn Gln Leu Tyr Asn
   625 630 635 640
   Glu Leu Asn Leu Gly Arg Arg Glu Glu Tyr Asp Val Leu Asp Lys Arg
   645 650 655
   Arg Gly Arg Asp Pro Glu Met Gly Gly Lys Pro Arg Arg Lys Asn Pro
   660 665 670
   Gln Glu Gly Leu Tyr Asn Glu Leu Gln Lys Asp Lys Met Ala Glu Ala
   675 680 685
   Tyr Ser Glu Ile Gly Met Lys Gly Glu Arg Arg Arg Gly Lys Gly His
   690 695 700
   Asp Gly Leu Tyr Gln Gly Leu Ser Thr Ala Thr Lys Asp Thr Tyr Asp
   705 710 715 720
   Ala Leu His Met Gln Ala Leu Pro Pro Arg
   725 730
<210> 82
   <211> 494
   <212> PRT
   <213> Artificial Sequence
<220>
   <223> chimeric antigen receptor sequence
<400> 82
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Val Lys Lys Pro Gly Ser Ser Val Lys Val Ser Cys Lys Ala Ser Gly
   35 40 45
   Tyr Thr Phe Thr Asp His Phe Ile Ser Trp Val Arg Gln Ala Pro Gly
   50 55 60
   Gln Gly Leu Glu Trp Met Gly Gly Leu Asn Pro Tyr Ser Gly Gly Thr
   65 70 75 80
   Asn Tyr Ala Gln Lys Phe Gln Gly Arg Val Thr Ile Thr Ala Asp Glu
   85 90 95
   Ser Thr Ser Thr Ala Tyr Met Glu Leu Ser Ser Leu Arg Ser Glu Asp
               100                 105                 110
   Thr Ala Val Tyr Tyr Cys Ala Arg His Asn Trp Gly Ala Tyr Phe Asp
           115                 120                 125
   Tyr Trp Trp Gly Gln Gly Thr Met Val Thr Val Ser Ser Gly Gly Gly
       130                 135                 140
   Gly Ser Gly Gly Gly Gly Ser Gly Gly Gly Gly Ser Glu Ile Val Leu
   145                 150                 155                 160
   Thr Gln Ser Pro Ala Thr Leu Ser Leu Ser Pro Gly Glu Arg Ala Thr
                   165                 170                 175
   Leu Ser Cys Arg Ala Ser Lys Ser Leu Leu His Ser Asn Gly Asn Thr
               180                 185                 190
   Tyr Ala Trp Tyr Gln Gln Lys Pro Gly Gln Ala Pro Arg Leu Leu Ile
           195                 200                 205
   Tyr Tyr Met Ser Arg Ala Thr Gly Ile Pro Ala Arg Phe Ser Gly Ser
       210                 215                 220
   Gly Ser Gly Thr Asp Phe Thr Leu Thr Ile Ser Ser Leu Glu Pro Glu
   225                 230                 235                 240
   Asp Phe Ala Val Tyr Tyr Cys Met Gln Gly Leu Glu Asp Pro Tyr Thr
                   245                 250                 255
   Phe Phe Gly Gln Gly Thr Lys Leu Glu Ile Lys Arg Ala Ala Ala Thr
               260                 265                 270
   Thr Thr Pro Ala Pro Arg Pro Pro Thr Pro Ala Pro Thr Ile Ala Ser
           275                 280                 285
   Gln Pro Leu Ser Leu Arg Pro Glu Ala Cys Arg Pro Ala Ala Gly Gly
       290                 295                 300
   Ala Val His Thr Arg Gly Leu Asp Phe Ala Cys Asp Ile Tyr Ile Trp
   305                 310                 315                 320
   Ala Pro Leu Ala Gly Thr Cys Gly Val Leu Leu Leu Ser Leu Val Ile
                   325                 330                 335
   Thr Leu Tyr Cys Lys Arg Gly Arg Lys Lys Leu Leu Tyr Ile Phe Lys
               340                 345                 350
   Gln Pro Phe Met Arg Pro Val Gln Thr Thr Gln Glu Glu Asp Gly Cys
   355 360 365
   Ser Cys Arg Phe Pro Glu Glu Glu Glu Gly Gly Cys Glu Leu Arg Val
   370 375 380
   Lys Phe Ser Arg Ser Ala Asp Ala Pro Ala Tyr Lys Gln Gly Gln Asn
   385 390 395 400
   Gln Leu Tyr Asn Glu Leu Asn Leu Gly Arg Arg Glu Glu Tyr Asp Val
   405 410 415
   Leu Asp Lys Arg Arg Gly Arg Asp Pro Glu Met Gly Gly Lys Pro Arg
   420 425 430
   Arg Lys Asn Pro Gln Glu Gly Leu Tyr Asn Glu Leu Gln Lys Asp Lys
   435 440 445
   Met Ala Glu Ala Tyr Ser Glu Ile Gly Met Lys Gly Glu Arg Arg Arg
   450 455 460
   Gly Lys Gly His Asp Gly Leu Tyr Gln Gly Leu Ser Thr Ala Thr Lys
   465 470 475 480
   Asp Thr Tyr Asp Ala Leu His Met Gln Ala Leu Pro Pro Arg
   485 490
<210> 83
   <211> 790
   <212> PRT
   <213> Artificial Sequence
<220>
   <223> chimeric antigen receptor sequence
<400> 83
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Val Lys Lys Pro Gly Ser Ser Val Lys Val Ser Cys Lys Ala Ser Gly
   35 40 45
   Tyr Thr Phe Thr Asp His Phe Ile Ser Trp Val Arg Gln Ala Pro Gly
   50 55 60
   Gln Gly Leu Glu Trp Met Gly Gly Leu Asn Pro Tyr Ser Gly Gly Thr
   65 70 75 80
   Asn Tyr Ala Gln Lys Phe Gln Gly Arg Val Thr Ile Thr Ala Asp Glu
   85 90 95
   Ser Thr Ser Thr Ala Tyr Met Glu Leu Ser Ser Leu Arg Ser Glu Asp
   100 105 110
   Thr Ala Val Tyr Tyr Cys Ala Arg His Asn Trp Gly Ala Tyr Phe Asp
   115 120 125
   Tyr Trp Trp Gly Gln Gly Thr Met Val Thr Val Ser Ser Gly Gly Gly
   130 135 140
   Gly Ser Gly Gly Gly Gly Ser Gly Gly Gly Gly Ser Glu Ile Val Leu
   145 150 155 160
   Thr Gln Ser Pro Ala Thr Leu Ser Leu Ser Pro Gly Glu Arg Ala Thr
   165 170 175
   Leu Ser Cys Arg Ala Ser Lys Ser Leu Leu His Ser Asn Gly Asn Thr
   180 185 190
   Tyr Ala Trp Tyr Gln Gln Lys Pro Gly Gln Ala Pro Arg Leu Leu Ile
   195 200 205
   Tyr Tyr Met Ser Arg Ala Thr Gly Ile Pro Ala Arg Phe Ser Gly Ser
   210 215 220
   Gly Ser Gly Thr Asp Phe Thr Leu Thr Ile Ser Ser Leu Glu Pro Glu
   225 230 235 240
   Asp Phe Ala Val Tyr Tyr Cys Met Gln Gly Leu Glu Asp Pro Tyr Thr
   245 250 255
   Phe Phe Gly Gln Gly Thr Lys Leu Glu Ile Lys Arg Glu Pro Lys Ser
   260 265 270
   Cys Asp Lys Thr His Thr Cys Pro Pro Cys Pro Ala Pro Glu Leu Leu
   275 280 285
   Gly Gly Pro Ser Val Phe Leu Phe Pro Pro Lys Pro Lys Asp Thr Leu
   290 295 300
   Met Ile Ser Arg Thr Pro Glu Val Thr Cys Val Val Val Asp Val Ser
   305 310 315 320
   His Glu Asp Pro Glu Val Lys Phe Asn Trp Tyr Val Asp Gly Val Glu
                   325                 330                 335
   Val His Asn Ala Lys Thr Lys Pro Arg Glu Glu Gln Tyr Asn Ser Thr
               340                 345                 350
   Tyr Arg Val Val Ser Val Leu Thr Val Leu His Gln Asp Trp Leu Asn
           355                 360                 365
   Gly Lys Glu Tyr Lys Cys Lys Val Ser Asn Lys Ala Leu Pro Ala Pro
       370                 375                 380
   Ile Glu Lys Thr Ile Ser Lys Ala Lys Gly Gln Pro Arg Glu Pro Gln
   385                 390                 395                 400
   Val Tyr Thr Leu Pro Pro Ser Arg Asp Glu Leu Thr Lys Asn Gln Val
                   405                 410                 415
   Ser Leu Thr Cys Leu Val Lys Gly Phe Tyr Pro Ser Asp Ile Ala Val
               420                 425                 430
   Glu Trp Glu Ser Asn Gly Gln Pro Glu Asn Asn Tyr Lys Thr Thr Pro
           435                 440                 445
   Pro Val Leu Asp Ser Asp Gly Ser Phe Phe Leu Tyr Ser Lys Leu Thr
       450                 455                 460
   Val Asp Lys Ser Arg Trp Gln Gln Gly Asn Val Phe Ser Cys Ser Val
   465                 470                 475                 480
   Met His Glu Ala Leu His Asn His Tyr Thr Gln Lys Ser Leu Ser Leu
                   485                 490                 495
   Ser Pro Gly Lys Lys Asp Pro Lys Ala Ala Ala Phe Val Pro Val Phe
               500                 505                 510
   Leu Pro Ala Lys Pro Thr Thr Thr Pro Ala Pro Arg Pro Pro Thr Pro
           515                 520                 525
   Ala Pro Thr Ile Ala Ser Gln Pro Leu Ser Leu Arg Pro Glu Ala Cys
       530                 535                 540
   Arg Pro Ala Ala Gly Gly Ala Val His Thr Arg Gly Leu Asp Phe Ala
   545                 550                 555                 560
   Cys Asp Ile Tyr Ile Trp Ala Pro Leu Ala Gly Thr Cys Gly Val Leu
                   565                 570                 575
   Leu Leu Ser Leu Val Ile Thr Leu Tyr Cys Asn His Arg Asn Arg Ser
   580 585 590
   Lys Arg Ser Arg Leu Leu His Ser Asp Tyr Met Asn Met Thr Pro Arg
   595 600 605
   Arg Pro Gly Pro Thr Arg Lys His Tyr Gln Pro Tyr Ala Pro Pro Arg
   610 615 620
   Asp Phe Ala Ala Tyr Arg Ser Arg Phe Ser Val Val Lys Arg Gly Arg
   625 630 635 640
   Lys Lys Leu Leu Tyr Ile Phe Lys Gln Pro Phe Met Arg Pro Val Gln
   645 650 655
   Thr Thr Gln Glu Glu Asp Gly Cys Ser Cys Arg Phe Pro Glu Glu Glu
   660 665 670
   Glu Gly Gly Cys Glu Leu Arg Val Lys Phe Ser Arg Ser Ala Asp Ala
   675 680 685
   Pro Ala Tyr Gln Gln Gly Gln Asn Gln Leu Tyr Asn Glu Leu Asn Leu
   690 695 700
   Gly Arg Arg Glu Glu Tyr Asp Val Leu Asp Lys Arg Arg Gly Arg Asp
   705 710 715 720
   Pro Glu Met Gly Gly Lys Pro Arg Arg Lys Asn Pro Gln Glu Gly Leu
   725 730 735
   Tyr Asn Glu Leu Gln Lys Asp Lys Met Ala Glu Ala Tyr Ser Glu Ile
   740 745 750
   Gly Met Lys Gly Glu Arg Arg Arg Gly Lys Gly His Asp Gly Leu Tyr
   755 760 765
   Gln Gly Leu Ser Thr Ala Thr Lys Asp Thr Tyr Asp Ala Leu His Met
   770 775 780
   Gln Ala Leu Pro Pro Arg
   785 790
<210> 84
   <211> 554
   <212> PRT
   <213> Artificial Sequence
<220>
   <223> chimeric antigen receptor sequence
<400> 84
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp His Phe Ile Ser Trp Val Arg Gln Ala Pro Gly
       50                  55                  60
   Gln Gly Leu Glu Trp Met Gly Gly Leu Asn Pro Tyr Ser Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg His Asn Trp Gly Ala Tyr Phe Asp
           115                 120                 125
   Tyr Trp Trp Gly Gln Gly Thr Met Val Thr Val Ser Ser Gly Gly Gly
       130                 135                 140
   Gly Ser Gly Gly Gly Gly Ser Gly Gly Gly Gly Ser Glu Ile Val Leu
   145                 150                 155                 160
   Thr Gln Ser Pro Ala Thr Leu Ser Leu Ser Pro Gly Glu Arg Ala Thr
                   165                 170                 175
   Leu Ser Cys Arg Ala Ser Lys Ser Leu Leu His Ser Asn Gly Asn Thr
               180                 185                 190
   Tyr Ala Trp Tyr Gln Gln Lys Pro Gly Gln Ala Pro Arg Leu Leu Ile
           195                 200                 205
   Tyr Tyr Met Ser Arg Ala Thr Gly Ile Pro Ala Arg Phe Ser Gly Ser
       210                 215                 220
   Gly Ser Gly Thr Asp Phe Thr Leu Thr Ile Ser Ser Leu Glu Pro Glu
   225                 230                 235                 240
   Asp Phe Ala Val Tyr Tyr Cys Met Gln Gly Leu Glu Asp Pro Tyr Thr
                   245                 250                 255
   Phe Phe Gly Gln Gly Thr Lys Leu Glu Ile Lys Arg Ala Ala Ala Phe
               260                 265                 270
   Val Pro Val Phe Leu Pro Ala Lys Pro Thr Thr Thr Pro Ala Pro Arg
           275                 280                 285
   Pro Pro Thr Pro Ala Pro Thr Ile Ala Ser Gln Pro Leu Ser Leu Arg
       290                 295                 300
   Pro Glu Ala Cys Arg Pro Ala Ala Gly Gly Ala Val His Thr Arg Gly
   305                 310                 315                 320
   Leu Asp Phe Ala Cys Asp Ile Tyr Ile Trp Ala Pro Leu Ala Gly Thr
                   325                 330                 335
   Cys Gly Val Leu Leu Leu Ser Leu Val Ile Thr Leu Tyr Cys Asn His
               340                 345                 350
   Arg Asn Arg Ser Lys Arg Ser Arg Leu Leu His Ser Asp Tyr Met Asn
           355                 360                 365
   Met Thr Pro Arg Arg Pro Gly Pro Thr Arg Lys His Tyr Gln Pro Tyr
       370                 375                 380
   Ala Pro Pro Arg Asp Phe Ala Ala Tyr Arg Ser Arg Phe Ser Val Val
   385                 390                 395                 400
   Lys Arg Gly Arg Lys Lys Leu Leu Tyr Ile Phe Lys Gln Pro Phe Met
                   405                 410                 415
   Arg Pro Val Gln Thr Thr Gln Glu Glu Asp Gly Cys Ser Cys Arg Phe
               420                 425                 430
   Pro Glu Glu Glu Glu Gly Gly Cys Glu Leu Arg Val Lys Phe Ser Arg
           435                 440                 445
   Ser Ala Asp Ala Pro Ala Tyr Gln Gln Gly Gln Asn Gln Leu Tyr Asn
       450                 455                 460
   Glu Leu Asn Leu Gly Arg Arg Glu Glu Tyr Asp Val Leu Asp Lys Arg
   465                 470                 475                 480
   Arg Gly Arg Asp Pro Glu Met Gly Gly Lys Pro Arg Arg Lys Asn Pro
   485 490 495
   Gln Glu Gly Leu Tyr Asn Glu Leu Gln Lys Asp Lys Met Ala Glu Ala
   500 505 510
   Tyr Ser Glu Ile Gly Met Lys Gly Glu Arg Arg Arg Gly Lys Gly His
   515 520 525
   Asp Gly Leu Tyr Gln Gly Leu Ser Thr Ala Thr Lys Asp Thr Tyr Asp
   530 535 540
   Ala Leu His Met Gln Ala Leu Pro Pro Arg
   545 550
<210> 85
   <211> 724
   <212> PRT
   <213> Artificial Sequence
<220>
   <223> chimeric antigen receptor sequence
<400> 85
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp Tyr Glu Ile Ser Trp Val Arg Gln Ala Pro Gly
       50                  55                  60
   Gln Gly Leu Glu Trp Met Gly Gly Ile Asp Pro Glu Thr Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg Arg Arg Tyr Tyr Gly Ser Ser Ser
           115                 120                 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Gln Asp Ile Gly Asn Tyr Ala Trp
               180                 185                 190
   Tyr Gln Gln Lys Pro Gly Gln Ala Pro Arg Leu Leu Ile Tyr Tyr Thr
           195                 200                 205
   Ser Arg Ala Thr Gly Ile Pro Ala Arg Phe Ser Gly Ser Gly Ser Gly
       210                 215                 220
   Thr Asp Phe Thr Leu Thr Ile Ser Ser Leu Glu Pro Glu Asp Phe Ala
   225                 230                 235                 240
   Val Tyr Tyr Cys Gln Gln Gly Ser Ala Leu Pro Pro Thr Phe Phe Gly
                   245                 250                 255
   Gln Gly Thr Lys Leu Glu Ile Lys Arg Ala Glu Pro Lys Ser Cys Asp
               260                 265                 270
   Lys Thr His Thr Cys Pro Pro Cys Pro Ala Pro Glu Leu Leu Gly Gly
           275                 280                 285
   Pro Ser Val Phe Leu Phe Pro Pro Lys Pro Lys Asp Thr Leu Met Ile
       290                 295                 300
   Ser Arg Thr Pro Glu Val Thr Cys Val Val Val Asp Val Ser His Glu
   305                 310                 315                 320
   Asp Pro Glu Val Lys Phe Asn Trp Tyr Val Asp Gly Val Glu Val His
                   325                 330                 335
   Asn Ala Lys Thr Lys Pro Arg Glu Glu Gln Tyr Asn Ser Thr Tyr Arg
               340                 345                 350
   Val Val Ser Val Leu Thr Val Leu His Gln Asp Trp Leu Asn Gly Lys
           355                 360                 365
   Glu Tyr Lys Cys Lys Val Ser Asn Lys Ala Leu Pro Ala Pro Ile Glu
       370                 375                 380
   Lys Thr Ile Ser Lys Ala Lys Gly Gln Pro Arg Glu Pro Gln Val Tyr
   385 390 395 400
   Thr Leu Pro Pro Ser Arg Asp Glu Leu Thr Lys Asn Gln Val Ser Leu
   405 410 415
   Thr Cys Leu Val Lys Gly Phe Tyr Pro Ser Asp Ile Ala Val Glu Trp
   420 425 430
   Glu Ser Asn Gly Gln Pro Glu Asn Asn Tyr Lys Thr Thr Pro Pro Val
   435 440 445
   Leu Asp Ser Asp Gly Ser Phe Phe Leu Tyr Ser Lys Leu Thr Val Asp
   450 455 460
   Lys Ser Arg Trp Gln Gln Gly Asn Val Phe Ser Cys Ser Val Met His
   465 470 475 480
   Glu Ala Leu His Asn His Tyr Thr Gln Lys Ser Leu Ser Leu Ser Pro
   485 490 495
   Gly Lys Lys Asp Pro Lys Ala Ala Ala Ile Glu Val Met Tyr Pro Pro
   500 505 510
   Pro Tyr Leu Asp Asn Glu Lys Ser Asn Gly Thr Ile Ile His Val Lys
   515 520 525
   Gly Lys His Leu Cys Pro Ser Pro Leu Phe Pro Gly Pro Ser Lys Pro
   530 535 540
   Phe Trp Val Leu Val Val Val Gly Gly Val Leu Ala Cys Tyr Ser Leu
   545 550 555 560
   Leu Val Thr Val Ala Phe Ile Ile Phe Trp Val Arg Ser Lys Arg Ser
   565 570 575
   Arg Leu Leu His Ser Asp Tyr Met Asn Met Thr Pro Arg Arg Pro Gly
   580 585 590
   Pro Thr Arg Lys His Tyr Gln Pro Tyr Ala Pro Pro Arg Asp Phe Ala
   595 600 605
   Ala Tyr Arg Ser Arg Val Lys Phe Ser Arg Ser Ala Asp Ala Pro Ala
   610 615 620
   Tyr Gln Gln Gly Gln Asn Gln Leu Tyr Asn Glu Leu Asn Leu Gly Arg
   625 630 635 640
   Arg Glu Glu Tyr Asp Val Leu Asp Lys Arg Arg Gly Arg Asp Pro Glu
   645 650 655
   Met Gly Gly Lys Pro Arg Arg Lys Asn Pro Gln Glu Gly Leu Tyr Asn
   660 665 670
   Glu Leu Gln Lys Asp Lys Met Ala Glu Ala Tyr Ser Glu Ile Gly Met
   675 680 685
   Lys Gly Glu Arg Arg Arg Gly Lys Gly His Asp Gly Leu Tyr Gln Gly
   690 695 700
   Leu Ser Thr Ala Thr Lys Asp Thr Tyr Asp Ala Leu His Met Gln Ala
   705 710 715 720
   Leu Pro Pro Arg
<210> 86
   <211> 487
   <212> PRT
   <213> Artificial Sequence
<220>
   <223> chimeric antigen receptor sequence
<400> 86
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp Tyr Glu Ile Ser Trp Val Arg Gln Ala Pro Gly
       50                  55                  60
   Gln Gly Leu Glu Trp Met Gly Gly Ile Asp Pro Glu Thr Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg Arg Arg Tyr Tyr Gly Ser Ser Ser
   115 120 125
   Phe Asp Tyr Trp Trp Gly Gln Gly Thr Met Val Thr Val Ser Ser Gly
   130 135 140
   Gly Gly Gly Ser Gly Gly Gly Gly Ser Gly Gly Gly Gly Ser Glu Ile
   145 150 155 160
   Val Leu Thr Gln Ser Pro Ala Thr Leu Ser Leu Ser Pro Gly Glu Arg
   165 170 175
   Ala Thr Leu Ser Cys Arg Ala Ser Gln Asp Ile Gly Asn Tyr Ala Trp
   180 185 190
   Tyr Gln Gln Lys Pro Gly Gln Ala Pro Arg Leu Leu Ile Tyr Tyr Thr
   195 200 205
   Ser Arg Ala Thr Gly Ile Pro Ala Arg Phe Ser Gly Ser Gly Ser Gly
   210 215 220
   Thr Asp Phe Thr Leu Thr Ile Ser Ser Leu Glu Pro Glu Asp Phe Ala
   225 230 235 240
   Val Tyr Tyr Cys Gln Gln Gly Ser Ala Leu Pro Pro Thr Phe Phe Gly
   245 250 255
   Gln Gly Thr Lys Leu Glu Ile Lys Arg Ala Ala Ala Ile Glu Val Met
   260 265 270
   Tyr Pro Pro Pro Tyr Leu Asp Asn Glu Lys Ser Asn Gly Thr Ile Ile
   275 280 285
   His Val Lys Gly Lys His Leu Cys Pro Ser Pro Leu Phe Pro Gly Pro
   290 295 300
   Ser Lys Pro Phe Trp Val Leu Val Val Val Gly Gly Val Leu Ala Cys
   305 310 315 320
   Tyr Ser Leu Leu Val Thr Val Ala Phe Ile Ile Phe Trp Val Arg Ser
   325 330 335
   Lys Arg Ser Arg Leu Leu His Ser Asp Tyr Met Asn Met Thr Pro Arg
   340 345 350
   Arg Pro Gly Pro Thr Arg Lys His Tyr Gln Pro Tyr Ala Pro Pro Arg
   355 360 365
   Asp Phe Ala Ala Tyr Arg Ser Arg Val Lys Phe Ser Arg Ser Ala Asp
       370                 375                 380
   Ala Pro Ala Tyr Gln Gln Gly Gln Asn Gln Leu Tyr Asn Glu Leu Asn
   385                 390                 395                 400
   Leu Gly Arg Arg Glu Glu Tyr Asp Val Leu Asp Lys Arg Arg Gly Arg
                   405                 410                 415
   Asp Pro Glu Met Gly Gly Lys Pro Arg Arg Lys Asn Pro Gln Glu Gly
               420                 425                 430
   Leu Tyr Asn Glu Leu Gln Lys Asp Lys Met Ala Glu Ala Tyr Ser Glu
           435                 440                 445
   Ile Gly Met Lys Gly Glu Arg Arg Arg Gly Lys Gly His Asp Gly Leu
       450                 455                 460
   Tyr Gln Gly Leu Ser Thr Ala Thr Lys Asp Thr Tyr Asp Ala Leu His
   465                 470                 475                 480
   Met Gln Ala Leu Pro Pro Arg
                   485
<210> 87
   <211> 728
   <212> PRT
   <213> Artificial Sequence
<220>
   <223> chimeric antigen receptor sequence
<400> 87
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp Tyr Glu Ile Ser Trp Val Arg Gln Ala Pro Gly
       50                  55                  60
   Gln Gly Leu Glu Trp Met Gly Gly Ile Asp Pro Glu Thr Gly Gly Thr
   65                  70                  75                  80
   Asn Tyr Ala Gln Lys Phe Gln Gly Arg Val Thr Ile Thr Ala Asp Glu
   85 90 95
   Ser Thr Ser Thr Ala Tyr Met Glu Leu Ser Ser Leu Arg Ser Glu Asp
   100 105 110
   Thr Ala Val Tyr Tyr Cys Ala Arg Arg Arg Tyr Tyr Gly Ser Ser Ser
   115 120 125
   Phe Asp Tyr Trp Trp Gly Gln Gly Thr Met Val Thr Val Ser Ser Gly
   130 135 140
   Gly Gly Gly Ser Gly Gly Gly Gly Ser Gly Gly Gly Gly Ser Glu Ile
   145 150 155 160
   Val Leu Thr Gln Ser Pro Ala Thr Leu Ser Leu Ser Pro Gly Glu Arg
   165 170 175
   Ala Thr Leu Ser Cys Arg Ala Ser Gln Asp Ile Gly Asn Tyr Ala Trp
   180 185 190
   Tyr Gln Gln Lys Pro Gly Gln Ala Pro Arg Leu Leu Ile Tyr Tyr Thr
   195 200 205
   Ser Arg Ala Thr Gly Ile Pro Ala Arg Phe Ser Gly Ser Gly Ser Gly
   210 215 220
   Thr Asp Phe Thr Leu Thr Ile Ser Ser Leu Glu Pro Glu Asp Phe Ala
   225 230 235 240
   Val Tyr Tyr Cys Gln Gln Gly Ser Ala Leu Pro Pro Thr Phe Phe Gly
   245 250 255
   Gln Gly Thr Lys Leu Glu Ile Lys Arg Ala Glu Pro Lys Ser Cys Asp
   260 265 270
   Lys Thr His Thr Cys Pro Pro Cys Pro Ala Pro Glu Leu Leu Gly Gly
   275 280 285
   Pro Ser Val Phe Leu Phe Pro Pro Lys Pro Lys Asp Thr Leu Met Ile
   290 295 300
   Ser Arg Thr Pro Glu Val Thr Cys Val Val Val Asp Val Ser His Glu
   305 310 315 320
   Asp Pro Glu Val Lys Phe Asn Trp Tyr Val Asp Gly Val Glu Val His
   325 330 335
   Asn Ala Lys Thr Lys Pro Arg Glu Glu Gln Tyr Asn Ser Thr Tyr Arg
               340                 345                 350
   Val Val Ser Val Leu Thr Val Leu His Gln Asp Trp Leu Asn Gly Lys
           355                 360                 365
   Glu Tyr Lys Cys Lys Val Ser Asn Lys Ala Leu Pro Ala Pro Ile Glu
       370                 375                 380
   Lys Thr Ile Ser Lys Ala Lys Gly Gln Pro Arg Glu Pro Gln Val Tyr
   385                 390                 395                 400
   Thr Leu Pro Pro Ser Arg Asp Glu Leu Thr Lys Asn Gln Val Ser Leu
                   405                 410                 415
   Thr Cys Leu Val Lys Gly Phe Tyr Pro Ser Asp Ile Ala Val Glu Trp
               420                 425                 430
   Glu Ser Asn Gly Gln Pro Glu Asn Asn Tyr Lys Thr Thr Pro Pro Val
           435                 440                 445
   Leu Asp Ser Asp Gly Ser Phe Phe Leu Tyr Ser Lys Leu Thr Val Asp
       450                 455                 460
   Lys Ser Arg Trp Gln Gln Gly Asn Val Phe Ser Cys Ser Val Met His
   465                 470                 475                 480
   Glu Ala Leu His Asn His Tyr Thr Gln Lys Ser Leu Ser Leu Ser Pro
                   485                 490                 495
   Gly Lys Lys Asp Pro Lys Ala Ala Ala Thr Thr Thr Pro Ala Pro Arg
               500                 505                 510
   Pro Pro Thr Pro Ala Pro Thr Ile Ala Ser Gln Pro Leu Ser Leu Arg
           515                 520                 525
   Pro Glu Ala Cys Arg Pro Ala Ala Gly Gly Ala Val His Thr Arg Gly
       530                 535                 540
   Leu Asp Phe Ala Cys Asp Ile Tyr Ile Trp Ala Pro Leu Ala Gly Thr
   545                 550                 555                 560
   Cys Gly Val Leu Leu Leu Ser Leu Val Ile Thr Leu Tyr Cys Lys Arg
                   565                 570                 575
   Gly Arg Lys Lys Leu Leu Tyr Ile Phe Lys Gln Pro Phe Met Arg Pro
               580                 585                 590
   Val Gln Thr Thr Gln Glu Glu Asp Gly Cys Ser Cys Arg Phe Pro Glu
   595 600 605
   Glu Glu Glu Gly Gly Cys Glu Leu Arg Val Lys Phe Ser Arg Ser Ala
   610 615 620
   Asp Ala Pro Ala Tyr Lys Gln Gly Gln Asn Gln Leu Tyr Asn Glu Leu
   625 630 635 640
   Asn Leu Gly Arg Arg Glu Glu Tyr Asp Val Leu Asp Lys Arg Arg Gly
   645 650 655
   Arg Asp Pro Glu Met Gly Gly Lys Pro Arg Arg Lys Asn Pro Gln Glu
   660 665 670
   Gly Leu Tyr Asn Glu Leu Gln Lys Asp Lys Met Ala Glu Ala Tyr Ser
   675 680 685
   Glu Ile Gly Met Lys Gly Glu Arg Arg Arg Gly Lys Gly His Asp Gly
   690 695 700
   Leu Tyr Gln Gly Leu Ser Thr Ala Thr Lys Asp Thr Tyr Asp Ala Leu
   705 710 715 720
   His Met Gln Ala Leu Pro Pro Arg
   725
<210> 88
   <211> 491
   <212> PRT
   <213> Artificial Sequence
<220>
   <223> chimeric antigen receptor sequence
<400> 88
   Leu Leu Val Thr Ser Leu Leu Leu Cys Glu Leu Pro His Pro Ala Phe
   1 5 10 15
   Leu Leu Ile Pro Asp Thr Gln Val Gln Leu Gln Gln Ser Gly Ala Glu
   20 25 30
   Val Lys Lys Pro Gly Ser Ser Val Lys Val Ser Cys Lys Ala Ser Gly
   35 40 45
   Tyr Thr Phe Thr Asp Tyr Glu Ile Ser Trp Val Arg Gln Ala Pro Gly
   50 55 60
   Gln Gly Leu Glu Trp Met Gly Gly Ile Asp Pro Glu Thr Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg Arg Arg Tyr Tyr Gly Ser Ser Ser
           115                 120                 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Gln Asp Ile Gly Asn Tyr Ala Trp
               180                 185                 190
   Tyr Gln Gln Lys Pro Gly Gln Ala Pro Arg Leu Leu Ile Tyr Tyr Thr
           195                 200                 205
   Ser Arg Ala Thr Gly Ile Pro Ala Arg Phe Ser Gly Ser Gly Ser Gly
       210                 215                 220
   Thr Asp Phe Thr Leu Thr Ile Ser Ser Leu Glu Pro Glu Asp Phe Ala
   225                 230                 235                 240
   Val Tyr Tyr Cys Gln Gln Gly Ser Ala Leu Pro Pro Thr Phe Phe Gly
                   245                 250                 255
   Gln Gly Thr Lys Leu Glu Ile Lys Arg Ala Ala Ala Thr Thr Thr Pro
               260                 265                 270
   Ala Pro Arg Pro Pro Thr Pro Ala Pro Thr Ile Ala Ser Gln Pro Leu
           275                 280                 285
   Ser Leu Arg Pro Glu Ala Cys Arg Pro Ala Ala Gly Gly Ala Val His
       290                 295                 300
   Thr Arg Gly Leu Asp Phe Ala Cys Asp Ile Tyr Ile Trp Ala Pro Leu
   305                 310                 315                 320
   Ala Gly Thr Cys Gly Val Leu Leu Leu Ser Leu Val Ile Thr Leu Tyr
                   325                 330                 335
   Cys Lys Arg Gly Arg Lys Lys Leu Leu Tyr Ile Phe Lys Gln Pro Phe
               340                 345                 350
   Met Arg Pro Val Gln Thr Thr Gln Glu Glu Asp Gly Cys Ser Cys Arg
           355                 360                 365
   Phe Pro Glu Glu Glu Glu Gly Gly Cys Glu Leu Arg Val Lys Phe Ser
       370                 375                 380
   Arg Ser Ala Asp Ala Pro Ala Tyr Lys Gln Gly Gln Asn Gln Leu Tyr
   385                 390                 395                 400
   Asn Glu Leu Asn Leu Gly Arg Arg Glu Glu Tyr Asp Val Leu Asp Lys
                   405                 410                 415
   Arg Arg Gly Arg Asp Pro Glu Met Gly Gly Lys Pro Arg Arg Lys Asn
               420                 425                 430
   Pro Gln Glu Gly Leu Tyr Asn Glu Leu Gln Lys Asp Lys Met Ala Glu
           435                 440                 445
   Ala Tyr Ser Glu Ile Gly Met Lys Gly Glu Arg Arg Arg Gly Lys Gly
       450                 455                 460
   His Asp Gly Leu Tyr Gln Gly Leu Ser Thr Ala Thr Lys Asp Thr Tyr
   465                 470                 475                 480
   Asp Ala Leu His Met Gln Ala Leu Pro Pro Arg
                   485                 490
<210> 89
   <211> 788
   <212> PRT
   <213> Artificial Sequence
<220>
   <223> chimeric antigen receptor sequence
<400> 89 785
<210> 90
   <211> 551
   <212> PRT
   <213> Artificial Sequence
<220>
   <223> chimeric antigen receptor sequence
<400> 90
   Leu Leu Val Thr Ser Leu Leu Leu Cys Glu Leu Pro His Pro Ala Phe
   1               5                   10                  15
   Leu Leu Ile Pro Asp Thr Gln Val Gln Leu Gln Gln Ser Gly Ala Glu
               20                  25                  30
   Val Lys Lys Pro Gly Ser Ser Val Lys Val Ser Cys Lys Ala Ser Gly
           35                  40                  45
   Tyr Thr Phe Thr Asp Tyr Glu Ile Ser Trp Val Arg Gln Ala Pro Gly
       50                  55                  60
   Gln Gly Leu Glu Trp Met Gly Gly Ile Asp Pro Glu Thr Gly Gly Thr
   65                  70                  75                  80
   Asn Tyr Ala Gln Lys Phe Gln Gly Arg Val Thr Ile Thr Ala Asp Glu
                   85                  90                  95
   Ser Thr Ser Thr Ala Tyr Met Glu Leu Ser Ser Leu Arg Ser Glu Asp
               100                 105                 110
   Thr Ala Val Tyr Tyr Cys Ala Arg Arg Arg Tyr Tyr Gly Ser Ser Ser
           115                 120                 125
   Phe Asp Tyr Trp Trp Gly Gln Gly Thr Met Val Thr Val Ser Ser Gly
       130                 135                 140
   Gly Gly Gly Ser Gly Gly Gly Gly Ser Gly Gly Gly Gly Ser Glu Ile
   145                 150                 155                 160
   Val Leu Thr Gln Ser Pro Ala Thr Leu Ser Leu Ser Pro Gly Glu Arg
                   165                 170                 175
   Ala Thr Leu Ser Cys Arg Ala Ser Gln Asp Ile Gly Asn Tyr Ala Trp
               180                 185                 190
   Tyr Gln Gln Lys Pro Gly Gln Ala Pro Arg Leu Leu Ile Tyr Tyr Thr
           195                 200                 205
   Ser Arg Ala Thr Gly Ile Pro Ala Arg Phe Ser Gly Ser Gly Ser Gly
       210                 215                 220
   Thr Asp Phe Thr Leu Thr Ile Ser Ser Leu Glu Pro Glu Asp Phe Ala
   225                 230                 235                 240
   Val Tyr Tyr Cys Gln Gln Gly Ser Ala Leu Pro Pro Thr Phe Phe Gly
                   245                 250                 255
   Gln Gly Thr Lys Leu Glu Ile Lys Arg Ala Ala Ala Phe Val Pro Val
               260                 265                 270
   Phe Leu Pro Ala Lys Pro Thr Thr Thr Pro Ala Pro Arg Pro Pro Thr
           275                 280                 285
   Pro Ala Pro Thr Ile Ala Ser Gln Pro Leu Ser Leu Arg Pro Glu Ala
       290                 295                 300
   Cys Arg Pro Ala Ala Gly Gly Ala Val His Thr Arg Gly Leu Asp Phe
   305                 310                 315                 320
   Ala Cys Asp Ile Tyr Ile Trp Ala Pro Leu Ala Gly Thr Cys Gly Val
                   325                 330                 335
   Leu Leu Leu Ser Leu Val Ile Thr Leu Tyr Cys Asn His Arg Asn Arg
               340                 345                 350
   Ser Lys Arg Ser Arg Leu Leu His Ser Asp Tyr Met Asn Met Thr Pro
           355                 360                 365
   Arg Arg Pro Gly Pro Thr Arg Lys His Tyr Gln Pro Tyr Ala Pro Pro
       370                 375                 380
   Arg Asp Phe Ala Ala Tyr Arg Ser Arg Phe Ser Val Val Lys Arg Gly
   385                 390                 395                 400
   Arg Lys Lys Leu Leu Tyr Ile Phe Lys Gln Pro Phe Met Arg Pro Val
                   405                 410                 415
   Gln Thr Thr Gln Glu Glu Asp Gly Cys Ser Cys Arg Phe Pro Glu Glu
               420                 425                 430
   Glu Glu Gly Gly Cys Glu Leu Arg Val Lys Phe Ser Arg Ser Ala Asp
           435                 440                 445
   Ala Pro Ala Tyr Gln Gln Gly Gln Asn Gln Leu Tyr Asn Glu Leu Asn
   450 455 460
   Leu Gly Arg Arg Glu Glu Tyr Asp Val Leu Asp Lys Arg Arg Gly Arg
   465 470 475 480
   Asp Pro Glu Met Gly Gly Lys Pro Arg Arg Lys Asn Pro Gln Glu Gly
   485 490 495
   Leu Tyr Asn Glu Leu Gln Lys Asp Lys Met Ala Glu Ala Tyr Ser Glu
   500 505 510
   Ile Gly Met Lys Gly Glu Arg Arg Arg Gly Lys Gly His Asp Gly Leu
   515 520 525
   Tyr Gln Gly Leu Ser Thr Ala Thr Lys Asp Thr Tyr Asp Ala Leu His
   530 535 540
   Met Gln Ala Leu Pro Pro Arg
   545 550
<210> 91
   <211> 2203
   <212> DNA
   <213> Artificial Sequence
<220>
   <223> chimeric antigen receptor sequence
<400> 91
   ccctcgagcc gccaccatgg ttctgctcgt gacaagcctg ctgctgtgcg agctgcccca      60
   ccctgccttt ctgctgatcc ccgacaccga cgtgaagctg caggaatctg gccctggact     120
   ggtggcccct agccagagcc tgagcatcac ctgtaccgtg tccggcttca gcctgaccag     180
   ctacgccgtg tcttgggtgc gccagcctcc tggcaaaggc ctggaatggc tgggcatcat     240
   ttggagcggc ggagccacca actacaacag cgccctgaag tcccggctga gcatctccaa     300
   ggacaacagc aagagccagg tgttcctgaa gatgaacggc ctgcagaccg acgacaccgc     360
   ccggtactat tgcgccagag agcactacta cggcagcagc gctatggact actggggcca     420
   gggcgccagc atcacagtgt ctagcggagg cggaggatct ggcggcggag gaagtggcgg     480
   agggggatct ggaatcgtga tgacccagag ccctctgagc ctgcctgtgt ccctgggaga     540
   tcaggcctcc atcagctgca gatccagcca gagcatcgtg cacagctacg gcaacaccta     600
   cctgttctgg tatctgcaga agcccggcca gagccccaag ctgctgatct accgggtgtc     660
   caaccggttc agcggcgtgc ccgatagatt ttccggcagc ggctccggca ccaacttcac     720
   cctgaagatc agccgggtgg aagccgagga catgggcgtg tactactgtt ttcaaggcac     780
   ccacgtgccc tacaccttcg gaggcggcac caagctggaa atcaaagagc ccaagagctg     840
   cgacaagacc cacacctgtc ccccttgtcc tgcccctgaa ctgctgggcg gacctagcgt 900
   gttcctgttc cccccaaagc ccaaggacac cctgatgatc tcccggaccc ccgaagtgac 960
   ctgcgtggtg gtggatgtgt cccacgagga ccctgaagtg aagttcaatt ggtacgtgga 1020
   cggcgtggaa gtgcacaacg ccaagaccaa gcccagagag gaacagtaca acagcaccta 1080
   cagagtggtg tccgtgctga ccgtgctgca ccaggactgg ctgaacggca aagagtacaa 1140
   gtgcaaagtg tccaacaagg ccctgcctgc ccccatcgag aaaaccatca gcaaggccaa 1200
   gggccagccc cgcgaacccc aggtgtacac actgccccct agcagggacg agctgaccaa 1260
   gaatcaggtg tccctgacct gtctcgtgaa gggcttctac ccctccgata tcgccgtgga 1320
   atgggagagc aacggccagc ccgagaacaa ctacaagacc accccccctg tgctggacag 1380
   cgacggctca ttcttcctgt acagcaagct gacagtggac aagagccggt ggcagcaggg 1440
   caacgtgttc agctgctccg tgatgcacga ggccctgcac aaccactaca cccagaagtc 1500
   cctgtccctg agccccggca agaaggaccc caaagcggcc gcaattgaag ttatgtatcc 1560
   tcctccttac ctagacaatg agaagagcaa tggaaccatt atccatgtga aagggaaaca 1620
   cctttgtcca agtcccctat ttcccggacc ttctaagccc ttttgggtgc tggtggtggt 1680
   tgggggagtc ctggcttgct atagcttgct agtaacagtg gcctttatta ttttctgggt 1740
   gaggagtaag aggagcaggc tcctgcacag tgactacatg aacatgactc cccgccgccc 1800
   cgggcccacc cgcaagcatt accagcccta tgccccacca cgcgacttcg cagcctatcg 1860
   ctccagagtg aagttcagca ggagcgcaga cgcccccgcg taccagcagg gccagaacca 1920
   gctctataac gagctcaatc taggacgaag agaggagtac gatgttttgg acaagagacg 1980
   tggccgggac cctgagatgg ggggaaagcc gagaaggaag aaccctcagg aaggcctgta 2040
   caatgaactg cagaaagata agatggcgga ggcctacagt gagattggga tgaaaggcga 2100
   gcgccggagg ggcaaggggc acgatggcct ttaccagggt ctcagtacag ccaccaagga 2160
   cacctacgac gcccttcaca tgcaggccct gccccctcgc taa 2203
<210> 92
   <211> 1495
   <212> DNA
   <213> Artificial Sequence
<220>
   <223> chimeric antigen receptor sequence
<400> 92
   ccctcgagcc gccaccatgg ttctcctcgt gacttccctt ctgctgtgcg agctcccaca 60
   ccccgccttc ctgctcattc ctgataccga tgtgaagctg caagaatccg gccccggact 120
   ggtcgcgcca agccaatcgc tgagcattac ttgcacggtg tccggatttt cgttgacctc 180
   ctacgctgtg tcctgggtca gacagccgcc gggtaaagga ctcgaatggc ttggcatcat 240
   ctggtcgggc ggagcgacta actacaactc agcgctgaaa tcgcggctgt ccatctcaaa 300
   ggataattca aaaagccagg tgtttctgaa gatgaatggc ctgcagactg acgacaccgc 360
   tcgctactac tgcgcccgcg agcattacta cggatcatcc gcaatggact attgggggca 420
   gggcgcatct atcaccgtca gcagcggggg cggaggttct ggcggagggg gttcgggcgg 480
   gggagggagc ggaatcgtga tgacccagtc gccgctttcc ttgcctgtca gcctgggaga 540
   tcaggccagc atctcatgtc ggtcgtccca gagcatcgtg cactcgtacg gtaacacgta 600
   cctcttctgg tacctccaaa agcctggaca gtcaccaaag ctgttgatct atagggtgtc 660
   caatcgcttc tcgggtgtgc cggaccggtt ctcgggctcg ggatcaggaa ccaactttac 720
   tctgaagatc tccagagtgg aagccgagga catgggagtc tactactgct tccaaggaac 780
   tcatgttccg tacaccttcg gaggagggac caagctggaa atcaaggcgg ccgcaattga 840
   agttatgtat cctcctcctt acctagacaa tgagaagagc aatggaacca ttatccatgt 900
   gaaagggaaa cacctttgtc caagtcccct atttcccgga ccttctaagc ccttttgggt 960
   gctggtggtg gttgggggag tcctggcttg ctatagcttg ctagtaacag tggcctttat 1020
   tattttctgg gtgaggagta agaggagcag gctcctgcac agtgactaca tgaacatgac 1080
   tccccgccgc cccgggccca cccgcaagca ttaccagccc tatgccccac cacgcgactt 1140
   cgcagcctat cgctccagag tgaagttcag caggagcgca gacgcccccg cgtaccagca 1200
   gggccagaac cagctctata acgagctcaa tctaggacga agagaggagt acgatgtttt 1260
   ggacaagaga cgtggccggg accctgagat ggggggaaag ccgagaagga agaaccctca 1320
   ggaaggcctg tacaatgaac tgcagaaaga taagatggcg gaggcctaca gtgagattgg 1380
   gatgaaaggc gagcgccgga ggggcaaggg gcacgatggc ctttaccagg gtctcagtac 1440
   agccaccaag gacacctacg acgcccttca catgcaggcc ctgccccctc gctaa 1495
<210> 93
   <211> 2215
   <212> DNA
   <213> Artificial Sequence
<220>
   <223> chimeric antigen receptor sequence
<400> 93
   ccctcgagcc gccaccatgg ttctgctcgt gacaagcctg ctgctgtgcg agctgcccca      60
   ccctgccttt ctgctgatcc ccgacaccga cgtgaagctg caggaatctg gccctggact     120
   ggtggcccct agccagagcc tgagcatcac ctgtaccgtg tccggcttca gcctgaccag     180
   ctacgccgtg tcttgggtgc gccagcctcc tggcaaaggc ctggaatggc tgggcatcat     240
   ttggagcggc ggagccacca actacaacag cgccctgaag tcccggctga gcatctccaa     300
   ggacaacagc aagagccagg tgttcctgaa gatgaacggc ctgcagaccg acgacaccgc 360
   ccggtactat tgcgccagag agcactacta cggcagcagc gctatggact actggggcca 420
   gggcgccagc atcacagtgt ctagcggagg cggaggatct ggcggcggag gaagtggcgg 480
   agggggatct ggaatcgtga tgacccagag ccctctgagc ctgcctgtgt ccctgggaga 540
   tcaggcctcc atcagctgca gatccagcca gagcatcgtg cacagctacg gcaacaccta 600
   cctgttctgg tatctgcaga agcccggcca gagccccaag ctgctgatct accgggtgtc 660
   caaccggttc agcggcgtgc ccgatagatt ttccggcagc ggctccggca ccaacttcac 720
   cctgaagatc agccgggtgg aagccgagga catgggcgtg tactactgtt ttcaaggcac 780
   ccacgtgccc tacaccttcg gaggcggcac caagctggaa atcaaagagc ccaagagctg 840
   cgacaagacc cacacctgtc ccccttgtcc tgcccctgaa ctgctgggcg gacctagcgt 900
   gttcctgttc cccccaaagc ccaaggacac cctgatgatc tcccggaccc ccgaagtgac 960
   ctgcgtggtg gtggatgtgt cccacgagga ccctgaagtg aagttcaatt ggtacgtgga 1020
   cggcgtggaa gtgcacaacg ccaagaccaa gcccagagag gaacagtaca acagcaccta 1080
   cagagtggtg tccgtgctga ccgtgctgca ccaggactgg ctgaacggca aagagtacaa 1140
   gtgcaaagtg tccaacaagg ccctgcctgc ccccatcgag aaaaccatca gcaaggccaa 1200
   gggccagccc cgcgaacccc aggtgtacac actgccccct agcagggacg agctgaccaa 1260
   gaatcaggtg tccctgacct gtctcgtgaa gggcttctac ccctccgata tcgccgtgga 1320
   atgggagagc aacggccagc ccgagaacaa ctacaagacc accccccctg tgctggacag 1380
   cgacggctca ttcttcctgt acagcaagct gacagtggac aagagccggt ggcagcaggg 1440
   caacgtgttc agctgctccg tgatgcacga ggccctgcac aaccactaca cccagaagtc 1500
   cctgtccctg agccccggca agaaggaccc caaagcggcc gcaaccacga cgccagcgcc 1560
   gcgaccacca acaccggcgc ccaccatcgc gtcgcagccc ctgtccctgc gcccagaggc 1620
   gtgccggcca gcggcggggg gcgcagtgca cacgaggggg ctggacttcg cctgtgatat 1680
   ctacatctgg gcgcccttgg ccgggacttg tggggtcctt ctcctgtcac tggttatcac 1740
   cctttactgc aaacggggca gaaagaaact cctgtatata ttcaaacaac catttatgag 1800
   accagtacaa actactcaag aggaagatgg ctgtagctgc cgatttccag aagaagaaga 1860
   aggaggatgt gaactgagag tgaagttcag caggagcgca gacgcccccg cgtacaagca 1920
   gggccagaac cagctctata acgagctcaa tctaggacga agagaggagt acgatgtttt 1980
   ggacaagaga cgtggccggg accctgagat ggggggaaag ccgagaagga agaaccctca 2040
   ggaaggcctg tacaatgaac tgcagaaaga taagatggcg gaggcctaca gtgagattgg 2100
   gatgaaaggc gagcgccgga ggggcaaggg gcacgatggc ctttaccagg gtctcagtac 2160
   agccaccaag gacacctacg acgcccttca catgcaggcc ctgccccctc gctaa 2215
<210> 94
   <211> 1507
   <212> DNA
   <213> Artificial Sequence
<220>
   <223> chimeric antigen receptor sequence
<400> 94
   ccctcgagcc gccaccatgg ttctcctcgt gacttccctt ctgctgtgcg agctcccaca 60
   ccccgccttc ctgctcattc ctgataccga tgtgaagctg caagaatccg gccccggact 120
   ggtcgcgcca agccaatcgc tgagcattac ttgcacggtg tccggatttt cgttgacctc 180
   ctacgctgtg tcctgggtca gacagccgcc gggtaaagga ctcgaatggc ttggcatcat 240
   ctggtcgggc ggagcgacta actacaactc agcgctgaaa tcgcggctgt ccatctcaaa 300
   ggataattca aaaagccagg tgtttctgaa gatgaatggc ctgcagactg acgacaccgc 360
   tcgctactac tgcgcccgcg agcattacta cggatcatcc gcaatggact attgggggca 420
   gggcgcatct atcaccgtca gcagcggggg cggaggttct ggcggagggg gttcgggcgg 480
   gggagggagc ggaatcgtga tgacccagtc gccgctttcc ttgcctgtca gcctgggaga 540
   tcaggccagc atctcatgtc ggtcgtccca gagcatcgtg cactcgtacg gtaacacgta 600
   cctcttctgg tacctccaaa agcctggaca gtcaccaaag ctgttgatct atagggtgtc 660
   caatcgcttc tcgggtgtgc cggaccggtt ctcgggctcg ggatcaggaa ccaactttac 720
   tctgaagatc tccagagtgg aagccgagga catgggagtc tactactgct tccaaggaac 780
   tcatgttccg tacaccttcg gaggagggac caagctggaa atcaaggcgg ccgcaaccac 840
   gacgccagcg ccgcgaccac caacaccggc gcccaccatc gcgtcgcagc ccctgtccct 900
   gcgcccagag gcgtgccggc cagcggcggg gggcgcagtg cacacgaggg ggctggactt 960
   cgcctgtgat atctacatct gggcgccctt ggccgggact tgtggggtcc ttctcctgtc 1020
   actggttatc accctttact gcaaacgggg cagaaagaaa ctcctgtata tattcaaaca 1080
   accatttatg agaccagtac aaactactca agaggaagat ggctgtagct gccgatttcc 1140
   agaagaagaa gaaggaggat gtgaactgag agtgaagttc agcaggagcg cagacgcccc 1200
   cgcgtacaag cagggccaga accagctcta taacgagctc aatctaggac gaagagagga 1260
   gtacgatgtt ttggacaaga gacgtggccg ggaccctgag atggggggaa agccgagaag 1320
   gaagaaccct caggaaggcc tgtacaatga actgcagaaa gataagatgg cggaggccta 1380
   cagtgagatt gggatgaaag gcgagcgccg gaggggcaag gggcacgatg gcctttacca 1440
   gggtctcagt acagccacca aggacaccta cgacgccctt cacatgcagg ccctgccccc 1500
   tcgctaa 1507
<210> 95
   <211> 2395
   <212> DNA
   <213> Artificial Sequence
<220>
   <223> chimeric antigen receptor sequence
<400> 95
   ccctcgagcc gccaccatgg ttctgctcgt gacaagcctg ctgctgtgcg agctgcccca      60
   ccctgccttt ctgctgatcc ccgacaccga cgtgaagctg caggaatctg gccctggact     120
   ggtggcccct agccagagcc tgagcatcac ctgtaccgtg tccggcttca gcctgaccag     180
   ctacgccgtg tcttgggtgc gccagcctcc tggcaaaggc ctggaatggc tgggcatcat     240
   ttggagcggc ggagccacca actacaacag cgccctgaag tcccggctga gcatctccaa     300
   ggacaacagc aagagccagg tgttcctgaa gatgaacggc ctgcagaccg acgacaccgc     360
   ccggtactat tgcgccagag agcactacta cggcagcagc gctatggact actggggcca     420
   gggcgccagc atcacagtgt ctagcggagg cggaggatct ggcggcggag gaagtggcgg     480
   agggggatct ggaatcgtga tgacccagag ccctctgagc ctgcctgtgt ccctgggaga     540
   tcaggcctcc atcagctgca gatccagcca gagcatcgtg cacagctacg gcaacaccta     600
   cctgttctgg tatctgcaga agcccggcca gagccccaag ctgctgatct accgggtgtc     660
   caaccggttc agcggcgtgc ccgatagatt ttccggcagc ggctccggca ccaacttcac     720
   cctgaagatc agccgggtgg aagccgagga catgggcgtg tactactgtt ttcaaggcac     780
   ccacgtgccc tacaccttcg gaggcggcac caagctggaa atcaaagagc ccaagagctg     840
   cgacaagacc cacacctgtc ccccttgtcc tgcccctgaa ctgctgggcg gacctagcgt     900
   gttcctgttc cccccaaagc ccaaggacac cctgatgatc tcccggaccc ccgaagtgac     960
   ctgcgtggtg gtggatgtgt cccacgagga ccctgaagtg aagttcaatt ggtacgtgga    1020
   cggcgtggaa gtgcacaacg ccaagaccaa gcccagagag gaacagtaca acagcaccta    1080
   cagagtggtg tccgtgctga ccgtgctgca ccaggactgg ctgaacggca aagagtacaa    1140
   gtgcaaagtg tccaacaagg ccctgcctgc ccccatcgag aaaaccatca gcaaggccaa    1200
   gggccagccc cgcgaacccc aggtgtacac actgccccct agcagggacg agctgaccaa    1260
   gaatcaggtg tccctgacct gtctcgtgaa gggcttctac ccctccgata tcgccgtgga    1320
   atgggagagc aacggccagc ccgagaacaa ctacaagacc accccccctg tgctggacag    1380
   cgacggctca ttcttcctgt acagcaagct gacagtggac aagagccggt ggcagcaggg    1440
   caacgtgttc agctgctccg tgatgcacga ggccctgcac aaccactaca cccagaagtc    1500
   cctgtccctg agccccggca agaaggaccc caaagcggcc gcattcgtgc cggtcttcct    1560
   gccagcgaag cccaccacga cgccagcgcc gcgaccacca acaccggcgc ccaccatcgc 1620
   gtcgcagccc ctgtccctgc gcccagaggc gtgccggcca gcggcggggg gcgcagtgca 1680
   cacgaggggg ctggacttcg cctgtgatat ctacatctgg gcgcccttgg ccgggacttg 1740
   tggggtcctt ctcctgtcac tggttatcac cctttactgc aaccacagga acaggagtaa 1800
   gaggagcagg ctcctgcaca gtgactacat gaacatgact ccccgccgcc ccgggcccac 1860
   ccgcaagcat taccagccct atgccccacc acgcgacttc gcagcctatc gctcccgttt 1920
   ctctgttgtt aaacggggca gaaagaagct cctgtatata ttcaaacaac catttatgag 1980
   accagtacaa actactcaag aggaagatgg ctgtagctgc cgatttccag aagaagaaga 2040
   aggaggatgt gaactgagag tgaagttcag caggagcgca gacgcccccg cgtaccagca 2100
   gggccagaac cagctctata acgagctcaa tctaggacga agagaggagt acgatgtttt 2160
   ggacaagaga cgtggccggg accctgagat ggggggaaag ccgagaagga agaaccctca 2220
   ggaaggcctg tacaatgaac tgcagaaaga taagatggcg gaggcctaca gtgagattgg 2280
   gatgaaaggc gagcgccgga ggggcaaggg gcacgatggc ctttaccagg gtctcagtac 2340
   agccaccaag gacacctacg acgcccttca catgcaggcc ctgccccctc gctaa 2395
<210> 96
   <211> 1687
   <212> DNA
   <213> Artificial Sequence
<220>
   <223> chimeric antigen receptor sequence
<400> 96
   ccctcgagcc gccaccatgg ttctcctcgt gacttccctt ctgctgtgcg agctcccaca        60
   ccccgccttc ctgctcattc ctgataccga tgtgaagctg caagaatccg gccccggact       120
   ggtcgcgcca agccaatcgc tgagcattac ttgcacggtg tccggatttt cgttgacctc       180
   ctacgctgtg tcctgggtca gacagccgcc gggtaaagga ctcgaatggc ttggcatcat       240
   ctggtcgggc ggagcgacta actacaactc agcgctgaaa tcgcggctgt ccatctcaaa       300
   ggataattca aaaagccagg tgtttctgaa gatgaatggc ctgcagactg acgacaccgc       360
   tcgctactac tgcgcccgcg agcattacta cggatcatcc gcaatggact attgggggca       420
   gggcgcatct atcaccgtca gcagcggggg cggaggttct ggcggagggg gttcgggcgg       480
   gggagggagc ggaatcgtga tgacccagtc gccgctttcc ttgcctgtca gcctgggaga       540
   tcaggccagc atctcatgtc ggtcgtccca gagcatcgtg cactcgtacg gtaacacgta       600
   cctcttctgg tacctccaaa agcctggaca gtcaccaaag ctgttgatct atagggtgtc       660
   caatcgcttc tcgggtgtgc cggaccggtt ctcgggctcg ggatcaggaa ccaactttac       720
   tctgaagatc tccagagtgg aagccgagga catgggagtc tactactgct tccaaggaac       780
   tcatgttccg tacaccttcg gaggagggac caagctggaa atcaaggcgg ccgcattcgt       840
   gccggtcttc ctgccagcga agcccaccac gacgccagcg ccgcgaccac caacaccggc       900
   gcccaccatc gcgtcgcagc ccctgtccct gcgcccagag gcgtgccggc cagcggcggg       960
   gggcgcagtg cacacgaggg ggctggactt cgcctgtgat atctacatct gggcgcccct      1020
   ggccgggact tgtggggtcc ttctcctgtc actggttatc accctttact gcaaccacag      1080
   gaacaggagt aagaggagca ggctcctgca cagtgactac atgaacatga ctccccgccg      1140
   ccccgggccc acccgcaagc attaccagcc ctatgcccca ccacgcgact tcgcagccta      1200
   tcgctcccgt ttctctgttg ttaaacgggg cagaaagaag ctcctgtata tattcaaaca      1260
   accatttatg agaccagtac aaactactca agaggaagat ggctgtagct gccgatttcc      1320
   agaagaagaa gaaggaggat gtgaactgag agtgaagttc agcaggagcg cagacgcccc      1380
   cgcgtaccag cagggccaga accagctcta taacgagctc aatctaggac gaagagagga      1440
   gtacgatgtt ttggacaaga gacgtggccg ggaccctgag atggggggaa agccgagaag      1500
   gaagaaccct caggaaggcc tgtacaatga actgcagaaa gataagatgg cggaggccta      1560
   cagtgagatt gggatgaaag gcgagcgccg gaggggcaag gggcacgatg gcctttacca      1620
   gggtctcagt acagccacca aggacaccta cgacgccctt cacatgcagg ccctgccccc      1680
   tcgctaa                                                                1687
<210> 97
   <211> 2209
   <212> DNA
   <213> Artificial Sequence
<220>
   <223> chimeric antigen receptor sequence
<400> 97
   ccctcgagcc gccaccatgg ttcttctcgt gacaagcctt cttctctgcg aattgcccca      60
   cccagccttt ttgcttatcc ccgacaccca agtccagctg cagcaatcag gggccgagtt     120
   ggtcaagcct ggggcatcgg tcaaaatctc atgtaaagcc tcgggatatg cgttctcgtc     180
   atactggatg aattgggtca agcagcggcc aggaaaggga ctggaatgga tcgggcaaat     240
   ctacccaggg gatggagata caacatataa cgggaagttt aaagggaaag caactctcac     300
   tgcggacaag tcatcatcga cggtatacat gcagcttaac tcattgacaa gcgaggactc     360
   ggcggtctat ttctgcgtac ggtattacta cggatcgtcg gggtacttcg attattgggg      420
   tcagggaacc acgctgacag tgtccagcgg aggtggcggg tccggaggcg gaggatccgg     480
   tggcggtgga agcgatgtgc agatgatcca gacgccggac tcactcgcgg tgtcactcgg     540
   gcagcgggcg acgatttcat gcagagcctc cgagtcggtg gacaattacg gtatctcctt     600
   catgcattgg tatcagcaga aacccgggca gtcgcccaag ctgttgatct acagagcgtc 660
   caaccttgag tcggggattc ccgctaggtt ctccgggtca ggatcccgca cggacttcac 720
   cttgacgatt aacccggtgg aaactgatga cgtcgccact tactactgtc agcagaacaa 780
   taaggaccct cccacatttg gcggaggtac gaagcttgaa atcaagaggg cggagccgaa 840
   gagctgcgat aaaacgcaca catgccctcc atgccctgca ccggagctct tgggcggacc 900
   ttccgtgttt ctgttcccac cgaaacccaa agacaccctg atgatttcgc gcacgccgga 960
   ggtaacttgt gtggtggtgg acgtaagcca tgaggacccg gaagtaaagt tcaactggta 1020
   tgtcgatggc gtggaggtcc acaatgcgaa aaccaagccg agagaggaac agtataactc 1080
   cacgtaccga gtcgtaagcg tgcttacagt gcttcatcaa gattggttga atggtaaaga 1140
   atacaaatgc aaggtgtcga acaaagctct gcccgcacca attgagaaaa ctattagcaa 1200
   ggcgaagggg cagcccaggg aaccccaagt gtatactttg ccgccctcgc gcgatgaact 1260
   cactaagaat caagtctcgc tgacgtgtct cgtcaagggg ttttacccga gcgacatcgc 1320
   ggtggagtgg gagtcgaacg gtcaaccgga gaacaattac aaaaccacac ctcccgtgct 1380
   cgattcggac ggatcgtttt tcctctattc caaattgacc gtcgataagt cgcgatggca 1440
   gcagggtaat gtattttcgt gttcggtaat gcacgaagcc ctccacaacc attatacgca 1500
   gaagtcgctg tccctgtcgc ccggaaagaa agacccgaag gcggccgcaa ttgaagttat 1560
   gtatcctcct ccttacctag acaatgagaa gagcaatgga accattatcc atgtgaaagg 1620
   gaaacacctt tgtccaagtc ccctatttcc cggaccttct aagccctttt gggtgctggt 1680
   ggtggttggg ggagtcctgg cttgctatag cttgctagta acagtggcct ttattatttt 1740
   ctgggtgagg agtaagagga gcaggctcct gcacagtgac tacatgaaca tgactccccg 1800
   ccgccccggg cccacccgca agcattacca gccctatgcc ccaccacgcg acttcgcagc 1860
   ctatcgctcc agagtgaagt tcagcaggag cgcagacgcc cccgcgtacc agcagggcca 1920
   gaaccagctc tataacgagc tcaatctagg acgaagagag gagtacgatg ttttggacaa 1980
   gagacgtggc cgggaccctg agatgggggg aaagccgaga aggaagaacc ctcaggaagg 2040
   cctgtacaat gaactgcaga aagataagat ggcggaggcc tacagtgaga ttgggatgaa 2100
   aggcgagcgc cggaggggca aggggcacga tggcctttac cagggtctca gtacagccac 2160
   caaggacacc tacgacgccc ttcacatgca ggccctgccc cctcgctaa 2209
<210> 98
   <211> 1501
   <212> DNA
   <213> Artificial Sequence
<220>
   <223> chimeric antigen receptor sequence
<400> 98
   ccctcgagcc gccaccatgg ttctgcttgt gacctccctg cttctctgcg aactccctca      60
   tccggcattc ctgctcatcc ccgacaccca agtccaactc cagcagagcg gagccgagct     120
   ggtgaagccg ggagcgagcg tcaaaatcag ctgtaaagcc tccggctacg ccttcagctc     180
   atactggatg aactgggtga agcaaagacc gggaaagggg ttggaatgga tcggacaaat     240
   ctacccggga gatggagata ctacctacaa tgggaagttt aaaggaaagg ccactctgac     300
   cgctgataag tcctcgtcca cggtctacat gcagctcaac tcactgactt cggaggatag     360
   cgccgtgtac ttctgcgtgc gctactacta cggatcatca ggatacttcg actactgggg     420
   ccaaggtacc actctcaccg tgtcgtcggg aggaggcggc tccggcggtg gaggatccgg     480
   aggcggaggc tcagacgtgc agatgattca gactcccgac tcgctggcgg tgtccctcgg     540
   tcagagggcc accatttcgt gccgggcttc ggagtcagtg gacaattacg gcatcagctt     600
   tatgcactgg tatcagcaaa agccaggcca gtccccaaag ttgctgatct accgcgcatc     660
   gaatctggag tccggcatcc cagctcggtt cagcgggagc ggatcgagaa ctgactttac     720
   gctgaccatc aacccggtcg aaaccgatga cgtcgcaact tattactgcc agcagaacaa     780
   caaggaccct ccgaccttcg gtggagggac taagctggaa atcaaacgcg cggcggccgc     840
   aattgaagtt atgtatcctc ctccttacct agacaatgag aagagcaatg gaaccattat     900
   ccatgtgaaa gggaaacacc tttgtccaag tcccctattt cccggacctt ctaagccctt     960
   ttgggtgctg gtggtggttg ggggagtcct ggcttgctat agcttgctag taacagtggc     1020
   ctttattatt ttctgggtga ggagtaagag gagcaggctc ctgcacagtg actacatgaa    1080
   catgactccc cgccgccccg ggcccacccg caagcattac cagccctatg ccccaccacg    1140
   cgacttcgca gcctatcgct ccagagtgaa gttcagcagg agcgcagacg cccccgcgta    1200
   ccagcagggc cagaaccagc tctataacga gctcaatcta ggacgaagag aggagtacga    1260
   tgttttggac aagagacgtg gccgggaccc tgagatgggg ggaaagccga gaaggaagaa    1320
   ccctcaggaa ggcctgtaca atgaactgca gaaagataag atggcggagg cctacagtga    1380
   gattgggatg aaaggcgagc gccggagggg caaggggcac gatggccttt accagggtct    1440
   cagtacagcc accaaggaca cctacgacgc ccttcacatg caggccctgc cccctcgcta    1500
   a                                                                    1501
<210> 99
   <211> 2221
   <212> DNA
   <213> Artificial Sequence
<220>
   <223> chimeric antigen receptor sequence
<400> 99
   ccctcgagcc gccaccatgg ttcttctcgt gacaagcctt cttctctgcg aattgcccca      60
   cccagccttt ttgcttatcc ccgacaccca agtccagctg cagcaatcag gggccgagtt      120
   ggtcaagcct ggggcatcgg tcaaaatctc atgtaaagcc tcgggatatg cgttctcgtc     180
   atactggatg aattgggtca agcagcggcc aggaaaggga ctggaatgga tcgggcaaat     240
   ctacccaggg gatggagata caacatataa cgggaagttt aaagggaaag caactctcac     300
   tgcggacaag tcatcatcga cggtatacat gcagcttaac tcattgacaa gcgaggactc     360
   ggcggtctat ttctgcgtac ggtattacta cggatcgtcg gggtacttcg attattgggg      420
   tcagggaacc acgctgacag tgtccagcgg aggtggcggg tccggaggcg gaggatccgg     480
   tggcggtgga agcgatgtgc agatgatcca gacgccggac tcactcgcgg tgtcactcgg     540
   gcagcgggcg acgatttcat gcagagcctc cgagtcggtg gacaattacg gtatctcctt     600
   catgcattgg tatcagcaga aacccgggca gtcgcccaag ctgttgatct acagagcgtc     660
   caaccttgag tcggggattc ccgctaggtt ctccgggtca ggatcccgca cggacttcac     720
   cttgacgatt aacccggtgg aaactgatga cgtcgccact tactactgtc agcagaacaa     780
   taaggaccct cccacatttg gcggaggtac gaagcttgaa atcaagaggg cggagccgaa     840
   gagctgcgat aaaacgcaca catgccctcc atgccctgca ccggagctct tgggcggacc     900
   ttccgtgttt ctgttcccac cgaaacccaa agacaccctg atgatttcgc gcacgccgga     960
   ggtaacttgt gtggtggtgg acgtaagcca tgaggacccg gaagtaaagt tcaactggta    1020
   tgtcgatggc gtggaggtcc acaatgcgaa aaccaagccg agagaggaac agtataactc    1080
   cacgtaccga gtcgtaagcg tgcttacagt gcttcatcaa gattggttga atggtaaaga    1140
   atacaaatgc aaggtgtcga acaaagctct gcccgcacca attgagaaaa ctattagcaa    1200
   ggcgaagggg cagcccaggg aaccccaagt gtatactttg ccgccctcgc gcgatgaact    1260
   cactaagaat caagtctcgc tgacgtgtct cgtcaagggg ttttacccga gcgacatcgc    1320
   ggtggagtgg gagtcgaacg gtcaaccgga gaacaattac aaaaccacac ctcccgtgct    1380
   cgattcggac ggatcgtttt tcctctattc caaattgacc gtcgataagt cgcgatggca    1440
   gcagggtaat gtattttcgt gttcggtaat gcacgaagcc ctccacaacc attatacgca    1500
   gaagtcgctg tccctgtcgc ccggaaagaa agacccgaag gcggccgcaa ccacgacgcc    1560
   agcgccgcga ccaccaacac cggcgcccac catcgcgtcg cagcccctgt ccctgcgccc    1620
   agaggcgtgc cggccagcgg cggggggcgc agtgcacacg agggggctgg acttcgcctg    1680
   tgatatctac atctgggcgc ccttggccgg gacttgtggg gtccttctcc tgtcactggt    1740
   tatcaccctt tactgcaaac ggggcagaaa gaaactcctg tatatattca aacaaccatt    1800
   tatgagacca gtacaaacta ctcaagagga agatggctgt agctgccgat ttccagaaga    1860
   agaagaagga ggatgtgaac tgagagtgaa gttcagcagg agcgcagacg cccccgcgta    1920
   caagcagggc cagaaccagc tctataacga gctcaatcta ggacgaagag aggagtacga 1980
   tgttttggac aagagacgtg gccgggaccc tgagatgggg ggaaagccga gaaggaagaa 2040
   ccctcaggaa ggcctgtaca atgaactgca gaaagataag atggcggagg cctacagtga 2100
   gattgggatg aaaggcgagc gccggagggg caaggggcac gatggccttt accagggtct 2160
   cagtacagcc accaaggaca cctacgacgc ccttcacatg caggccctgc cccctcgcta 2220
   a 2221
<210> 100
   <211> 1513
   <212> DNA
   <213> Artificial Sequence
<220>
   <223> chimeric antigen receptor sequence
<400> 100
   ccctcgagcc gccaccatgg ttctgcttgt gacctccctg cttctctgcg aactccctca      60
   tccggcattc ctgctcatcc ccgacaccca agtccaactc cagcagagcg gagccgagct     120
   ggtgaagccg ggagcgagcg tcaaaatcag ctgtaaagcc tccggctacg ccttcagctc     180
   atactggatg aactgggtga agcaaagacc gggaaagggg ttggaatgga tcggacaaat     240
   ctacccggga gatggagata ctacctacaa tgggaagttt aaaggaaagg ccactctgac     300
   cgctgataag tcctcgtcca cggtctacat gcagctcaac tcactgactt cggaggatag     360
   cgccgtgtac ttctgcgtgc gctactacta cggatcatca ggatacttcg actactgggg     420
   ccaaggtacc actctcaccg tgtcgtcggg aggaggcggc tccggcggtg gaggatccgg     480
   aggcggaggc tcagacgtgc agatgattca gactcccgac tcgctggcgg tgtccctcgg     540
   tcagagggcc accatttcgt gccgggcttc ggagtcagtg gacaattacg gcatcagctt     600
   tatgcactgg tatcagcaaa agccaggcca gtccccaaag ttgctgatct accgcgcatc     660
   gaatctggag tccggcatcc cagctcggtt cagcgggagc ggatcgagaa ctgactttac     720
   gctgaccatc aacccggtcg aaaccgatga cgtcgcaact tattactgcc agcagaacaa     780
   caaggaccct ccgaccttcg gtggagggac taagctggaa atcaaacgcg cggcggccgc     840
   aaccacgacg ccagcgccgc gaccaccaac accggcgccc accatcgcgt cgcagcccct     900
   gtccctgcgc ccagaggcgt gccggccagc ggcggggggc gcagtgcaca cgagggggct     960
   ggacttcgcc tgtgatatct acatctgggc gcccttggcc gggacttgtg gggtccttct    1020
   cctgtcactg gttatcaccc tttactgcaa acggggcaga aagaaactcc tgtatatatt    1080
   caaacaacca tttatgagac cagtacaaac tactcaagag gaagatggct gtagctgccg    1140
   atttccagaa gaagaagaag gaggatgtga actgagagtg aagttcagca ggagcgcaga    1200
   cgcccccgcg tacaagcagg gccagaacca gctctataac gagctcaatc taggacgaag    1260
   agaggagtac gatgttttgg acaagagacg tggccgggac cctgagatgg ggggaaagcc    1320
   gagaaggaag aaccctcagg aaggcctgta caatgaactg cagaaagata agatggcgga    1380
   ggcctacagt gagattggga tgaaaggcga gcgccggagg ggcaaggggc acgatggcct    1440
   ttaccagggt ctcagtacag ccaccaagga cacctacgac gcccttcaca tgcaggccct    1500
   gccccctcgc taa                                                       1513
<210> 101
   <211> 2401
   <212> DNA
   <213> Artificial Sequence
<220>
   <223> chimeric antigen receptor sequence
<400> 101
   ccctcgagcc gccaccatgg ttcttctcgt gacaagcctt cttctctgcg aattgcccca      60
   cccagccttt ttgcttatcc ccgacaccca agtccagctg cagcaatcag gggccgagtt     120
   ggtcaagcct ggggcatcgg tcaaaatctc atgtaaagcc tcgggatatg cgttctcgtc     180
   atactggatg aattgggtca agcagcggcc aggaaaggga ctggaatgga tcgggcaaat     240
   ctacccaggg gatggagata caacatataa cgggaagttt aaagggaaag caactctcac     300
   tgcggacaag tcatcatcga cggtatacat gcagcttaac tcattgacaa gcgaggactc     360
   ggcggtctat ttctgcgtac ggtattacta cggatcgtcg gggtacttcg attattgggg      420
   tcagggaacc acgctgacag tgtccagcgg aggtggcggg tccggaggcg gaggatccgg     480
   tggcggtgga agcgatgtgc agatgatcca gacgccggac tcactcgcgg tgtcactcgg     540
   gcagcgggcg acgatttcat gcagagcctc cgagtcggtg gacaattacg gtatctcctt     600
   catgcattgg tatcagcaga aacccgggca gtcgcccaag ctgttgatct acagagcgtc     660
   caaccttgag tcggggattc ccgctaggtt ctccgggtca ggatcccgca cggacttcac     720
   cttgacgatt aacccggtgg aaactgatga cgtcgccact tactactgtc agcagaacaa     780
   taaggaccct cccacatttg gcggaggtac gaagcttgaa atcaagaggg cggagccgaa     840
   gagctgcgat aaaacgcaca catgccctcc atgccctgca ccggagctct tgggcggacc     900
   ttccgtgttt ctgttcccac cgaaacccaa agacaccctg atgatttcgc gcacgccgga     960
   ggtaacttgt gtggtggtgg acgtaagcca tgaggacccg gaagtaaagt tcaactggta    1020
   tgtcgatggc gtggaggtcc acaatgcgaa aaccaagccg agagaggaac agtataactc    1080
   cacgtaccga gtcgtaagcg tgcttacagt gcttcatcaa gattggttga atggtaaaga    1140
   atacaaatgc aaggtgtcga acaaagctct gcccgcacca attgagaaaa ctattagcaa    1200
   ggcgaagggg cagcccaggg aaccccaagt gtatactttg ccgccctcgc gcgatgaact    1260
   cactaagaat caagtctcgc tgacgtgtct cgtcaagggg ttttacccga gcgacatcgc 1320
   ggtggagtgg gagtcgaacg gtcaaccgga gaacaattac aaaaccacac ctcccgtgct 1380
   cgattcggac ggatcgtttt tcctctattc caaattgacc gtcgataagt cgcgatggca 1440
   gcagggtaat gtattttcgt gttcggtaat gcacgaagcc ctccacaacc attatacgca 1500
   gaagtcgctg tccctgtcgc ccggaaagaa agacccgaag gcggccgcat tcgtgccggt 1560
   cttcctgcca gcgaagccca ccacgacgcc agcgccgcga ccaccaacac cggcgcccac 1620
   catcgcgtcg cagcccctgt ccctgcgccc agaggcgtgc cggccagcgg cggggggcgc 1680
   agtgcacacg agggggctgg acttcgcctg tgatatctac atctgggcgc ccttggccgg 1740
   gacttgtggg gtccttctcc tgtcactggt tatcaccctt tactgcaacc acaggaacag 1800
   gagtaagagg agcaggctcc tgcacagtga ctacatgaac atgactcccc gccgccccgg 1860
   gcccacccgc aagcattacc agccctatgc cccaccacgc gacttcgcag cctatcgctc 1920
   ccgtttctct gttgttaaac ggggcagaaa gaagctcctg tatatattca aacaaccatt 1980
   tatgagacca gtacaaacta ctcaagagga agatggctgt agctgccgat ttccagaaga 2040
   agaagaagga ggatgtgaac tgagagtgaa gttcagcagg agcgcagacg cccccgcgta 2100
   ccagcagggc cagaaccagc tctataacga gctcaatcta ggacgaagag aggagtacga 2160
   tgttttggac aagagacgtg gccgggaccc tgagatgggg ggaaagccga gaaggaagaa 2220
   ccctcaggaa ggcctgtaca atgaactgca gaaagataag atggcggagg cctacagtga 2280
   gattgggatg aaaggcgagc gccggagggg caaggggcac gatggccttt accagggtct 2340
   cagtacagcc accaaggaca cctacgacgc ccttcacatg caggccctgc cccctcgcta 2400
   a 2401
<210> 102
   <211> 1693
   <212> DNA
   <213> Artificial Sequence
<220>
   <223> chimeric antigen receptor sequence
<400> 102
   ccctcgagcc gccaccatgg ttctgcttgt gacctccctg cttctctgcg aactccctca 60
   tccggcattc ctgctcatcc ccgacaccca agtccaactc cagcagagcg gagccgagct 120
   ggtgaagccg ggagcgagcg tcaaaatcag ctgtaaagcc tccggctacg ccttcagctc 180
   atactggatg aactgggtga agcaaagacc gggaaagggg ttggaatgga tcggacaaat 240
   ctacccggga gatggagata ctacctacaa tgggaagttt aaaggaaagg ccactctgac 300
   cgctgataag tcctcgtcca cggtctacat gcagctcaac tcactgactt cggaggatag 360
   cgccgtgtac ttctgcgtgc gctactacta cggatcatca ggatacttcg actactgggg 420
   ccaaggtacc actctcaccg tgtcgtcggg aggaggcggc tccggcggtg gaggatccgg 480
   aggcggaggc tcagacgtgc agatgattca gactcccgac tcgctggcgg tgtccctcgg 540
   tcagagggcc accatttcgt gccgggcttc ggagtcagtg gacaattacg gcatcagctt 600
   tatgcactgg tatcagcaaa agccaggcca gtccccaaag ttgctgatct accgcgcatc 660
   gaatctggag tccggcatcc cagctcggtt cagcgggagc ggatcgagaa ctgactttac 720
   gctgaccatc aacccggtcg aaaccgatga cgtcgcaact tattactgcc agcagaacaa 780
   caaggaccct ccgaccttcg gtggagggac taagctggaa atcaaacgcg cggcggccgc 840
   attcgtgccg gtcttcctgc cagcgaagcc caccacgacg ccagcgccgc gaccaccaac 900
   accggcgccc accatcgcgt cgcagcccct gtccctgcgc ccagaggcgt gccggccagc 960
   ggcggggggc gcagtgcaca cgagggggct ggacttcgcc tgtgatatct acatctgggc 1020
   gcccttggcc gggacttgtg gggtccttct cctgtcactg gttatcaccc tttactgcaa 1080
   ccacaggaac aggagtaaga ggagcaggct cctgcacagt gactacatga acatgactcc 1140
   ccgccgcccc gggcccaccc gcaagcatta ccagccctat gccccaccac gcgacttcgc 1200
   agcctatcgc tcccgtttct ctgttgttaa acggggcaga aagaagctcc tgtatatatt 1260
   caaacaacca tttatgagac cagtacaaac tactcaagag gaagatggct gtagctgccg 1320
   atttccagaa gaagaagaag gaggatgtga actgagagtg aagttcagca ggagcgcaga 1380
   cgcccccgcg taccagcagg gccagaacca gctctataac gagctcaatc taggacgaag 1440
   agaggagtac gatgttttgg acaagagacg tggccgggac cctgagatgg ggggaaagcc 1500
   gagaaggaag aaccctcagg aaggcctgta caatgaactg cagaaagata agatggcgga 1560
   ggcctacagt gagattggga tgaaaggcga gcgccggagg ggcaaggggc acgatggcct 1620
   ttaccagggt ctcagtacag ccaccaagga cacctacgac gcccttcaca tgcaggccct 1680
   gccccctcgc taa 1693
<210> 103
   <211> 2200
   <212> DNA
   <213> Artificial Sequence
<220>
   <223> chimeric antigen receptor sequence
<400> 103
   ccctcgagcc gccaccatgg ttctgctcgt gacaagcctg ctgctgtgcg agctgcccca      60
   ccctgccttt ctgctgatcc ccgacaccga cgtgcagctg caggaatctg gacccgtgct     120
   cgtgaaaacc ggcgccagcg tgaagatgag ctgtaccgcc agcggctaca ccttcaccga     180
   ccacttcatg gactgggtca agcagagcca cggcaagagc ctggaatgga tcggcagcct     240
   gaacccctac agcggcggca ccagctacaa ccagaagttc aagggcaagg ccaccctgac       300
   cgtggacaag agcagcagca ccgcctacat ggaactgaac agcctgacca gcgtggacag       360
   cgccgtgtac tactgcgcca gacacaactg gggcgcctac ttcgactatt ggggccaggg        420
   cacaaccctg acagtgtcta gcggaggcgg aggatctggc ggcggaggaa gtggcggagg       480
   gggatctgat atcgtgatga cccaggccgc tcccagcgtg ccagtgacac ctggcgagag       540
   cgtgtccatc agctgcagaa gcagcaagtc cctgctgcac agcaacggca atacctacct       600
   gtactggttc ctgcagaggc ctggccagag cccccagcgg ctgatctact acatgagcaa       660
   cctggccagc ggcgtgcccg acagattttc tggcagaggc agcggcaccg acttcaccct       720
   gagaatcagc cgggtggaag ccgaggacgt gggcgtgtac tattgcatgc agggcctgga       780
   agatccttac accttcggcg gaggcaccaa gctggaaatc aaagagccca agagctgcga       840
   caagacccac acctgtcccc cttgtcctgc ccctgaactg ctgggcggac ctagcgtgtt       900
   cctgttcccc ccaaagccca aggataccct gatgatcagc aggacccccg aagtgacctg       960
   cgtggtggtg gatgtgtccc acgaggaccc tgaagtgaag ttcaattggt acgtggacgg      1020
   cgtggaagtg cacaacgcca agaccaagcc cagagaggaa cagtacaaca gcacctaccg      1080
   ggtggtgtcc gtgctgaccg tgctgcacca ggactggctg aacggcaaag agtacaagtg      1140
   caaggtgtcc aacaaggccc tgcctgcccc catcgagaaa accatcagca aggccaaggg      1200
   ccagccccgc gaaccccagg tgtacacact gccccctagc agggacgagc tgaccaagaa       1260
   ccaggtgtcc ctgacctgtc tcgtgaaggg cttctacccc tccgatatcg ccgtggaatg      1320
   ggagagcaac ggccagcccg agaacaacta caagaccacc ccccctgtgc tggactccga      1380
   cggctcattc ttcctgtaca gcaagctgac agtggataag tcccggtggc agcagggcaa      1440
   cgtgttcagc tgctccgtga tgcacgaagc cctgcacaac cactacaccc agaaaagcct      1500
   gtccctgagc cctggcaaga aggaccccaa agcggccgca attgaagtta tgtatcctcc      1560
   tccttaccta gacaatgaga agagcaatgg aaccattatc catgtgaaag ggaaacacct      1620
   ttgtccaagt cccctatttc ccggaccttc taagcccttt tgggtgctgg tggtggttgg      1680
   gggagtcctg gcttgctata gcttgctagt aacagtggcc tttattattt tctgggtgag      1740
   gagtaagagg agcaggctcc tgcacagtga ctacatgaac atgactcccc gccgccccgg      1800
   gcccacccgc aagcattacc agccctatgc cccaccacgc gacttcgcag cctatcgctc      1860
   cagagtgaag ttcagcagga gcgcagacgc ccccgcgtac cagcagggcc agaaccagct      1920
   ctataacgag ctcaatctag gacgaagaga ggagtacgat gttttggaca agagacgtgg      1980
   ccgggaccct gagatggggg gaaagccgag aaggaagaac cctcaggaag gcctgtacaa      2040
   tgaactgcag aaagataaga tggcggaggc ctacagtgag attgggatga aaggcgagcg      2100
   ccggaggggc aaggggcacg atggccttta ccagggtctc agtacagcca ccaaggacac      2160
   ctacgacgcc cttcacatgc aggccctgcc ccctcgctaa                            2200
<210> 104
   <211> 1492
   <212> DNA
   <213> Artificial Sequence
<220>
   <223> chimeric antigen receptor sequence
<400> 104
   ccctcgagcc gccaccatgg ttctgctcgt gacaagcctg ctgctgtgcg agctgcccca      60
   ccctgccttt ctgctgatcc ccgacaccga cgtgcagctg caggaatctg gacccgtgct     120
   cgtgaaaacc ggcgccagcg tgaagatgag ctgtaccgcc agcggctaca ccttcaccga     180
   ccacttcatg gactgggtca agcagagcca cggcaagagc ctggaatgga tcggcagcct     240
   gaacccctac agcggcggca ccagctacaa ccagaagttc aagggcaagg ccaccctgac     300
   cgtggacaag agcagcagca ccgcctacat ggaactgaac agcctgacca gcgtggacag     360
   cgccgtgtac tactgcgcca gacacaactg gggcgcctac ttcgactatt ggggccaggg     420
   cacaaccctg acagtgtcta gcggaggcgg aggatctggc ggcggaggaa gtggcggagg     480
   gggatctgat atcgtgatga cccaggccgc tcccagcgtg ccagtgacac ctggcgagag     540
   cgtgtccatc agctgcagaa gcagcaagtc cctgctgcac agcaacggca atacctacct     600
   gtactggttc ctgcagaggc ctggccagag cccccagcgg ctgatctact acatgagcaa     660
   cctggccagc ggcgtgcccg acagattttc tggcagaggc agcggcaccg acttcaccct     720
   gagaatcagc cgggtggaag ccgaggacgt gggcgtgtac tattgcatgc agggcctgga     780
   agatccttac accttcggcg gaggcaccaa gctggaaatc aaagcggccg caattgaagt     840
   tatgtatcct cctccttacc tagacaatga gaagagcaat ggaaccatta tccatgtgaa     900
   agggaaacac ctttgtccaa gtcccctatt tcccggacct tctaagccct tttgggtgct     960
   ggtggtggtt gggggagtcc tggcttgcta tagcttgcta gtaacagtgg cctttattat    1020
   tttctgggtg aggagtaaga ggagcaggct cctgcacagt gactacatga acatgactcc    1080
   ccgccgcccc gggcccaccc gcaagcatta ccagccctat gccccaccac gcgacttcgc    1140
   agcctatcgc tccagagtga agttcagcag gagcgcagac gcccccgcgt accagcaggg    1200
   ccagaaccag ctctataacg agctcaatct aggacgaaga gaggagtacg atgttttgga    1260
   caagagacgt ggccgggacc ctgagatggg gggaaagccg agaaggaaga accctcagga    1320
   aggcctgtac aatgaactgc agaaagataa gatggcggag gcctacagtg agattgggat    1380
   gaaaggcgag cgccggaggg gcaaggggca cgatggcctt taccagggtc tcagtacagc    1440
   caccaaggac acctacgacg cccttcacat gcaggccctg ccccctcgct aa            1492
<210> 105
   <211> 2212
   <212> DNA
   <213> Artificial Sequence
<220>
   <223> chimeric antigen receptor sequence
<400> 105
   ccctcgagcc gccaccatgg ttctgctcgt gacaagcctg ctgctgtgcg agctgcccca      60
   ccctgccttt ctgctgatcc ccgacaccga cgtgcagctg caggaatctg gacccgtgct     120
   cgtgaaaacc ggcgccagcg tgaagatgag ctgtaccgcc agcggctaca ccttcaccga     180
   ccacttcatg gactgggtca agcagagcca cggcaagagc ctggaatgga tcggcagcct     240
   gaacccctac agcggcggca ccagctacaa ccagaagttc aagggcaagg ccaccctgac     300
   cgtggacaag agcagcagca ccgcctacat ggaactgaac agcctgacca gcgtggacag     360
   cgccgtgtac tactgcgcca gacacaactg gggcgcctac ttcgactatt ggggccaggg     420
   cacaaccctg acagtgtcta gcggaggcgg aggatctggc ggcggaggaa gtggcggagg     480
   gggatctgat atcgtgatga cccaggccgc tcccagcgtg ccagtgacac ctggcgagag     540
   cgtgtccatc agctgcagaa gcagcaagtc cctgctgcac agcaacggca atacctacct     600
   gtactggttc ctgcagaggc ctggccagag cccccagcgg ctgatctact acatgagcaa     660
   cctggccagc ggcgtgcccg acagattttc tggcagaggc agcggcaccg acttcaccct     720
   gagaatcagc cgggtggaag ccgaggacgt gggcgtgtac tattgcatgc agggcctgga     780
   agatccttac accttcggcg gaggcaccaa gctggaaatc aaagagccca agagctgcga     840
   caagacccac acctgtcccc cttgtcctgc ccctgaactg ctgggcggac ctagcgtgtt     900
   cctgttcccc ccaaagccca aggataccct gatgatcagc aggacccccg aagtgacctg     960
   cgtggtggtg gatgtgtccc acgaggaccc tgaagtgaag ttcaattggt acgtggacgg    1020
   cgtggaagtg cacaacgcca agaccaagcc cagagaggaa cagtacaaca gcacctaccg    1080
   ggtggtgtcc gtgctgaccg tgctgcacca ggactggctg aacggcaaag agtacaagtg    1140
   caaggtgtcc aacaaggccc tgcctgcccc catcgagaaa accatcagca aggccaaggg    1200
   ccagccccgc gaaccccagg tgtacacact gccccctagc agggacgagc tgaccaagaa    1260
   ccaggtgtcc ctgacctgtc tcgtgaaggg cttctacccc tccgatatcg ccgtggaatg    1320
   ggagagcaac ggccagcccg agaacaacta caagaccacc ccccctgtgc tggactccga    1380
   cggctcattc ttcctgtaca gcaagctgac agtggataag tcccggtggc agcagggcaa    1440
   cgtgttcagc tgctccgtga tgcacgaagc cctgcacaac cactacaccc agaaaagcct    1500
   gtccctgagc cctggcaaga aggaccccaa agcggccgca accacgacgc cagcgccgcg    1560
   accaccaaca ccggcgccca ccatcgcgtc gcagcccctg tccctgcgcc cagaggcgtg 1620
   ccggccagcg gcggggggcg cagtgcacac gagggggctg gacttcgcct gtgatatcta 1680
   catctgggcg cccttggccg ggacttgtgg ggtccttctc ctgtcactgg ttatcaccct 1740
   ttactgcaaa cggggcagaa agaaactcct gtatatattc aaacaaccat ttatgagacc 1800
   agtacaaact actcaagagg aagatggctg tagctgccga tttccagaag aagaagaagg 1860
   aggatgtgaa ctgagagtga agttcagcag gagcgcagac gcccccgcgt acaagcaggg 1920
   ccagaaccag ctctataacg agctcaatct aggacgaaga gaggagtacg atgttttgga 1980
   caagagacgt ggccgggacc ctgagatggg gggaaagccg agaaggaaga accctcagga 2040
   aggcctgtac aatgaactgc agaaagataa gatggcggag gcctacagtg agattgggat 2100
   gaaaggcgag cgccggaggg gcaaggggca cgatggcctt taccagggtc tcagtacagc 2160
   caccaaggac acctacgacg cccttcacat gcaggccctg ccccctcgct aa 2212
<210> 106
   <211> 1504
   <212> DNA
   <213> Artificial Sequence
<220>
   <223> chimeric antigen receptor sequence
<400> 106
   ccctcgagcc gccaccatgg ttctgctcgt gacaagcctg ctgctgtgcg agctgcccca      60
   ccctgccttt ctgctgatcc ccgacaccga cgtgcagctg caggaatctg gacccgtgct     120
   cgtgaaaacc ggcgccagcg tgaagatgag ctgtaccgcc agcggctaca ccttcaccga     180
   ccacttcatg gactgggtca agcagagcca cggcaagagc ctggaatgga tcggcagcct     240
   gaacccctac agcggcggca ccagctacaa ccagaagttc aagggcaagg ccaccctgac     300
   cgtggacaag agcagcagca ccgcctacat ggaactgaac agcctgacca gcgtggacag     360
   cgccgtgtac tactgcgcca gacacaactg gggcgcctac ttcgactatt ggggccaggg     420
   cacaaccctg acagtgtcta gcggaggcgg aggatctggc ggcggaggaa gtggcggagg     480
   gggatctgat atcgtgatga cccaggccgc tcccagcgtg ccagtgacac ctggcgagag     540
   cgtgtccatc agctgcagaa gcagcaagtc cctgctgcac agcaacggca atacctacct     600
   gtactggttc ctgcagaggc ctggccagag cccccagcgg ctgatctact acatgagcaa     660
   cctggccagc ggcgtgcccg acagattttc tggcagaggc agcggcaccg acttcaccct     720
   gagaatcagc cgggtggaag ccgaggacgt gggcgtgtac tattgcatgc agggcctgga     780
   agatccttac accttcggcg gaggcaccaa gctggaaatc aaagcggccg caaccacgac     840
   gccagcgccg cgaccaccaa caccggcgcc caccatcgcg tcgcagcccc tgtccctgcg     900
   cccagaggcg tgccggccag cggcgggggg cgcagtgcac acgagggggc tggacttcgc     960
   ctgtgatatc tacatctggg cgcccttggc cgggacttgt ggggtccttc tcctgtcact    1020
   ggttatcacc ctttactgca aacggggcag aaagaaactc ctgtatatat tcaaacaacc    1080
   atttatgaga ccagtacaaa ctactcaaga ggaagatggc tgtagctgcc gatttccaga    1140
   agaagaagaa ggaggatgtg aactgagagt gaagttcagc aggagcgcag acgcccccgc    1200
   gtacaagcag ggccagaacc agctctataa cgagctcaat ctaggacgaa gagaggagta    1260
   cgatgttttg gacaagagac gtggccggga ccctgagatg gggggaaagc cgagaaggaa    1320
   gaaccctcag gaaggcctgt acaatgaact gcagaaagat aagatggcgg aggcctacag    1380
   tgagattggg atgaaaggcg agcgccggag gggcaagggg cacgatggcc tttaccaggg    1440
   tctcagtaca gccaccaagg acacctacga cgcccttcac atgcaggccc tgccccctcg    1500
   ctaa                                                                 1504
<210> 107
   <211> 2392
   <212> DNA
   <213> Artificial Sequence
<220>
   <223> chimeric antigen receptor sequence
<400> 107
   ccctcgagcc gccaccatgg ttctgctcgt gacaagcctg ctgctgtgcg agctgcccca     60
   ccctgccttt ctgctgatcc ccgacaccga cgtgcagctg caggaatctg gacccgtgct    120
   cgtgaaaacc ggcgccagcg tgaagatgag ctgtaccgcc agcggctaca ccttcaccga    180
   ccacttcatg gactgggtca agcagagcca cggcaagagc ctggaatgga tcggcagcct    240
   gaacccctac agcggcggca ccagctacaa ccagaagttc aagggcaagg ccaccctgac    300
   cgtggacaag agcagcagca ccgcctacat ggaactgaac agcctgacca gcgtggacag    360
   cgccgtgtac tactgcgcca gacacaactg gggcgcctac ttcgactatt ggggccaggg    420
   cacaaccctg acagtgtcta gcggaggcgg aggatctggc ggcggaggaa gtggcggagg    480
   gggatctgat atcgtgatga cccaggccgc tcccagcgtg ccagtgacac ctggcgagag    540
   cgtgtccatc agctgcagaa gcagcaagtc cctgctgcac agcaacggca atacctacct    600
   gtactggttc ctgcagaggc ctggccagag cccccagcgg ctgatctact acatgagcaa    660
   cctggccagc ggcgtgcccg acagattttc tggcagaggc agcggcaccg acttcaccct    720
   gagaatcagc cgggtggaag ccgaggacgt gggcgtgtac tattgcatgc agggcctgga    780
   agatccttac accttcggcg gaggcaccaa gctggaaatc aaagagccca agagctgcga    840
   caagacccac acctgtcccc cttgtcctgc ccctgaactg ctgggcggac ctagcgtgtt    900
   cctgttcccc ccaaagccca aggataccct gatgatcagc aggacccccg aagtgacctg    960
   cgtggtggtg gatgtgtccc acgaggaccc tgaagtgaag ttcaattggt acgtggacgg     1020
   cgtggaagtg cacaacgcca agaccaagcc cagagaggaa cagtacaaca gcacctaccg     1080
   ggtggtgtcc gtgctgaccg tgctgcacca ggactggctg aacggcaaag agtacaagtg     1140
   caaggtgtcc aacaaggccc tgcctgcccc catcgagaaa accatcagca aggccaaggg     1200
   ccagccccgc gaaccccagg tgtacacact gccccctagc agggacgagc tgaccaagaa     1260
   ccaggtgtcc ctgacctgtc tcgtgaaggg cttctacccc tccgatatcg ccgtggaatg     1320
   ggagagcaac ggccagcccg agaacaacta caagaccacc ccccctgtgc tggactccga     1380
   cggctcattc ttcctgtaca gcaagctgac agtggataag tcccggtggc agcagggcaa     1440
   cgtgttcagc tgctccgtga tgcacgaagc cctgcacaac cactacaccc agaaaagcct     1500
   gtccctgagc cctggcaaga aggaccccaa agcggccgca ttcgtgccgg tcttcctgcc     1560
   agcgaagccc accacgacgc cagcgccgcg accaccaaca ccggcgccca ccatcgcgtc     1620
   gcagcccctg tccctgcgcc cagaggcgtg ccggccagcg gcggggggcg cagtgcacac     1680
   gagggggctg gacttcgcct gtgatatcta catctgggcg cccttggccg ggacttgtgg     1740
   ggtccttctc ctgtcactgg ttatcaccct ttactgcaac cacaggaaca ggagtaagag     1800
   gagcaggctc ctgcacagtg actacatgaa catgactccc cgccgccccg ggcccacccg     1860
   caagcattac cagccctatg ccccaccacg cgacttcgca gcctatcgct cccgtttctc     1920
   tgttgttaaa cggggcagaa agaagctcct gtatatattc aaacaaccat ttatgagacc     1980
   agtacaaact actcaagagg aagatggctg tagctgccga tttccagaag aagaagaagg     2040
   aggatgtgaa ctgagagtga agttcagcag gagcgcagac gcccccgcgt accagcaggg     2100
   ccagaaccag ctctataacg agctcaatct aggacgaaga gaggagtacg atgttttgga     2160
   caagagacgt ggccgggacc ctgagatggg gggaaagccg agaaggaaga accctcagga     2220
   aggcctgtac aatgaactgc agaaagataa gatggcggag gcctacagtg agattgggat     2280
   gaaaggcgag cgccggaggg gcaaggggca cgatggcctt taccagggtc tcagtacagc     2340
   caccaaggac acctacgacg cccttcacat gcaggccctg ccccctcgct aa             2392
<210> 108
   <211> 1684
   <212> DNA
   <213> Artificial Sequence
<220>
   <223> chimeric antigen receptor sequence
<400> 108
   ccctcgagcc gccaccatgg ttctgctcgt gacaagcctg ctgctgtgcg agctgcccca      60
   ccctgccttt ctgctgatcc ccgacaccga cgtgcagctg caggaatctg gacccgtgct     120
   cgtgaaaacc ggcgccagcg tgaagatgag ctgtaccgcc agcggctaca ccttcaccga     180
   ccacttcatg gactgggtca agcagagcca cggcaagagc ctggaatgga tcggcagcct     240
   gaacccctac agcggcggca ccagctacaa ccagaagttc aagggcaagg ccaccctgac     300
   cgtggacaag agcagcagca ccgcctacat ggaactgaac agcctgacca gcgtggacag     360
   cgccgtgtac tactgcgcca gacacaactg gggcgcctac ttcgactatt ggggccaggg     420
   cacaaccctg acagtgtcta gcggaggcgg aggatctggc ggcggaggaa gtggcggagg     480
   gggatctgat atcgtgatga cccaggccgc tcccagcgtg ccagtgacac ctggcgagag     540
   cgtgtccatc agctgcagaa gcagcaagtc cctgctgcac agcaacggca atacctacct     600
   gtactggttc ctgcagaggc ctggccagag cccccagcgg ctgatctact acatgagcaa     660
   cctggccagc ggcgtgcccg acagattttc tggcagaggc agcggcaccg acttcaccct     720
   gagaatcagc cgggtggaag ccgaggacgt gggcgtgtac tattgcatgc agggcctgga     780
   agatccttac accttcggcg gaggcaccaa gctggaaatc aaagcggccg cattcgtgcc     840
   ggtcttcctg ccagcgaagc ccaccacgac gccagcgccg cgaccaccaa caccggcgcc     900
   caccatcgcg tcgcagcccc tgtccctgcg cccagaggcg tgccggccag cggcgggggg     960
   cgcagtgcac acgagggggc tggacttcgc ctgtgatatc tacatctggg cgcccttggc    1020
   cgggacttgt ggggtccttc tcctgtcact ggttatcacc ctttactgca accacaggaa    1080
   caggagtaag aggagcaggc tcctgcacag tgactacatg aacatgactc cccgccgccc    1140
   cgggcccacc cgcaagcatt accagcccta tgccccacca cgcgacttcg cagcctatcg    1200
   ctcccgtttc tctgttgtta aacggggcag aaagaagctc ctgtatatat tcaaacaacc    1260
   atttatgaga ccagtacaaa ctactcaaga ggaagatggc tgtagctgcc gatttccaga    1320
   agaagaagaa ggaggatgtg aactgagagt gaagttcagc aggagcgcag acgcccccgc    1380
   gtaccagcag ggccagaacc agctctataa cgagctcaat ctaggacgaa gagaggagta    1440
   cgatgttttg gacaagagac gtggccggga ccctgagatg gggggaaagc cgagaaggaa    1500
   gaaccctcag gaaggcctgt acaatgaact gcagaaagat aagatggcgg aggcctacag    1560
   tgagattggg atgaaaggcg agcgccggag gggcaagggg cacgatggcc tttaccaggg    1620
   tctcagtaca gccaccaagg acacctacga cgcccttcac atgcaggccc tgccccctcg    1680
   ctaa                                                                 1684
<210> 109
   <211> 2197
   <212> DNA
   <213> Artificial Sequence
<220>
   <223> chimeric antigen receptor sequence
<400> 109
   ccctcgagcc gccaccatgg ttcttctcgt gactagcctc ctgctgtgcg aacttccaca      60
   tccagctttc ctgcttatcc cagacaccgc cctccaactc cagcaaagcg gtgctgaact     120
   tgtgaggcct ggcgcttctg tcaccctgag ctgcaaagcc agcggttata ccttcaccga     180
   ttacgaaatg cattgggtga agcagacccc agtgcatggt ctggagtgga ttggagctat     240
   cgaccccgaa actggaggga ctgcctacaa ccagaagttt gagggaaagg ccatccttac     300
   tgccgacaag tcatcatcta ccgcatacat ggagctgagg tcactgacct ccgaggactc     360
   ccccgtgtac tattgcgcca gaaggaggta ctacggttca tcttccttcg attattgggg     420
   acagggaact actctgaccg tcagctctgg cggtggtgga tcaggtggag gcggaagcgg     480
   agggggaggt tcagacgtcc agatgattca gactccttcc agcctttctg cctcactcgg     540
   ggaccgcgtg accatctcat gtagagcctc ccaagacatc ggcaattacc ttaattggta     600
   tcaacaaaaa cctgatggca ctgtgaagct cctgatctac tacacctctc ggcttcactc     660
   aggggtcccc agccggttct ctggctctgg ttcagggacc gaatactctc tcaccattag     720
   caatctcgaa caagaggaca tcgcaactta cttctgccag cagggaagcg cactgccgcc     780
   caccttcgga ggaggaacca agctggaaat caatcgggcc gagccgaaga gctgcgacaa     840
   gactcatact tgtcctcctt gtccagcccc ggaactgctc ggcggaccct ccgtgttcct 900
   gttcccgccc aagcccaagg acactcttat gatcagccgc acccccgaag tgacttgcgt 960
   cgtcgtggac gtgagccacg aggaccctga agtgaagttc aactggtatg tggacggagt    1020
   cgaagtgcat aacgccaaaa ccaaaccccg cgaggagcaa tacaattcaa cctatcgcgt    1080
   ggtgagcgtg ctcaccgtgc tgcaccagga ctggcttaac ggtaaagagt acaagtgtaa    1140
   agtgagcaac aaagctctgc ccgctcctat tgagaaaact atcagcaagg ctaagggaca 1200
   gcctcgggaa cctcaagtgt atacccttcc ccctagccgg gatgaactga ccaagaatca 1260
   agtcagcctt acttgtctgg tcaaggggtt ctacccatcc gacattgcag tggaatggga    1320
   gtcaaacggg cagcccgaga acaattacaa gaccaccccg cctgtgctgg acagcgacgg    1380
   atcattcttt ctttactcaa agctgactgt ggataagtca agatggcagc agggtaacgt    1440
   gttttcttgc agcgtcatgc acgaggccct gcacaaccat tatacccaga agagcctgtc    1500
   actgtctccg ggaaagaagg accctaaggc ggccgcaatt gaagttatgt atcctcctcc    1560
   ttacctagac aatgagaaga gcaatggaac cattatccat gtgaaaggga aacacctttg    1620
   tccaagtccc ctatttcccg gaccttctaa gcccttttgg gtgctggtgg tggttggggg    1680
   agtcctggct tgctatagct tgctagtaac agtggccttt attattttct gggtgaggag    1740
   taagaggagc aggctcctgc acagtgacta catgaacatg actccccgcc gccccgggcc    1800
   cacccgcaag cattaccagc cctatgcccc accacgcgac ttcgcagcct atcgctccag    1860
   agtgaagttc agcaggagcg cagacgcccc cgcgtaccag cagggccaga accagctcta    1920
   taacgagctc aatctaggac gaagagagga gtacgatgtt ttggacaaga gacgtggccg      1980
   ggaccctgag atggggggaa agccgagaag gaagaaccct caggaaggcc tgtacaatga      2040
   actgcagaaa gataagatgg cggaggccta cagtgagatt gggatgaaag gcgagcgccg      2100
   gaggggcaag gggcacgatg gcctttacca gggtctcagt acagccacca aggacaccta      2160
   cgacgccctt cacatgcagg ccctgccccc tcgctaa                               2197
<210> 110
   <211> 1489
   <212> DNA
   <213> Artificial Sequence
<220>
   <223> chimeric antigen receptor sequence
<400> 110
   ccctcgagcc gccaccatgg ttctccttgt gacctcactc ctgctgtgcg aactgccgca      60
   tccagccttc ctgctgatcc ccgacactgc gctccaactg cagcaatccg gagctgaatt     120
   ggtgcggcca ggtgcgtccg tgacgttgag ctgcaaggca tccggataca cctttaccga     180
   ctacgagatg cactgggtca aacagactcc tgtccacggc ctcgaatgga ttggagcaat     240
   cgacccagaa actggaggga ccgcgtacaa ccagaagttt gaaggaaagg ccattttgac     300
   tgccgacaaa tcctcctcga ccgcctacat ggaactgaga tccctgactt cggaggattc     360
   gccggtgtac tactgtgcac gccgcagata ctacgggagc tcgtcgttcg actactgggg     420
   tcagggaacc actctgactg tctcatccgg tggaggcgga tcaggcggtg gagggtcagg     480
   cggaggcggc tccgacgtgc agatgatcca gaccccgtcc tcgctctccg cttcgcttgg     540
   agatcgggtc acgatcagct gccgcgcttc acaagatatc ggaaactatc tcaactggta     600
   ccaacagaag ccggacggaa ctgtgaagct gctcatctac tacacctcgc gccttcatag     660
   cggagtgcct tcaaggttca gcggctcggg gtcgggaacc gagtacagcc tgaccatctc     720
   aaatctggag caggaagata tcgccactta tttctgccag caaggtagcg ccctccctcc     780
   gaccttcgga ggcgggacga agctggagat caatcgggcg gcggccgcaa ttgaagttat     840
   gtatcctcct ccttacctag acaatgagaa gagcaatgga accattatcc atgtgaaagg     900
   gaaacacctt tgtccaagtc ccctatttcc cggaccttct aagccctttt gggtgctggt     960
   ggtggttggg ggagtcctgg cttgctatag cttgctagta acagtggcct ttattatttt    1020
   ctgggtgagg agtaagagga gcaggctcct gcacagtgac tacatgaaca tgactccccg    1080
   ccgccccggg cccacccgca agcattacca gccctatgcc ccaccacgcg acttcgcagc    1140
   ctatcgctcc agagtgaagt tcagcaggag cgcagacgcc cccgcgtacc agcagggcca    1200
   gaaccagctc tataacgagc tcaatctagg acgaagagag gagtacgatg ttttggacaa    1260
   gagacgtggc cgggaccctg agatgggggg aaagccgaga aggaagaacc ctcaggaagg    1320
   cctgtacaat gaactgcaga aagataagat ggcggaggcc tacagtgaga ttgggatgaa    1380
   aggcgagcgc cggaggggca aggggcacga tggcctttac cagggtctca gtacagccac    1440
   caaggacacc tacgacgccc ttcacatgca ggccctgccc cctcgctaa                1489
<210> 111
   <211> 2209
   <212> DNA
   <213> Artificial Sequence
<220>
   <223> chimeric antigen receptor sequence
<400> 111
   ccctcgagcc gccaccatgg ttcttctcgt gactagcctc ctgctgtgcg aacttccaca      60
   tccagctttc ctgcttatcc cagacaccgc cctccaactc cagcaaagcg gtgctgaact     120
   tgtgaggcct ggcgcttctg tcaccctgag ctgcaaagcc agcggttata ccttcaccga     180
   ttacgaaatg cattgggtga agcagacccc agtgcatggt ctggagtgga ttggagctat     240
   cgaccccgaa actggaggga ctgcctacaa ccagaagttt gagggaaagg ccatccttac     300
   tgccgacaag tcatcatcta ccgcatacat ggagctgagg tcactgacct ccgaggactc     360
   ccccgtgtac tattgcgcca gaaggaggta ctacggttca tcttccttcg attattgggg     420
   acagggaact actctgaccg tcagctctgg cggtggtgga tcaggtggag gcggaagcgg     480
   agggggaggt tcagacgtcc agatgattca gactccttcc agcctttctg cctcactcgg     540
   ggaccgcgtg accatctcat gtagagcctc ccaagacatc ggcaattacc ttaattggta     600
   tcaacaaaaa cctgatggca ctgtgaagct cctgatctac tacacctctc ggcttcactc     660
   aggggtcccc agccggttct ctggctctgg ttcagggacc gaatactctc tcaccattag     720
   caatctcgaa caagaggaca tcgcaactta cttctgccag cagggaagcg cactgccgcc     780
   caccttcgga ggaggaacca agctggaaat caatcgggcc gagccgaaga gctgcgacaa     840
   gactcatact tgtcctcctt gtccagcccc ggaactgctc ggcggaccct ccgtgttcct     900
   gttcccgccc aagcccaagg acactcttat gatcagccgc acccccgaag tgacttgcgt     960
   cgtcgtggac gtgagccacg aggaccctga agtgaagttc aactggtatg tggacggagt    1020
   cgaagtgcat aacgccaaaa ccaaaccccg cgaggagcaa tacaattcaa cctatcgcgt    1080
   ggtgagcgtg ctcaccgtgc tgcaccagga ctggcttaac ggtaaagagt acaagtgtaa    1140
   agtgagcaac aaagctctgc ccgctcctat tgagaaaact atcagcaagg ctaagggaca    1200
   gcctcgggaa cctcaagtgt atacccttcc ccctagccgg gatgaactga ccaagaatca    1260
   agtcagcctt acttgtctgg tcaaggggtt ctacccatcc gacattgcag tggaatggga    1320
   gtcaaacggg cagcccgaga acaattacaa gaccaccccg cctgtgctgg acagcgacgg    1380
   atcattcttt ctttactcaa agctgactgt ggataagtca agatggcagc agggtaacgt      1440
   gttttcttgc agcgtcatgc acgaggccct gcacaaccat tatacccaga agagcctgtc      1500
   actgtctccg ggaaagaagg accctaaggc ggccgcaacc acgacgccag cgccgcgacc      1560
   accaacaccg gcgcccacca tcgcgtcgca gcccctgtcc ctgcgcccag aggcgtgccg      1620
   gccagcggcg gggggcgcag tgcacacgag ggggctggac ttcgcctgtg atatctacat      1680
   ctgggcgccc ttggccggga cttgtggggt ccttctcctg tcactggtta tcacccttta      1740
   ctgcaaacgg ggcagaaaga aactcctgta tatattcaaa caaccattta tgagaccagt      1800
   acaaactact caagaggaag atggctgtag ctgccgattt ccagaagaag aagaaggagg      1860
   atgtgaactg agagtgaagt tcagcaggag cgcagacgcc cccgcgtaca agcagggcca      1920
   gaaccagctc tataacgagc tcaatctagg acgaagagag gagtacgatg ttttggacaa      1980
   gagacgtggc cgggaccctg agatgggggg aaagccgaga aggaagaacc ctcaggaagg      2040
   cctgtacaat gaactgcaga aagataagat ggcggaggcc tacagtgaga ttgggatgaa      2100
   aggcgagcgc cggaggggca aggggcacga tggcctttac cagggtctca gtacagccac      2160
   caaggacacc tacgacgccc ttcacatgca ggccctgccc cctcgctaa                  2209
<210> 112
   <211> 1501
   <212> DNA
   <213> Artificial Sequence
<220>
   <223> chimeric antigen receptor sequence
<400> 112
   ccctcgagcc gccaccatgg ttctccttgt gacctcactc ctgctgtgcg aactgccgca      60
   tccagccttc ctgctgatcc ccgacactgc gctccaactg cagcaatccg gagctgaatt     120
   ggtgcggcca ggtgcgtccg tgacgttgag ctgcaaggca tccggataca cctttaccga     180
   ctacgagatg cactgggtca aacagactcc tgtccacggc ctcgaatgga ttggagcaat     240
   cgacccagaa actggaggga ccgcgtacaa ccagaagttt gaaggaaagg ccattttgac     300
   tgccgacaaa tcctcctcga ccgcctacat ggaactgaga tccctgactt cggaggattc     360
   gccggtgtac tactgtgcac gccgcagata ctacgggagc tcgtcgttcg actactgggg     420
   tcagggaacc actctgactg tctcatccgg tggaggcgga tcaggcggtg gagggtcagg     480
   cggaggcggc tccgacgtgc agatgatcca gaccccgtcc tcgctctccg cttcgcttgg     540
   agatcgggtc acgatcagct gccgcgcttc acaagatatc ggaaactatc tcaactggta     600
   ccaacagaag ccggacggaa ctgtgaagct gctcatctac tacacctcgc gccttcatag     660
   cggagtgcct tcaaggttca gcggctcggg gtcgggaacc gagtacagcc tgaccatctc     720
   aaatctggag caggaagata tcgccactta tttctgccag caaggtagcg ccctccctcc     780
   gaccttcgga ggcgggacga agctggagat caatcgggcg gcggccgcaa ccacgacgcc     840
   agcgccgcga ccaccaacac cggcgcccac catcgcgtcg cagcccctgt ccctgcgccc     900
   agaggcgtgc cggccagcgg cggggggcgc agtgcacacg agggggctgg acttcgcctg     960
   tgatatctac atctgggcgc ccttggccgg gacttgtggg gtccttctcc tgtcactggt    1020
   tatcaccctt tactgcaaac ggggcagaaa gaaactcctg tatatattca aacaaccatt    1080
   tatgagacca gtacaaacta ctcaagagga agatggctgt agctgccgat ttccagaaga    1140
   agaagaagga ggatgtgaac tgagagtgaa gttcagcagg agcgcagacg cccccgcgta    1200
   caagcagggc cagaaccagc tctataacga gctcaatcta ggacgaagag aggagtacga    1260
   tgttttggac aagagacgtg gccgggaccc tgagatgggg ggaaagccga gaaggaagaa    1320
   ccctcaggaa ggcctgtaca atgaactgca gaaagataag atggcggagg cctacagtga    1380
   gattgggatg aaaggcgagc gccggagggg caaggggcac gatggccttt accagggtct    1440
   cagtacagcc accaaggaca cctacgacgc ccttcacatg caggccctgc cccctcgcta    1500
   a                                                                    1501
<210> 113
   <211> 2389
   <212> DNA
   <213> Artificial Sequence
<220>
   <223> chimeric antigen receptor sequence
<400> 113
   ccctcgagcc gccaccatgg ttcttctcgt gactagcctc ctgctgtgcg aacttccaca      60
   tccagctttc ctgcttatcc cagacaccgc cctccaactc cagcaaagcg gtgctgaact     120
   tgtgaggcct ggcgcttctg tcaccctgag ctgcaaagcc agcggttata ccttcaccga     180
   ttacgaaatg cattgggtga agcagacccc agtgcatggt ctggagtgga ttggagctat     240
   cgaccccgaa actggaggga ctgcctacaa ccagaagttt gagggaaagg ccatccttac     300
   tgccgacaag tcatcatcta ccgcatacat ggagctgagg tcactgacct ccgaggactc     360
   ccccgtgtac tattgcgcca gaaggaggta ctacggttca tcttccttcg attattgggg     420
   acagggaact actctgaccg tcagctctgg cggtggtgga tcaggtggag gcggaagcgg     480
   agggggaggt tcagacgtcc agatgattca gactccttcc agcctttctg cctcactcgg     540
   ggaccgcgtg accatctcat gtagagcctc ccaagacatc ggcaattacc ttaattggta     600
   tcaacaaaaa cctgatggca ctgtgaagct cctgatctac tacacctctc ggcttcactc     660
   aggggtcccc agccggttct ctggctctgg ttcagggacc gaatactctc tcaccattag     720
   caatctcgaa caagaggaca tcgcaactta cttctgccag cagggaagcg cactgccgcc     780
   caccttcgga ggaggaacca agctggaaat caatcgggcc gagccgaaga gctgcgacaa 840
   gactcatact tgtcctcctt gtccagcccc ggaactgctc ggcggaccct ccgtgttcct 900
   gttcccgccc aagcccaagg acactcttat gatcagccgc acccccgaag tgacttgcgt 960
   cgtcgtggac gtgagccacg aggaccctga agtgaagttc aactggtatg tggacggagt 1020
   cgaagtgcat aacgccaaaa ccaaaccccg cgaggagcaa tacaattcaa cctatcgcgt 1080
   ggtgagcgtg ctcaccgtgc tgcaccagga ctggcttaac ggtaaagagt acaagtgtaa 1140
   agtgagcaac aaagctctgc ccgctcctat tgagaaaact atcagcaagg ctaagggaca 1200
   gcctcgggaa cctcaagtgt atacccttcc ccctagccgg gatgaactga ccaagaatca 1260
   agtcagcctt acttgtctgg tcaaggggtt ctacccatcc gacattgcag tggaatggga 1320
   gtcaaacggg cagcccgaga acaattacaa gaccaccccg cctgtgctgg acagcgacgg 1380
   atcattcttt ctttactcaa agctgactgt ggataagtca agatggcagc agggtaacgt 1440
   gttttcttgc agcgtcatgc acgaggccct gcacaaccat tatacccaga agagcctgtc 1500
   actgtctccg ggaaagaagg accctaaggc ggccgcattc gtgccggtct tcctgccagc 1560
   gaagcccacc acgacgccag cgccgcgacc accaacaccg gcgcccacca tcgcgtcgca 1620
   gcccctgtcc ctgcgcccag aggcgtgccg gccagcggcg gggggcgcag tgcacacgag 1680
   ggggctggac ttcgcctgtg atatctacat ctgggcgccc ttggccggga cttgtggggt 1740
   ccttctcctg tcactggtta tcacccttta ctgcaaccac aggaacagga gtaagaggag 1800
   caggctcctg cacagtgact acatgaacat gactccccgc cgccccgggc ccacccgcaa 1860
   gcattaccag ccctatgccc caccacgcga cttcgcagcc tatcgctccc gtttctctgt 1920
   tgttaaacgg ggcagaaaga agctcctgta tatattcaaa caaccattta tgagaccagt 1980
   acaaactact caagaggaag atggctgtag ctgccgattt ccagaagaag aagaaggagg 2040
   atgtgaactg agagtgaagt tcagcaggag cgcagacgcc cccgcgtacc agcagggcca 2100
   gaaccagctc tataacgagc tcaatctagg acgaagagag gagtacgatg ttttggacaa 2160
   gagacgtggc cgggaccctg agatgggggg aaagccgaga aggaagaacc ctcaggaagg 2220
   cctgtacaat gaactgcaga aagataagat ggcggaggcc tacagtgaga ttgggatgaa 2280
   aggcgagcgc cggaggggca aggggcacga tggcctttac cagggtctca gtacagccac 2340
   caaggacacc tacgacgccc ttcacatgca ggccctgccc cctcgctaa 2389
<210> 114
   <211> 1681
   <212> DNA
   <213> Artificial Sequence
<220>
   <223> chimeric antigen receptor sequence
<400> 114
   ccctcgagcc gccaccatgg ttctccttgt gacctcactc ctgctgtgcg aactgccgca      60
   tccagccttc ctgctgatcc ccgacactgc gctccaactg cagcaatccg gagctgaatt     120
   ggtgcggcca ggtgcgtccg tgacgttgag ctgcaaggca tccggataca cctttaccga     180
   ctacgagatg cactgggtca aacagactcc tgtccacggc ctcgaatgga ttggagcaat     240
   cgacccagaa actggaggga ccgcgtacaa ccagaagttt gaaggaaagg ccattttgac     300
   tgccgacaaa tcctcctcga ccgcctacat ggaactgaga tccctgactt cggaggattc     360
   gccggtgtac tactgtgcac gccgcagata ctacgggagc tcgtcgttcg actactgggg     420
   tcagggaacc actctgactg tctcatccgg tggaggcgga tcaggcggtg gagggtcagg     480
   cggaggcggc tccgacgtgc agatgatcca gaccccgtcc tcgctctccg cttcgcttgg     540
   agatcgggtc acgatcagct gccgcgcttc acaagatatc ggaaactatc tcaactggta     600
   ccaacagaag ccggacggaa ctgtgaagct gctcatctac tacacctcgc gccttcatag     660
   cggagtgcct tcaaggttca gcggctcggg gtcgggaacc gagtacagcc tgaccatctc     720
   aaatctggag caggaagata tcgccactta tttctgccag caaggtagcg ccctccctcc     780
   gaccttcgga ggcgggacga agctggagat caatcgggcg gcggccgcat tcgtgccggt     840
   cttcctgcca gcgaagccca ccacgacgcc agcgccgcga ccaccaacac cggcgcccac     900
   catcgcgtcg cagcccctgt ccctgcgccc agaggcgtgc cggccagcgg cggggggcgc     960
   agtgcacacg agggggctgg acttcgcctg tgatatctac atctgggcgc ccttggccgg    1020
   gacttgtggg gtccttctcc tgtcactggt tatcaccctt tactgcaacc acaggaacag    1080
   gagtaagagg agcaggctcc tgcacagtga ctacatgaac atgactcccc gccgccccgg    1140
   gcccacccgc aagcattacc agccctatgc cccaccacgc gacttcgcag cctatcgctc    1200
   ccgtttctct gttgttaaac ggggcagaaa gaagctcctg tatatattca aacaaccatt    1260
   tatgagacca gtacaaacta ctcaagagga agatggctgt agctgccgat ttccagaaga    1320
   agaagaagga ggatgtgaac tgagagtgaa gttcagcagg agcgcagacg cccccgcgta    1380
   ccagcagggc cagaaccagc tctataacga gctcaatcta ggacgaagag aggagtacga    1440
   tgttttggac aagagacgtg gccgggaccc tgagatgggg ggaaagccga gaaggaagaa    1500
   ccctcaggaa ggcctgtaca atgaactgca gaaagataag atggcggagg cctacagtga    1560
   gattgggatg aaaggcgagc gccggagggg caaggggcac gatggccttt accagggtct    1620
   cagtacagcc accaaggaca cctacgacgc ccttcacatg caggccctgc cccctcgcta    1680
   a                                                                    1681
<210> 115
   <211> 1620
   <212> PRT
   <213> homo sapiens
<400> 115
<210> 116
   <211> 6267
   <212> DNA
   <213> homo sapiens
<400> 116
   agctgcaagt ggcgggcgcc caggcagatg cgatccagcg gctctggggg cggcagcggt     60
   ggtagcagct ggtacctccc gccgcctctg ttcggagggt cgcggggcac cgaggtgctt    120
   tccggccgcc ctctggtcgg ccacccaaag ccgcgggcgc tgatgatggg tgaggagggg    180
   gcggcaagat ttcgggcgcc cctgccctga acgccctcag ctgctgccgc cggggccgct    240
   ccagtgcctg cgaactctga ggagccgagg cgccggtgag agcaaggacg ctgcaaactt    300
   gcgcagcgcg ggggctggga ttcacgccca gaagttcagc aggcagacag tccgaagcct    360
   tcccgcagcg gagagatagc ttgagggtgc gcaagacggc agcctccgcc ctcggttccc    420
   gcccagaccg ggcagaagag cttggaggag ccaaaaggaa cgcaaaaggc ggccaggaca 480
   gcgtgcagca gctgggagcc gccgttctca gccttaaaag ttgcagagat tggaggctgc 540
   cccgagaggg gacagacccc agctccgact gcggggggca ggagaggacg gtacccaact 600
   gccacctccc ttcaaccata gtagttcctc tgtaccgagc gcagcgagct acagacgggg 660
   gcgcggcact cggcgcggag agcgggaggc tcaaggtccc agccagtgag cccagtgtgc 720
   ttgagtgtct ctggactcgc ccctgagctt ccaggtctgt ttcatttaga ctcctgctcg 780
   cctccgtgca gttgggggaa agcaagagac ttgcgcgcac gcacagtcct ctggagatca 840
   ggtggaagga gccgctgggt accaaggact gttcagagcc tcttcccatc tcggggagag 900
   cgaagggtga ggctgggccc ggagagcagt gtaaacggcc tcctccggcg ggatgggagc 960
   catcgggctc ctgtggctcc tgccgctgct gctttccacg gcagctgtgg gctccgggat 1020
   ggggaccggc cagcgcgcgg gctccccagc tgcggggccg ccgctgcagc cccgggagcc 1080
   actcagctac tcgcgcctgc agaggaagag tctggcagtt gacttcgtgg tgccctcgct 1140
   cttccgtgtc tacgcccggg acctactgct gccaccatcc tcctcggagc tgaaggctgg 1200
   caggcccgag gcccgcggct cgctagctct ggactgcgcc ccgctgctca ggttgctggg 1260
   gccggcgccg ggggtctcct ggaccgccgg ttcaccagcc ccggcagagg cccggacgct 1320
   gtccagggtg ctgaagggcg gctccgtgcg caagctccgg cgtgccaagc agttggtgct 1380
   ggagctgggc gaggaggcga tcttggaggg ttgcgtcggg ccccccgggg aggcggctgt 1440
   ggggctgctc cagttcaatc tcagcgagct gttcagttgg tggattcgcc aaggcgaagg 1500
   gcgactgagg atccgcctga tgcccgagaa gaaggcgtcg gaagtgggca gagagggaag 1560
   gctgtccgcg gcaattcgcg cctcccagcc ccgccttctc ttccagatct tcgggactgg 1620
   tcatagctcc ttggaatcac caacaaacat gccttctcct tctcctgatt attttacatg 1680
   gaatctcacc tggataatga aagactcctt ccctttcctg tctcatcgca gccgatatgg 1740
   tctggagtgc agctttgact tcccctgtga gctggagtat tcccctccac tgcatgacct 1800
   caggaaccag agctggtcct ggcgccgcat cccctccgag gaggcctccc agatggactt 1860
   gctggatggg cctggggcag agcgttctaa ggagatgccc agaggctcct ttctccttct 1920
   caacacctca gctgactcca agcacaccat cctgagtccg tggatgagga gcagcagtga 1980
   gcactgcaca ctggccgtct cggtgcacag gcacctgcag ccctctggaa ggtacattgc 2040
   ccagctgctg ccccacaacg aggctgcaag agagatcctc ctgatgccca ctccagggaa 2100
   gcatggttgg acagtgctcc agggaagaat cgggcgtcca gacaacccat ttcgagtggc 2160
   cctggaatac atctccagtg gaaaccgcag cttgtctgca gtggacttct ttgccctgaa 2220
   gaactgcagt gaaggaacat ccccaggctc caagatggcc ctgcagagct ccttcacttg 2280
   ttggaatggg acagtcctcc agcttgggca ggcctgtgac ttccaccagg actgtgccca 2340
   gggagaagat gagagccaga tgtgccggaa actgcctgtg ggtttttact gcaactttga 2400
   agatggcttc tgtggctgga cccaaggcac actgtcaccc cacactcctc aatggcaggt 2460
   caggacccta aaggatgccc ggttccagga ccaccaagac catgctctat tgctcagtac 2520
   cactgatgtc cccgcttctg aaagtgctac agtgaccagt gctacgtttc ctgcaccgat 2580
   caagagctct ccatgtgagc tccgaatgtc ctggctcatt cgtggagtct tgaggggaaa 2640
   cgtgtccttg gtgctagtgg agaacaaaac cgggaaggag caaggcagga tggtctggca 2700
   tgtcgccgcc tatgaaggct tgagcctgtg gcagtggatg gtgttgcctc tcctcgatgt 2760
   gtctgacagg ttctggctgc agatggtcgc atggtgggga caaggatcca gagccatcgt 2820
   ggcttttgac aatatctcca tcagcctgga ctgctacctc accattagcg gagaggacaa 2880
   gatcctgcag aatacagcac ccaaatcaag aaacctgttt gagagaaacc caaacaagga 2940
   gctgaaaccc ggggaaaatt caccaagaca gacccccatc tttgacccta cagttcattg 3000
   gctgttcacc acatgtgggg ccagcgggcc ccatggcccc acccaggcac agtgcaacaa 3060
   cgcctaccag aactccaacc tgagcgtgga ggtggggagc gagggccccc tgaaaggcat 3120
   ccagatctgg aaggtgccag ccaccgacac ctacagcatc tcgggctacg gagctgctgg 3180
   cgggaaaggc gggaagaaca ccatgatgcg gtcccacggc gtgtctgtgc tgggcatctt 3240
   caacctggag aaggatgaca tgctgtacat cctggttggg cagcagggag aggacgcctg 3300
   ccccagtaca aaccagttaa tccagaaagt ctgcattgga gagaacaatg tgatagaaga 3360
   agaaatccgt gtgaacagaa gcgtgcatga gtgggcagga ggcggaggag gagggggtgg 3420
   agccacctac gtatttaaga tgaaggatgg agtgccggtg cccctgatca ttgcagccgg 3480
   aggtggtggc agggcctacg gggccaagac agacacgttc cacccagaga gactggagaa 3540
   taactcctcg gttctagggc taaacggcaa ttccggagcc gcaggtggtg gaggtggctg 3600
   gaatgataac acttccttgc tctgggccgg aaaatctttg caggagggtg ccaccggagg 3660
   acattcctgc ccccaggcca tgaagaagtg ggggtgggag acaagagggg gtttcggagg 3720
   gggtggaggg gggtgctcct caggtggagg aggcggagga tatataggcg gcaatgcagc 3780
   ctcaaacaat gaccccgaaa tggatgggga agatggggtt tccttcatca gtccactggg 3840
   catcctgtac accccagctt taaaagtgat ggaaggccac ggggaagtga atattaagca 3900
   ttatctaaac tgcagtcact gtgaggtaga cgaatgtcac atggaccctg aaagccacaa 3960
   ggtcatctgc ttctgtgacc acgggacggt gctggctgag gatggcgtct cctgcattgt 4020
   gtcacccacc ccggagccac acctgccact ctcgctgatc ctctctgtgg tgacctctgc 4080
   cctcgtggcc gccctggtcc tggctttctc cggcatcatg attgtgtacc gccggaagca 4140
   ccaggagctg caagccatgc agatggagct gcagagccct gagtacaagc tgagcaagct 4200
   ccgcacctcg accatcatga ccgactacaa ccccaactac tgctttgctg gcaagacctc 4260
   ctccatcagt gacctgaagg aggtgccgcg gaaaaacatc accctcattc ggggtctggg 4320
   ccatggcgcc tttggggagg tgtatgaagg ccaggtgtcc ggaatgccca acgacccaag 4380
   ccccctgcaa gtggctgtga agacgctgcc tgaagtgtgc tctgaacagg acgaactgga 4440
   tttcctcatg gaagccctga tcatcagcaa attcaaccac cagaacattg ttcgctgcat 4500
   tggggtgagc ctgcaatccc tgccccggtt catcctgctg gagctcatgg cggggggaga 4560
   cctcaagtcc ttcctccgag agacccgccc tcgcccgagc cagccctcct ccctggccat 4620
   gctggacctt ctgcacgtgg ctcgggacat tgcctgtggc tgtcagtatt tggaggaaaa 4680
   ccacttcatc caccgagaca ttgctgccag aaactgcctc ttgacctgtc caggccctgg 4740
   aagagtggcc aagattggag acttcgggat ggcccgagac atctacaggg cgagctacta 4800
   tagaaaggga ggctgtgcca tgctgccagt taagtggatg cccccagagg ccttcatgga 4860
   aggaatattc acttctaaaa cagacacatg gtcctttgga gtgctgctat gggaaatctt 4920
   ttctcttgga tatatgccat accccagcaa aagcaaccag gaagttctgg agtttgtcac 4980
   cagtggaggc cggatggacc cacccaagaa ctgccctggg cctgtatacc ggataatgac 5040
   tcagtgctgg caacatcagc ctgaagacag gcccaacttt gccatcattt tggagaggat 5100
   tgaatactgc acccaggacc cggatgtaat caacaccgct ttgccgatag aatatggtcc 5160
   acttgtggaa gaggaagaga aagtgcctgt gaggcccaag gaccctgagg gggttcctcc 5220
   tctcctggtc tctcaacagg caaaacggga ggaggagcgc agcccagctg ccccaccacc 5280
   tctgcctacc acctcctctg gcaaggctgc aaagaaaccc acagctgcag agatctctgt 5340
   tcgagtccct agagggccgg ccgtggaagg gggacacgtg aatatggcat tctctcagtc 5400
   caaccctcct tcggagttgc acaaggtcca cggatccaga aacaagccca ccagcttgtg 5460
   gaacccaacg tacggctcct ggtttacaga gaaacccacc aaaaagaata atcctatagc 5520
   aaagaaggag ccacacgaca ggggtaacct ggggctggag ggaagctgta ctgtcccacc 5580
   taacgttgca actgggagac ttccgggggc ctcactgctc ctagagccct cttcgctgac 5640
   tgccaatatg aaggaggtac ctctgttcag gctacgtcac ttcccttgtg ggaatgtcaa 5700
   ttacggctac cagcaacagg gcttgccctt agaagccgct actgcccctg gagctggtca 5760
   ttacgaggat accattctga aaagcaagaa tagcatgaac cagcctgggc cctgagctcg 5820
   gtcgcacact cacttctctt ccttgggatc cctaagaccg tggaggagag agaggcaatg 5880
   gctccttcac aaaccagaga ccaaatgtca cgttttgttt tgtgccaacc tattttgaag 5940
   taccaccaaa aaagctgtat tttgaaaatg ctttagaaag gttttgagca tgggttcatc 6000
   ctattctttc gaaagaagaa aatatcataa aaatgagtga taaatacaag gcccagatgt 6060
   ggttgcataa ggtttttatg catgtttgtt gtatacttcc ttatgcttct ttcaaattgt 6120
   gtgtgctctg cttcaatgta gtcagaatta gctgcttcta tgtttcatag ttggggtcat      6180
   agatgtttcc ttgccttgtt gatgtggaca tgagccattt gaggggagag ggaacggaaa      6240
   taaaggagtt atttgtaatg actaaaa                                          6267